(19) Europäisches Patentamt
European Patent Office
Office européen des brevets

(11) EP 2 865 266 A1

(12) # EUROPEAN PATENT APPLICATION
published in accordance with Art. 153(4) EPC

(43) Date of publication:
**29.04.2015 Bulletin 2015/18**

(21) Application number: **13804499.5**

(22) Date of filing: **05.06.2013**

(51) Int Cl.:
***A01N 43/52*** (2006.01) ***A01N 37/18*** (2006.01)
***A01N 37/24*** (2006.01) ***A01N 37/46*** (2006.01)
***A01N 37/50*** (2006.01) ***A01N 43/12*** (2006.01)
***A01N 43/16*** (2006.01) ***A01N 43/32*** (2006.01)
***A01N 43/36*** (2006.01) ***A01N 43/40*** (2006.01)
***A01N 43/42*** (2006.01) ***A01N 43/54*** (2006.01)
***A01N 43/56*** (2006.01) ***A01N 43/653*** (2006.01)
***A01N 43/76*** (2006.01) ***A01N 43/78*** (2006.01)
***A01N 43/80*** (2006.01) ***A01N 43/88*** (2006.01)
***A01N 43/90*** (2006.01) ***A01N 47/02*** (2006.01)

(86) International application number:
**PCT/JP2013/066128**

(87) International publication number:
**WO 2013/187423 (19.12.2013 Gazette 2013/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.06.2012 JP 2012135538**

(71) Applicant: **Sumitomo Chemical Company, Limited**
**Tokyo 104-8260 (JP)**

(72) Inventors:
- **SUZUKI, Tatsuya**
  **Takarazuka-shi**
  **Hyogo 665-8555 (JP)**
- **IWATA, Atsushi**
  **Tokyo 104-8260 (JP)**
- **NOKURA, Yoshihiko**
  **Takarazuka-shi**
  **Hyogo 665-8555 (JP)**

(74) Representative: **Vossius & Partner**
**Patentanwälte Rechtsanwälte mbB**
**Siebertstrasse 3**
**81675 München (DE)**

(54) **PEST CONTROL COMPOSITION FOR HARMFUL ARTHROPODS, AND PEST CONTROL METHOD FOR HARMFUL ARTHROPODS**

(57) A harmful arthropod control composition comprising a compound represented by the following Formula (1)

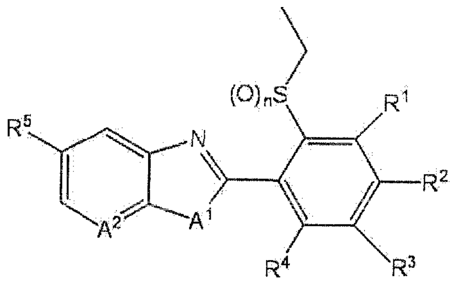

(1)

wherein each symbol represents definitions described in the specification, and one or more fungicidal compounds, which are selected from among azoles, strobilurins, phenylamides, rice blast controlling compounds, rice sheath blight disease controlling compounds, and other fungicidal compounds. The composition has an excellent controlling effect against harmful arthropods.

EP 2 865 266 A1

## Description

### Technical Field

The present invention relates to a harmful arthropod control composition and a method for controlling the harmful arthropods.

### Background Art

In the related art, various compounds are known as active ingredients of the harmful arthropod control composition (for example, refer to NPL 1).

### Citation List

### Non-Patent Literature

[Non-Patent Literature 1] The Pesticide Manual-15th edition (published by BCPC), ISBN 978-1-901396-18-8

### Summary of Invention

### Technical Problem

The object of the present invention is to provide a harmful arthropod control composition having an excellent controlling effect against harmful arthropods.

### Solution to Problem

The present inventors have conducted extensive studies in order to find harmful arthropod control composition having an excellent controlling effect against harmful arthropods, and as a result, they have found that a composition which comprises a compound represented by the following Formula (1) and one or more fungicidal compounds selected from the following Group (A) has an excellent controlling effect against the harmful arthropods.

The present invention is as follows.

[1] A harmful arthropod control composition comprising:

a compound represented by Formula (1)

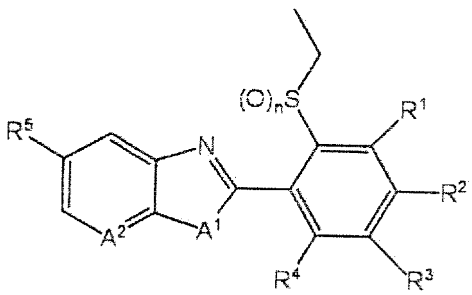

(1)

wherein

A1 represents -NR6-, an oxygen atom, or a sulfur atom,
A2 represents a nitrogen atom or =CH-,
R1, R2, R3, and R4 are the same or different and each represent a C1-C3 chain hydrocarbon group optionally having one or more halogen atoms, a phenyl group optionally having one or more atoms or groups selected from Group Z, a 6-membered heterocyclic group optionally having one or more atoms or groups selected from Group Z, -OR7, -S(O)mR7, a halogen atom, or a hydrogen atom, provided that at least two of R1, R2, R3 and R4 represent hydrogen atoms,
R5 represents a C1-C3 chain hydrocarbon group optionally having one or more atoms or groups selected

from Group X, -OR7, -S(O)mR7, or a halogen atom,
R6 represents a C1-C3 chain hydrocarbon group optionally having one or more atoms or groups selected from Group W, a C3-C6 alicyclic hydrocarbon group optionally having one or more atoms or groups selected from Group W, or a hydrogen atom,
R7 represents a C1-C3 chain hydrocarbon group optionally having one or more halogen atoms, or a hydrogen atom,
m represents 0, 1, or 2 and n represents 0, 1, or 2, provided that, in -S(O)mR7, R7 is not a hydrogen atom when m is 1 or 2,
Group X is a group consisting of a C1-C3 alkoxy group optionally having one or more halogen atoms, a C2-C3 alkenyloxy group optionally having one or more halogen atoms, a C2-C3 alkynyloxy group optionally having one or more halogen atoms, a C1-C3 alkyl sulfanyl group optionally having one or more halogen atoms, a C1-C3 alkyl sulfinyl group optionally having one or more halogen atoms, a C1-C3 alkyl sulfanyl group optionally having one or more halogen atoms, a cyano group, a hydroxy group, and a halogen atom,
Group Z is a group consisting of a C1-C3 chain hydrocarbon group optionally having one or more halogen atoms, a C1-C3 alkoxy group optionally having one or more halogen atoms, a C1-C3 alkyl sulfanyl group optionally having one or more halogen atoms, a C1-C3 alkyl sulfinyl group optionally having one or more halogen atoms, a C1-C3 alkyl sulfonyl group optionally having one or more halogen atoms, a cyano group, a nitro group, and a halogen atom,
Group W is a group consisting of a C1-C3 alkoxy group optionally having one or more halogen atoms, a C2-C3 alkenyloxy group optionally having one or more halogen atoms, C2-C3 alkynyloxy group optionally having one or more halogen atoms, a halogen atom, and hydroxy group, and

one or more fungicidal compounds selected from the following Group (A),
Group (A)

A-1: azoles
tebuconazole, metconazole, difenoconazole, triticonazole, imazalil, triadimenol, fluquinconazole, prochloraz, prothioconazole, diniconazole, diniconazole M, cyproconazole, tetraconazole, ipconazole, triforine, pyrifenox, fenarimol, nuarimol, oxpoconazole fumarate, pefurazoate, triflumizole, azaconazole, bitertanol, bromuconazole, epoxiconazole, fenbuconazole, flusilazole, flutriafol, hexaconazole, imibenconazole, myclobutanil, penconazole, propiconazole, simeconazole, and triadimefon
A-2: strobilurins
kresoxim-methyl, azoxystrobin, pyraclostrobin, picoxystrobin, enestrobin, trifloxystrobin, dimoxystrobin, fluoxastrobin, orysastrobin, famoxadone, fenamidone, and metominostrobin, and a compound represented by the following Formula (2)
Formula (2)

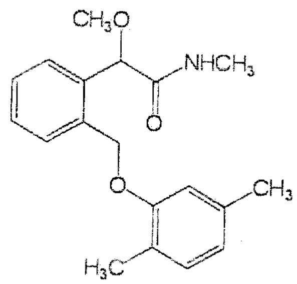

(2)

A-3: phenylamides
metalaxyl, metalaxyl-M, furalaxyl-M, benalaxyl, benalaxyl-M, ofurace, and oxadixyl
A-4: rice blast controlling compounds
Probenazole, tiadinil, tricyclazole, pyroquilon, kasugamycin hydrochloride, ferimzone, isotianil, fthalide, and tebufloquin
A-5: rice sheath blight disease controlling compounds Pencycuron, furametpyr, and validamycin
A-6: carboxamides
carboxin, flutolanil, penthiopyrad, fluopyram, penflufen, sedaxane, and fluxapyroxad
A-7: others
fludioxonil, ethaboxam, tolclofos-methyl, and captan.

[2] The harmful arthropod control composition according to [1], wherein the weight ratio of the content of the compound represented by Formula (1) to the content of the one or more fungicidal compounds selected from Group (A) is 10000:1 to 1:100.

[3] The harmful arthropod control composition according to [1], wherein the weight ratio of the content of the compound represented by Formula (1) to the content of the one or more fungicidal compounds selected from Group (A) is 1000:1 to 1:10.

[4] A method for controlling harmful arthropod, comprising the step of applying an effective amount of the harmful arthropod control composition according to any one of [1] to [3] to a plant or soil for cultivating a plant. Description of Embodiments

A harmful arthropod control composition of the present invention comprises a compound (hereinafter, referred to as the present condensed heterocyclic compound) represented by Formula (1)

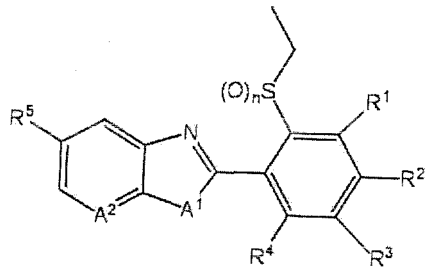

(1)

wherein

A1 represents -NR6-, an oxygen atom, or a sulfur atom,
A2 represents a nitrogen atom or =CH-,
R1, R2, R3, and R4 are the same or different and each represent a C1-C3 chain hydrocarbon group optionally having one or more halogen atoms, a phenyl group optionally having one or more atoms or groups selected from Group Z, a 6-membered heterocyclic group optionally having one or more atoms or groups selected from Group Z, -OR7, -S(O)mR7, a halogen atom, or a hydrogen atom, provided that at least two of R1, R2, R3 and R4 represent hydrogen atoms,
R5 represents a C1-C3 chain hydrocarbon group optionally having one or more atoms or groups selected from Group X, -OR7, -S(O)mR7, or a halogen atom,
R6 represents a C1-C3 chain hydrocarbon group optionally having one or more atoms or groups selected from Group W, a C3-C6 alicyclic hydrocarbon group optionally having one or more atoms or groups selected from Group W, or a hydrogen atom,
R7 represents a C1-C3 chain hydrocarbon group optionally having one or more halogen atoms, or a hydrogen atom,
m represents 0, 1, or 2 and n represents 0, 1, or 2, provided that, in -S(O)mR7, R7 is not a hydrogen atom when n is 1 or 2,
Group X is a group consisting of a C1-C3 alkoxy group optionally having one or more halogen atoms, a C2-C3 alkenyloxy group optionally having one or more halogen atoms, a C2-C3 alkynyloxy group optionally having one or more halogen atoms, a C1-C3 alkyl sulfanyl group optionally having one or more halogen atoms, a C1-C3 alkyl sulfinyl group optionally having one or more halogen atoms, a C1-C3 alkyl sulfonyl group optionally having one or more halogen atoms, a cyano group, a hydroxy group, and a halogen atom,
Group Z is a group consisting of a C1-C3 chain hydrocarbon group optionally having one or more halogen atoms, a C1-C3 alkoxy group optionally having one or more halogen atoms, a C1-C3 alkyl sulfanyl group optionally having one or more halogen atoms, a C1-C3 alkyl sulfinyl group optionally having one or more halogen atoms, a C1-C3 alkyl sulfonyl group optionally having one or more halogen atoms, a cyano group, a nitro group, and a halogen atom,
Group W is a group consisting of a C1-C3 alkoxy group optionally having one or more halogen atoms, a C2-C3 alkenyloxy group optionally having one or more halogen atoms, C2-C3 alkynyloxy group optionally having one or more halogen atoms, a halogen atom, and a hydroxy group, and one or more fungicidal compounds (hereinafter, referred to as the present fungicidal compounds) selected from the group (hereinafter, in some cases, referred to as Group (A)) consisting of

(A-1) azoles, such as tebuconazole, metconazole, difenoconazole, triticonazole, imazalil, triadimenol, fluquinconazole, prochloraz, prothioconazole, diniconazole, diniconazole M, cyproconazole, tetraconazole, ipconazole, triforine, pyrifenox, fenarimol, nuarimol, oxpoconazole fumarate, pefurazoate, triflumizole, azaconazole, bitertanol, bromuconazole, epoxiconazole, fenbuconazole, flusilazole, flutriafol, hexaconazole, imibenconazole, myclobutanil, penconazole, propiconazole, simeconazole, and triadimefon; (A-2) strobilurins, such as kresoxim-methyl, azoxystrobin, pyraclostrobin, picoxystrobin, enestrobin, trifloxystrobin, dimoxystrobin, fluoxastrobin, orysastrobin, famoxadone, fenamidone, and metominostrobin, and a compound represented by the following Formula (2)

Formula (2)

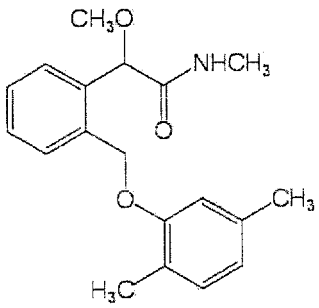

(2)

;

(A-3) phenylamides, such as metalaxyl, metalaxyl-M, furalaxyl-M, benalaxyl, benalaxyl-M, ofurace, and oxadixyl; (A-4) rice blast controlling compounds, such as probenazole, tiadinil, tricyclazole, pyroquilon, kasugamycin hydrochloride, ferimzone, isotianil, fthalide, and tebufloquin; (A-5) rice sheath blight disease controlling compounds, such as pencycuron, furametpyr, and validamycin; (A-6) carboxamides, such as carboxin, flutolanil, penthiopyrad, fluopyram, penflufen, sedaxane, and fluxapyroxad; and (A-7) compounds, such as fludioxonil, ethaboxam, tolclofos-methyl, and captan.

Examples of substituent groups used in description of the present specification will be described hereinbelow.

The expression "C1-C3 chain hydrocarbon group" in the present specification represents a saturated or unsaturated hydrocarbon group which has a straight chain shape or a branched chain shape and has 1 to 3 carbon atoms, and examples thereof include C1-C3 alkyl groups, such as a methyl group, an ethyl group, a propyl group, and an isopropyl group; C2-C3 alkenyl groups, such as a vinyl group, a 1-propenyl group, a 2-propenyl group, and a 1-methylvinyl group; and C2-C3 alkynyl groups, such as an ethynyl group, a propargyl group, and a 2-butynyl group. The expression "C1-C2 chain hydrocarbon group" represents a methyl group, an ethyl group, a vinyl group, and an ethynyl group.

The expression "C1-C3 alkyl group" in the present specification represents an alkyl group which has a straight chain shape or a branched chain shape and has 1 to 3 carbon atoms, and examples thereof include a methyl group, an ethyl group, a propyl group, and an isopropyl group. The expression "C1-C2 alkyl group" represents a methyl group or an ethyl group.

The expression "C2-C3 alkenyl group" in the present specification represents an unsaturated hydrocarbon group which has 2 to 3 carbon atoms, has a straight chain shape or a branched chain shape, and has one or more double bonds in a molecule, and examples thereof include a vinyl group, a 1-propenyl group, a 2-propenyl group, and a 1-methylvinyl group.

The expression "C2-C3 alkynyl group" in the present specification represents an unsaturated hydrocarbon group which has 2 to 3 carbon atoms, has a straight chain shape or a branched chain shape, and has one or more triple bonds in a molecule, and examples thereof include an ethynyl group and a propargyl group.

The expression "C1-C3 alkoxy group" in the present specification represents a group which is represented by alkyl-O- which has a straight chain shape or a branched chain shape and has 1 to 3 carbon atoms, and examples thereof include a methoxy group, an ethoxy group, a propyloxy group, and an isopropyloxy group.

The expression "C2-C3 alkenyloxy group" in the present specification represents a group which is represented by alkenyl-O- which has 2 to 3 carbon atoms, has a straight chain shape or a branched chain shape, and has one or more double bonds in a molecule, and examples thereof include a vinyloxy group, a 1-propenyloxy group, a 2-propenyloxy group, and a 1-methylvinyloxy group.

The expression "C2-C3 alkynyloxy group" in the present specification represents a group which is represented by alkynyl-O- which has 2 to 3 carbon atoms, has a straight chain shape or a branched chain shape, and has one or more triple bonds in a molecule, and examples thereof include an ethynyloxy group and a propargyloxy group.

The expression "C1-C3 alkyl sulfanyl group" in the present specification represents a group which is represented by alkyl-S- which has 1 to 3 carbon atoms, and has straight chain shape or a branched chain shape, and examples thereof include a methylsulfanyl group, an ethyl sulfanyl group, a propyl sulfanyl group, and an isopropyl sulfanyl group.

The expression "C1-C3 alkyl sulfanyl group" in the present specification represents a group which is represented by alkyl-S(O)- which has 1 to 3 carbon atoms, and has a straight chain shape or a branched chain shape, and examples

thereof include a methyl sulfinyl group, an ethyl sulfinyl group, a propyl sulfinyl group, and an isopropyl sulfinyl group.

The expression "C1-C3 alkyl sulfonyl group" in the present specification represents a group which is represented by alkyl-S(O)2- which has 1 to 3 carbon atoms, and has a straight chain shape or a branched chain shape, and examples thereof include a methyl sulfonyl group, an ethyl sulfonyl group, a propyl sulfonyl group, and an isopropyl sulfonyl group.

The expression "C3-C6 alicyclic hydrocarbon group" in the present specification represents a nonaromatic cyclic hydrocarbon group which has 3 to 6 carbon atoms, and examples thereof include C3-C6 cycloalkyl groups, such as a cyclopropyl group, a cyclobutyl group, a cyclopentyl group, and a cyclohexyl group; and C3-C6 cycloalkenyl groups, such as a cyclopropenyl group, a cyclobutenyl group, a cyclopentenyl group, and a cyclohexenyl group.

The expression "C3-C6 cycloalkyl group" in the present specification represents a cyclic alkyl group which has 3 to 6 carbon atoms, and examples thereof include a cyclopropyl group, a cyclobutyl group, a cyclopentyl group, and a cyclohexyl group.

In the expression "optionally having one or more atoms or groups selected from Group X" in the present specification, when there are two or more atoms or groups selected from Group X, these atoms or groups selected from Group X may be the same or different.

In the expression optionally having one or more atoms or groups selected from Group Z" in the present specification, when there are two or more atoms or groups selected from Group Z, these atoms or groups selected from Group Z may be the same or different.

In the expression "optionally having one or more atoms or groups selected from Group W" in the present specification, when there are two or more atoms or groups selected from Group W, these atoms or groups selected from Group W may be the same or different.

In the expression optionally having one or more halogen atoms" in the present specification, when there are two or more halogen atoms, these halogen atoms may be the same or different.

The expression "6-membered heterocyclic group" in the present specification represents a 6-membered heterocyclic compound residue which contains one or more atoms selected from the group consisting of nitrogen atoms, oxygen atoms, and sulfur atoms except for the carbon atoms of a cyclic structure, and examples thereof include 6-membered aromatic heterocyclic group and 6-membered nonaromatic heterocyclic group.

Examples of the "6-membered aromatic heterocyclic group" include a pyrazinyl group, a pyrimidinyl group, a pyridyl group, and a pyridazinyl group.

Examples of the "6-membered nonaromatic heterocyclic group" include a piperidyl group, a morpholinyl group, a piperazinyl group, and a thiomorpholinyl group.

The "halogen atom" in the present specification means a fluorine atom, a chlorine atom, a bromine atoms, and an iodine atom.

Examples of the "C1-C3 chain hydrocarbon group optionally having one or more atoms cr groups selected from Group X" in the present condensed heterocyclic compound include C1-C3 alkyl groups, such as methyl group, an ethyl group, a propyl group, an isopropyl group, a methoxy methyl group, an ethoxy methyl group, a propyloxy methyl group, an isopropyloxy methyl group, a 2-methoxyethyl group, a 2-ethoxyethyl group, a 2-propyloxyethyl group, a 2-isopropyloxyethyl group, a difluoromethyl group, a trifluoromethyl group, a trichloromethyl group, a 2-fluoroethyl group, a 2,2-difluoroethyl group, a 1,2,2,2-tetrafluoro ethyl group, a 2,2,2-trifluoroethyl group, a pentafluoroethyl group, a 2-chloro-1,1,1,3,3,3-hexafluoropropane-2-yl group (-C(C1)(CF3)2), a heptafluoropropyl group, a heptafluoroisopropyl group, a methyl sulfinyl ethyl group, an ethyl sulfanylethyl group, a methyl sulfinylethyl group, a methyl sulfonylethyl group, a methoxycarbonylmethyl group, and a cyanomethyl group, optionally having one or more atoms or groups selected from Group X; C2-C3 alkenyl groups, such as a vinyl group, a 1-propenyl group, a 2-propenyl group, a 1-methylvinyl group, a 1,1-difluoroallyl group, and a pentafluoro allyl group, optionally having one or more atoms or groups selected from Group X; and C2-C3 alkynyl groups, such as an ethynyl group and a propargyl group, optionally having one or more atoms or groups selected from Group X.

Examples of the "C1-C3 chain hydrocarbon group optionally having one or more halogen atoms" in the present condensed heterocyclic compound include C1-C3 alkyl groups, such as a methyl group, an ethyl group, a propyl group, an isopropyl group, a trifluoromethyl group, a trichloromethyl group, a 2-fluoroethyl group, a 2,2-difluoromethyl group, a 2,2,2-trifluoroethyl group, a pentafluoroethyl group, and a heptafluoroisopropyl group, optionally having one or more halogen atoms; C2-C3 alkenyl groups, such as a vinyl group, a 1-propenyl group, a 2-propenyl group, a 1-methyl-vinyl group, a 1,1-difluoroallyl group, and a pentafluoro allyl group, optionally having one or more halogen atoms; and C2-C3 alkynyl groups, such as an ethynyl group and a propargyl group, optionally having one or more halogen atoms.

Examples of the "C1-C3 alkyl group optionally having one or more halogen atoms" in the present condensed heterocyclic compound include a methyl group, an ethyl group, a propyl group, an isopropyl group, a trifluoromethyl group, a trichloromethyl group, a 2-fluoroethyl group, a 2,2-difluoroethyl group, a 2,2,2-trifluoroethyl group, a pentafluoroethyl group, and a heptafluoroisopropyl group.

Examples of the "phenyl group optionally having one or more atoms or groups selected from Group Z" in the present condensed heterocyclic compound include a phenyl group, a 2-fluorophenyl group, a 3-fluorophenyl group, a

4-fluorophenyl group, a 2,3-difluorophenyl group, a 2,4-difluorophenyl group, a 2,5-difluorophenyl group, a 2,6-diflucrcphenyl group, a 3,4-difluorophenyl group, a 3,5-difluorophenyl group, a 2,3,4,5,6-pentafluorophenyl group, a 2-chlorophenyl group, a 3-chlorophenyl group, a 4-chlorophenyl group, a 2-bromophenyl group, a 3-bromophenyl group, a 4-bromophenyl group, a 2-iodophenyl group, a 3-iodophenyl group, a 4-iodophenyl group, a 2-trifluoromethylphenyl group, a 3-trifluoromethylphenyl group, a 4-trifluoromethylphenyl group, a 2-trifluoromethoxy phenyl group, a 3-trifluoromethoxy phenyl group, a 4-trifluoromethoxy phenyl group, a 2-trifluoromethyl sulfanylphenyl group, a 3-trifluoromethyl sulfanylphenyl group, a 4-trifluoromethyl sulfanylphenyl group, a 4-methoxycarbonylphenyl group, a 4-nitrophenyl group, a 4-cyanophenyl group, a 4-methylamino-phenyl group, a 4-dimethylamino-phenyl group, a 4-methylsulfinyl-phenyl group, and a 4-methylsulfonyl-phenyl group.

Examples of the "6-memberad heterocyclic group optionally having one or more atoms or groups selected from Group Z" in the present condensed heterocyclic compound include 6-membered nonaromatic heterocyclic groups, such as a piperidyl group, a morpholyl group, and a thiomorpholyl group, optionally having one or more atoms or groups selected from Group Z; and 6-membered aromatic heterocyclic groups, such as a pyrazinyl group, a 2-pyrimidinyl group, a 4-pyrimidinyl group, a 5-pyrimidinyl group, a 2-pyridyl group, a 3-pyridyl group, a 4-pyridyl group, a 3-fluoro-2-pyridyl group, a 4-fluoro-2-pyridyl group, 5-fluoro-2-pyridyl group, a 6-fluoro-2-pyridyl group, a 2-pyrimidinyl group, a 4-trifluoromethylpyridine-2-yl group, and a 5-trifluoromethylpyridine-2-yl group, optionally having one or more atoms or groups selected from Group Z.

Examples of the "C1-C3 chain hydrocarbon group optionally having one or more atoms or groups selected from Group W" in the present condensed heterocyclic compound include C1-C3 alkyl groups, such as a methyl group, a ethyl group, a propyl group, a isopropyl group, a trifluoromethyl group, a trichloromethyl group, a 2-fluoroethyl group, a 2,2-difluoroethyl group, a 2,2,2-trifluoroethyl group, a pentafluoroethyl group, a methoxy methyl group, a ethoxy methyl group, a propylcxy methyl group, a isopropyloxy methyl group, a methoxyethyl group, a ethoxyethyl group, a propyloxyethyl group, an isopropyloxyethyl group, a methyl sulfanyl ethyl group, an ethyl sulfanyl ethyl group, a methyl sulfinyl ethyl group, and a methyl sulfonyl ethyl group, optionally having one or more atoms or groups selected from Group W; C2-C3 alkenyl groups, such as a vinyl group, a 1-propenyl group, a 2-propenyl group, a 1,1-difluoroallyl group, and a pentafluoro allyl group, optionally having one or more atoms or groups selected from Group W; and C2-C3 alkynyl groups, such as an ethynyl group and a propargyl group, optionally having one or more atoms or groups selected from Group W.

Examples of the "C3-C6 alicyclic hydrocarbon group optionally having one or more atoms or groups selected from Group W" in the present condensed heterocyclic compound include a cyclopropyl group, a cyclobutyl group, a cyclopentyl group, a cyclohexyl group, a cyclopropenyl group, a cyclobutenyl group, a cyclopentenyl group, a 1-cyclohexenyl group, a 2-cyclohexenyl group, and a 3-cyclohexenyl group.

Examples of the "C1-C3 alkyl sulfanyl group optionally having one or more halogen atoms" in the present condensed heterocyclic compound include a methylsulfanyl group, an ethyl sulfanyl group, a propyl sulfanyl group, an isopropyl sulfanyl group, a trifluoromethyl sulfanyl group, a 2,2,2-trifluoroethyl sulfanyl group, and a pentafluoro ethyl sulfanyl group.

Examples of the "C1-C3 alkyl sulfinyl group optionally having one or more halogen atoms" in the present condensed heterocyclic compound include a methyl sulfinyl group, an ethyl sulfinyl group, a propyl sulfinyl group, an isopropyl sulfinyl group, a trifluoromethyl sulfinyl group, a 2,2,2-trifluoroethyl sulfinyl group, and a pentafluoroethyl sulfinyl group.

Examples of the "C1-C3 alkyl sulfonyl group optionally having one or more halogen atoms" in the present condensed heterocyclic compound include a methyl sulfonyl group, an ethyl sulfonyl group, a propyl sulfonyl group, an isopropyl sulfanyl group, a trifluoromethyl sulfonyl group, a 2,2,2-trifluoroethyl sulfonyl group, and a pentafluoroethyl sulfonyl group.

Examples of the "C1-C3 alkoxy group optionally having one or more halogen atoms" in the present condensed heterocyclic compound include a methoxy group, a, trifluoromethoxy group, an ethoxy group, a 2,2,2-trifluoroethoxy group, a propyloxy group, and an isopropyloxy group.

Examples of the "C2-C3 alkenyloxy group optionally having one or more halogen atoms" in the present condensed heterocyclic compound include a 2-propenyl group, a 2-methyl-2-propenyloxy group, and a 3,3-difluoroallyloxy group, and a 3,3-dichloroallyloxy group.

Examples of the "C2-C3 alkynyloxy group optionally having one or more halogen atoms" in the present condensed heterocyclic compound include a propargyloxy group.

The "one or more" in the present condensed heterocyclic compound refers to one or more atoms or groups and a number of atoms or groups less than or equal to the maximum number of atoms or groups which can be bonded to each other, unless otherwise noted.

Examples of the present condensed heterocyclic compound include the following compounds.

A compound in which A1 is -NR6-, an oxygen atom, or a sulfur atom, and R5 is a C1-C3 chain hydrocarbon group having one C1-C3 alkoxy group optionally having one or more halogen atoms or a C1-C3 chain hydrocarbon

group optionally having one or more halogen atoms, in Formula (1);
a compound in which A1 is -NR6- or a sulfur atom, and R5 is a C1-C3 chain hydrocarbon group optionally having one or more halogen atoms, in Formula (1);
a compound in which A1 is -NR6-, an oxygen atom, or a sulfur atom, A2 is N, and R5 is a C1-C3 chain hydrocarbon group having one C1-C3 alkoxy group optionally having one or more halogen atoms or a C1-C3 chain hydrocarbon group optionally having one or more halogen atoms, in Formula (1);
a compound in which A1 is -NR6- or a sulfur atom, A2 is N, and R5 is a C1-C3 chain hydrocarbon group optionally having one or more halogen atoms, in Formula (1);
a compound in which A1 is -NR6-, an oxygen atom, or a sulfur atom, R5 is a C1-C3 chain hydrocarbon group having one C1-C3 alkoxy group optionally having one or more halogen atoms or a C1-C3 chain hydrocarbon group optionally having one or more halogen atoms, and R6 is a methyl group, in Formula (1);
a compound in which A1 is -NR6- or a sulfur atom, R5 is a C1-C3 chain hydrocarbon group optionally having one or more halogen atoms, and R6 is a methyl group, in Formula (1);
a compound in which A1 is -NR6-, an oxygen atom, or a sulfur atom, A2 is N, R5 is a C1-C3 chain hydrocarbon group having one C1-C3 alkoxy group optionally having one or more halogen atoms or a C1-C3 chain hydrocarbon group optionally having one or more halogen atoms, and R6 is a methyl group, in Formula (1) ;
a compound in which A1 is -NR6- or a sulfur atom, A2 is N, R5 is a C1-C3 chain hydrocarbon group optionally having one or more halogen atoms, and R6 is a methyl group, in Formula (1);
a compound in which A1 is -NR6-, A2 is N, R5 is a C1-C2 chain hydrocarbon group optionally having one or more halogen atoms, and R6 is a methyl group, in Formula (1);
a compound in which A1 is -NR6- or a sulfur atom, R5 is -S(O)mR7, and R7 is a C1-C3 chain hydrocarbon group optionally having halogen atoms, in Formula (1);
a compound in which A1 is -NR6- or a sulfur atom, A2 is N, R5 is -S(O)mR7, and R7 is a C1-C3 chain hydrocarbon group optionally having one or more halogen atoms, in Formula (1);
a compound in which A1 is -NR6- or a sulfur atom, R5 is -S(O)mR7, R6 is a methyl group, and R7 is a C1-C3 chain hydrocarbon group optionally having one or more halogen atoms, in Formula (1);
a compound in which A1 is -NR6- or a sulfur atom, A2 is N, R5 is -S(O)mR7, R6 is a methyl group, and R7 is a C1-C3 chain hydrocarbon group optionally having one or more halogen atoms, in Formula (1);
a compound in which A1 is a sulfur atom, A2 is N, R5 is -S(O)mR7, R6 is a methyl group, and R7 is a C1-C2 chain hydrocarbon group optionally having one or more halogen atoms, in Formula (1);
a compound in which R2, R3, and R4 each are the same or different and are a C1-C3 chain hydrocarbon group, optionally having one or more halogen atoms, -OR7, a halogen atom, or a hydrogen atom, and R5 is a C1-C3 chain hydrocarbon group optionally having one or more halogen atoms, -OR7, or a halogen atom, in Formula (1);
a compound in which R2, R3, and R4 each are the same or different and are a 6-membered heterocyclic group, optionally having one or more halogen atoms or may have a C1-C2 alkyl group optionally having one or more halogen atoms, or a hydrogen atom, and R5 is a 6-membered heterocyclic group optionally having one or more halogen atoms or may have a C1-C2 alkyl group optionally having one or more halogen atoms, in Formula (1);
a compound in which R2, R3, and R4 each are the same or different and are a methyl group, an ethyl group, a trifluoromethyl group, a trifluoromethoxy group, or a hydrogen atom, and R5 is a methyl group, ethyl group, a trifluoromethyl group, or a trifluoromethoxy group,, in Formula (1);
a compound in which R2, R3, and R4 each are the same or different and are a fluorine atom, a chlorine atom, a bromine atom, an iodine atom, or a hydrogen atom, and R5 is a fluorine atom, a chlorine atom, a bromine atom, or an iodine atom, in Formula (1); and
a compound in which R2, R3, and R4 each are the same or different and are a fluorine atom, a chlorine atom, a bromine atom, a methyl group, a trifluoromethyl group, a trifluoromethoxy group, a pentafluoroethyl group, or a hydrogen atom, and R5 is a fluorine atom, a chlorine atom, a bromine atom, a methyl group, a trifluoromethyl group, a trifluorométhcxy group, or a pentafluoroethyl group, in Formula (1).
a compound in which A1 is -NR6- in Formula (1);
a compound in which A1 is an oxygen atom in Formula (1);
a compound in which A1 is a sulfur atom in Formula (1);
a compound in which A2 is a nitrogen atom in Formula (1);
a compound in which A2 is =CH- in Formula (1);
a compound in which R1, R3, and R4 each are the same or different and are a halogen atom or a hydrogen atom, in Formula (1);
a compound in which R2 is a C1-C3 alkyl group optionally having one or more halogen atoms, a C1-C3 alkoxy group optionally having one or more halogen atoms, and a 6-membered aromatic heterocyclic group optionally having the C1-C3 alkyl group having one or more halogen atoms, a halogen atom, or a hydrogen atom, in Formula (1);
a compound in which R1, R3, and R4 each are the same or different and are a halogen atom or a hydrogen atom, and

R2 is a C1-C3 alkyl group optionally having one or more halogen atoms, a C1-C3 alkoxy group optionally having one or more halogen atoms, and a 6-membered aromatic heterocyclic group optionally having the C1-C3 alkyl group having one or more halogen atoms, a halogen atom, or a hydrogen atom, in Formula (1);
a compound represented by Formula (1A)

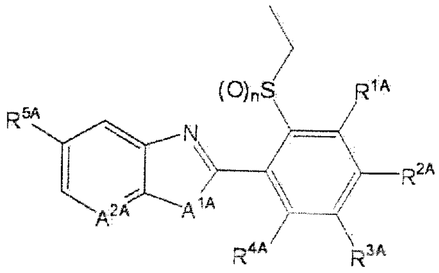
(1A)

[wherein,

A1A represents -NR6-, an oxygen atom, or a sulfur atom, A2A represents a nitrogen atom or -CH-,
R1A, R3A, and R4A each are the same or different and represent a halogen atom or a hydrogen atom, provided that at least two of R1A, R3A, and R4A represent hydrogen atoms,
R2A represents a C1-C3 alkyl group optionally having one or more halogen atoms, a C1-C3 alkoxy group optionally having one or more halogen atoms, a 6-membered aromatic heterocyclic group optionally having a C1-C3 alkyl group having one or more halogen atoms, a halogen atom, or a hydrogen atom,
R5A represents a C1-C3 alkyl group optionally having one or more halogen atoms, or -S(O)mR7A,
R6A represents a C1-C3 alkyl group or a C3-C6 cycloalkyl group,
R7A represents a C1-C3 alkyl group optionally having one or more halogen atoms,
m represents 0, 1, or 2, and n represents 0, 1, or 2.],

a compound in which A1A is -NR6A- in Formula (1A);
a compound in which A1A is an oxygen atom in Formula (1A); a compound in which A1A is a sulfur atom in Formula (1A):
a compound in which A2A is a nitrogen atom in Formula (1A);
a compound in which A2A is -CH- in Formula (1A);
a compound represented by Formula (1B)

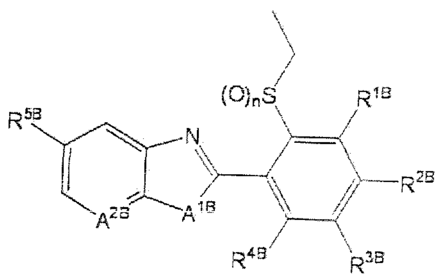
(1B)

[wherein,

A1B represents -NR6-, an oxygen atom, or a sulfur atoms,
A2B represents a nitrogen atom or =CH-,
R1B, R3B, and R4B each are the same or different and represent a fluorine atom, a chloride atom, or a hydrogen atom, provided that at least two of R1B, R3B, and R4B represent hydrogen atoms,
R2B represents a C1-C3 alkyl group (particularly, a methyl group, an ethyl group, or an isopropyl group), a C1-C3 alkyl group having one or more halogen atoms (particularly, a difluoromethyl group, a trifluoromethyl group, a pentafluoroethyl group, a heptafluoropropyl group, and the like), C1-C3 alkoxy group having one or more halogen atoms (particularly, a trifluoromethoxy group, and the like), a nitrogen-containing 6-membered aromatic heterocyclic group (particularly, a 2-, 3-, or 4-pyridyl group, a 2-pyrimidinyl group, and the like), a halogen atom (particularly, a fluorine atom, a chloride atom, a bromine atom, and the like), or a hydrogen atom,
R5B represents a C1-C3 alkyl group having one or more halogen atoms (particularly, a difluoromethyl group, a trifluoromethyl group, a pentafluoroethyl group, a 2-chloro-1,1,1,3,3,3-hexafluoropropane-2-yl group (-C(Cl)(CF3)2),

a heptafluoroisopropyl group, and the like), or -S(O)mR7B,
R6B represents a C1-C3 alkyl group (a methyl group, an ethyl group, a propyl group, or an isopropyl group, and particularly a methyl group),
R7B represents a C1-C3 alkyl group having one or more halogen atoms (a trifluoromethyl group, a pentafluoroethyl group, and the like, and particularly, a trifluoromethyl group),
m represents 0, 1, or 2, and n represents 0, 1, or 2.];

a compound in which A1B is -NR6B- in Formula (1B);
a compound in which A1B is an oxygen atom in Formula (1B);
a compound in which A1B is a sulfur atom in Formula (1B);
a compound in which A2B is a nitrogen atom in Formula (1B);
a compound in which A2B is =CH- in Formula (1B).

The compounds represented by the above-described General Formulas (1A) and (1B) are included in the compounds represented by General Formula (1).

Next, methods of producing the present condensed heterocyclic compound will be described.

The present condensed heterocyclic compound can be produced by, for example, the following (Production Method A) to (Production Method F).

(Production Method A)

A present condensed heterocyclic compound (1) can be produced by reacting a compound (M1) with a compound (M2). Alternately, the present condensed heterocyclic compound (1) can be preapared by cyclizing a compound (M3) which has been produced by reacting the compound (M1) with the compound (M2).

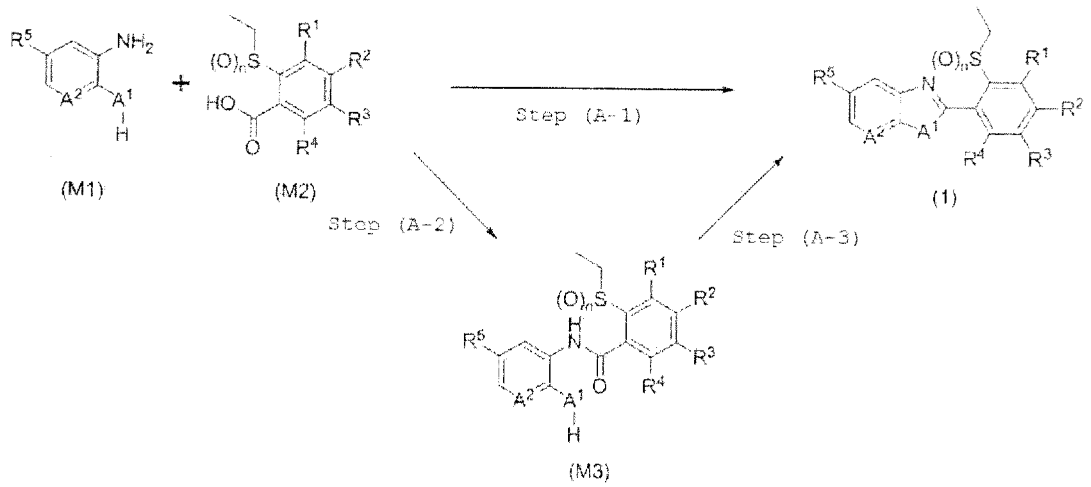

(M1) (M2) (1) (M3)

[wherein, R1, R2, R3, R4, R5, A1, A2, and n each represent the same meanings as those referred to above.]

(Production Method B)

The present condensed heterocyclic compound (1) can be produced by reacting a compound (M1) with a compound (M4). Alternately, the present condensed heterocyclic compound (1) can be preapared by cyclizing a compound (M3) which has been produced by reacting the compound (M1) with the compound (M4).

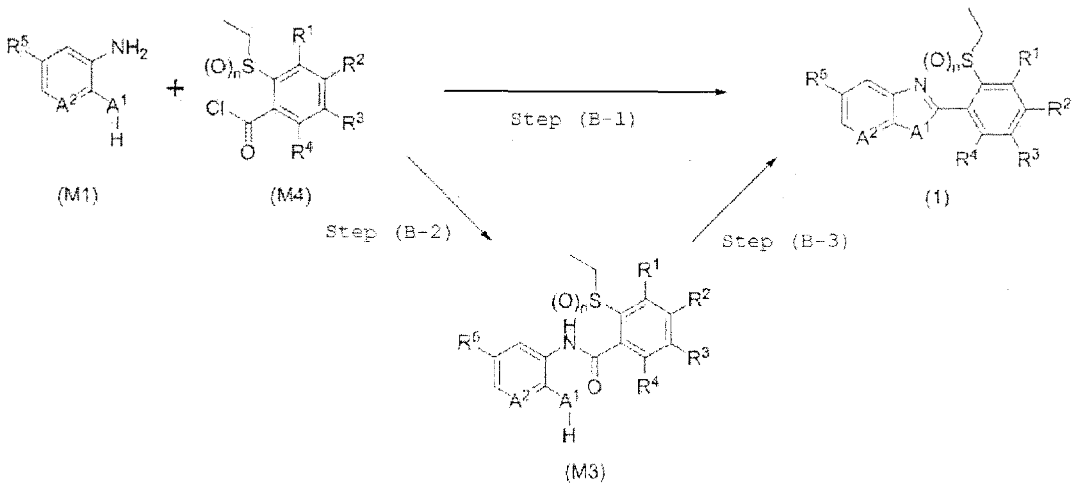


[wherein, R1, R2, R3, R4, R5, A1, A2, and n each represent the same meanings as those referred to above.]

(Production Method C)

The present condensed heterocyclic compound (1) can be produced by reacting a compound (M1) with a compound (M5). Alternately, the present condensed heterocyclic compound (1) can be preapared by cyclizing a compound (M6) which has been produced by reacting the compound (M1) with the compound (M5).

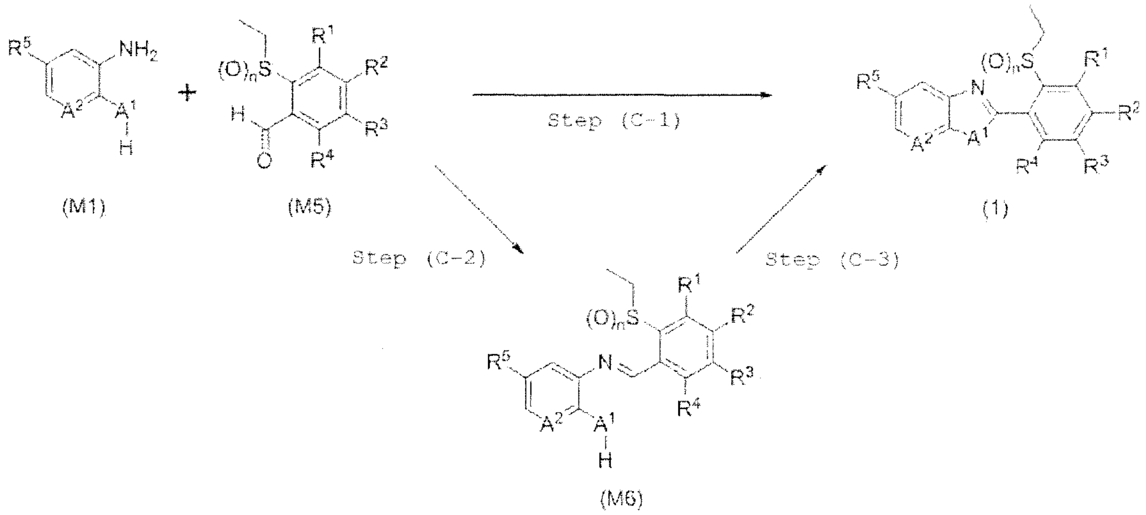


[wherein, R1, R2, R3, R4, R5, A1, A2, and n each represent the same meanings as those referred to above.]

(Production Method D)

A compound (2) in which A1 is a sulfur atom in Formula (1) can be produced by reacting a compound (M7) with a sulfurizing agent.

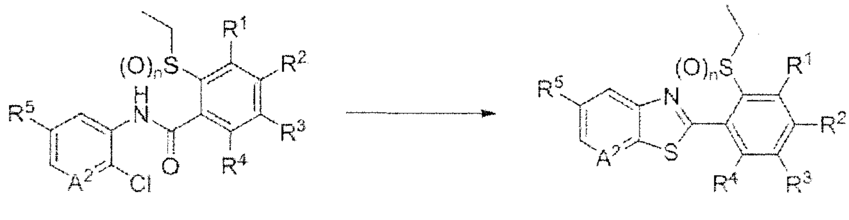

(M7)

(2)

[wherein, R1, R2, R3, R4, R5, A2, and n each represent the same meanings as those referred to above.]

(Production Method E)

A compound (3) in which n is 0 in Formula (1) can be produced through the following method, for example.

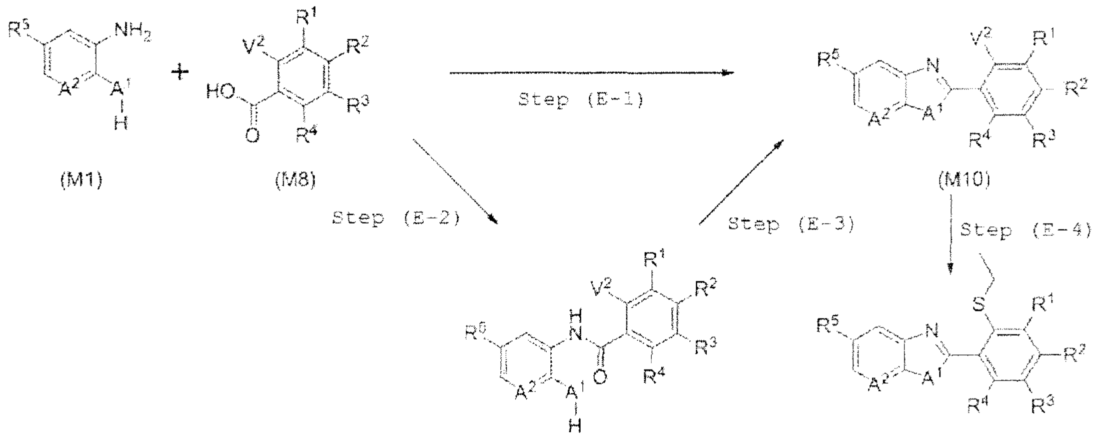


[wherein, R1, R2, R3, R4, R5, A1, and A2 each represent the same meanings as those referred to above and V2 represents a fluorine atom or a chlorine atom.]

(Production Method F)

A compound (3) in which n is 0 in Formula (1) can be produced through the following method, for example.

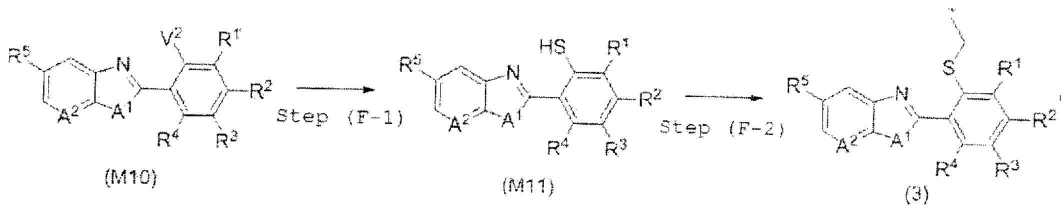


[wherein, R1, R2, R3, R4, R5, A1, A2, and V2 each represent the same meanings as those referred to above.]

The production methods (Production Method A) to (Production Method F) of the present condensed heterocyclic compound will be described in more detail hereinbelow. In addition, production methods of a different present condensed heterocyclic compound from the present condensed heterocyclic compound will also be described. For example, the present condensed heterocyclic compound can be produced through the following (Production Method 1) to (Production Method 23).

(Production Method 1) (Step (C-1))

A compound (4) in which A1 is -NR6- in Formula (1) can be produced by reacting a compound (M12) with a compound (M5) in accordance with Step (C-1).

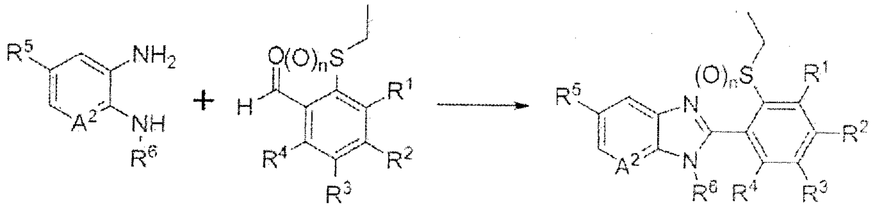

(M12) (M5) (4)

[wherein, R1, R2, R3, R4, R5, R6, A2, and n each represent the same meanings as those referred to above.]

In general, the reaction is performed in the presence of a base, an acid, sulfite, or bisulfite.

In general, the reaction is performed in the presence of a solvent.

Examples of the base used for the reaction include hydrogen carbonates, such as sodium hydrogen carbonate and potassium hydrogen carbonate; carbonates, such as sodium carbonate and potassium carbonate; and a mixture thereof.

Examples of the acid used for the reaction include sulfonic acids, such as p-toluenesulfonic acid; and carboxylic acids, such as acetic acid.

Examples of the sulfite used for the reaction include sodium sulfite and potassium sulfite.

Examples of the bisulfite used for the reaction include sodium bisulfite and potassium bisulfite.

Examples of the solvent used for the reaction include ethers, such as tetrahydrofuran (hereinafter, referred to as THF), ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as N,N-dimethylformamide (hereinafter, referred to as DMF) and N-methylpyrrolidone (hereinafter, referred to as NMP); sulfoxides, such as dimethyl sulfoxide (hereinafter, referred to as DMSO); nitrogen-containing aromatic compounds, such as pyridine and quinoline; and a mixture thereof.

The reaction can be performed by adding an oxidant as necessary.

Examples of the oxidant used for the reaction include oxygen, cupric chloride, and 2,3-dichloro-5,6-dicyano-p-benzoquinone.

In the reaction, with respect to 1 mole of the compound (M12), the compound (M5) is generally used at a ratio of 1 mole to 3 moles, the base is generally used at a ratio of 1 mole to 5 moles, the acid is generally used at a ratio of 1 mole to 5 moles, the sulfite is generally used at a ratio of 1 mole to 5 moles, the bisulfite is generally used at a ratio of 1 mole to 5 moles, and the oxidant is generally used at a ratio of 1 mole to 5 moles.

The temperature of the reaction is generally within the range of 30°C to 200°C. The reaction time of the reaction is generally within the range of 0.1 hours to 24 hours.

After completion of the reaction, it is possible to isolate the compound (4) by performing post-treatment operations, for example, subjecting the reaction mixture to extraction using an organic solvent after adding water to the reaction mixture; and drying and condensing an organic layer thereof. The isolated a compound (4) can be further purified through chromatography, recrystallization, etc.

(Production Method 2) (Step A-1)

A compound (4) in which A1 is -NR6- in Formula (1) can be produced by reacting a compound (M12) with a compound (M2) in the presence of a dehydration condensation agent in accordance with Step (A-1).

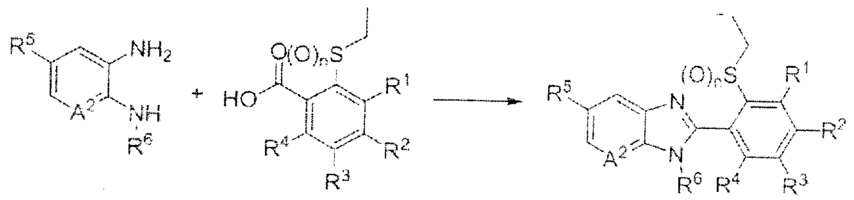

(M12) (M2) (4)

[wherein, R1, R2, R3, R4, R5, R6, A2, and n each represent the same meanings as those referred to above.]

In general, the reaction is performed in the presence of a solvent.

Examples of the solvent used for the reaction include ethers, such as THF, tert-butyl methyl ether, ethylene glycol dimethyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.

Examples of the dehydration condensation agent used for the reaction include carbodiimides, such as 1-ethyl-3-(3-dimethylaminopropyl) carbodiimide hydrochloride (hereinafter, referred to as WSC) and 1,3-dicyclohexylcarbodiimide; and (benzotriazol-1-yloxy)tris(dimethylamino)phosphonium hexafluorophosphate (hereinafter, referred to as a BOP reagent).

The reaction can be performed by adding a catalyst as necessary.

Examples of the catalyst used for the reaction include 1-hydroxybenzotriazole (hereinafter, referred to as HOBt).

In the reaction, with respect to 1 mole of the compound (M12), the compound (M2) is generally used at a ratio of 1 mole to 3 moles, the dehydration condensation agent is generally used at a ratio of 1 mole to 5 moles, and the catalyst is generally used at a ratio of 0.01 moles to 0.1 noles.

The temperature of the reaction is generally within the range of 30°C to 200°C. The reaction time of the reaction is generally within the range of 0.1 hours to 24 hours. After completion of the reaction, it is possible to isolate the compound (4) by performing post-treatment operations, for example, subjecting the reaction mixture to extraction using an organic solvent after adding water to the reaction mixture; and drying and condensing an organic layer thereof. The isolated a compound (4) can be further purified through chromatography, recrystallization, etc.

In addition, it is possible to produce the compound (4) based on the above-described method using the compound (M4) instead of the compound (M2) in accordance with Step (B-1).

When using the compound (M4), in general, the reaction is performed without adding the dehydration condensation agent. The reaction can be performed by adding a base as necessary.

Examples of the base include alkali metal carbonates, such as sodium carbonate and potassium carbonate; tertiary amines, such as triethylamine and diisopropylethylamine; and nitrogen-containing aromatic compounds, such as pyridine and 4-dimethylaminopyridine.

In the reaction, with respect to 1 mole of the compound (M12), the compound (M4) is generally used at a ratio of 1 mole to 3 moles and the base is generally used at a ratio of 1 mole to 10 moles.

(Production Method 3) (Step (A-3) and Step (B-3))

A compound (4) in which A1 is -NR6- in Formula (1) can be produced by dehydrating and condensing a compound (M13) in accordance with Step (A-3) or Step (B-3).

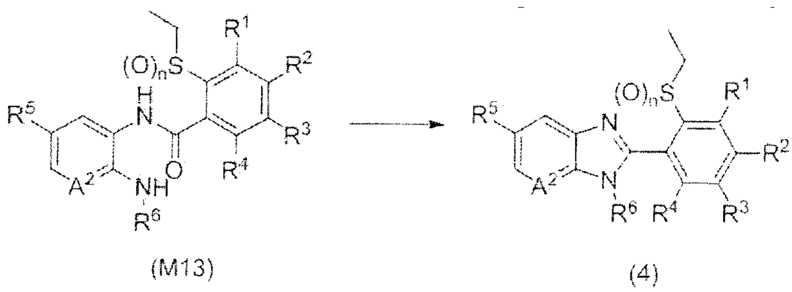

(M13) (4)

[wherein, R1, R2, R3, R4, R5, R6, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; alcohols, such as methanol, ethanol, propanol, butanol, and pentanol; and a mixture thereof.

It is possible to use an acid or a dehydrating agent for the reaction as necessary. Examples of the acid used for the reaction include sulfonic acids, such as p-toluenesulfonic acid; and carboxylic acids, such as acetic acid. Examples of the dehydrating agent used for the reaction include phosphorus oxychloride, acetic anhydride, and trifluoroacetic anhydride.

In the reaction, with respect to 1 mole of the compound (M13), the acid or the dehydrating agent is generally used at a ratio cf 1 mole to 10 moles.

The temperature of the reaction is generally within the range of 30°C to 200°C. The reaction tine of the reaction is generally within the range of 0.1 hours to 24 hours.

After completion of the reaction, it is possible to isolate the compound (4) by performing post-treatment operations, for example, subjecting the reaction mixture to extraction using an organic solvent; and drying and condensing an organic layer thereof. The isolated a compound (4) can be further purified through chromatography, recrystallization, etc.

(Production Method 4) (Step (A-3) and Step (B-3))

A compound (4) in which A1 is -NR6- in Formula (1) can be produced by subjecting a compound (M13) to a reaction in the presence of a base in accordance with Step (A-3) or Step (B-3).

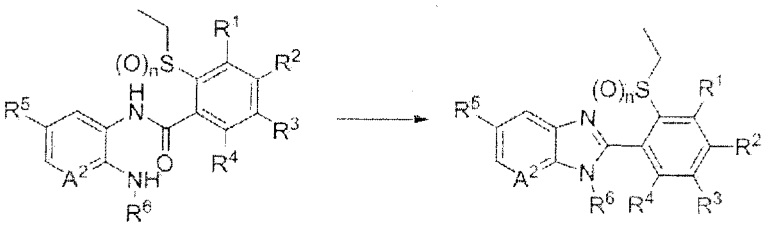

(M13) (4)

[wherein, R1, R2, R3, R4, R5, R6, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; alcohols, such as methanol, ethanol, propanol, butanol, tert-butanol, and pentanol; and a mixture thereof.

Examples of the base used for the reaction include tripotassium phosphate.

In the reaction, with respect to 1 mole of the compound (M13), the base is generally used at a ratio of 1 mole to 10 moles.

The temperature of the reaction is generally within the range of 30°C to 200°C. The reaction time of the reaction is generally within the range of 0.1 hours to 24 hours.

After completion of the reaction, it is possible to isolate the compound (4) by performing post-treatment operations, for example, subjecting the reaction mixture to extraction using an organic solvent; and drying and condensing an organic layer thereof. The isolated a compound (4) can be further purified through chromatography, recrystallization, etc.

(Production Method 5)

A compound (4) in which A1 is -NR6- in Formula (1) can be produced by reacting a compound (5) and a compound (M14) in the presence of a base.

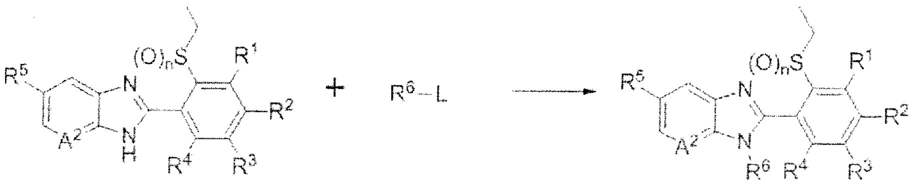

(5) (M14) (4)

[wherein, R1, R2, R3, R4, R5, A2, and n each represent the same meanings as those referred to above; L represents leaving groups, such as a chlorine atom, a bromine atom, an iodine atom, a trifluoromethylsulfonyloxy group, and a methylsulfonyloxy group; and here R6 represents a C1-C3 chain hydrocarbon group optionally having one or more atoms or groups selected from Group W.]

In general, the reaction is performed in the presence of a solvent.

Examples of the solvent used for the reaction include ethers, such as THF, ethylene glycol dimethyl ether, tert-butyl methyl ether, and 1,4-dioxane; aromatic hydrocarbons, such as toluene and xylene; nitriles, such as acetonitrile;

acid amides, such as DMF and NMP; sulfoxides, such as DMSO; and a mixture thereof.

Examples of the base used for the reaction include alkali metal or alkaline earth metal hydrides, such as sodium hydride, potassium hydride, and calcium hydride; inorganic bases, such as sodium carbonate and potassium carbonate; or organic bases such as triethylamine.

In the reaction, with respect to 1 mole of the compound (5), the compound (M14) is generally used at a ratio of 1 mole to 5 moles.

In the reaction, with respect to 1 mole of the compound (5), the base is generally used at a ratio of 1 mole to 3 moles.

The temperature of the reaction is generally within the range of 0°C to 100°C. The reaction time of the reaction is generally within the range of 0.1 hours to 24 hours.

After completion of the reaction, it is possible to isolate the compound (4) by performing post-treatment operations, for example, subjecting the reaction mixture to extraction using an organic solvent; and drying and condensing an organic layer thereof. The isolated a compound (4) can be further purified through chromatography, recrystallization, etc.

(Production Method 6) (Step (A-1))

A compound (6) in which A1 is an oxygen atom in Formula (1) can be produced by reacting a compound (M15) with a compound (M2) in the presence of an acid in accordance with Step (A-1).

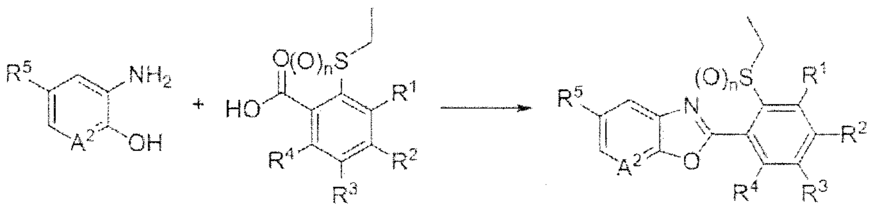

(M15) (M2) (6)

[wherein, R1, R2, R3, R4, R5, A2, and n each represent the same meanings as those referred to above.]

In general, the reaction is performed in the presence or absence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene or dichlorobenzene; and a mixture thereof.

Examples of the acid used for the reaction include polyphosphoric acid or trimethylsilyl polyphosphate.

The reaction is generally performed in the absence of solvent when using polyphosphoric acid as the acid, but may be performed in a solvent.

In the reaction, with respect to 1 mole of the compound (M15), the compound (M2) is generally used at a ratio of 1 mole to 3 moles and the acid is generally used at a ratio of 1 mole to 10 moles.

The temperature of the reaction is generally within the range of 50°C to 200°C. The reaction time of the reaction is generally within the range of 0.5 hours to 24 hours.

After completion of the reaction, it is possible to isolate the compound (6) by performing post-treatment operations, for example, subjecting the reaction mixture to extraction using an organic solvent after adding the reaction mixture to water; and drying and condensing an organic layer thereof. The isolated a compound (6) can be further purified through chromatography, recrystallization, etc.

In addition, it is possible to produce the compound (6) based on the above-described method using the compound (M4) instead of the compound (M2) in accordance with Step (B-1).

When using the compound (M4), in general, the reaction is performed without adding a dehydration condensation agent. The reaction can be performed by adding a base as necessary.

Examples of the base include alkali metal carbonates, such as sodium carbonate and potassium carbonate; tertiary amines, such as triethylamine and diisopropylethylamine; and nitrogen-containing aromatic compounds, such as pyridine and 4-dimethylaminopyridine.

In the reaction, with respect to 1 mole of the compound (M15), the compound (M4) is generally used at a ratio of 1 mole to 3 moles and the base is generally used at a ratio of 1 mole to 10 moles.

(Production Method 7) (Step (C-3))

A compound (6) in which A1 is an oxygen atom in Formula (1) can be produced by subjecting a compound (M16) 48 to an oxidation reaction in accordance with Step (C-3).

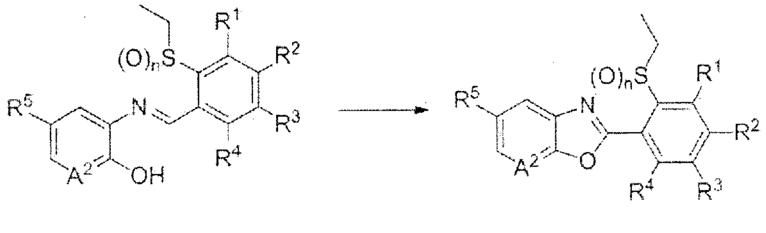

(M16) (6)

[wherein, R1, R2, R3, R4, R5, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as dichloromethane, chloroform, and chlorobenzene; esters, such as ethyl acetate and butyl acetate; alcohols, such as methanol and ethanol; nitriles, such as acetonitrile; acid amides, such as DMF and NMP; sulfoxides, such as DMSO; acetic acid; and a mixture thereof.

Examples of the oxidant used for the reaction include metal oxidants, such as lead (IV) acetate and lead (IV) oxide; and hypervalent iodine compounds, such as iodobenzene diacetate.

In the reaction, with respect to 1 mole of the compound (M16), the oxidant is generally used at a ratio of 1 mole to 3 moles.

The temperature of the reaction is generally within the range of 0°C to 100°C. The reaction time of the reaction is generally within the range of 0.1 hours to 24 hours.

After completion of the reaction, it is possible to isolate the compound (6) by performing post-treatment operations, for example, subjecting the reaction mixture to extraction using an organic solvent; and drying and condensing an organic layer thereof. The isolated a compound (6) can be further purified through chromatography, recrystallization, etc.

(Production Method 8) (Step (A-3) and Step (B-3))

A compound (6) in which A1 is an oxygen atom in Formula (1) can be produced by subjecting a compound (M17) to a reaction in the presence of a dehydration condensation agent in accordance with Step (A-3) or Step (B-3).

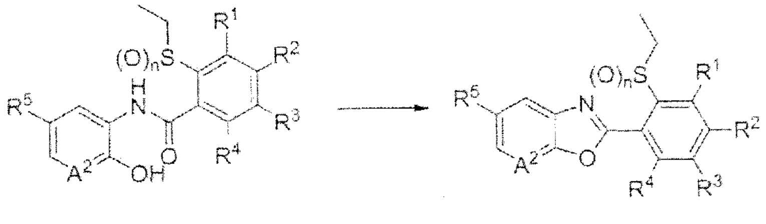

(M17) (6)

[wherein, R1, R2, R3, R4, R5, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aromatic hydrocarbons, such as toluene and xylene; halogenated hydrocarbons, such as dichloromethane, chloroform, carbon tetrachloride, and chlorobenzene; esters, such as ethyl acetate and butyl acetate; nitriles, such as acetonitrile; and a mixture thereof. Among these, carbon tetrachloride can also be used as the dehydration condensation agent.

Examples of the dehydration condensation agent used for the reaction include a mixture of a triphenylphosphine, a base, and carbon tetrachloride or carbon tetrabromide; and a mixture of triphenylphosphine and azo diesters, such as azodicarboxylic acid diethyl ester.

Examples of the base used for the reaction include tertiary amines, such as triethylamine and diisopropylethylamine.

In the reaction, with respect to 1 mole of the compound (M17), the dehydration condensation agent is generally used at a ratio of 1 mole to 3 moles. When using a base, the base is generally used at a ratio of 1 mole to 5 moles with respect to 1 mole of the compound (M17).

The temperature of the reaction is generally within the range of -30°C to 100°C. The reaction time of the reaction is generally within the range of 0.5 hours to 24 hours.

After completion of the reaction, it is possible to isolate the compound (6) by performing post-treatment operations, for example, subjecting the reaction mixture to extraction using an organic solvent; and drying and condensing an organic layer thereof. The isolated a compound (6) can be further purified through chromatography, recrystallization, etc.

(Production Method 9) (Step (A-3) and Step (B-3))

A compound (6) in which A1 is an oxygen atom in Formula (1) can be produced by subjecting a compound (M17) to a reaction in the presence of an acid in accordance with Step (A-3) or Step (B-3).

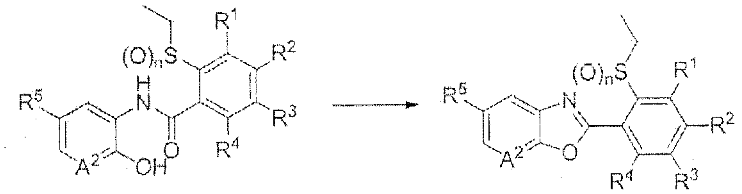

(M17) (6)

[wherein, R1, R2, R3, R4, R5, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aromatic hydrocarbons, such as toluene and xylene; halogenated hydrocarbons, such as dichloromethane, chloroform, and chlorobenzene; and a mixture thereof.

Examples of the above-described acid include sulfonic acids, such as p-toluene sulfonic acid; and polyphosphoric acid.

In the reaction, with respect to 1 mole of the compound (M17), the acid is generally used at a ratio of 0.1 moles to 3 moles.

The temperature of the reaction is generally within the range of 50°C to 200°C. The reaction time of the reaction is generally within the range of 1 hour to 24 hours.

After completion of the reaction, it is possible to isolate the compound (6) by performing post-treatment operations, for example, subjecting the reaction mixture to extraction using an organic solvent; and drying and condensing an organic layer thereof. The isolated a compound (6) can be further purified through chromatography, recrystallization, etc.

(Production Method 10) (Step (C-1))

A compound (2) in which A1 is a sulfur atom in Formula (1) can be produced by reacting a compound (M18) with a compound (M5) in accordance with Step (C-1).

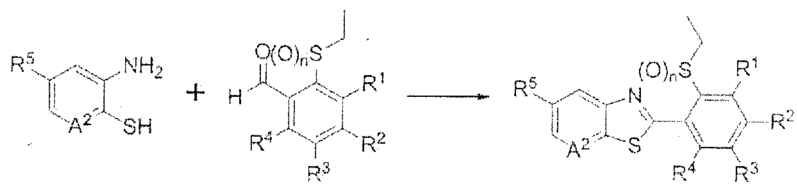

(M18) (M5) (2)

[wherein, R1, R2, R3, R4, R5, A2, and n each represent the same meanings as those referred to above.]

In general, the reaction is performed in the presence of a base, an acid, sulfite, or bisulfite. Examples of the base used for the reaction include hydrogen carbonates, such as sodium hydrogen carbonate and potassium hydrogen

carbonate; carbonates, such as sodium carbonate and potassium carbonate; and a mixture thereof.

Examples of the acid used for the reaction include sulfonic acids, such as p-toluenesulfonic acid; and carboxylic acids, such as acetic acid.

Examples of the sulfite used for the reaction include sodium sulfite and potassium sulfite.

Examples of the bisulfite used for the reaction include sodium bisulfite and potassium bisulfite.

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; halogenated hydrocarbons, such as chlorobenzene; esters, such as ethyl acetate and butyl acetate; nitriles, such as acetonitrile; acid amides, such as DMF, N-, and NMP; sulfoxides, such as DMSO; aromatic hydrocarbons, such as toluene, xylene, and nitrobenzene; and a mixture thereof.

The reaction can be performed by adding an oxidant as necessary.

Examples of the oxidant used for the reaction include oxygen, cupric chloride, and 2,3-dichloro-5,6-dicyano-p-benzoquinone.

In the reaction, with respect to 1 mole of the compound (M18), the compound (M5) is generally used at a ratio of 1 mole to 3 moles, the base is used at a ratio of 1 mole to 5 moles, the acid is generally used at a ratio of 1 mole to 5 moles, the sulfite is generally used at a ratio of 1 mole to 5 moles, the bisulfite is generally used at a ratio of 1 mole to 5 moles, and the oxidant is generally used at a ratio of 1 mole to 5 moles.

The temperature of the reaction is generally within the range of 50°C to 200°C. The reaction time of the reaction is generally within the range of 0.1 hours to 24 hours.

After completion of the reaction, it is possible to isolate the compound (2) by performing post-treatment operations, for example, subjecting the reaction mixture to extraction using an organic solvent after adding the reaction mixture to water; and drying and condensing an organic layer thereof. The isolated a compound (2) can be further purified through chromatography, recrystallization, etc.

(Production Method 11) (Step (C-1))

A compound (2) in which A1 is a sulfur atom in Formula (1) can be produced by reacting hydrochloride of a compound (M18) with a compound (M5) in accordance with Step (C-1).

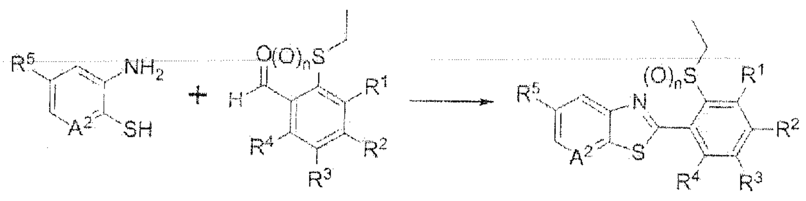

(M18) (M5) (2)

[wherein, R1, R2, R3, R4, R5, A2, and n each represent the same meanings as those referred to above.]

The reaction is performed in the presence of a base, and is generally performed in the presence of a solvent.

Examples of the base used for the reaction include tertiary amines, such as diisopropylethylamine and triethylamine.

Examples of the solvent used for the reaction include sulfoxides, such as DMSO; aromatic hydrocarbons, such as nitrobenzene; and a mixture thereof.

In the reaction, with respect to 1 mole of the compound (M18), the compound (M5) is generally used at a ratio of 0.5 moles to 3 moles and the base is generally used at a ratio of 1 mole to 2 moles.

The temperature of the reaction is generally within the range of 50°C to 200°C. The reaction time of the reaction is generally within the range of 0.1 hours to 24 hours.

After completion of the reaction, it is possible to isolate the compound (2) by performing post-treatment operations, for example, subjecting the reaction mixture to extraction using an organic solvent after adding the reaction mixture to water; and drying and condensing an organic layer thereof. The isolated a compound (2) can be purified through chromatography, recrystallization, etc.

(Production Method 12)

A compound (8) in which R5 is a cyano group or a C1-C3 alkyl group in Formula (1) can be produced by reacting a compound (7) with a cyanating agent or di(C1-C3 alkyl) zinc in the presence of a palladium compound.

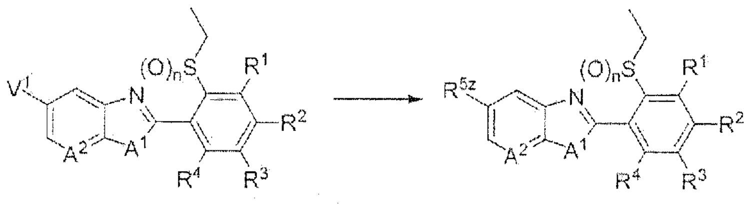

(7) (8)

[wherein, R1, R2, R3, R4,, A1, A2, and n each represent the same meanings as those referred to above; V1 represents leaving groups, such as a bromine atom or an iodine atom; and R5z represents a cyano group or a C1-C3 alkyl group.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; alcohols, such as methanol and ethanol; acid amides, such as DMF and NMP; sulfoxides, such as DMSO; and a mixture thereof.

Examples of the cyanating agent used for the reaction include zinc cyanide and examples of the di(C1-C3 alkyl) zinc include dimethyl zinc, diethyl zinc, and diisopropyl zinc.

Examples of the palladium compound used for the reaction include tetrakis (triphenylphosphine) palladium.

In the reaction, with respect to 1 mole of the compound (7), the cyanating agent or the di(C1-C3 alkyl) zinc is generally used at a ratio of 1 mole to 5 moles and the palladium compound is generally used at a ratio of 0.01 moles to 0.5 moles.

The temperature of the reaction is generally within the range of 50°C to 200°C. The reaction time of the reaction is generally within the range of 0.5 hours to 24 hours.

After completion of the reaction, it is possible to isolate the compound (8) by performing post-treatment operations, for example, subjecting the reaction mixture to extraction using an organic solvent; and drying and condensing an organic layer thereof. The isolated a compound (8) can be further purified through chromatography, recrystallization, etc.

(Production Method 13)

A compound (9) in which R5 is a C1-C3 perfluoroalkyl group in Formula (1) can be produced by reacting a compound (7) with a compound (M19) in the presence of copper iodide.

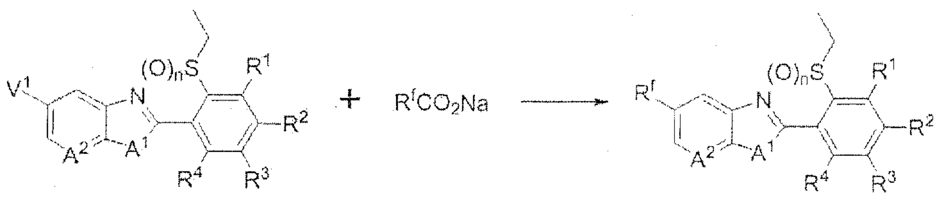

(7) (M19) (9)

[wherein, R1, R2, R3, R4, A1, A2, n, and V1 each represent the same meanings as those referred to above and Rf represents a C1-C3 perfluoroalkyl group.]

In general, the reaction is performed in the presence of a solvent.

Examples of the solvent used for the reaction include aromatic hydrocarbons, such as toluene and xylene; acid amides, such as DMF and NMP; and a mixture thereof.

In the reaction, with respect to 1 mole of the compound (7), the compound (M19) is generally used at a ratio of 1 mole to 10 moles and the copper iodide is generally used at a ratio of 1 mole to 5 moles.

The temperature of the reaction is generally within the range of 50°C to 200°C. The reaction time of the reaction is generally within the range of 0.5 hours to 24 hours.

After completion of the reaction, it is possible to isolate the compound (9) by performing post-treatment operations, for example, subjecting the reaction mixture to extraction using an organic solvent; and drying and condensing an organic layer thereof. The isolated a compound (9) can be further purified through chromatography, recrystallization, etc.

(Production Method 14) (Step (E-4))

A compound (3) in which n is 0 in Formula (1) can be produced by reacting a compound (M10) with a compound (M20) in the presence of a base in accordance with Step (E-4).

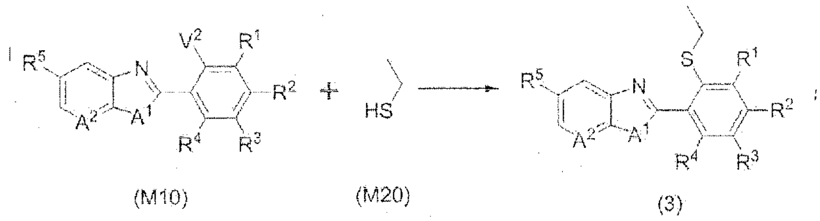

(M10) (M20) (3)

[wherein, R1, R2, R3, R4, R5, A1, A2, and V2 each represent the same meanings as those referred to above.]
In general, the reaction is performed in the presence of a solvent.
Examples of the solvent used for the reaction of the solvent used for the reaction include water,' ethers, such as THF, ethylene glycol dimethyl ether, tert-butyl methyl ether, and 1,4-dioxane; aromatic hydrocarbons, such as toluene and xylene; alcohols, such as methanol and ethanol; nitriles, such as acetonitrile; acid amides, such as DMF and NMP; sulfoxides, such as DMSO; and a mixture thereof.
Examples of the base used for the reaction include alkali metal hydrides, such as sodium hydride.
In the reaction, with respect to 1 mole of the compound (M10), the compound (M20) is generally used at a ratio of 1 mole to 10 moles and the base is generally used at a ratio of 1 mole tc 10 moles.
The temperature of the reaction is generally within the range of 0°C to 150°C. The reaction time of the reaction is generally within the range of 0.5 hours to 24 hours.
After completion of the reaction, it is possible to isolate the compound (3) by performing post-treatment operations, for example, subjecting the reaction mixture to extraction using an organic solvent; and drying and condensing an organic layer thereof. The isolated a compound (3) can be further purified through chromatography, recrystallization, etc.

(Production Method 15) (Step (F-2))

A compound (3) in which n is 0 in Formula (1) can be produced by reacting a compound (M11) with a compound (M21) in the presence of a base in accordance with Step (F-2).

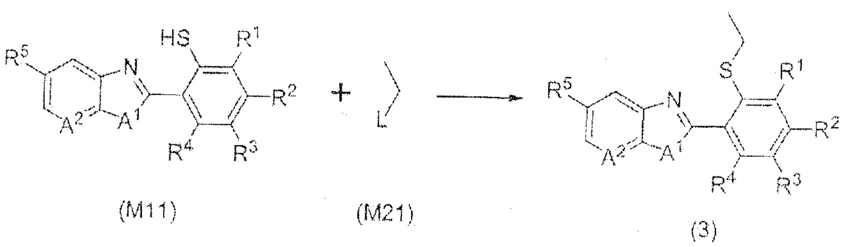

(M11) (M21) (3)

[wherein, R1, R2, R3, R4, R5, A1, A2, and L each represent the same meanings as those referred to above.]
In general, the reaction is performed in the presence of a solvent.
Examples of the solvent used for the reaction include ethers, such as THF, ethylene glycol dimethyl ether, tert-butyl methyl ether, and 1,4-dioxane; aromatic hydrocarbons, such as toluene and xylene; nitriles, such as acetonitrile; acid amides, such as DMF and NMP; sulfoxides, such as DMSO; and a mixture thereof.
Examples of the base used for the reaction include alkali metal or alkaline earth metal hydrides, such as sodium hydride, potassium hydride, and calcium hydride; inorganic bases, such as sodium carbonate and potassium carbonate; or organic bases such as triethylamine.
In the reaction, with respect to 1 mole of the compound (M11), the base is generally used at a ratio of 1 mole to 3 moles and the compound (M21) is generally used at a ratio of 1 mole to 3 moles.
The temperature of the reaction is generally within the range of 0°C to 100°C. The reaction time of the reaction is generally within the range of 0.1 hours to 24 hours. After completion of the reaction, it is possible to isolate the compound (3) by performing post-treatment operations, for example, subjecting the reaction mixture to extraction using

an organic solvent; and drying and condensing an organic layer thereof. The isolated a compound (3) can be further purified through chromatography, recrystallization, etc.

(Production Method 16)

A compound (10) in which n is 1 in Formula (1) can be produced by subjecting a compound (3) to an oxidation reaction.

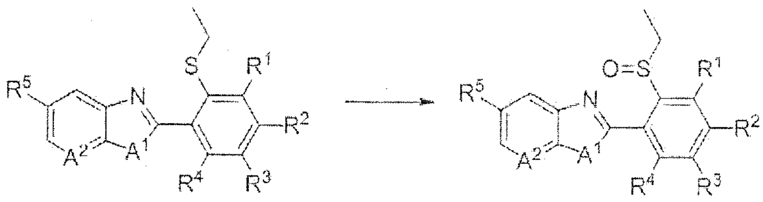

(3) (10)

[wherein, R1, R2, R3, R4, R5, A1, and A2 each represent the same meanings as those referred to above.]
In general, the reaction is performed in the presence of a solvent.
Examples of the solvent used for the reaction include halogenated hydrocarbons, such as dichloromethane and chloroform; alcohols, such as methanol and ethanol; acetic acid; water; and a mixture thereof.
Examples of the oxidant used for the reaction include sodium periodate or M-chloroperbenzoic acid.
In the reaction, with respect to 1 mole of the compound (3), the oxidant is generally used at a ratio of 1 mole to 3 moles. With respect to 1 mole of the compound (3), and the oxidant is preferably used at a ratio of 1 mole to 1.2 moles.
The temperature of the reaction is generally within the range of -20°C to 80°C. The reaction time of the reaction is generally within the range of 0.1 hours to 12 hours.
After completion of the reaction, it is possible to isolate the compound (10) by performing post-treatment operations, for example, subjecting the reaction mixture to extraction using an organic solvent; washing an organic layer thereof with an aqueous solution of a reducing agent (for example, sodium sulfite and sodium thiosulfate) and an aqueous solution of a base (for example, sodium hydrogen carbonate) as necessary; and drying and condensing the organic layer. The isolated a compound (10) can be further purified through chromatography, recrystallization, etc.

(Production Method 17)

A compound (11) in which n is 2 in Formula (1) can be produced by subjecting a compound (3) to a reaction in the presence of an oxidant.

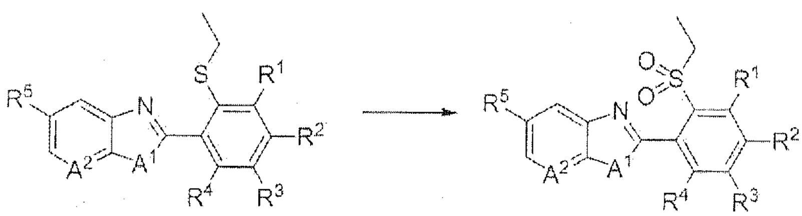

(3) (11)

[wherein, R1, R2, R3, R4, R5, A1, and A2 each represent the same meanings as those referred to above.]
In general, the reaction is performed in the presence of a solvent.
Examples of the solvent used for the reaction include halogenated hydrocarbons, such as dichloromethane and chloroform; alcohols, such as methanol and ethanol; acetic acid; water; and a mixture thereof.
Examples of the oxidant used for the reaction include m-chloroperbenzoic acid or hydrogen peroxide solution.
In the reaction, with respect to 1 mole of the compound (3), the oxidant is generally used at a ratio of 2 moles to 5 moles. With respect to 1 mole of the compound (3), the oxidant is preferably used at a ratio of 2 moles to 3 moles.
The temperature of the reaction is generally within the range of -20°C to 120°C. The reaction time of the reaction is generally within the range of 0.1 hours to 12 hours.
After completion of the reaction, it is possible to isolate the compound (11) by performing post-treatment operations, for example, subjecting the reaction mixture to extraction using an organic solvent; washing an organic layer thereof with an aqueous solution of a reducing agent (for example, sodium sulfite and sodium thiosulfate) and an aqueous solution of a base (for example, sodium hydrogen carbonate) as necessary; and drying and condensing the organic layer. The isolated a compound (11) can be further purified through chromatography, recrystallization, etc.

(Production Method 18)

A compound (11) in which n is 2 in Formula (1) can be produced by subjecting a compound (10) to a reaction in the presence of an oxidant.

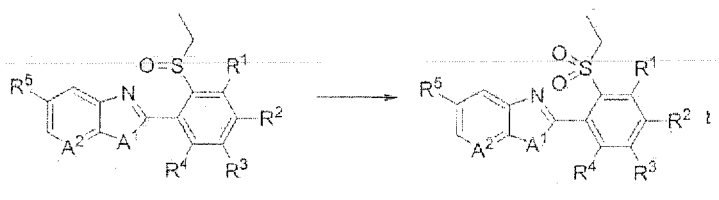

(10) (11)

[wherein, R1, R2, R3, R4, R5, A1, and A2 each represent the same meanings as those referred to above.]

In general, the reaction is performed in the presence of a solvent.

Examples of the solvent used for the reaction include halogenated hydrocarbons, such as dichloromethane and chloroform; alcohols, such as methanol and ethanol; acetic acid; water; and a mixture thereof.

Examples of the oxidant used for the reaction include m-chloroperbenzoic acid or hydrogen peroxide solution.

In the reaction, with respect to 1 mole of the compound (10), the oxidant is generally used at a ratio of 1 mole to 4 moles. With respect to 1 mole of the compound (10), the oxidant is preferably used at a ratio of 1 mole to 2 moles.

The temperature of the reaction is generally within the range of -20°C to 120°C. The reaction time of the reaction is generally within the range of 0.1 hours to 12 hours.

After completion of the reaction, it is possible to isolate the compound (11) by performing post-treatment operations, for example, subjecting the reaction mixture to extraction using an organic solvent; washing an organic layer thereof with an aqueous solution of a reducing agent (for example, sodium sulfite and sodium thiosulfate) and an aqueous solution of a base (for example, sodium hydrogen carbonate) as necessary; and drying and condensing the organic layer. The isolated a compound (11) can be further purified through chromatography, recrystallization, etc.

(Production Method 19) (Step (A-3) and Step (B-3))

A compound (2) in which A1 is a sulfur atom in Formula (1) can be produced by subjecting a compound (M22) to a reaction in the presence of an acid in accordance with Step (A-3) and Step (B-3).

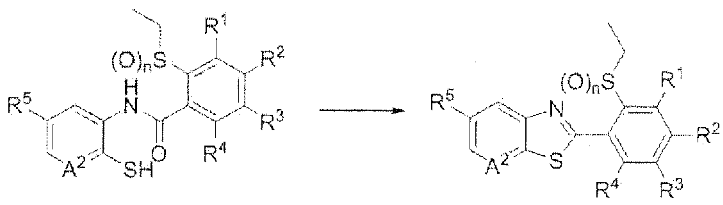

(M22) (2)

[wherein, R1, R2, R3, R4, R5, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aromatic hydrocarbons, such as toluene and xylene; halogenated hydrocarbons, such as dichloromethane, chloroform, and chlorobenzene; and a mixture thereof.

Examples of the acid used for the reaction include sulfonic acids such as p-toluene sulfonic acid; and polyphosphoric acid.

In the reaction, with respect to 1 mole of the compound (M22), the acid is generally used at a ratio of 1 mole to 3 moles.

The temperature of the reaction is generally within the range of 50°C to 200°C. The reaction time of the reaction is generally within the range of 1 hour to 24 hours.

After completion of the reaction, it is possible to isolate the compound (2) by performing post-treatment operations, for example, subjecting the reaction mixture to extraction using an organic solvent; and drying and condensing an organic layer thereof. The isolated a compound (2) can be further purified through chromatography, recrystallization, etc.

(Production Method 20)

A compound (12-a) in which R5 is represented by -SH, a compound (12-b) which is a compound of a disulfide body of the compound (12-a), a compound (13) in which R5 is represented by -SR7, and a compound (14) in which R5 is represented by -S(O)mR7 in Formula (1) can be produced by the following method, for example.

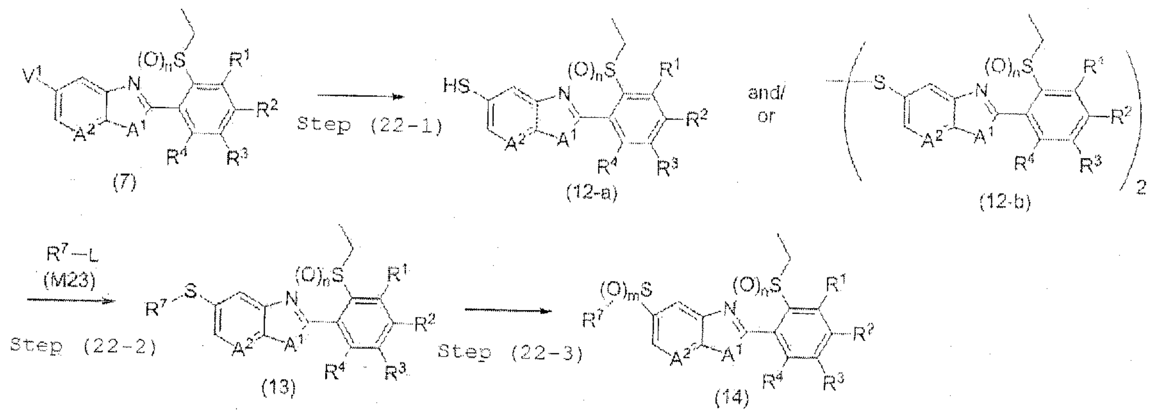

(7) (12-a) (12-b) (13) (14)

[wherein, R1, R2, R3, R4, R7, A1, A2, n, m, V1, and L each represent the same meanings as those referred to above.]

Step (22-1)

The compound (12-a) and/or the compound (12-b) can be produced by reacting a compound (7) with a thiolating agent in the presence of a catalyst.

In general, the reaction is performed in the presence of a solvent.

Examples of the solvent used for the reaction include aromatic hydrocarbons, such as toluene and xylene; acid amides, such as DMF and NMP; sulfoxides, such as DMSO; and a mixture thereof.

Examples of the thiolating agent used for the reaction include sodium sulfide, sodium sulfide 9-hydrate, and thiourea.

Examples of the catalyst used for the reaction include copper (I) chloride, copper (I) bromide, and copper (I) iodide.

The reaction can be performed in the presence of a base as necessary.

Examples of the base used for the reaction include potassium carbonate, cesium carbonate, tripotassium phosphate, and triethylamine.

In the reaction, with respect to 1 mole of the compound (7), the thiolating agent is generally used at a ratio of 1 mole to 10 moles and the catalyst is generally used at a ratio of 0.1 moles to 5 moles.

The temperature of the reaction is generally within the range of 50°C to 200°C. The reaction time of the reaction is generally within the range of 0.5 hours to 24 hours.

After completion of the reaction, it is possible to isolate the compound (12-a) and/or the compound (12-b) by performing post-treatment operations, for example, subjecting the reaction mixture to extraction using an organic solvent; and drying and condensing an organic layer thereof. The isolated a compound (12-a) and/or a compound (12-b) can be further purified through chromatography, recrystallization, etc.

Step (22-2)

The compound (13) can be produced by reacting the compound (12-a) and/or the compound (12-b) with a compound (M23) in the presence of a base.

In general, the reaction is performed in the presence of a solvent.

Examples of the solvent used for the reaction include alcohols, such as methanol and ethanol; ethers, such as 1,4-dioxane, diethyl ether, THF, and tert-butyl methyl ether; halogenated hydrocarbons, such as dichloromethane, chloroform, carbon tetrachloride, 1,2-dichloroethane, and chlorobenzene; aromatic hydrocarbons, such as toluene, benzene, and xylene; esters, such as ethyl acetate and butyl acetate; nitriles, such as acetonitrile; aprotic polar solvents, such as DMF, NMP, 1,3-dimethyl-2-imidazolidinone, and dimethyl sulfoxide; nitrogen-containing aromatic compounds, such as pyridine and quinoline; water; and a mixture thereof.

Examples of the base used for the reaction include nitrogen-containing heterocyclic compounds, such as pyridine, picoline, 2,6-lutidine, diazabicycloundecene (hereinafter, referred to as DBU), and 1,5-diazabicyclo [4.3.0]-5-nonene; tertiary amines, such as triethylamine and N-ethyldiisopropylamine; inorganic bases, such as tripotassium phosphate, potassium carbonate, sodium hydride, sodium hydroxide, and potassium hydroxide.

In the reaction, with respect to 1 mole of the compound (12-a) and/or the compound (12-b), the compound (M23) is generally used at a ratio of 1 mole to 10 moles and the base is generally used at a ratio of 1 mole to 5 moles.

The temperature of the reaction is generally within the range of 0°C to 120°C. The reaction time of the reaction is generally within the range of 0.5 hours to 24 hours.

After completion of the reaction, it is possible to isolate the compound (13) by performing post-treatment operations, for example, subjecting the reaction mixture to extraction using an organic solvent; and drying and condensing an organic layer thereof. The isolated a compound (13) can be further purified through chromatography, recrystallization, etc.

Step (22-3)

The compound (14) in which m is 1 or 2 can be produced by subjecting the compound (13) to an oxidation reaction.

In general, the reaction is performed in the presence of a solvent.

Examples of the solvent used for the reaction include halogenated hydrocarbons, such as dichloromethane and chloroform; alcohols, such as methanol and ethanol; acetic acid; water; and a mixture thereof.

Examples of the oxidant used for the reaction include m-chloroperbenzoic acid or hydrogen peroxide solution.

The reaction can be performed in the presence of a catalyst as necessary.

Examples of the catalyst used for the reaction include sodium tungstate.

In the reaction, with respect to 1 mole of the compound (13), the oxidant is generally used at a ratio of 1 mole to 5 moles.

The temperature of the reaction is generally within the range of -20°C to 120°C. The reaction time of the reaction is generally within the range of 0.1 hours to 12 hours.

After completion of the reaction, it is possible to isolate the compound (14) by performing post-treatment operations, for example, subjecting the reaction mixture to extraction using an organic solvent; washing an organic layer thereof with an aqueous solution of a reducing agent (for example, sodium sulfite and sodium thiosulfate) and an aqueous solution of a base (for example, sodium hydrogen carbonate) as necessary; and drying and condensing the organic layer. The isolated a compound (14) can be further purified through chromatography, recrystallization, etc.

(Production Method 21)

A compound (15) in which R5 is -OR7 in Formula (1) can be produced by the following method, for example.

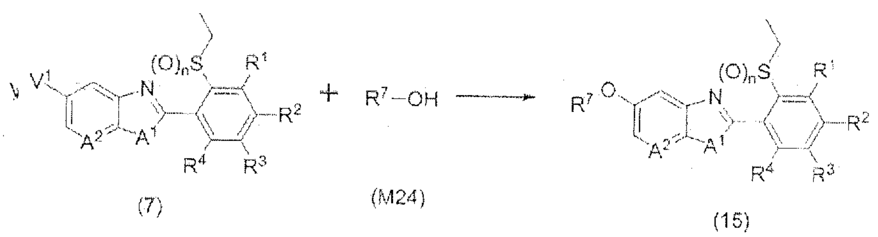

(7) (M24) (15)

[wherein, R1, R2, R3, R4, R7, A1, A2, V1, and n each represent the same meanings as those referred to above.]

Examples of the solvent used for the reaction include ethers, such as THF, ethylene glycol dimethyl ether, tert-butyl methyl ether, and 1,4-dioxane; aromatic hydrocarbons, such as toluene and xylene; nitriles, such as acetonitrile; acid amides, such as DMF and NMP; sulfoxides, such as DMSO; and a mixture thereof.

Examples of the base used for the reaction include alkali metal or alkaline earth metal hydrides, such as sodium hydride, potassium hydride, and calcium hydride; inorganic bases, such as sodium carbonate, potassium carbonate,

and cesium carbonate; or organic bases such as triethylamine.

The reaction can be performed by adding a copper compound as necessary.

Examples of the copper compound used for the reaction include copper, copper (I) iodide, copper (I) bromide, and copper (I) chloride.

In the reaction, with respect to 1 mole of the compound (7), a compound (M24) is generally used at a ratio of 1 mole to 5 moles and the base is generally used at a ratio of 1 mole to 3 moles.

The temperature of the reaction is generally within the range of 20°C to 250°C. The reaction time of the reaction is generally within the range of 0.1 hours to 48 hours.

After completion of the reaction, it is possible to isolate the compound (15) by subjecting the reaction mixture to extraction using an organic solvent after adding the reaction mixture to water and condensing an organic layer thereof; collecting the solid, which has been generated by adding the reaction mixture to water, through filtering; or collecting the solid, which has been generated in the reaction mixture, through filtering. The isolated a compound (15) can be further purified through reorystallization, chromatography, etc.

(Production Method 22) (Step (A-1))

A compound (2) in which A1 is a sulfur atom in Formula (1) can be produced by reacting a compound (M18) with a compound (M2) in accordance with Step (A-1).

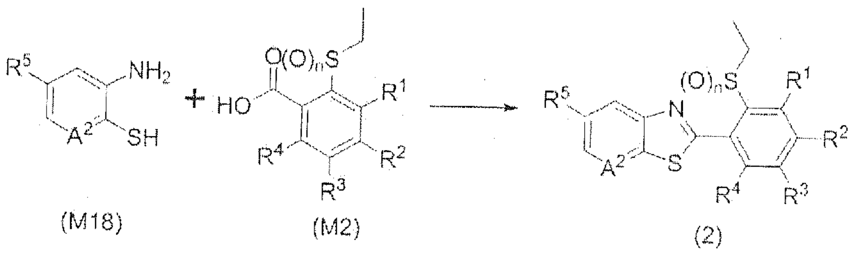

[wherein, R1, R2, R3, R4, R5, A2, and n each represent the same meanings as those referred to above.]

In general, the reaction is performed in the presence of a solvent.

Examples of the solvent used for the reaction include ethers, such as THF, tert-butyl methyl ether, ethylene glycol dimethyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.

Examples of dehydration condensation agents used for the reaction include WSC, carbodiimides, such as 1,3-dicyclohexylcarbodiimide; and a BOP reagent.

The reaction can be performed by adding a catalyst as necessary.

The catalyst used for the reaction includes HOBt.

In the reaction, with respect to 1 mole of the compound (M18), the compound (M2) is generally used at a ratio of 1 mole to 3 moles, the dehydration condensation agent is generally used at a ratio of 1 mole to 5 moles, and the catalyst is generally used at a ratio of 0.01 moles to 0.1 moles.

The temperature of the reaction is generally within the range of 30°C to 200°C. The reaction time of the reaction is generally within the range of 0.1 hours to 24 hours.

After completion of the reaction, it is possible to isolate the compound (2) by performing post-treatment operations, for example, subjecting the reaction mixture to extraction using an organic solvent after adding water to the reaction mixture; and drying and condensing an organic layer thereof. The isolated a compound (2) can be further purified through chromatography, recrystallization, etc.

In addition, it is possible to produce the compound (2) based on the above-described method using the compound (M4) instead of the compound (M2) in accordance with Step (B-1).

When using the compound (M4), in general, the reaction is performed without adding the dehydration condensation agent. The reaction can be performed by adding a base as necessary.

Examples of the base include alkali metal carbonates, such as sodium carbonate and potassium carbonate; tertiary amines, such as triethylamine and diisopropylethylamine; and nitrogen-containing aromatic compounds, such as pyridine and 4-dimethylaminopyridine.

In the reaction, with respect to 1 mole of the compound (M18), the compound (M4) is generally used at a ratio of 1 mole to 3 moles and the base is generally used at a ratio of 1 mole to 10 moles.

(Production Method 23) (Production Method D)

A compound (2) in which A1 is a sulfur atom in Formula (1) can be produced by reacting a compound (M7) and a sulfurizing agent in accordance with (Production Method D).

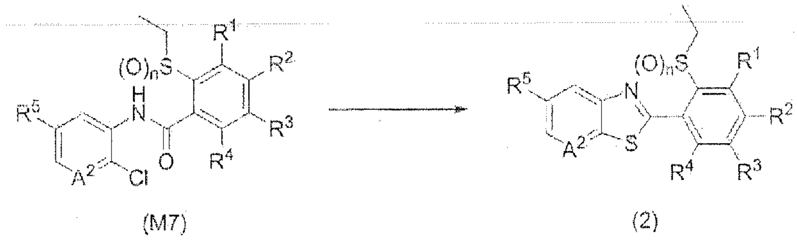

[wherein, R1, R2, R3, R4, R5, A2, and n each represent the same meanings as those referred to above.]

The reaction is performed in the presence or absence of a solvent.

Examples of the solvent used for the reaction include ethers, such as 1,4-dioxane, diethyl ether, tetrahydrofuran, tert-butyl methyl ether, and diglyme; halogenated hydrocarbons, such as dichloromethane, chloroform, carbon tetrachloride, 1,2-dichloroethane, and chlorobenzene; hydrocarbons, such as toluene, benzene, and xylene; nitriles, such as acetonitrile; pyridines such as pyridine, picoline, and lutidine; and a mixture thereof.

Examples of the sulfurizing agent used for the reaction include diphosphorus pentasulfide, Lawesson's reagent (2,4-bis-(4-methoxyphenyl)-1,3-dithia-2,4-diphosphetane-2,4-disulfide).

The amount of the sulfurizing agent used for the reaction is generally greater than or equal to 1 mole with respect to 1 mole of the compound (M7).

The temperature of the reaction is generally within the range of 0°C to 200°C. The reaction time of the reaction is generally within the range of 1 hour to 24 hours.

After completion of the reaction, it is possible to isolate the compound (2) by subjecting the reaction mixture to extraction using an organic solvent after adding the reaction mixture to water and condensing an organic layer thereof; collecting the solid, which has been generated by adding the reaction mixture to water, through filtering; or collecting the solid, which has been generated in the reaction mixture, through filtering. The isolated a compound (2) can be further purified through recrystallization, chromatography, etc.

An intermediate of the present invention can be produced through the following method, for example.

(Intermediate Production Method 1)

A compound (M12) can be produced through the following method.

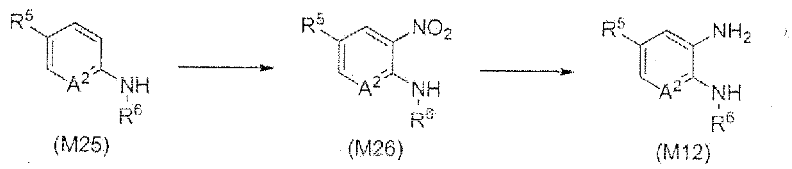

[wherein, R5, R6, and A2 each represent the same meanings as those referred to above.]

(Step 1)

A compound (M26) can be produced by subjecting a compound (M25) to a reaction in the presence of a nitrating agent.

In general, the reaction is performed in the presence of a solvent.

Examples of the solvent used for the reaction include halogenated hydrocarbons, such as dichloromethane and chloroform; acetic acid; concentrated sulfuric acid; concentrated nitric acid; water; and a mixture thereof.

Examples of the nitrating agent used for the reaction include concentrated nitric acid.

In the reaction, with respect to 1 mole of the compound (M25), the nitrating agent is generally used at a ratio of 1 mole to 3 moles.

The temperature of the reaction is generally within the range of -10°C to 100°C. The reaction time of the reaction is generally within the range of 0.1 hours to 24 hours.

After completion of the reaction, it is possible to isolate the compound (M26) by performing post-treatment operations, for example, adding the reaction mixture to water; subjecting the reaction mixture to extraction using an organic solvent; and drying and condensing an organic layer thereof. The isolated a compound (M26) can be further purified through chromatography, recrystallization, etc.

(Step 2)

A compound (M12) can be produced by reacting the compound (M26) and hydrogen in the presence of a hydrogenation catalyst.
In general, the reaction is performed in the presence of a solvent under a hydrogen atmosphere of 1 to 100 times atmospheric pressure.
Examples of the solvent used for the reaction include ethers, such as THF, ethylene glycol dimethyl ether, tert-butyl methyl ether, and 1,4-dioxane; esters, such as ethyl acetate and butyl acetate; alcohols, such as methanol and ethanol; water; and a mixture thereof.
Examples of the hydrogenation catalyst used for the reaction include transition metal compounds, such as palladium carbon, palladium hydroxide, raney nickel, and platinum oxide.
In the reaction, with respect to 1 mole of the compound (M26), hydrogen is generally used at a ratio of 3 moles and the hydrogenation catalyst is generally used at a ratio of 0.001 moles to 0.5 moles.
The reaction can be performed by adding an acid (base, etc.) as necessary.
The temperature of the reaction is generally within the range of -20°C to 100°C. The reaction time of the reaction is generally within the range of 0.1 hours to 24 hours.
After completion of the reaction, it is possible to isolate the compound (M12) by performing post-treatment operations, for example, filtering the reaction mixture; subjecting the reaction mixture to extraction using an organic solvent as necessary; and drying and condensing an organic layer thereof. The isolated a compound (M12) can be further purified through chromatography, recrystallization, etc.

(Intermediate Production Method 2) (Step (A-2))

A compound (M13) can be produced by reacting a compound (M12) and a compound (M2) in the presence of a dehydration condensation agent in accordance with Step (A-2).

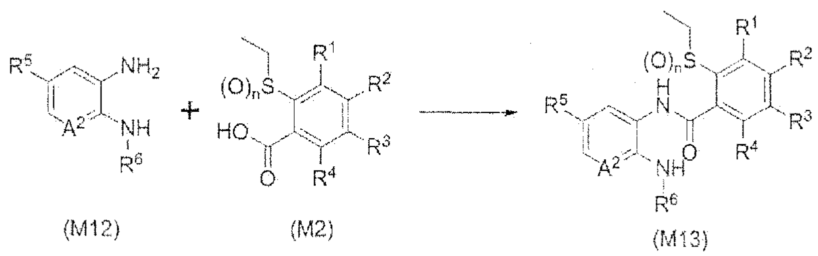

[wherein, R1, R2, R3, R4, R5, R6, A2, and n each represent the same meanings as those referred to above.]
In general, the reaction is performed in the presence of a solvent.
Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.
Examples of the dehydration condensation agent used for the reaction include WSC, carbodiimides, such as 1,3-dicyclohexylcarbodiimide; and a BOP reagent.
In the reaction, with respect to 1 mole of the compound (M12), the compound (M2) is generally used at a ratio of 1 mole to 3 moles and the dehydration condensation agent is generally used at a ratio of 1 mole to 5 moles.
The temperature of the reaction is generally within the range of 0°C to 140°C. The reaction time of the reaction is generally within the range of 0.1 hours to 24 hours.
After completion of the reaction, it is possible to isolate the compound (M13) by performing post-treatment operations, for example, subjecting the reaction mixture to extraction using an organic solvent after adding water to the reaction mixture; and drying and condensing an organic layer thereof. The isolated a compound (M13) can be further purified through chromatography, reorystallization, etc.

(Intermediate Production Method 3) (Step (B-2))

A compound (M13) can be produced by reacting a compound (M12) and a compound (M4) in the presence of a base in accordance with Step (B-2).

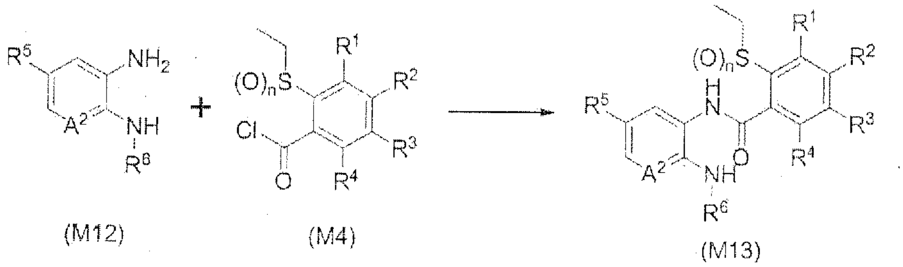

[wherein, R1, R2, R3, R4, R5, R6, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; and a mixture thereof.

Examples of the base include alkali metal carbonates, such as sodium carbonate and potassium carbonate; tertiary amines, such as triethylamine and diisopropylethylamine; and nitrogen-containing aromatic compounds, such as pyridine and 4-dimethylaminopyridine.

In the reaction, with respect to 1 mole of the compound (M12), the compound (M4) is generally used at a ratio of 1 mole to 3 moles and the base is generally used at a ratio of 1 mole to 10 moles.

The temperature of the reaction is generally within the range of -20°C to 100°C. The reaction time of the reaction is generally within the range of 0.1 hours to 24 hours.

After completion of the reaction, it is possible to isolate the compound (M13) by performing post-treatment operations, for example, subjecting the reaction mixture to extraction using an organic solvent after adding water to the reaction mixture; and drying and condensing an organic layer thereof. The isolated a compound (M13) can be further purified through chromatography, recrystallization, etc.

(Intermediate Production Method 4)

Compound (M15) can be produced through the following method.

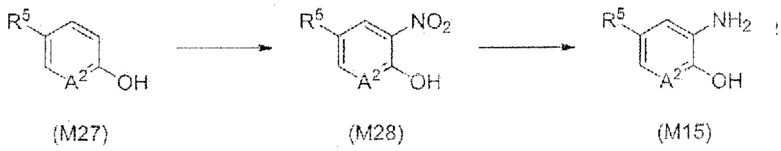

[wherein, R5 and A2 each represent the same meanings as those referred to above.]'

(Step 1)

A compound (M28) can be produced by subjecting a compound (M27) to a reaction in the presence of a nitrating agent.

In general, the reaction is performed in the presence of a solvent.

Examples of the solvent used for the reaction include aliphatic halogenated hydrocarbons, such as chloroform; acetic acid; concentrated sulfuric acid; concentrated nitric acid; water; and a mixture thereof.

Examples of the nitrating agent used for the reaction include concentrated nitric acid.

In the reaction, with respect to 1 mole of the compound (M27), the nitrating agent is generally used at a ratio of 1 mole to 3 moles.

The temperature of the reaction is generally within the range of -10°C to 100°C. The reaction time of the reaction

is generally within the range of 0.1 hours to 24 hours.

After completion of the reaction, it is possible to isolate the compound (M28) by performing post-treatment operations, for example, addiing the reaction mixture to water; subjecting the reaction mixture to extraction using an organic solvent; and drying and condensing an organic layer thereof. The isolated a compound (28) can be further purified through chromatography, recrystallization, etc.

(Step 2)

The compound (M15) can be produced by reacting the compound (M28) and hydrogen in the presence of a hydrogenation catalyst.

In general, the reaction is performed in the presence of a solvent under a hydrogen atmosphere of 1 to 100 times atmospheric pressure.

Examples of the solvent used for the reaction include ethers, such as THF, ethylene glycol dimethyl ether, tert-butyl methyl ether, and 1,4-dioxane; esters, such as ethyl acetate and butyl acetate; alcohols, such as methanol and ethanol; water; and a mixture thereof.

Examples of the hydrogenation catalyst used for the reaction include transition metal compounds, such as palladium carbon, palladium hydroxide, raney nickel, and platinum oxide.

In the reaction, with respect to 1 mole of the compound (M28), hydrogen is generally used at a ratio of 3 moles and the hydrogenation catalyst is generally used at a ratio of 0.001 moles to 0.5 moles.

The reaction can be performed by adding an acid (base, etc.) as necessary.

The temperature of the reaction is generally within the range of -20°C to 100°C. The reaction time of the reaction is generally within the range of 0.1 hours to 24 hours.

After completion of the reaction, it is possible to isolate the compound (M15) by performing post-treatment operations, for example, filtering the reaction mixture; subjecting the reaction mixture to extraction using an organic solvent as necessary; and drying and condensing an organic layer thereof. The isolated a compound (M15) can be further purified through chromatography, recrystallization, etc.

(Intermediate Production Method 5) (Step (C-2))

A compound (M16) can be produced by reacting a compound (M15) and a compound (M5) in accordance with Step (C-2).

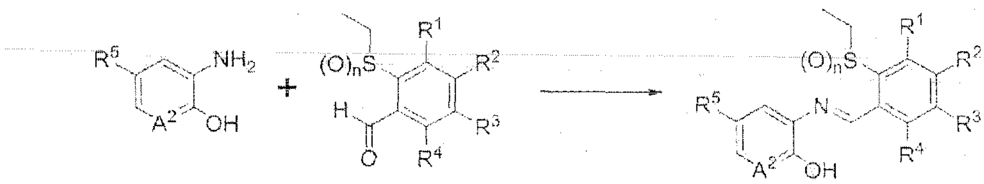

(M15) (M5) (M16)

[wherein, R1, R2, R3, R4, R5, A2, and n each represent the same meanings as those referred to above.]

In general, the reaction is performed in the presence of a solvent.

Examples of the solvent used for the reaction include alcohols, such as methanol and ethanol;' ethers, such as THF, ethylene glycol dimethyl ether, tert-butyl methyl ether, and 1,4-dioxane; aromatic hydrocarbons, such as toluene and xylene; and a mixture thereof.

In the reaction, with respect to 1 mole of the compound (M15), the compound (M5) is generally used at a ratio of 1 mole to 3 moles.

The reaction can be performed by adding an acid, a base, etc. as necessary.

The temperature of the reaction is generally within the range of 0°C to 150°C. The reaction time of the reaction is generally within the range of 0.1 hours to 24 hours.

After completion of the reaction, it is possible to isolate the compound (M16) by performing post-treatment operations, for example, subjecting the reaction mixture to extraction using an organic solvent; and drying and condensing an organic layer thereof. The isolated a compound (M16) can be further purified through chromatography, recrystallization, etc.

((Intermediate Production Method 6) (Step (A-2))

A compound (M17) can be produced by reacting a compound (M15) and a compound (M2) in the presence of a dehydration condensation agent in accordance with Step (A-2).

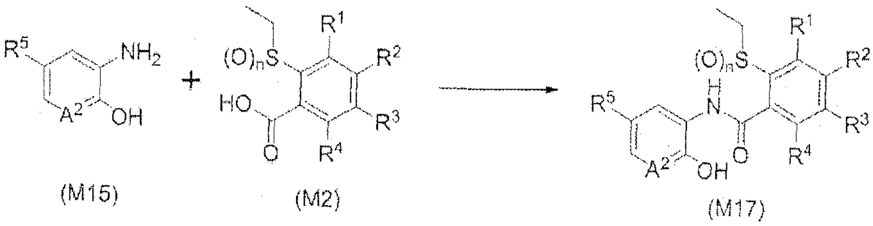

(M15) (M2) (M17)

[wherein, R1, R2, R3, R4, R5, A2, and n each represent the same meanings as those referred to above.]
In general, the reaction is performed in the presence of a solvent.
Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.
Examples of the dehydration condensation agent used for the reaction include WSC, carbodiimides, such as 1,3-dicyclohexylcarbodiimide; and a BOP reagent.
In the reaction, with respect to 1 mole of the compound (M15), the compound (M2) is generally used at a ratio of 1 mole to 3 moles and the dehydration condensation agent is generally used at a ratio of 1 mole to 5 moles.
The temperature of the reaction is generally within the range of 0°C to 140°C. The reaction time of the reaction is generally within the range of 0.1 hours to 24 hours.
After completion of the reaction, it is possible to isolate the compound (M17) by performing post-treatment operations, for example, subjecting the reaction mixture to extraction using an organic solvent after adding water to the reaction mixture; and drying and condensing an organic layer thereof. The isolated a compound (M17) can be further purified through chromatography, recrystallization, etc.

(Intermediate Production Method 7) - (Step (B-2))

A compound (M17) can be produced by reacting a compound (M15) and a compound (M4) in the presence of a base in accordance with Step (B-2).

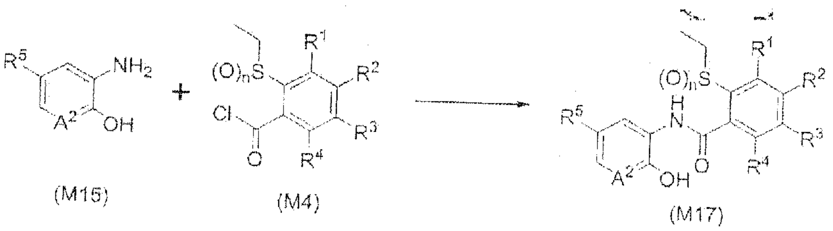

(M15) (M4) (M17)

[wherein, R1, R2, R3, R4, R5, A2, and n each represent the same meanings as those referred to above.]
In general, the reaction is performed in the presence of a solvent.
Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; and a mixture thereof.
Examples of the base used for the reaction include alkali metal carbonates, such as sodium carbonate and potassium carbonate; tertiary amines, such as triethylamine and diisopropylethylamine; and nitrogen-containing aromatic compounds, such as pyridine and 4-dimethylaminopyridine.
In the reaction, with respect to 1 mole of the compound (M15), the compound (M4) is generally used at a ratio of 1 mole to 3 moles and the base is generally used at a ratio of 1 mole to 10 moles.

The temperature of the reaction is generally within the range of -20°C to 100°C. The reaction time of the reaction is generally within the range of 0.1 hours to 24 hours.
After completion of the reaction, it is possible to isolate the compound (M17) by performing post-treatment operations, for example, subjecting the reaction mixture to extraction using an organic solvent after adding water to the reaction mixture; and drying and condensing an organic layer thereof. The isolated a compound (M17) can be further purified through chromatography, recrystallization, etc.

(Intermediate Production Method 8)

A compound (M18) can be produced through the following method.

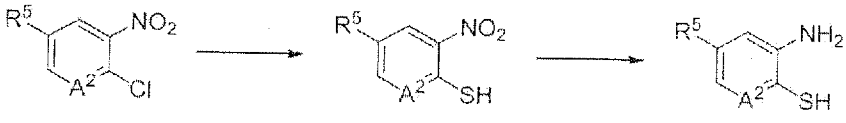

(M29) (M30) (M18)

[wherein, R5 and A2 each represent the same meanings as those referred to above.]

(Step 1)

A compound (M30) can be produced by reacting a compound (M29) and thiourea in the presence of a base.
In general, the reaction is performed in the presence of a solvent.
Examples of the solvent used for the reaction include alcohols, such as methanol and ethanol; water; and a mixture thereof.
Examples of the base used for the reaction include alkali metal hydroxides, such as sodium hydroxide and potassium hydroxide.
In the reaction, with respect to 1 mole of the compound (M29), thiourea is generally used at a ratio of 0.5 moles to 3 moles and the base is generally used at a ratio of 1 mole to 10 moles.
The temperature of the reaction is generally within the range of 0°C to 100°C. The reaction time of the reaction is generally within the range of 0.1 hours to 24 hours.
After completion of the reaction, it is possible to isolate the compound (M30) by performing post-treatment operations, for example, subjecting the reaction mixture to extraction using an organic solvent after adding an acid to the reaction mixture; and drying and condensing an organic layer thereof. The isolated a compound (M30) can be further purified through chromatography, recrystallization, etc.

(Step 2)

The compound (M18) can be produced by subjecting the compound (M30) to a reduction reaction.
The reduction reaction can be performed in the presence of reducing agents, such as iron powder and zinc powder; acids such as hydrochloric acid and acetic acid; and water.
In general, the reaction is performed in the presence of a solvent.
Examples of the solvent used for the reaction include ethers, such as THF, ethylene glycol dimethyl ether, tert-butyl methyl ether, and 1,4-dioxane; esters, such as ethyl acetate and butyl acetate; alcohols, such as methanol and ethanol; acid amides, such as DMF and NMP; and a mixture thereof.
In the reaction, with respect to 1 mole of the compound (M30), the reducing agent is generally used at a ratio of 3 moles to 10 moles.
The temperature of the reaction is generally within the range of 0°C to 100°C. The reaction time of the reaction is generally within the range of 0.1 hours to 24 hours.
After completion of the reaction, it is possible to isolate the compound (M18) by performing post-treatment operations, for example, subjecting the reaction mixture to extraction using an organic solvent after adding water to the reaction mixture; and drying and condensing an organic layer thereof. The isolated a compound (M18) can be purified through chromatography, recrystallization, etc.

(Intermediate Production Method 9)

A compound (M33) in which A1 is -NR6- in a compound (M10) can be produced by reacting a compound (M12)

and a compound (M31) in the presence of a base.

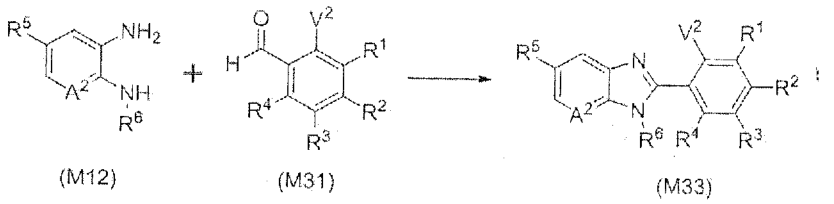

(M12) (M31) (M33)

[wherein, R1, R2, R3, R4, R5, R6, A2, and V2 each represent the same meanings as those referred to above.]

In general, the reaction is performed in the presence of a solvent.

Examples of the base used for the reaction include hydrogen carbonates, such as sodium hydrogen carbonate and potassium hydrogen carbonate; carbonates, such as sodium carbonate and potassium carbonate; sulfite, such as sodium sulfite and potassium sulfite; and a mixture thereof.

Examples of the solvent used for the reaction include ethers, such as THF, ethylene glycol dimethyl ether, tert-butyl methyl ether, and 1,4-dioxane: aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.

In the reaction, with respect to 1 mole of the compound (M12), the compound (M31) is generally used at a ratio of 1 mole to 3 moles and the base is generally used at a ratio of 1 mole to 5 moles.

The temperature of the reaction is generally within the range of 30°C to 200°C. The reaction time of the reaction is generally within the range of 0.1 hours to 24 hours.

After completion of the reaction, it is possible to isolate the compound (M33) by performing post-treatment operations, for example, subjecting the reaction mixture to extraction using an organic solvent after adding water to the reaction mixture; and drying and condensing an organic layer thereof. The isolated a compound (M33) can be further purified through chromatography, recrystallization, etc.

(Intermediate Production Method 10) (Step (E-1))

A compound (M33) in which A1 is -NR6- in a compound (M10) can be produced by reacting a compound (M12) and a compound (M8) in the presence of a dehydration condensation agent in accordance with Step (E-1).

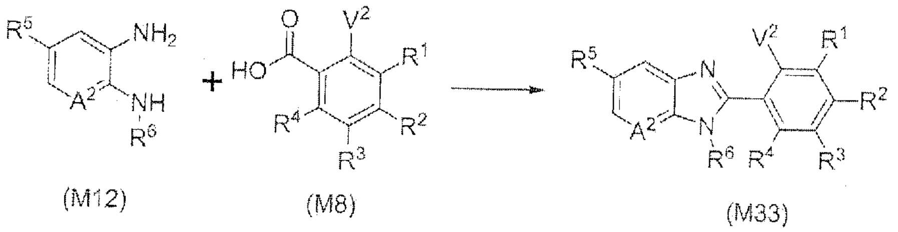

(M12) (M8) (M33)

[wherein, R1, R2, R3, R4, R5, R6, A2, and V2 each represent the same meanings as those referred to above.]

In general, the reaction is performed in the presence of a solvent.

Examples of the solvent used for the reaction include ethers, such as THF, tert-butyl methyl ether, ethylene glycol dimethyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.

Examples of dehydration condensation agents used for the reaction include WSC, carbodiimides, such as 1,3-dicyclohexylcarbodiimide; and a BOP reagent. The reaction can be performed by adding a catalyst as necessary. The catalyst used for the reaction includes HOBt.

In the reaction, with respect to 1 mole of the compound (M12), the compound (M8) is generally used at a ratio of 1 mole to 3 moles, the dehydration condensation agent is generally used at a ratio of 1 mole to 5 moles, and the catalyst is generally used at a ratio of 0.01 moles to 0.1 moles.

The temperature of the reaction is generally within the range of 30°C to 200°C. The reaction time of the reaction is generally within the range of 0.1 hours to 24 hours.

After completion of the reaction, it is possible to isolate the compound (M33) by performing post-treatment operations, for example, subjecting the reaction mixture to extraction using an organic solvent after adding water to the reaction mixture; and drying and condensing an organic layer thereof. The isolated a compound (M33) can be further purified through chromatography, recrystallization, etc.

(Intermediate Production Method 11) (Step (E-3))

A compound (M33) in which A1 is -NR6- in a compound (M10) can be produced by dehydrating and condensing a compound (M32) in accordance with Step (E-3).

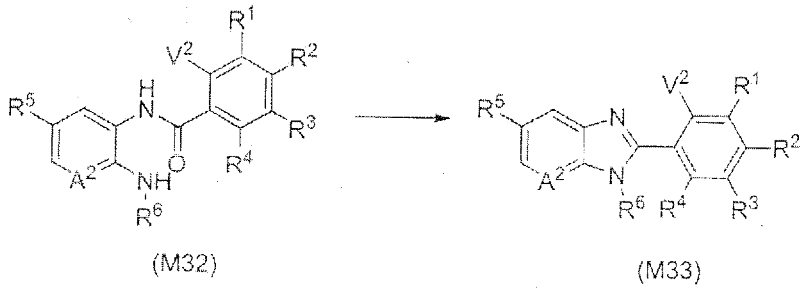

[wherein, R1, R2, R3, R4, R5, R6, A2, and V2 each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; alcohols, such as methanol, ethanol, propanol, butanol, and pentanol; and a mixture thereof.

It is possible to use an acid or a dehydrating agent for the reaction as necessary. Examples of the acid used for the reaction include sulfonic acids, such as p-toluenesulfonic acid; and carboxylic acids, such as acetic acid. Examples of the dehydrating agent used for the reaction include phosphorus oxychloride, acetic anhydride, and trifluoroacetic anhydride.

In the reaction, with respect to 1 mole of the compound (M32), the acid or the dehydrating agent is generally used at a ratio of 1 mole to 10 moles.

The temperature of the reaction is generally within the range of 30°C to 200°C. The reaction time of the reaction is generally within the range of 0.1 hours to 24 hours.

After completion of the reaction, it is possible to isolate the compound (M33) by performing post-treatment operations, for example, subjecting the reaction mixture to extraction using an organic solvent; and drying and condensing an organic layer thereof. The isolated a compound (M33) can be further purified through chromatography, recrystallization, etc.

(Intermediate Production Method 12) (Step (E-3))

A compound (M33) in which A1 is -NR6- in a compound (M10) can be produced by subjecting a compound (M32) to a reaction in the presence of a base in accordance with Step (E-3).

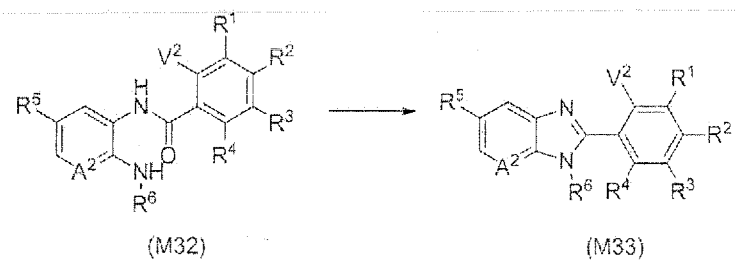

[wherein, R1, R2, R3, R4, R5, R6, A2, and V2 each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; alcohols, such as methanol, ethanol, propanol, butanol, tert-butanol, and pentanol; and a mixture thereof.

Examples of the base used for the reaction include tripotassium phosphate.

In the reaction, with respect to 1 mole of the compound (M32), the base is generally used at a ratio of 1 mole to 10 moles.

The temperature of the reaction is generally within the range of 30°C to 200°C. The reaction time of the reaction is generally within the range of 0.1 hours to 24 hours.

After completion of the reaction, it is possible to isolate the compound (M33) by performing post-treatment operations, for example, subjecting the reaction mixture to extraction using an organic solvent; and drying and condensing an organic layer thereof. The isolated a compound (M33) can be further purified through chromatography, recrystallization, etc.

(Intermediate Production Method 13) (Step (E-2))

A compound (M32) can be produced by reacting a compound (M12) and a compound (M8) in the presence of a dehydration condensation agent in accordance with Step (E-2).

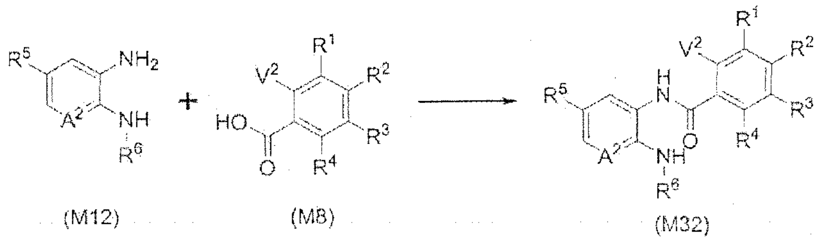

(M12) (M8) (M32)

[wherein, R1, R2, R3, R4, R5, R6, A2, and V2 each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.

Examples of the dehydration condensation used for the reaction agent include WSC, carbodiimides, such as 1,3-dicyclohexylcarbodiimide; and a BOP reagent.

In the reaction, with respect to 1 mole of the compound (M12), the compound (M8) is generally used at a ratio of 1 mole to 3 moles and the dehydration condensation agent is generally used at a ratio of 1 mole to 5 moles.

The temperature of the reaction is generally within the range of 0°C to 140°C. The reaction time of the reaction is generally within the range of 0.1 hours to 24 hours.

After completion of the reaction, it is possible to isolate the compound (M32) by performing post-treatment operations, for example, subjecting the reaction mixture to extraction using an organic solvent after adding water to the reaction mixture; and drying and condensing an organic layer thereof. The isolated a compound (M32) can be further purified through chromatography, recrystallization, etc.

(Intermediate Production Method 14)

A compound (M32) can be produced by reacting a compound (M12) and a compound (M34) in the presence of a base.

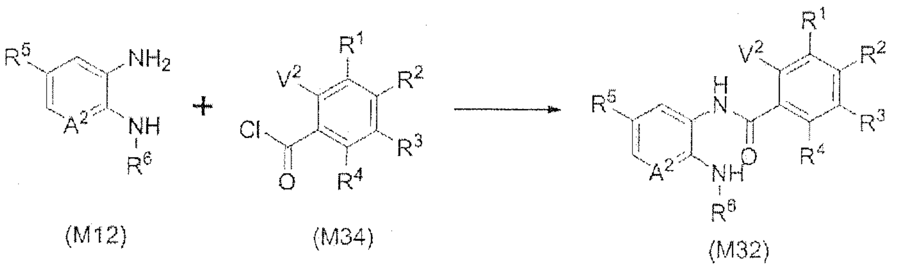

(M12) (M34) (M32)

[wherein, R1, R2, R3, R4, R5, R6, A2, and V2 each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; and a mixture thereof.

Examples of the base include alkali metal carbonates, such as sodium carbonate and potassium carbonate; tertiary amines, such as triethylamine and diisopropylethylamine; and nitrogen-containing aromatic compounds, such as pyridine and 4-dimethylaminopyridine.

In the reaction, with respect to 1 mole of the compound (M12), the compound (M34) is generally used at a ratio of 1 mole to 3 moles and the base is generally used at a ratio of 1 mole to 10 moles.

The temperature of the reaction is generally within the range of -20°C to 100°C. The reaction time of the reaction is generally within the range of 0.1 hours to 24 hours.

After completion of the reaction, it is possible to isolate the compound (M32) by performing post-treatment operations, for example, subjecting the reaction mixture to extraction using an organic solvent after adding water to the reaction mixture; and drying and condensing an organic layer thereof. The isolated a compound (M32) can be further purified through chromatography, recrystallization, etc.

(Intermediate Production Method 15)

A compound (M37) in which n is 0 in a compound (M5) can be produced through the following method.

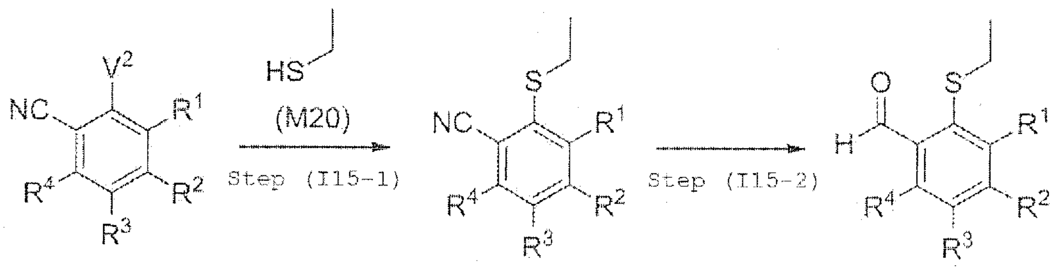


(M35) (M36) (M37)

[wherein, R1, R2, R3, R4, and V2 each represent the same meanings as those referred to above.]

(Step I15-1)

A compound (M36) can be produced by reacting a compound (M35) and a compound (M20) in the presence of a base.

In general, the reaction is performed in the presence of a solvent.

Examples of the solvent used for the reaction include ethers, such as THF, ethylene glycol dimethyl ether, tert-butyl methyl ether, and 1,4-dioxane; aromatic hydrocarbons, such as toluene and xylene; nitriles, such as acetonitrile; acid amides, such as DMF and NMP; sulfoxides, such as DMSO; and a mixture thereof.

Examples of the base used for the reaction include alkali metal hydrides, such as sodium hydride.

In the reaction, with respect to 1 mole of the compound (M35), the compound (M20) is generally used at a ratio of 1 mole to 10 moles and the base is generally used at a ratio of 1 mole to 10 moles.

The temperature of the reaction is generally within the range of 0°C to 150°C. The reaction time of the reaction is generally within the range of 0.5 hours to 24 hours.

After completion of the reaction, it is possible to isolate the compound (M36) by performing post-treatment operations, for example, subjecting the reaction mixture to extraction using an organic solvent; and drying and condensing an organic layer thereof. The isolated a compound (M36) can be further purified through chromatography, recrystallization, etc.

(Step I15-2)

The compound (M37) can be produced by subjecting the compound (M36) to a reduction reaction.

In general, the reaction is performed in the presence of a solvent.

Examples of the solvent used for the reaction include ethers, such as THF, ethylene glycol dimethyl ether, tert-butyl methyl ether, and 1,4-dioxane; aromatic hydrocarbons, such as toluene and xylene; halogenated hydrocarbons, such as dichloromethane and chloroform; and a mixture thereof.

Examples of a reducing agent used for the reaction include diisobutylaluminum hydride.

In the reaction, with respect to 1 mole of the compound (M36), the reducing agent is generally used at a ratio of 1 mole to 2 moles.

The temperature of the reaction is generally within the range of -20°C to 100°C. The reaction time of the reaction is generally within the range of 0.5 hours to 24 hours.

After completion of the reaction, it is possible to isolate the compound (M37) by performing post-treatment operations, for example, subjecting the reaction mixture to extraction using an organic solvent; and drying and condensing an organic layer thereof. The isolated a compound (M37) can be further purified through chromatography, recrystallization, etc.

(Intermediate Production Method 16)

A compound (M37) in which n is 0 in a compound (M5) can be produced by reacting a compound (M38) and a compound (M20) in the presence of a base.

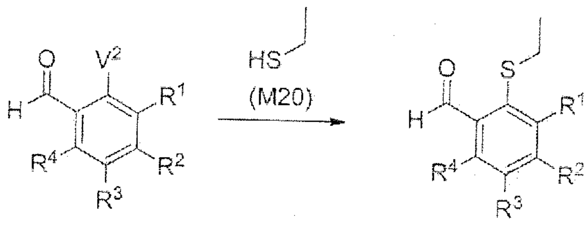


(M38) (M37)

[wherein, R1, R2, R3, R4, and V2 each represent the same meanings as those referred to above.]

In general, the reaction is performed in the presence of a solvent.

Examples of the solvent used for the reaction include, ethers, such as THF, ethylene glycol dimethyl ether, tert-butyl methyl ether, and 1,4-dioxane; aromatic hydrocarbons, such as toluene and xylene; nitriles, such as acetonitrile; acid amides, such as DMF and NMP; sulfoxides, such as DMSO; and a mixture thereof.

Examples of the base used for the reaction include alkali metal hydrides, such as sodium hydride.

In the reaction, with respect to 1 mole of the compound (M38), the compound (M20) is generally used at a ratio of 1 mole to 10 moles and the base is generally used at a ratio of 1 mole to 10 moles.

The temperature of the reaction is generally within the range of 0°C to 150°C. The reaction time of the reaction is generally within the range of 0.5 hours to 24 hours.

After completion of the reaction, it is possible to isolate the compound (M37) by performing post-treatment operations, for example, subjecting the reaction mixture to extraction using an organic solvent; and drying and condensing an organic layer thereof. The isolated a compound (M37) can be further purified through chromatography, recrystallization, etc.

(Intermediate Production Method 17)

A compound (M39) in which n is 0 in a compound (M2) can be produced by subjecting a compound (M36) to a hydrolysis reaction in the presence of a base, and a compound (M41) in which n is 1 or 2 in a compound (M2) can be

produced by the following method.

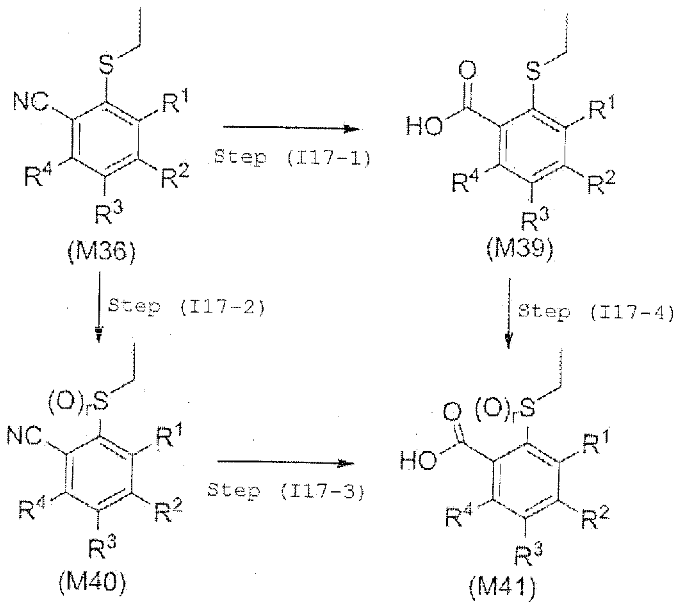


[wherein, R1, R2, R3, and R4 each represent the same meanings as those referred to above, and r represents 1 or 2.]

(Step I17-1)

In general, the reaction is performed in the presence of a solvent.

Examples of the solvent used for the reaction includes ethers, such as THF, ethylene glycol dimethyl ether, tert-butyl methyl ether, and 1,4-dioxane; alcohols, such as methanol and ethanol; water; and a mixture thereof.

Examples of the base used for the reaction include alkali metal hydroxides, such as sodium hydroxide and potassium hydroxide.

In the reaction, with respect to 1 mole of the compound (M36), the base is generally used at a ratio of 1 mole to 10 moles.

The temperature of the reaction is generally within the range of 0°C to 120°C. The reaction time of the reaction is generally within the range of 0.1 hours to 24 hours.

After completion of the reaction, it is possible to isolate the compound (M39) by performing post-treatment operations, for example, subjecting the reaction mixture to extraction using an organic solvent after making the reaction liquid acidic; and drying and condensing an organic layer thereof. The isolated a compound (M39) can be further purified through chromatography, recrystallization, etc.

(Step I17-2)

In general, the reaction is performed in the presence of a solvent.

Examples of the solvent used for the reaction include halogenated hydrocarbons, such as dichloromethane and chloroform; alcohols, such as methanol and ethanol; acetic acid; water; and a mixture thereof.

Examples of the oxidant used for the reaction include m-chloroperbenzoic acid or hydrogen peroxide solution.

The reaction can be performed in the presence of a catalyst as necessary.

Examples of the catalyst used for the reaction include sodium tungstate.

In the reaction, with respect to 1 mole of the compound (M36), the oxidant is generally used at a ratio of 1 mole to 5 moles.

The temperature of the reaction is generally within the range of -20°C to 120°C. The reaction time of the reaction is generally within the range of 0.1 hours to 12 hours.

After completion of the reaction, it is possible to isolate the compound (M40) by performing post-treatment operations, for example, subjecting the reaction mixture to extraction using an organic solvent; washing an organic layer thereof with an aqueous solution of a reducing agent (for example, sodium sulfite and sodium thiosulfate) and an aqueous solution of a base (for example, sodium hydrogen carbonate) as necessary; and drying and condensing the organic layer. The isolated a compound (M40) can be further purified through chromatography, recrystallization, etc.

(Step I17-3)

In general, the reaction is performed in the presence of a solvent.
Examples of the solvent used for the reaction include ethers, such as THF, ethylene glycol dimethyl ether, tert-butyl methyl ether, and 1,4-dioxane; alcohols, such as methanol and ethanol; water; and a mixture thereof.
Examples of the base used for the reaction include alkali metal hydroxides, such as sodium hydroxide and potassium hydroxide.
In the reaction, with respect to 1 mole of the compound (M40), the base is generally used at a ratio of 1 mole to 10 moles.
The temperature of the reaction is generally within the range of 0°C to 120°C. The reaction time of the reaction is generally within the range of 0.1 hours to 24 hours.
After completion of the reaction, it is possible to isolate the compound (M41) by performing post-treatment operations, for example, subjecting the reaction mixture to extraction using an organic solvent after making the reaction liquid acidic; and drying and condensing an organic layer thereof. The isolated a compound (M41) can be further purified through chromatography, recrystallization, etc.

(Step I17-4)

In general, the reaction is performed in the presence of a solvent.
Examples of the solvent used for the reaction include halogenated hydrocarbons, such as dichloromethane and chloroform; alcohols, such as methanol and ethanol; acetic acid; water; and a mixture thereof.
Examples of the oxidant used for the reaction include m-chloroperbenzoic acid or hydrogen peroxide solution.
The reaction can be performed in the presence of a catalyst as necessary.
Examples of the catalyst used for the reaction include sodium tungstate.
In the reaction, with respect to 1 mole of the compound (M39), the oxidant is generally used at a ratio of 1 mole to 5 moles.
The temperature of the reaction is generally within the range of -20°C to 120°C. The reaction time of the reaction is generally within the range of 0.1 hours to 12 hours.
After completion of the reaction, it is possible to isolate the compound (M41) by performing post-treatment operations, for example, subjecting the reaction mixture to extraction using an organic solvent; washing an organic layer thereof with an aqueous solution of a reducing agent (for example, sodium sulfite and sodium thiosulfate) and an aqueous solution of a base (for example, sodium hydrogen carbonate) as necessary; and drying and condensing the organic layer. The isolated a compound (M41) can be further purified through chromatography, recrystallization, etc.

(Intermediate Production Method 18)

A compound (M39) in which 1 is 0 in a compound (M2) can be produced by subjecting a compound (M36) to a hydrolysis reaction in the presence of an acid.

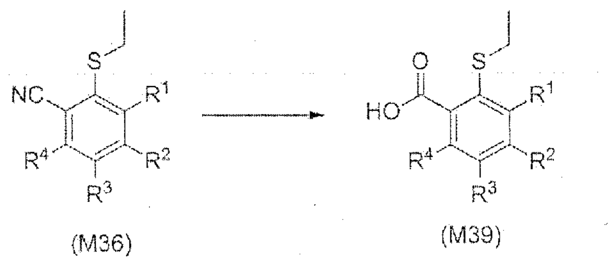

(M36) (M39)

[wherein, R1, R2, R3, and R4 each represent the same meanings as those referred to above.]
In general, the reaction is performed by having an aqueous solution of an acid as a solvent.
Examples of the acid used for the reaction include mineral acids, such as hydrochloric acid, nitric acid, phosphoric acid, and sulfuric acid; and carboxylic acids, such as acetic acid and trifluoroacetic acid.
The temperature of the reaction is generally within the range of 0°C to 100°C. The reaction time of the reaction is generally within the range of 0.1 hours to 24 hours.
After completion of the reaction, it is possible to isolate the compound (M39) by performing post-treatment operations, for example, subjecting the reaction mixture to extraction using an organic solvent; and drying and condensing an organic layer thereof. The isolated a compound (M39) can be further purified through chromatography, recrystallization,

etc.

(Intermediate Production Method 19)

A compound (M4) can be produced by chlorinating a compound (M2) in the presence of a chlorinating agent.

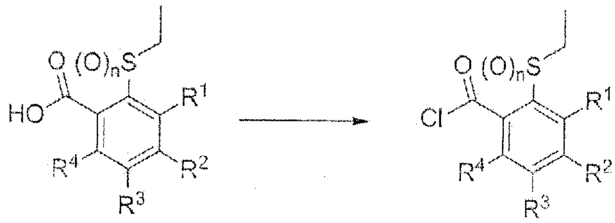

(M2) (M4)

[wherein, R1, R2, R3, R4, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aromatic hydrocarbons, such as toluene and xylene; halogenated hydrocarbons, such as dichloromethane and chloroform; and a mixture thereof.

Examples of the chlorinating agent used for the reaction include thionyl chloride and oxalyl dichloride.

In the reaction, with respect to 1 mole of the compound (M2), the chlorinating agent is generally used at a ratio of 1 mole to 5 moles.

The temperature of the reaction is generally within the range of 0°C to 100°C. The reaction time of the reaction is generally within the range of 0.1 hours to 24 hours.

After completion of the reaction, it is possible to isolate the compound (M4) by distilling the solvent.

(Intermediate Production Method 20)

A compound (M45) in which A2 is a nitrogen atom in a compound (M12) can be produced through the following method, for example.

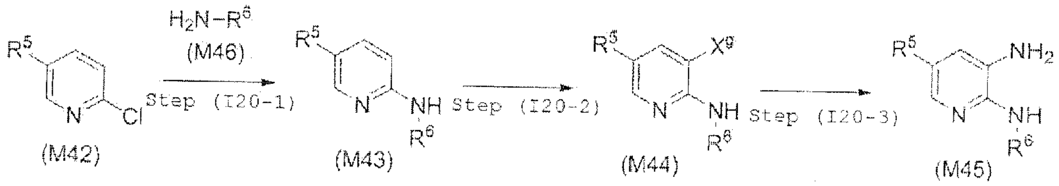


(M42) (M43) (M44) (M45)

[wherein, R5 and R6 each represent the same meanings as those referred to above, and Xg represents halogen atoms, such as a chlorine atom, a bromine atom, or an iodine atom.]

(Step I20-1)

A compound (M43) can be produced by reacting a compound (M42) and a compound (M46).

In general, the reaction is performed in the presence or absence of a solvent.

Examples of the solvent used for the reaction include water; alcohols, such as methanol and ethanol; ethers, such as 1,4-dioxane, diethyl ether, and THF; esters, such as ethyl acetate and butyl acetate; halogenated hydrocarbons, such as dichloromethane, chloroform, carbon tetrachloride, and 1,2-dichloroethane; nitriles, such as acetonitrile; aprotic polar solvents, such as DMF, NMP, and dimethyl sulfoxide; nitrogen-containing aromatic compounds, such as pyridine and quinoline; and a mixture thereof.

The reaction can be performed by adding a base as necessary.

As the base used for the reaction include nitrogen-containing heterocyclic compounds, such as pyridine, picoline, 2,6-lutidine, DBU, and 1,5-diazabicyclo [4.3.0]-5-nonene; tertiary amines, such as triethylamine and N-ethyldiisopropylamine; and inorganic bases, such as potassium carbonate, cesium carbonate, and sodium hydroxide.

With respect to 1 mole of the compound (M42), the compound (M46) is generally used at a ratio of 1 mole to 5 moles.

The temperature of the reaction is generally within the range of 0°C to 200°C. The reaction time of the reaction is generally within the range of 0.1 hours to 24 hours.

After completion of the reaction, it is possible to isolate the compound (M43) by subjecting the reaction mixture to extraction using an organic solvent after adding the reaction mixture to water and condensing an organic layer thereof; collecting the solid, which has been generated by adding the reaction mixture to water, through filtering; or collecting the solid, which has been generated in the reaction mixture, through filtering. The isolated a compound (M43) can be further purified through recrystallization, chromatography, etc.

(Step I20-2)

The compound (M4) can be produced by reacting the compound (M43) and a halogenating agent.

In general, the reaction is performed in the presence of a solvent.

Examples of the solvent used for the reaction include water; acetic acid,; ethers such as 1,4-dioxane, diethyl ether, and THF; esters, such as ethyl acetate and butyl acetate; halogenated hydrocarbons, such as dichlcromethane, chloroform, carbon tetrachloride, and 123 1,2-dichloroethane; nitriles, such as acetonitrile; aprotic polar solvents, such as DMF and NMP; and a mixture thereof.

Examples of the halogenating agent used for the reaction include chlorinating agents, such as N-chlorosuccinimide and chlorine; brominating agents, such as N-bromosuccinimide and bromine; and chlorinating agents, such as N-iodosuccinimide and iodine.

With respect to 1 mole of the compound (M43), the halogenating agent is generally used at a ratio of 1 mole to 3 moles.

The temperature of the reaction is generally within the range of -10°C to 100°C. The reaction time of the reaction is generally within the range of 0.1 hours to 24 hours.

After completion of the reaction, it is possible to isolate the compound (M44) by subjecting the reaction mixture to extraction using an organic solvent after adding the reaction mixture to water and condensing an organic layer thereof; collecting the solid, which has been generated by adding the reaction mixture to water, through filtering; or collecting the solid, which has been generated in the reaction mixture, through filtering. The isolated a compound (M44) can be further purified through recrystallization, chromatography, etc.

(Step I20-3)

The compound (M45) can be produced by reacting the compound (M44) and an aminating agent in the presence of a copper compound.

In general, the reaction is performed in the presence of a solvent.

Examples of the solvent used for the reaction include water; alcohols, such as methanol and ethanol; ethers, such as 1,4-dioxane, diethyl ether, and THF; esters, such as ethyl acetate and butyl acetate; halogenated hydrocarbons, such as dichloromethane, chloroform, carbon tetrachloride, and 1,2-dichloroethane; nitriles, such as acetonitrile; aprotic polar solvents, such as DMF, NMP, and dimethyl sulfoxide; nitrogen-containing aromatic compounds, such as pyridine and quinoline; and a mixture thereof.

Examples of the aminating agent used for the reaction include ammonia, ammonia water, and lithium amide.

Examples of the copper compound used for the reaction include copper, copper (I) iodide, copper (I) oxide, copper (II) oxide, copper (II) acetylacetone, copper (II) acetate, and copper (II) sulfate.

The reaction can be performed by adding a ligand as necessary.

Examples of the ligand used for the reaction include acetylacetone, salen, and phenanthroline.

The reaction can be performed by adding a base as necessary.

As the base used for the reaction include nitrogen-containing heterocyclic compounds, such as pyridine, picoline, 2,6-lutidine, DBU, and 1,5-diazabicyclo [4.3.0]-5-nonene; and inorganic bases, such as tripotassium phosphate, potassium carbonate, cesium carbonate, and sodium hydroxide.

With respect to 1 mole of the compound (M44), the aminating agent is generally used at a ratio of 1 mole to 5 moles, the copper compound is generally used at a ratio of 0.02 moles to 0.5 moles, and the ligand is used at a ratio of 0.02 moles to 2 moles as necessary, and the base is generally used at a ratio of 1 mole to 5 moles as necessary.

The temperature of the reaction is generally within the range of 30°C to 200°C. The reaction time of the reaction is generally within the range of 0.1 hours to 48 hours.

After completion of the reaction, it is possible to isolate the compound (M45) by subjecting the reaction mixture to extraction using an organic solvent after adding the reaction mixture to water and condensing an organic layer thereof; collecting the solid, which has been generated by adding the reaction mixture to water, through filtering; or collecting

the solid, which has been generated in .the reaction mixture, through filtering. The isolated a compound (M45) can be further purified through recrystallization, chromatography, etc.

(Intermediate Production Method 21) (Step (A-2))

A compound (M22) can be produced by reacting a compound (M18) and a compound (M2) in the presence of a dehydration condensation agent in accordance with Step (A-2).

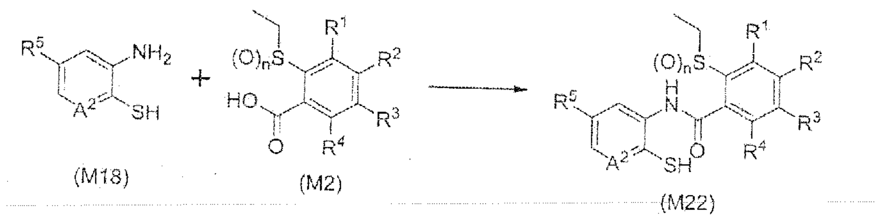

[wherein, R1, R2, R3, R4, R5, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.

Examples of dehydration condensation agents used for the reaction include carbodiimides, such as WSC and 1,3-dicyclohexylcarbodiimide; and a BOP reagent.

In the reaction, with respect to 1 mole of the compound (M18), the compound (M2) is generally used at a ratio of 1 mole to 3 moles and the dehydration condensation agent is generally used at a ratio of 1 mole to 5 moles.

The temperature of the reaction is generally within the range of 0°C to 140°C. The reaction time of the reaction is generally within the range of 0.1 hours to 24 hours.

After completion of the reaction, it is possible to isolate the compound (M22) by performing post-treatment operations, for example, subjecting the reaction mixture to extraction using an organic solvent after adding water to the reaction mixture; and drying and condensing an organic layer thereof. The isolated a compound (M22) can be further purified through chromatography, recrystallization, etc.

(Intermediate Production Method 22) (Step (3-2))

A compound (M22) can be produced by reacting a compound (M18) and a compound (M4) in the presence of a base in accordance with Step (B-2).

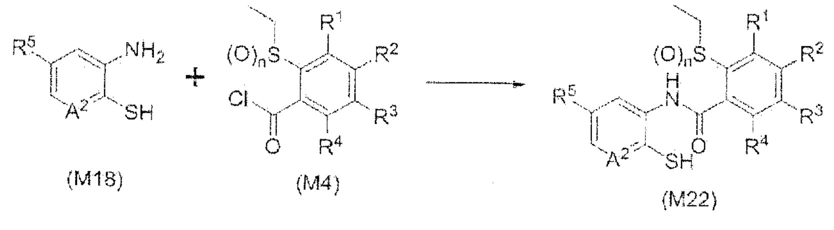

[wherein, R1, R2, R3, R4, R5, A2, and n each represent the same meanings as those referred to above.]

In general, the reaction is performed in the presence of a solvent.

Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; and a mixture thereof.

Examples of the base used for the reaction include alkali metal carbonates, such as sodium carbonate and potassium carbonate; tertiary amines, such as triethylamine and diisopropylethylamine; and nitrogen-containing aromatic compounds, such as pyridine and 4-dimethylaminopyridine.

In the reaction, with respect to 1 mole of the compound (M18), the compound (M4) is generally used at a ratio

of 1 mole to 3 moles and the base is generally used at a ratio of 1 mole to 10 moles.

The temperature of the reaction is generally within the range of -20°C to 100°C. The reaction time of the reaction is generally within the range of 0.1 hours to 24 hours.

After completion of the reaction, it is possible to isolate the compound (M22) by performing post-treatment operations, for example, subjecting the reaction mixture to extraction using an organic solvent after adding water to the reaction mixture; and drying and condensing an organic layer thereof. The isolated a compound (M22) can be further purified through chromatography, recrystallization, etc.

(Intermediate Production Example 23) (Step (E-1))

A compound (M47) in which A1 is an oxygen atom in a compound (M10) can be produced by reacting a compound (M15) and a a compound (M8) in accordance with Step (E-1).

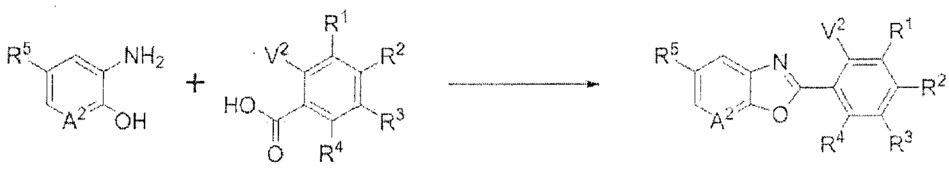

(M15) (M8) (M47)

[wherein, R1, R2, R3, R4, R5, A2, n, and V2 each represent the same meanings as those referred to above.]

It is possible to produce the compound (M47) based on the above-described method of (Production Method 6) using the compound (M8) instead of the compound (M2).

(Intermediate Production Example 24) (Step (E-2))

A compound (M48) in which A1 is an oxygen atom in a compound (M9) can be produced by reacting a compound (M15) and a compound (M8) in accordance with Step (E-2). In addition, a compound (M49) in which A1 is a sulfur atom in a compound (M9) can be produced by reacting a compound (M18) and a compound (M8).

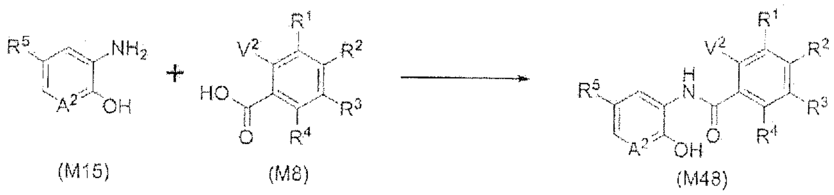

(M15) (M8) (M48)

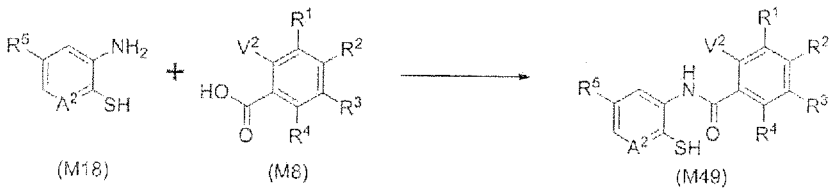

(M18) (M8) (M49)

[wherein, R1, R2, R3, R4, R5, A2, and V2 each represent the same meanings as those referred to above.]

It is possible to produce the compound (48) based on the above-described method of (Intermediate Production Method 6) using the compound (M8) instead of the compound (M2).

It is possible to produce the compound (49) based on the above-described method of (Intermediate Production Method 21) using the compound (M8) instead of the compound (M2).

(Intermediate Production Example 25) (Step (E-3))

A compound (M47) in which A1 is an oxygen atom in a compound (M10) can be produced by cyclizing a

compound (M48) in accordance with Step (E-3). In addition, a compound (M50) in which A1 is a sulfur atom in a compound (M10) can be produced by cyclizing a compound (M49) in accordance with Step (E-3).

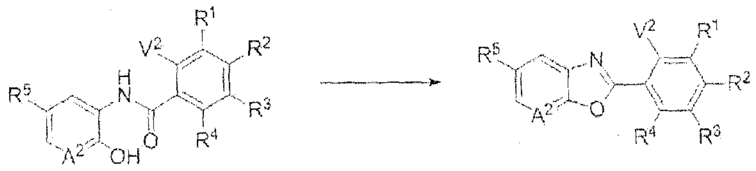

(M48) (M47)

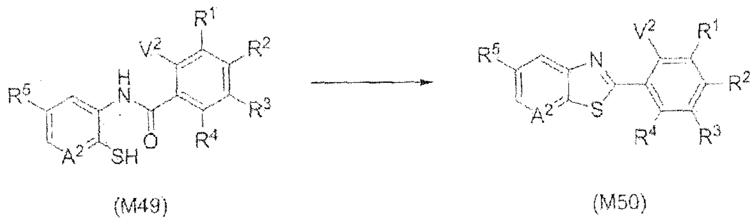

(M49) (M50)

[wherein, R1, R2, R3, R4, R5, A2, and V2 each represent the same meanings as those referred to above.]

It is possible to produce the compound (M47) based on the above-described method of (Production Method 8) or (Production Method 9) using the compound (M48) instead of the compound (M17).

It is possible to produce the compound (M50) based on the above-described method of (Production Method 21) using the compound (M49) instead of the compound (M22).

(Intermediate Production Method 26) (Step (F-1))

A compound (M11) can be produced by reacting a compound (M10) and sodium sulfide, sodium hydrogen sulfide, or hydrogen sulfide in accordance with Step (F-1).

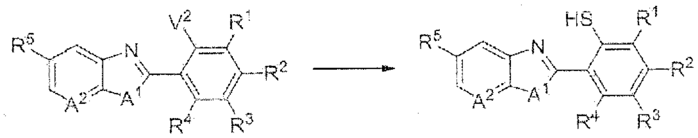

(M10) (M11)

[wherein, R1, R2, R3, R4, R5, A1, A2, and V2 each represent the same meanings as those referred to above.]

It is possible to produce the compound (M11) based on the above-described method of (Production Method 16) using sodium sulfide, sodium hydrogen sulfide, or hydrogen sulfide instead of the compound (M20).

When using sodium sulfide or sodium hydrogen sulfide, in general, the reaction is performed without adding a base.

(Intermediate Production Method 27) (Production Method D)

A compound (M7) can be produced by reacting a compound (M51) and a compound (M2).

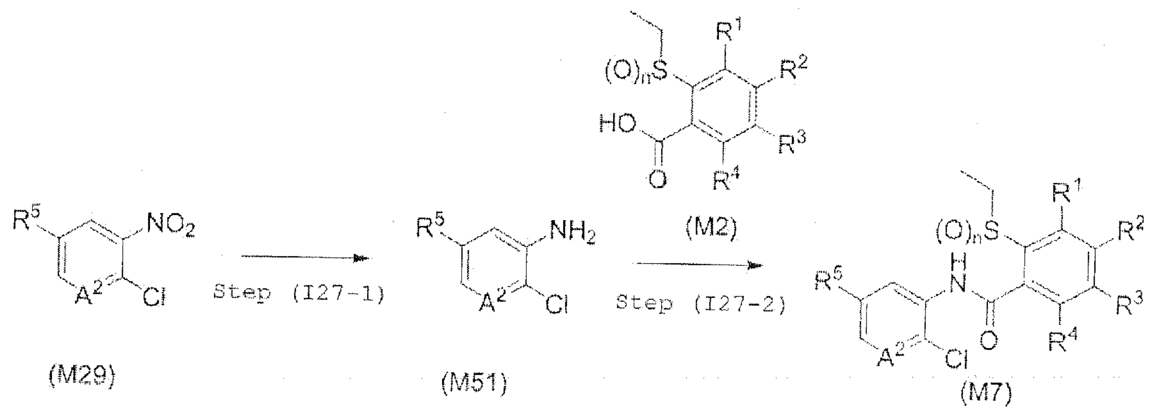


[wherein, R1, R2, R3, R4, R5, A2, and n each represent the same meanings as those referred to above.]

(Step (I27-1)

The compound (M51) can be produced by subjecting the compound (M29) to a reduction reaction.
The reduction reaction can be performed in the presence of reducing agents, such as iron powder and zinc powder; acids such as hydrochloric acid and acetic acid; and water.
In general, the reaction is performed in the presence of a solvent.
Examples of the solvent used for the reaction include ethers, such as THF, ethylene glycol dimethyl ether, tert-butyl methyl ether, and 1,4-dioxane; esters, such as ethyl acetate and butyl acetate; alcohols, such as methanol and ethanol; acid amides, such as DMF and NMP; and a mixture thereof.
In the reaction, with respect to 1 mole of the compound (M29), the reducing agent is generally used at a ratio of 3 moles to 10 moles.
The temperature of the reaction is generally within the range of 0°C to 100°C. The reaction time of the reaction is generally within the range of 0.1 hours to 24 hours.
After completion of the reaction, it is possible to isolate the compound (M51) by performing post-treatment operations, for example, subjecting the reaction mixture to extraction using an organic solvent after adding water to the reaction mixture; and drying and condensing an organic layer thereof. The isolated a compound (M51) can be purified through chromatography, recrystallization, etc.

(Step I27-2)

In general, the reaction is performed in the presence or absence of a solvent.
Examples of the solvent used for the reaction include ethers, such as THF, ethylene glycol dimethyl ether, tert-butyl methyl ether, and 1,4-dioxane; aliphatic hydrocarbons, such as hexane, heptane, and octane; aromatic hydrocarbons, such as toluene and xylene; halogenated hydrocarbons, such as chlorobenzene; esters, such as ethyl acetate and butyl acetate; nitriles, such as acetonitrile; acid amides, such as DMF and NMP; sulfoxides, such as DMSO; nitrogen-containing aromatic compounds, such as pyridine and quinoline; and a mixture thereof.
Examples of the dehydration condensation agent used for the reaction include carbodiimides, such as WSC and 1,3-dicyclohexylcarbodiimide; and a BOP reagent.
In the reaction, with respect to 1 mole of the compound (M51), the compound (M2) is generally used at a ratio of 1 mole to 3 moles and the dehydration condensation agent is generally used at a ratio of 1 mole to 5 moles.
The temperature of the reaction is generally within the range of 0°C to 140°C. The reaction time of the reaction is generally within the range of 0.1 hours to 24 hours.
After completion of the reaction, it is possible to isolate the compound (M7) by performing poet-treatment operations, for example, subjecting the reaction mixture to extraction using an organic solvent after adding water to the reaction mixture; and drying and condensing an organic layer thereof. The isolated a compound (M7) can be further purified through chromatography, recrystallization, etc.
In addition, it is possible to produce the compound (M7) based on the above-described method using the compound (M4) instead of the compound (M2).
When using the compound (M4), in general, the reaction is performed without adding the dehydration condensation agent. The reaction can be performed by adding a base as necessary.
Examples of the base used for the reaction include alkali metal carbonates, such as sodium carbonate and potassium carbonate; tertiary amines, such as triethylamine and diisopropylethylamine; and nitrogen-containing aromatic

compounds, such as pyridine and 4-dimethylaminopyridine.

In the reaction, with respect to 1 mole of the compound (M51), the compound (M4) is generally used at a ratio of 1 mole to 3 moles and the base is generally used at a ratio of 1 mole to 10 moles.

(Intermediate Production Method 28)

A compound (M1) can be produced by reacting a compound (M52) and an aminating agent.

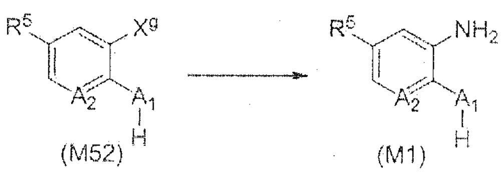

[wherein Xg, R5, A1, and A2 each represent the same meanings as those referred to above.]

It is possible to produce the compound (M1) based on the above-described method of Step (I20-3) of (Intermediate Production Method 20) using the compound (M52) instead of the compound (M44).

Next, specific examples of the present condensed heterocyclic compound are as follows. In Tables, R1 to R5, A1, A2, and n represent the symbols in the compound represented by Formula (1).

Table 1

| No. | $R^1$ | $R^2$ | $R^3$ | $R^4$ | $R^5$ | $A^1$ | $A^2$ | n |
|---|---|---|---|---|---|---|---|---|
| 1 | H | H | H | H | $CF_3$ | -NMe- | N | 0 |
| 2 | H | H | H | H | $CF_3$ | -NMe- | N | 1 |
| 3 | H | H | H | H | $CF_3$ | -NMe- | N | 2 |
| 4 | H | H | H | H | $CF_3$ | -NEt- | N | 0 |
| 5 | H | H | H | H | $CF_3$ | -NEt- | N | 1 |
| 6 | H | H | H | H | $CF_3$ | -NEt- | N | 2 |
| 7 | H | H | H | H | $CF_3$ | -NiPr- | N | 0 |
| 8 | H | H | H | H | $CF_3$ | -NiPr- | N | 1 |
| 9 | H | H | H | H | $CF_3$ | -NiPr- | N | 2 |
| 10 | H | H | H | H | $CF_3$ | -NCycPr- | N | 0 |
| 11 | H | H | H | H | $CF_3$ | -NCycPr- | N | 1 |
| 12 | H | H | H | H | $CF_3$ | -NCycPr- | N | 2 |
| 13 | H | H | H | H | $CF_2H$ | -NMe- | N | 0 |
| 14 | H | H | H | H | $CF_2H$ | -NMe- | N | 1 |
| 15 | H | H | H | H | $CF_2H$ | -NMe- | N | 2 |
| 16 | H | H | H | H | $CF_2CF_3$ | -NMe- | N | 0 |
| 17 | H | H | H | H | $CF_3$ | -NMe- | =CH- | 0 |
| 18 | H | H | H | H | $CF_3$ | -NMe- | =CH- | 1 |
| 19 | H | H | H | H | $CF_3$ | -NMe- | =CH- | 2 |
| 20 | H | H | H | H | $CF_3$ | O | =CH- | 0 |
| 21 | H | H | H | H | $CF_3$ | O | =CH- | 1 |
| 22 | H | H | H | H | $CF_3$ | O | =CH- | 2 |
| 23 | H | H | H | H | $CF_3$ | O | N | 0 |

(continued)

| No. | $R^1$ | $R^2$ | $R^3$ | $R^4$ | $R^5$ | $A^1$ | $A^2$ | n |
|---|---|---|---|---|---|---|---|---|
| 24 | H | H | H | H | $CF_3$ | O | N | 1 |
| 25 | H | H | H | H | $CF_3$ | O | N | 2 |
| 26 | H | H | H | H | $CF_3$ | S | =CH- | 0 |
| 27 | H | H | H | H | $CF_3$ | S | =CH- | 1 |
| 28 | H | H | H | H | $CF_3$ | S | =CH- | 2 |
| 29 | H | H | H | H | $CF_2CF_3$ | -NMe- | N | 1 |
| 30 | H | H | H | H | $CF_2CF_3$ | -NMe- | N | 2 |
| 31 | H | F | H | H | $CF_3$ | -NMe- | N | 0 |
| 32 | H | F | H | H | $CF_3$ | -NMe- | N | 1 |
| 33 | H | F | H | H | $CF_3$ | -NMe- | N | 2 |
| 34 | H | $CF_3$ | H | H | $CF_3$ | -NMe- | N | 0 |

Table 2

| No. | $R^1$ | $R^2$ | $R^3$ | $R^4$ | $R^5$ | $A^1$ | $A^2$ | n |
|---|---|---|---|---|---|---|---|---|
| 35 | H | H | H | H | $CF_2CF_2CF_3$ | -NMe- | N | 0 |
| 36 | H | H | H | H | $CF_2CF_2CF_3$ | -NMe- | N | 1 |
| 37 | H | H | H | H | $CF_2CF_2CF_3$ | -NMe- | N | 2 |
| 38 | H | $CF_3$ | H | H | $CF_3$ | -NMe- | N | 1 |
| 39 | H | $CF_3$ | H | H | $CF_3$ | -NMe- | N | 2 |
| 40 | H | H | H | H | $CF_3$ | S | N | 0 |
| 41 | H | H | H | H | $CF_3$ | S | N | 1 |
| 42 | H | H | H | H | $CF_3$ | S | N | 2 |
| 43 | H | H | H | H | $SCF_3$ | -NMe- | N | 2 |
| 44 | H | H | H | H | $SCF_3$ | -NMe- | N | 1 |
| 45 | H | H | H | H | $SOCF_3$ | -NMe- | N | 2 |
| 46 | H | H | H | H | $SO_2CF_3$ | -NMe- | N | 2 |
| 47 | H | $CF_3$ | H | H | $CF_3$ | S | N | 0 |
| 48 | H | $CF_3$ | H | H | $CF_3$ | S | N | 2 |
| 49 | H | H | H | H | SMe | -NMe- | N | 0 |
| 50 | H | H | H | H | $SO_2Me$ | -NMe- | N | 2 |
| 51 | H | $CF_3$ | H | H | $SCF_3$ | -NMe- | N | 0 |
| 52 | H | $CF_3$ | H | H | $SCF_3$ | -NMe- | N | 2 |
| 53 | H | $CF_3$ | H | H | $SO_2CF_3$ | -NMe- | N | 2 |
| 54 | H | $CF_3$ | H | H | $CF(CF_3)_2$ | -NH- | N | 0 |
| 55 | H | Br | H | H | $CF_3$ | -NMe- | N | 0 |
| 56 | H | Br | H | H | $CF_3$ | -NMe- | N | 1 |
| 57 | H | Br | H | H | $CF_3$ | -NMe- | N | 2 |

(continued)

| No. | $R^1$ | $R^2$ | $R^3$ | $R^4$ | $R^5$ | $A^1$ | $A^2$ | n |
|---|---|---|---|---|---|---|---|---|
| 58 | H | 2-pyrimidyl | H | H | $CF_3$ | -NMe- | N | 2 |
| 59 | H | $CF_3$ | H | H | $CF_3$ | -NH- | N | 0 |
| 60 | H | $CF_3$ | H | H | $CF_3$ | -NH- | N | 2 |
| 61 | H | H | H | H | $CF_3$ | -NH- | N | 0 |
| 62 | H | H | H | H | $CF_3$ | -NH- | N | 2 |
| 63 | H | $OCF_3$ | H | H | $CF_3$ | -NMe- | N | 1 |
| 64 | H | $OCF_3$ | H | H | $CF_3$ | -NMe- | N | 2 |
| 65 | H | $OCF_3$ | H | H | $CF_3$ | -NMe- | N | 0 |

Table 3

| No. | $R^1$ | $R^2$ | $R^3$ | $R^4$ | $R^5$ | $A^1$ | $A^2$ | n |
|---|---|---|---|---|---|---|---|---|
| 66 | H | H | H | H | $CHFCF_3$ | -NMe- | N | 0 |
| 67 | H | H | H | H | $CHFCF_3$ | -NMe- | N | 1 |
| 68 | H | H | H | H | $CHFCF_3$ | -NMe- | N | 2 |
| 69 | Cl | H | H | H | $CF_2CF_3$ | -NMe- | N | 0 |
| 70 | Cl | H | H | H | $CF_2CF_3$ | -NMe- | N | 1 |
| 71 | H | H | Cl | H | $CF_3$ | -NMe- | N | 0 |
| 72 | H | H | Cl | H | $CF_3$ | -NMe- | N | 1 |
| 73 | H | H | Cl | H | $CF_3$ | -NMe- | N | 2 |
| 74 | F | H | H | H | $CF_3$ | -NMe- | N | 0 |
| 75 | F | H | H | H | $CF_3$ | -NMe- | N | 1 |
| 76 | F | H | H | H | $CF_3$ | -NMe- | N | 2 |
| 77 | H | H | H | F | $CF_3$ | -NMe- | N | 0 |
| 78 | H | H | H | F | $CF_3$ | -NMe- | N | 1 |
| 79 | H | H | H | F | $CF_3$ | -NMe- | N | 2 |
| 80 | H | $CH_3$ | H | H | $CF_3$ | -NMe- | N | 0 |
| 81 | H | $CH_3$ | H | H | $CF_3$ | -NMe- | N | 1 |
| 82 | H | $CH_3$ | H | H | $CF_3$ | -NMe- | N | 2 |
| 83 | H | $CF_2CF_3$ | H | H | CF3 | -NMe- | N | 0 |
| 84 | H | $CF_2CF_3$ | H | H | $CF_3$ | -NMe- | N | 1 |
| 85 | H | $CF_2CF_3$ | H | H | $CF_3$ | -NMe- | N | 2 |
| 86 | H | H | H | H | SEt | -NMe- | N | 0 |
| 87 | H | H | H | H | $SO_2Et$ | -NMe- | N | 2 |
| 88 | H | H | H | H | SiPr | -NMe- | N | 0 |
| 89 | H | H | H | H | $SO_2iPr$ | -NMe- | N | 2 |
| 90 | H | $CF_3$ | H | H | $CF_2CF_3$ | -NMe- | N | 0 |

Table 4

| No. | $R^1$ | $R^2$ | $R^3$ | $R^4$ | $R^5$ | $A^1$ | $A^2$ | n |
|---|---|---|---|---|---|---|---|---|
| 91 | H | Cl | H | H | $CF_3$ | S | N | 2 |
| 92 | H | H | H | H | $C(Cl)(CF_3)_2$ | -NMe- | =CH- | 0 |
| 93 | H | H | H | H | $C(Cl)(CF_3)_2$ | -NMe- | =CH- | 1 |
| 94 | H | H | H | H | $C(Cl)(CF_3)_2$ | -NMe- | -CH- | 2 |
| 95 | H | $CF_3$ | H | H | $CF_2CF_3$ | -NMe- | N | 2 |
| 96 | H | H | H | H | $CF_2CF_3$ | -NMe- | =CH- | 0 |
| 97 | H | H | H | H | $CF_2CF_3$ | -NMe- | =CH- | 1 |
| 98 | H | H | H | H | $CF_2CF_3$ | -NMe- | =CH- | 2 |
| 99 | H | $CH_3$ | H | H | $CF_2CF_3$ | -NMe- | N | 0 |
| 100 | H | $CH_3$ | H | H | $CF_2CF_3$ | -NMe- | N | 1 |
| 101 | H | $CH_3$ | H | H | $CF_2CF_3$ | -NMe- | N | 2 |
| 102 | H | H | H | H | $CF_2CF_3$ | S | N | 0 |
| 103 | H | $CF_3$ | H | H | $CF_2CF_3$ | S | N | 0 |
| 104 | H | H | H | H | $CF_2CF_3$ | S | N | 2 |
| 105 | H | $CF_3$ | H | H | $CF_2CF_3$ | S | N | 2 |
| 106 | H | H | H | Cl | $CF_3$ | S | N | 0 |
| 107 | H | H | H | Cl | $CF_3$ | S | N | 2 |
| 108 | H | $C_2H_5$ | H | H | $CF_3$ | -NMe- | N | 2 |
| 109 | H | $CF_2CF_2CF_3$ | H | H | $CF_3$ | -NMe- | N | 2 |
| 110 | H | H | H | H | $CF_3$ | -NH- | N | 1 |
| 111 | H | $CF_3$ | H | H | $CF_3$ | -NEt- | N | 2 |
| 112 | H | $CF_3$ | H | H | $CF_3$ | -NMe- | -CH- | 0 |
| 113 | H | $CF_3$ | H | H | $CF_3$ | -NMe- | -CH- | 1 |
| 114 | H | $CF_3$ | H | H | $CF_3$ | -NMe- | =CH- | 2 |
| 115 | H | 2-pyridyl | H | H | $CF_3$ | -NMe- | N | 2 |
| 116 | H | iPr | H | H | $CF_3$ | -NMe- | N | 2 |
| 117 | H | $CF_2H$ | H | H | $CF_3$ | -NMe- | N | 2 |
| 118 | H | $OCF_3$ | H | H | $CF_2CF_3$ | -NMe- | N | 0 |
| 119 | H | $OCF_3$ | H | H | $CF_2CF_3$ | -NMe- | N | 1 |
| 120 | H | $OCF_3$ | H | H | $CF_2CF_3$ | -NMe- | N | 2 |
| 121 | H | $CF_3$ | H | H | $CF_2CF_3$ | -NMe- | =CH- | 0 |
| 122 | H | $CF_3$ | H | H | $CF_2CF_3$ | -NMe- | =CH- | 1 |
| 123 | H | $CF_3$ | H | H | $CF_2CF_3$ | -NMe- | =CH- | 2 |
| 124 | H | 4-trifluoromethyl-2-pyridyl | H | H | $CF_3$ | -NMe- | N | 2 |
| 125 | H | $OCF_3$ | H | H | $SCF_3$ | -NMe- | N | 0 |
| 126 | H | $OCF_3$ | H | H | $SCF_3$ | -NMe- | N | 1 |
| 127 | H | $OCF_3$ | H | H | $SCF_3$ | -NMe- | N | 2 |

(continued)

| No. | $R^1$ | $R^2$ | $R^3$ | $R^4$ | $R^5$ | $A^1$ | $A^2$ | n |
|---|---|---|---|---|---|---|---|---|
| 128 | H | $CF_3$ | H | H | $SCF_3$ | O | =CH- | 2 |
| 129 | H | $CF_3$ | H | H | $SOCF_3$ | O | =CH- | 2 |
| 130 | H | $CF_3$ | H | H | $CF_3$ | O | =CH- | 2 |
| 131 | H | H | H | H | $SOCF_3$ | O | =H- | 2 |

(In the above-described [Table 1] to [Table 4], Me represents a methyl group, Et represents an ethyl group, iPr represents an isopropyl group, and CycPr represents cyclopropyl group.)

Azoles, such as Tebuconazole, metconazole, difenoconazole, triticonazole, imazalil, triadimenol, fluquinconazole, prochloraz, prothioconazole, diniconazole, diniconazole M, cyproconazole, tetraconazole, ipconazole, triforine, pyrifenox, fenarimol, nuarimol, oxpoconazole fumarate, pefurazoate, triflumizole, azaconazole, bitertanol, bromuconazole, epoxiconazole, fenbuconazole, flusilazole, flutriafol, hexaconazole, imibenconazole, myclobutanil, penconazole, propiconazole, simeconazole, and triadimefon, which are used in the present invention, are well-known compounds. For example, the compounds are disclosed in pages 1072, 749, 354, 1182, 629, 1147, 543, 928, 965, 384, 384, 287, 1096, 663, 1177, 1255, 465, 1250, 854, 868, 1171, 52, 116, 134, 429, 468, 554, 560, 611, 643, 801, 869, 952, 1033, and 1145 of "The Pesticide Manual-15th edition (published by BCPC), ISBN 978-1-901396-18-8". These compounds can be obtained from commercially available formulations or can be obtained by being produced through well-known methods.

Strobilurins, such as kresoxim-methyl, azoxystrobin, pyraclostrobin, picoxystrobin, enestrobin, trifloxystrobin, dimoxystrobin, fluoxastrobin, orysastrobin, famoxadone, fenamidone, and metominostrobin, which are used in the present invention, are well-known compounds. For example, the compounds are disclosed in pages 688, 62, 971, 910, 1068, 1167, 383, 538, 840, 458, 462, and 783 of "The Pesticide Manual-15th edition (published by BCPC), ISBN 978-1-901396-18-8". These compounds can be obtained from commercially available formulations or can be produced through well-known methods.

The compound represented by the Formula (2) used in the present invention is a well-known strobilurin compound and is, for example, a compound disclosed in Pamphlet of International Publication No. 95/27693 and can be produced through the method disclosed in the publication.
Formula (2)

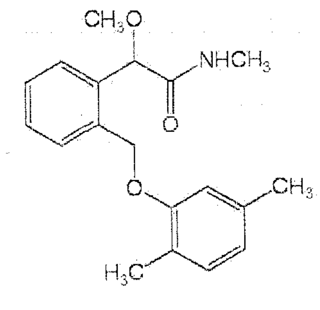

(2)

Phenylamides, such as metalaxyl, metalaxyl-M, furalaxyl-M, benalaxyl, benalaxyl-M, ofurace, and oxadixyl, which are used in the present invention, are well-known compounds. For example, the compounds are disclosed in pages 737, 739, 579, 74, 76, 834, and 847 of "The Pesticide Manual-15th edition' (published by BCPC), ISBN 978-1-901396-18-8". These compounds can be obtained from commercially available formulations or can be obtained by being produced throgh well-known methods.

All rice blast controlling compounds, such as probenazole, tiadinil, tricyclazole, pyroquilon, kasugamycin hydrochloride, and ferimzone, which are used in the present invention, are well-known compounds. For example, the compounds are disclosed in pages 927, 1134, 1163, 999, 685, and 497 of "The Pesticide Manual-15th edition (published by BCPC), ISBN 978-1-901396-18-8". These compounds can be obtained from commercially available formulations or can be obtained by being produced throgh well-known methods.

The isotianil used in the present invention is a well-known compound represented by the following Formula (3) and can be produced through the method disclosed in Pamphlet of International Publication No. 95/024413, for example.
Formula (3)

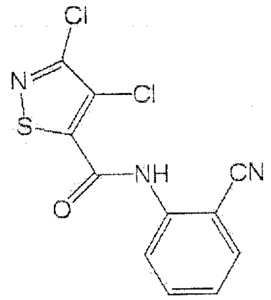

(3)

The fthalide used in the present invention is a well-known compound and is disclosed in page 147 of "SHIBUYA INDEX (Index of Pesticides), 13th edition, 2008 (published by SHIBUYA INDEX RESEARCH GROUP), ISBN 9784881371435", for example.

The tebufloquin used in the present invention is a well-known compound represented by the following Formula (4) and can be produced through the method disclosed in Pamphlet of International Publication No. 2001/092231, for example.

Formula (4)

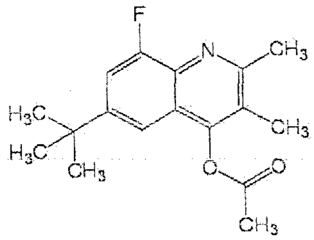

(4)

All rice sheath blight disease controlling compounds, such as pencycuron, furametpyr, and validamycin, which are used in the present invention, are well-known compounds. For example, the compounds are disclosed in pages 871, 580, and 1187 of "The Pesticide Manual-15th edition (published by BCPC), ISBN 978-1-901396-18-8". These compounds can be obtained from commercially available formulations or can be obtained by being produced throgh well-known methods.

All of carboxamides, such as carboxin, flutolanil, penthiopyrad, and fluopyram, which are used in the present invention, are well-known compounds. For example, the compounds are disclosed in pages 164, 559, 877, and 535 of "The Pesticide Manual-15th edition (published by BCPC), ISBN 978-1-901396-18-8". These compounds can be obtained from commercially available formulations or can be obtained by being produced through well-known methods.

The sedaxane used in the present invention is a well-known compound represented by the following Formula (5) and is disclosed in Pamphlet of International Publication No. 03/74491, for example. The compound can be obtained by being produced through the method disclosed in the publication.

Formula (5)

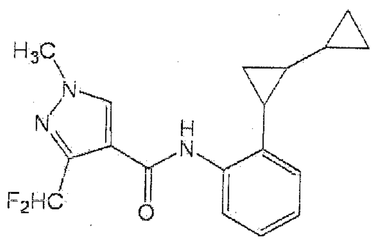

(5)

The penflufen used in the present invention is a well-known compound represented by the following Formula (6) and is disclosed in Pamphlet of International Publication No. 03/10149, for example. The compound can be obtained by being produced through the method disclosed in the publication.

Formula (6)

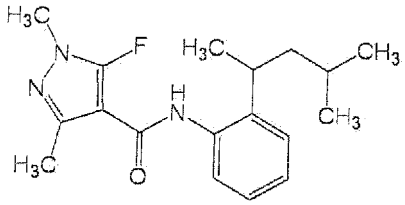

(6)

The fluxapyroxad used in the present invention is a well-known compound represented by the following Formula (7) and is disclosed in Pamphlet of International Publication No. 06/087343, for example. The compound can be obtained by being produced through the method disclosed in the publication.
Formula (7)

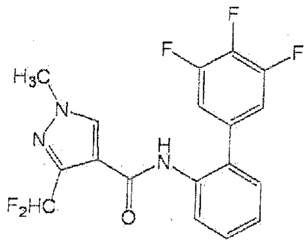

(7)

Fludioxonil, ethaboxam, tolclofos-methyl, and captan, which are used in the present invention, are well-known compounds. For example, the compounds are disclosed in pages 520, 435, 1135, and 154 of "The Pesticide Manual-15th edition (published by BCPC), ISBN 978-1-901396-18-8". These compounds can be obtained from commercially available formulations or can be obtained by being produced throghwell-known methods.

The harmful arthropod control composition of the present invention may be a compound in which the present condensed heterocyclic compound is simply mixied with the present fungicidal compound. However, in general, a compound which is formulated into an oil solution, an emulsion, a flowable agent, a water dispersible powder, a granular water dispersible powder, powder, granules, etc. by mixing the present condensed heterocyclic compound and the present fungicidal compound with an inert carrier, and as necessary, adding a surfactant or other auxiliary agents for a formulation is used.

In addition, the above-described formulated harmful arthropod control composition can be used as a harmful arthropod controlling agent as it is or by adding other inert components.

In the harmful arthropod control composition of the present invention, the total amount of the present condensed heterocyclic compound and the present fungicidal compound is generally within the range of 0.1 wt% to 100 wt%, preferably within the range of 0.2 wt% to 90 wt%, and more preferably within the range of 1 wt% to 80 wt%.

Examples of the inert carrier used during the formulation include a solid carrier and a liquid carrier. Examples of the solid carrier include fine powder and particulates, such as clays (kaolin clay, diatomaceous earth, bentonite, Fubasami clay, acid clay, and the like), synthetic hydrated silicon oxide, talc, ceramic, other inorganic minerals (sericite, quartz, sulfur, activated carbon, calcium carbonate, hydrated silica, and the like), and chemical fertilizers (ammonium sulfate, ammonium phosphate, ammonium nitrate, urea, ammonium chloride, and the like); and synthetic resins (polyester resins, such as polypropylene, polyacrylonitrile, polymethyl methacrylate, and polyethylene terephthalate, nylon resins, such as nylon-6, nylon-11, and nylon-66, a polyamide resin, polyvinyl chloride, polyvinylidene chloride, vinyl chloride-propylene copolymer, and the like).

Examples of the liquid carrier include water, alcohols (methanol, ethanol, isopropyl alcohol, butanol, hexanol, benzyl alcohol, ethylene glycol, propylene glycol, phenoxyethanol, and the like), ketones (acetone, methyl ethyl ketone, cyclohexanone, and the like), aromatic hydrocarbons (toluene, xylene, ethylbenzene, dodecylbenzene, phenylxylylethane, methylnaphthalene, and the like), aliphatic hydrocarbons (hexane, cyclohexane, kerosine, gas oil, and the like), esters (ethyl acetate, butyl acetate, isopropyl myristate, ethyl oleate, diisopropyl adipate, diisobutyl adipate, propylene glycol monomethyl ether acetate, and the like), nitriles

(acetonitrile, isobutyronitrile, and the like), ethers (diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, 3-methoxy-3-methyl-1-butanol, and the like), acid amides (N,N-dimethylformamide, N,N-dimethylacetamide, and the like), halogenated hydrocarbons (dichloromethane, trichloroethane, carbon tetrachloride, and the like), sulfoxides (dimethyl sulfoxide, and the like), propylene carbonate, and vegetable oils (soybean oil, cottonseed oil, and the like).

Examples of the surfactant include non-ionic surfactants, such as polyoxyethylene alkyl ether, polyoxyethylene alkylaryl ether, and polyethylene glycol fatty acid ester; and anionic sufactants, such as alkyl sulfonate, alkyl benzene sulfonate, and alkyl sulfate.

Examples of other auxiliary agents for a formulation include a sticking agent, a dispersing agent, colorant, and a stabilizer, and specific examples thereof include casein, gelatin, sugars (starch, gum arabic, cellulose derivatives, alginic acid, and the like), lignin derivatives, bentonite, synthetic water-soluble polymers (polyvinyl alcohol, polyvinyl pyrrolidone, polyacrylic acids, and the like), PAP (isopropyl acid phosphate), BHT (2,6-di-tert-butyl-4-methylphenol), and BHA (a mixture of 2-tert-butyl-4-methoxyphenol and 3-tert-butyl-4-methoxyphenol).

The content ratio of the present fungicidal compound to the present condensed heterocyclic compound in the harmful arthropod control composition of the present invention is not particularly limited. The present fungicidal compound with respect to 1000 parts by weight of the present condensed heterocyclic compound is generally 0.1 parts by weight to 100000 parts by weight and preferably 1 part by weight to 10000 parts by weight. That is, the content ratio of the present fungicidal compound to the present condensed heterocyclic compound is, by parts by weight, generally 10000:1 to 1:100 and particularly 1000:1 to 1:10.

It is possible to control the harmful arthropods by applying an effective amount of the harmful arthropod control composition of the present invention to a plant or soil for cultivation of a plant.

Examples of the harmful arthropods for which the composition of the present invention has an effect include harmful insects or harmful mites. Specific examples of the harmful arthropods include the following.

Hemiptera pests: planthoppers, such as small brown planthoppers (Laodelphax striatellus), brown planthoppers (Nilaparvata lugens), and white-backed planthoppers (Sogatella furcifera); leafhoppers, such as green rice leafhoppers (Nephotettix cincticeps), Taiwan green rice leafhoppers (Nephotettix virescens), and tea green leafhoppers (Empoasca onukii); aphids, such as cotton aphids (Aphis gossypii), green peach aphids (Myzus persicae), cabbage aphids (Brevicoryne brassicae), spiraea aphids (Aphis sprraecola), potato aphids (Macrosiphum euphorbiae), greenhouse potato aphids (Aulacorthum solani), bird-cherry oat aphids (Rhopalosiphum aphids padi), black citrus aphids (Toxoptera citricidus), and mealy plum aphids (Hyalopterus pruni); stink bugs, such as eastern green stink bugs (Nezara antennata), bean bugs (Riptortus clavetus), male rice bugs (Leptocorisa ohinensis), white-spotted spined bugs (Eysarcoris parvus), and brown marmorated stink bugs (Halyomorpha mista); whiteflies, such as greenhouse whiteflies (Trialeurodes vaporariorum), silverleaf whiteflies (Bemisia tabaci), citrus whiteflies (Dialeurodea citri), and orange spiny whiteflies (Aleurocanthus spiniferus); and the like.

Lepidoptera pests: pyralids, such as asiatic rice borers (Chilo suppressalis), yellow stem borers (Tryporyza incertulas), rice leafrollers (Cnaphalocrocis medinalis), cotton leaf rollers (Notarcha derogata), Indian meal moths (Plodia interpunctella), Asian corn borers (Ostrinia furnacalis), cabbage webworms (Hellula undalis), and bluegrass webworms (Pediasia teterrellus); noctuids, such as oriental leafworm moths (Spodoptera litura), beet armyworms (Spodoptera exigua), oriental armyworms (Pseudaletia separata), cabbage moths (Mamestra brassicae), black cutworms (Agrotis ipsilon), asiatic common loopers (Plusia nigrisigna), the genus Trichoplusia, the genus Heliothis, and the genus Helicoverpa; pieridae, such as small white butterflies (Pieris rapae); leaf roller moths, such as oriental fruit moths (Grapholita molesta), soybean pod borers (Leguminivora glycinivorella), adzuki bean podworms (Matsumuraeses azukivora), summer fruit tortrix moths (Adoxophyes orana fasciata), smaller tea tortrixes (Adoxophyes honmai.), oriental tea tortrix moths (Homona magnanima), apple tortrixes (Archips fuscocupreanus), and codling moths (Cydia pomonella); leaf miners, such as tea leaf rollers (Caloptilia theivora), and apple leaf miner (Phyllonorycter ringoneella); codling moths, such as peach fruit moths (Carposina niponensis); leafminer moths, such as the genus Rionetia; tussock moths, such as the genus Lymantria and the genus Euproctis; ermine moths, such as diamondback moths (Plutella xylostella); pink bollworms (Pectincphora gossypiella) and potato tuber moths (Phthorimaea operculella); tiger moths, such as fall webworms (Hyphantria cunea); clothes moths (Tinea translucens); and the like.

Thysanoptera pests: thrips, such as western flower thrips (Frankliniella occidentalis), southern yellow thrips (Thrips palmi), yellow tea thrips (Scirtothrips dorsalis), onion thrips (Thrips tabaci), and flower thrips (Frankliniella intonsa).

Diptera pests: root-maggots, such as bean seed flies (Delia platura) and onion flies (Delia antiqua); leafminer flies, such as Japanese rice leafminers (Agromyza oryzae), rice leafminers (Hydrellia griseola), vegetable leafminers, (Liriomyza sativae), American serpentine leafminers (Liriomyza trifolii), and garden pea leafminers (Chromatomyia horticola); grass flies, such as rice stem maggots (Chlorops oryzae); fruit flies, such as melon flies (Dacus cucurbitae) and Mediterranean fruit flies (Ceratitis capitata); and drosophilas.

Elytron pests: corn rootworms, such as western corn rootworms (Diabrotica virgifera virgifera) and southern corn rootworms (Diabrotica undecimpunctata howardi); scarabaeid beetles, such as scarab beetles (Anomala cuprea), soybean beetles (Anomala rufocuprea), and Japanese beetles (Popillia japonica); weevils, such as maize weevils (Sitophilus zeamais), rice water weevils (Lissorhoptrus oryzophilus), rice plant weevils (Echinocnemus squameus), boll weevils (Anthonomus grandis), and hunting billbugs (Sphenophorus venatus); mealworms, such as tallow mealworms (Tenebrio molitor) and red flour beetles (Tribolium castaneum); leaf beetles, such as rice leaf beetles (Oulema oryzae), cucurbit leaf beetles (Aulacophora femoralis), striped flea beetles (Phyllotreta striolata), and Colorado potato beetles

(Leptinotarsa decemlineata); ladybugs, such as 28-spotted ladybirds (Epilachna viginticctopunctata); bark beetles, such as powderpost beetles (Lyctus brunneus) and common pine shoot beetles (Tomicus piniperda); auger beetles; museum beetles; sawyer beetles, such as citrus long-horned beetles (Anoplophora malasiaca); click beetles (Agriotes spp.); and rove beetles (Paederus fuscipes).

In the case of using the harmful arthropod controlling agent of the present invention, the application amount thereof is generally 1 g to 10000 g of the present condensed heterocyclic compound per 10000 $m^2$. When the harmful arthropod controlling agent of the present invention has been formulated into an emulsion, a water dispersible powder, a flowable agent, etc., in general, the present condensed heterocyclic compound is applied by being diluted in water so that the concentration of the active ingredients becomes 0.01 ppm to 10000 ppm. Granules, powder, etc. are generally applied to the subject as they are.

These formulations or water-diluted solutions of the formulations may be scattered directly onto harmful arthropods or to plants, such as crops, to be protected from the harmful arthropods. Alternately, soil in cultivated land may be treated with the formulations or the water-diluted solutions in order to control harmful arthropods which live in the soil.

The harmful arthropod controlling agent of the present invention can be used in agricultural land in which the following "crops" have been cultivated.

Farm products: corn, rice, wheat, barley, rye, oats, sorghum, cotton, soybeans, peanuts, buckwheat, sugar beets, rapeseeds, sunflowers, sugar cane, tobacco, and the like.

Vegetables: solanaceous vegetables (eggplants, tomatoes, bell peppers, peppers, potatoes, and the like), cucurbit vegetables (cucumbers, pumpkins, zucchinis, watermelons, melons, and the like), cruciferous vegetables (radishes, turnips, horseradishes, kohlrabi, chinese cabbage, cabbage, mustard, broccoli, cauliflower, and the like), asteraceae vegetables (burdock, garland chrysanthemum, artichoke, lettuce, and the like), liliaceae vegetables (spring onions, onions, garlic, asparagus), umbelliferae vegetables (carrots, parsley, celery, parsnips, and the like), chenopodiaceae vegetables (spinach, chard, and the like), labiatae vegetables (perilla, mint, basil, and the like), strawberries, sweet potatoes, Japanese yams, taro, and the like.

Fruit trees: pomaceous fruits (apples, European pears, Japanese pears, Chinese quinces, quinces, and the like), stone fruits (peaches, Japanese plums, nectarines, Chinese plums, cherries, apricots, prunes, and the like), citrus (satsuma mandarins, oranges, lemons, limes, grapefruits, and the like), nut trees (chestnut, walnut, hazelnut, almond, pistachio, cashew nut, macadamia nut, and the like), berry fruits (blueberries, cranberries, blackberries, raspberries, and the like), grapes, Japanese persimmoms, olives, loquats, bananas, coffee, date palms, coconuts, oil palms, and the like.

Trees other than fruit trees: tea, mulberry, flowering trees (azalea, camellia, hydrangea, sasanqua, Japanese star anise, cherry tree, tulip tree, crape myrtle, fragrant olive, and the like), street trees (ash, birch tree, dogwood, eucalyptus, ginkgo, lilac, maple, oak, poplar, cercis, sweetgum, sycamore, Japanese zelkova, Japanese arborvitae, fir tree, hemlock, juniper, pine, spruce, yew, elm, horse chestnut, and the like), sweet viburnum, podocarpus, cedar, Japanese cypress, croton, Japanese spindle tree, Japanese photinia, and the like.

Lawn: grasses (zoysiagrass, zoysia matrella, and the like), bermuda grasses (bermuda grass, and the like), bent grasses (creeping bent, creeping bent grass high, thread creeping bent grass, and the like), blue grasses (kentucky bluegrass, rough bluegrass, and the like), fescues (festuca arundinacea, chewings fescue, creeping red fescue, and the like), ryegrasses (darnel, rye grass, and the like), orchard grass, timothy grass, and the like.

Others: flowering plants (rose, carnation, chrysanthemum, lisianthus, gypsophila, gerbera, marigold, salvia, petunia, verbena, tulip, aster, gentian, lily, pansies, cyclamen, orchid, lily of the valley, lavender, stock, ornamental cabbage, primula, poinsettia, gladiolus, cattleya, daisy, cymbidium, begonia, and the like), foliage plants, and the like.

Genetically modified crops are also included in the "crops".

[Examples]

Hereinafter, the present invention will be described in more detail with reference to production examples, formulation examples, reference production examples, and examples, but is not limited to these examples.

First, Production Examples in producing the present condensed heterocyclic compound will be shown.

Hereinafter, Praparation Examples of the present condensed heterocyclic compound will be shown, but the present condensed heterocyclic compound is not limited to these examples.

Production Example 1

A mixture of 1.0 g of N2-methyl-5-trifluoromethylpyridine-2,3-diamine, 0.96 g of 2-ethylsulfanyl benzaldehyde, 1.80 g of sodium hydrogen sulfite, and 10 ml of DMF was heated and stirred for 5 hours at 160°C. The reaction mixture was ice-cooled, and water was added thereto to filter deposited crystals which were subsequently washed with water, and then, with hexane. The obtained crystals were dried under reduced pressure to obtain 1.09 g of 2-(2-ethylsulfanyl-phenyl)-3-methyl-6-trifluoromethyl-3H-imidazo[4,5-b]pyridine (hereinafter, referred to as the present condensed heterocyclic compound 1).

Present Condensed Heterocyclic Compound 1

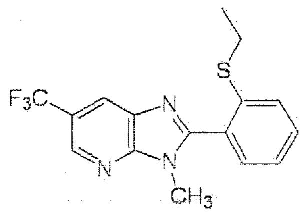

1H-NMR (CDC13) δ: 8.72-8.70 (1H, m), 8.33-8.31 (1H, m), 7.56-7.49 (2H, m), 7.47-7.43 (1H, m), 7.39-7.34 (1H, m), 3.77 (3H, s), 2.87 (2H, q), 1.24 (3H, t)

Production Example 2

A mixture of 0.25 g of 2-(2-ethylsulfanylphenyl)-3-methyl-6-trifluoromethyl-3H-imidazo[4,5-b]pyridine, 0.24 g of sodium periodate, 6 ml of methanol,' 2 ml of water, and 0.8 ml of THF was stirred for 20 minutes at room temperature. Then, the temperature was elevated to 50°C and the mixture was heated and stirred for 1.5 hours. After adding water to the reaction mixture which was ice-cooled, deposited crystals were filtered. The filtered crystals were dissolved in ethyl acetate, and were washed successively with a saturated aqueous sodium thiosulfate solution, a saturated aqueous sodium hydrogen carbonate solution, and a saturated saline solution. Then, an organic layer thereof was dried using magnesium sulfate and was subsequently condensed under reduced pressure. The obtained residue was washed with hexane to obtain 0.20 g of 2-(2-ethylsulfinylphenyl)-3-methyl-6-trifluoromethyl-3H-imidazo[4,5-b]pyridine (hereinafter, referred to as the present condensed heterocyclic compound 2).

Present Condensed Heterocyclic Compound 2

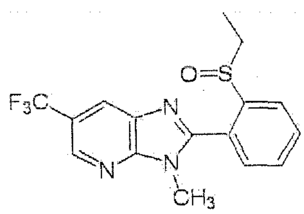

1H-NMR (CDCl3) δ: 8.76-8.74 (1H, m), 8.32-8.30 (1H, m), 8.27-8.24 (1H, m), 7.86-7.81 (1H, m), 7.72-7.68 (1H, m), 7.62-7.59 (1H, m), 3.89 (3H, s), 3.42-3.31 (1H, m), 3.02-2.92 (1H, m), 1.31 (3H, t)

Production Example 3

0.43 g of 3-chloroperbenzoic acid (with a purity greater than or equal to 65%) was added to a mixture of 0.25 g of 2-(2-ethyl-sulfanyl-phenyl)-3-methyl-6-trifluoromethyl-3H-imidazo[4,5-b]pyridine and 3 ml of chloroform under ice-cooling. Then, the temperature was elevated to room temperature and the mixture was stirred for 1 hour. Chloroform was added to the reaction mixture under ice-cooling, and then, a saturated aqueous sodium thiosulfate solution was added to the reaction mixture, which was then subjected to extraction using ethyl acetate. An organic layer thereof was washed with a saturated aqueous sodium hydrogen carbonate solution, and then, with a saturated saline solution. After drying the organic layer using sodium sulfate, the organic layer was condensed under reduced pressure. The obtained crystals were washed with hexane, and then, with methyl-tert-butyl ether, and 0.27 g of 2-(2-ethylsulfonylphenyl)-3-methyl-6-trifluoromethyl-3H-imidazo[4,5-b]pyridine (hereinafter, referred to as the present condensed heterocyclic compound 3) was obtained.

Present Condensed Heterocyclic Compound 3

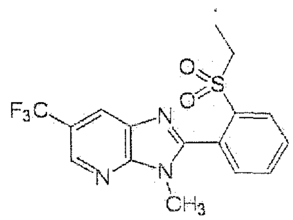

1H-NMR (CDC13) δ: 8.75-8.73 (1H, n), 8.29-8.27 (1H, m), 8.25-8.21 (1H, m), 7.87-7.79 (2H, m), 7.58-7.55 (1H, m), 3.72 (3H, s), 3.42 (2H, q), 1.26 (3H, t)

Production Example 4

720 mg of WSC was added to a mixture of 700 mg of N2-ethyl-5-trifluoromethylpyridine-2,3-diamine, 690 mg of 2-ethylsulfanyl benzoic acid, and 20 ml of pyridine at room temperature, and after the temperature was elevated to 95°C, the mixture was heated and stirred for 10 hours. A saturated aqueous sodium carbonate solution was added to the reaction mixture, which was cooled to room temperature, to subject the reaction mixture to extraction using ethyl acetate. An organic layer thereof was dried using sodium sulfate, and then, was condensed under reduced pressure.

The obtained residue was dissolved in 20 ml of xylene, and 1.6 g of p-toluenesulfonic acid monohydrate was added thereto. The temperature of the mixture was elevated to 170°C and the mixture was heated and stirred for 9.5 hours. A saturated aqueous sodium hydrogen carbonate solution was added to the reaction mixture, which was cooled to room temperature, to subject the reaction mixture to extraction using ethyl acetate. After drying the organic layer using sodium sulfate, the organic layer was condensed under reduced pressure. The obtained residue was subjected to silicagel column chromatography to obtain 295 mg of 3-ethyl-2-(2-ethylsulfanylphenyl)-6-trifluoromethyl-3H-imidazo[4,5-b]pyridine (hereinafter, referred to as the present condensed heterocyclic compound 4).

Present Condensed Heterocyclic Compound 4

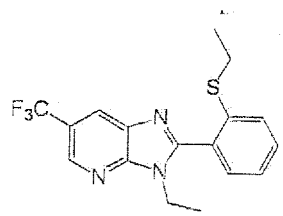

H-NMR (CDCl3) δ: 8.70 (1H, a), 8.32 (1H, a), 7.60-7.30 (4H, m), 4.25 (2H, q), 2.89 (2H, q), 1.35-1.30 (3H, m), 1.27-1.21 (3H, m)

Production Examples 5 and 6

140 mg of 2-(2-ethylaulfinylphenyl)-3-ethyl-6-trifluoromethyl-3H-imidazo[4,5-b]pyridine (hereinafter, referred to as a present condensed heterocyclic compound 5) and 60 mg of 2-(2-ethylsulfonylphenyl)-3-ethyl-6-trifluoromethyl-3H-imidazo[4,5-b]pyridine (hereinafter, referred to as a present condensed heterocyclic compound 6) were obtained based on the methods described in Production Examples 32 and 33 using 3-ethyl-2-(2-ethylsulfanylphenyl)-6-trifluoromethyl-3H-imidazo[4,5-b]pyridine instead of 2-(2-ethylsulfanyl-4-fluorophenyl)-3-methyl-6-trifluoromethyl-3H-imidazo[4,5-b]pyridine. Present Condensed Heterocyclic Compound 5

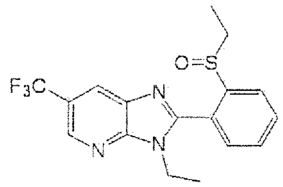

1H-NMR (CDC13) δ: 8.75 (1H, d), 8.31 (1H, d), 8.24 (1H, dd), 7.84 (1H, dt), 7.70 (1H, dt), 7.58 (1H, dd), 4.35 (2H, q), 3.43-3.30 (1H, m), 3.06-2.94 (1H, m), 1.41 (3H, t), 1.30 (3H, t)

Present Condensed Heterocyclic Compound 6

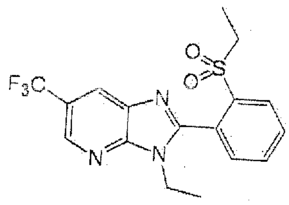

1H-NMR (CDC13) δ: 8.71 (1H, d), 8.32 (1H, d), 7.56-7.47 (2H, m), 7.43 (1H, dd), 7.39-7.31 (1H, m), 4.25 (2H, q), 2.89 (2H, q), 1.32 (3H, t), 1.25 (3H, t)

Production Examples 7

130 mg of 2-(2-ethyl-sulfanyl-phenyl)-3-isopropyl-6-trifluoromethyl-3H-imidazo[4,5-b]pyridine (hereinafter, referred to as the present condensed heterocyclic compound 7) was obtained based on the method described in the Production Example 4 using N2-isopropyl-5-trifluoromethylpyridine-2,3-diamine instead of N2-ethyl-5-trifluoromethylpyridine-2,3-diamine.

Present Condensed Heterocyclic Compound 7

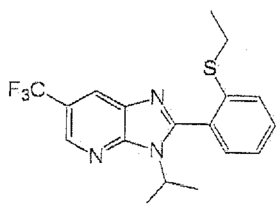

1H-NMR (CDC13) δ: 8.68 (1H, d), 8.28 (1H, d), 7.55-7.47 (2H, m), 7.39-7.31 (2H, m), 4.40-4.33 (1H, m), 2.91 (2H, q), 1.77-1.66 (6H, m), 1.25 (3H, t)

Production Examples 8 and 9

60 mg of 2-(2-ethylsulfinylphenyl)-3-isopropyl-6-trifluoromethyl-3H-imidazo[4,5-b]pyridine (hereinafter, referred to as the present condensed heterocyclic compound 8) and 55 mg of 2-(2-ethylsulfonylphenyl)-3-isopropyl-6-trifluoromethyl-3H-imidazo[4,5-b]pyridine (hereinafter, referred to as the present condensed heterocyclic compound 9) were obtained based on the methods described in Production Examples 32 and 33 using 2-(2-ethyl-sulfanyl-phenyl)-3-isopropyl-6-trifluoromethyl-3H-imidazo[4,5-b]pyridine instead of 2-(2-ethylsulfanyl-4-fluorophenyl)-3-methyl-6-trifluoromethyl-3H-imidazo[4,5-b]pyridine. Present Condensed Heterocyclic Compound 8

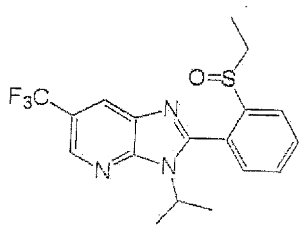

1H-NMR (CDC13) δ: 8.73 (1H, d), 8.28 (1H, d), 8.22 (1H, dd), 7.83 (1H, td), 7.69 (1H, td), 7.50 (1H, dd), 4.60-4.48 (1H, m), 3.38-3.26 (1H, m), 3.05-2.95 (1H, m), 1.77-1.71 (6H, m), 1.27 (3H, t)

Present Condensed Heterocyclic Compound 9

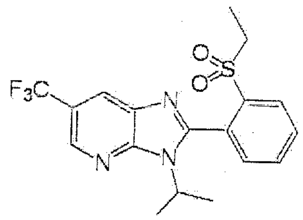

1H-NMR (CDCl3) δ: 8.71 (1H, d), 8.24-8.22 (2H, m), 7.85-7.78 (2H, m), 7.55-7.52 (1H, m), 4.33-4.26 (1H, m), 3.72-3.62 (1H, m), 3.44-3.34 (1H, m), 1.77-1.69 (6H, m), 1.28 (3H, t)

Production Example 10

280 mg of 3-cyclopropyl-2-(2-ethyl-sulfanyl-phenyl)-6-trifluoromethyl-3H-imidazo[4,5-b]pyridine (hereinafter, referred to as the present condensed heterocyclic compound 10) was obtained based on the method desoribed in Production Example 4 using N2-cyclopropyl-5-trifluoromethyl-pyridine-2,3-diamine instead of N2-ethyl-5-trifluoromethyl-pyridine-2,3-diamine.

Present Condensed Heterocyclic Compound 10

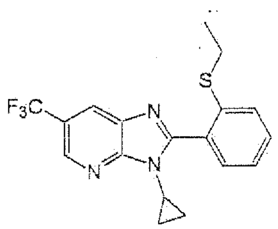

1H-NMR (CDC13) δ: 8.74 (1H, s), 8.31 (1H, s), 7.53-7.47 (3H, m), 7.39-7.31 (1H, m), 3.55-3.49 (1H, m), 2.90 (2H, q), 1.25 (3H, t), 1.01-0.94 (2H, m), 0.93-0.86 (2H, m)

Production Examples 11 and 12

114 mg of 3-cyclopropyl-2-(2-ethyl-sulfinyl-phenyl)-6-trifluoromethyl-3H-imidazo[4,5-b]pyridine (hereinafter, referred to as the present condensed heterocyclic compound 11) and 109 mg of 3-cyclopropyl-2-(2-ethyl-sulfonyl-phenyl)-6-trifluoromethyl-3H-imidazo[4,5-b]pyridine (hereinafter, referred to as the present condensed heterocyclic compound 12) were obtained based on the methods described in Production Examples 32 and 33 using 3-cyclopropyl-2-(2-ethyl-sulfanyl-phenyl)-6-trifluoromethyl-3H-imidazo[4,5-b]pyridine instead of 2-(2-ethylsulfanyl-4-fluorophenyl)-3-methyl-6-trifluoromethyl-3H-imidazo[4,5-b]pyridine.

Present Condensed Heterocyclic Compound 11

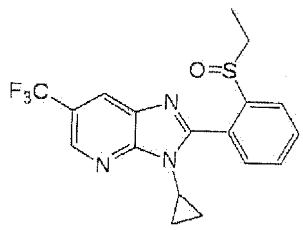

1H-NMR (CDC13) δ: 8.77 (1H, d), 8.29-8.26 (2H, m), 7.85-7.79 (2H, m), 7.72-7.67 (1H, m), 3.57-3.51 (1H, m), 3.49-3.39 (1H, m), 3.09-2.95 (1H, m), 1.34 (3H, t), 1.29-1.16 (1H, m), 1.09-0.92 (2H, m), 0.80-0.65 (1H, m)

Present Condensed Heterocyclic Compound 12

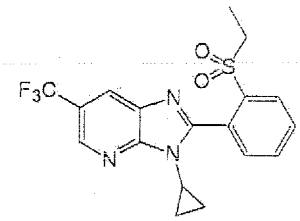

1H-NMR (CDC13) δ: 8.76 (1H, s), 8.27-8.22 (2H, m), 7.87-7.78 (2H, m), 7.65 (1H, dd), 3.60 (2H, brs), 3.39-3.33 (1H, m), 1.29 (3H, t), 1.11-1.11 (2H, m), 0.33 (2H, brs).

Production Example 13

700 μl of bis(2-methoxyethyl)aminosulfur trifluoride was added to a mixture of 122 mg of 2-(2-ethyl-sulfanyl-phenyl)-3-methyl-3H-imidazo[4,5-b]pyridine-6-carbaldehyde and 3 ml of chloroform under ice-cooling. The temperature was elevated to room temperature and the mixture was stirred for 3 hours. A saturated aqueous sodium hydrogen carbonate solution was added to the reaction mixture, which was then subjected to extraction successively using chloroform and ethyl acetate. An organic layer thereof was dried using sodium sulfate, and then, was condensed under reduced pressure. The obtained residue was subjected to silicagel column chromatography to obtain 49 mg of 6-difluoromethyl-2-(2-ethyl-sulfanyl-phenyl)-3-methyl-3H-imidazo[4,5-b]pyridine (hereinafter, referred to as the present condensed heterocyclic compound 13).

Present Condensed Heterocyclic Compound 13

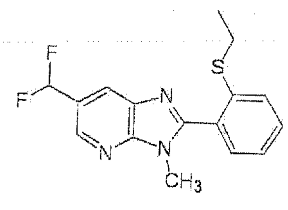

1H-NMR (CDCl3) δ: 8.59 (1H, s), 8.23 (1H, s), 7.56-7.43 (3H, m), 7.38-7.33 (1H, m), 6.88 (1H, t), 3.77 (3H, s), 2.8.7 (2H, q), 1.23 (3H, t)

Production Examples 14 and 15

110 mg of 6-difluoromethyl-2-(2-ethyl-sulfinyl-phenyl)-3-methyl-3H-imidazo[4,5-b]pyridine (hereinafter, referred to as the present condensed heterocyclic compound 14) and 101 mg of 6-difluoromethyl-2-(2-ethyl-sulfonyl-phenyl)-3-methyl-3H-imidazo[4,5-b]pyridine (hereinafter, referred to as the present condensed heterocyclic compound 15) were obtained based on the methods described in Production Examples 32 and 33 using 6-difluoromethyl-2-(2-ethyl-sulfanyl-phenyl)-3-methyl-3H-imidazo[4,5-b]pyridine instead of 2-(2-ethylsulfanyl-4-fluorophenyl)-3-methyl-6-trifluoromethyl-3H-imidazo[4,5-b]pyridine.

Present Condensed Heterocyclic Compound 14

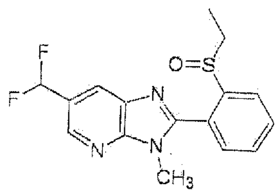

1H-NMR (CDC13) δ: 8.62 (1H, s), 8.26-8.23 (2H, m), 7.83 (1H, td), 7.70 (1H, td), 7.62 (1H, dd), 6.90 (1H, t), 3.89 (3H, s), 3.42-3.32 (1H, m), 3.02-2.92 (1H, m), 1.30 (3H, t)

Present Condensed Heterocyclic Compound 15

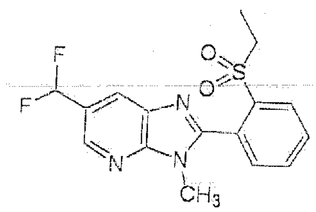

1H-NMR (CDCl3) δ: 8.61 (1H, s), 8.24-8.19 (2H, m), 7.87-7.78 (2H, m), 7.58 (1H, dd), 6.89 (1H, t), 3.71 (3H, s), 3.43 (2H, q), 1.25 (3H, t)

Production Example 16

A mixture of 500 mg of 6-bromo-2-(2-ethyl-sulfanyl-phenyl)-3-methyl-3H-imidazo[4,5-b]pyridine, 24 ml of NMP, 10 ml of xylene, 1.1 g of copper iodide, and 1.1 g of pentafluoropropionic acid sodium was heated to 170°C, and was heated and stirred for 3 days. A saturated aqueous sodium hydrogen carbonate solution was added to the reaction mixture, which was cooled to room temperature, to subject the reaction mixture to extraction using tert-butyl methyl ether. An organic layer thereof was dried using sodium sulfate, and then, was condensed under reduced pressure. The obtained residue was subjected to silicagel column chromatography to obtain 43 mg of 2-(2-ethyl-sulfanyl-phenyl)-3-methyl-6-pentafluoroethyl-3H-imidazo[4,5-b]pyridine (hereinafter, referred to as the present condensed heterocyclic compound 16).

Present Condensed Heterocyclic Compound 16)

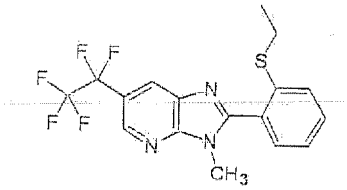

1H-NMR (CDC13) δ: 8.66 (1H, d), 8.30 (1H, d), 7.56-7.49 (2H, m), 7.47-7.43 (1H, m), 7.38-7.34 (1H, m), 3.78 (3H, s), 2.88 (2H, q), 1.24 (3H, t)

Production Example 17

A mixture of 1.64 g of 2-ethyl-sulfanyl-N-(2-methylamino-5-trifluoromethylphenyl)-benzamide, 1.76 g of p-toluenesulfonic acid monohydrate, and 50 ml of xylene was stirred for 1 hour at 150°C under heating and refluxing. A saturated aqueous sodium hydrogen carbonate solution was added to the reaction mixture, which was cooled to room temperature, to subject the reaction mixture to extraction using ethyl acetate. After washing an organic layer thereof with the saturated aqueous sodium hydrogen carbonate solution and a saturated saline solution, the organic layer was dried using sodium sulfate, and then was condensed under reduced pressure. The obtained residue was subjected to silicagel column chromatography to obtain 1.40 g of 2-(2-ethyl-sulfanyl-phenyl)-1-methyl-5-trifluoromethyl-1H-benzimidazole (hereinafter, referred to as the present condensed heterocyclic compound 17).

Present Condensed Heterocyclic Compound 17

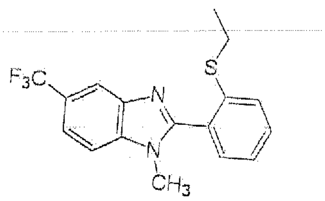

1H-NMR (CDCl3) δ: 8.12-8.10 (1H, m), 7.61-7.58 (1H, m), 7.53-7.44 (4H, m), 7.38-7.32 (1H, m), 3.69 (3H, s), 2.84 (2H, q), 1.22 (3H, t)

Production Example 18

0.30 g of 2-(2-ethyl-sulfinyl-phenyl)-1-methyl-5-trifluoromethyl-1H-benzimidazole (hereinafter, referred to as the present condensed heterocyclic compound 18) was obtained based on the method described in Production Example 2 using 2-(2-ethyl-sulfanyl-phenyl)-1-methyl-5-trifluoromethyl-1H-benzimidazole instead of 2-(2-ethyl-sulfanyl-phenyl)-3-methyl-6-trifluoromethyl-3H-imidazo[4,5-b]pyridine.

Present Condensed Heterocyclic Compound 18

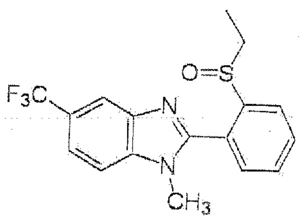

1H-NMR (CDC13) δ: 8.24-8.20 (1H, m), 8.10-8.07 (1H, m), 7.83-7.7B (1H, m), 7.70-7.62 (2H, m), 7.57-7.51 (2H, m), 3.79 (3H, s), 3.36-3.26 (1H, m), 2.98-2.88 (1H, m), 1.26 (3H, t)

Production Example 19

0.24 g of 2-(2-ethylsulfonyl-phenyl)-1-methyl-5-trifluoromethyl-1H-benzimidazole (hereinafter, referred to as the present condensed heterocyclic compound 19) was obtained based on the method described in Production Example 3 using 2-(2-ethyl-sulfanyl-phenyl)-1-methyl-5-trifluoromethyl-1H-benzimidazole instead of 2-(2-ethylsulfanyl-phenyl)-3-methyl-6-trifluoromethyl-3H-imidazo[4,5-b]pyridine.

Present Condensed Heterocyclic Compound 19

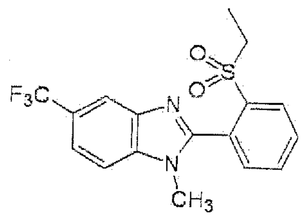

1H-NMR (CDC13) δ: 8.24-8.20 (1H, m), 8.07-8.05 (1H, m), 7.84-7.77 (2H, m), 7.64-7.61 (1H, m), 7.57-7.54 (1H, m), 7.53-7.49 (1H, m), 3.63 (3H, s), 3.46-3.34 (2H, m), 1.23 (3H, t)

Production Example 20

A mixture of 0.97 g of 2-amino-4-trifluoromethyl-phenol, 1.10 g of 2-ethylsulfanyl benzoic acid, 1.27 g of WSC, 37 mg of HOBt, and 5 ml of pyridine was stirred for 2.5 hours at 115°C under heating and refluxing. After allowing the mixture to stand overnight, the mixture was stirred for 6 hours at 115°C under heating and refluxing again. Water was added tc the reaction mixture, which was cooled to room temperature, to subject the reaction mixture to extraction using ethyl acetate. An organic layer thereof was washed successively with water, a saturated aqueous sodium hydrogen carbonate solution, and a saturated saline solution. After drying the organic layer using sodium sulfate, the organic layer was condensed under reduced pressure.

A mixture of the obtained residue, 2.09 g of p-toluenesulfonic acid monohydrate, and 50 ml of xylene was stirred for 2 hours at 153°C under heating and refluxing. A saturated aqueous sodium hydrogen carbonate solution was added to the reaction mixture, which was cooled to room temperature, to subject the reaction mixture to extraction using ethyl acetate. The organic layer was washed successively with a saturated aqueous sodium hydrogen carbonate solution, water, a 10% citric acid solution, water, a saturated aqueous sodium hydrogen carbonate solution, and a saturated saline solution. After drying the organic layer using sodium sulfate, the organic layer was condensed under reduced pressure. The obtained residue was subjected to silicagel column chromatography to obtain 0.98 g of 2-(2-ethyl-sulfanyl-phenyl)-5-trifluoromethyl-benzoxazole (hereinafter referred to as the present condensed heterocyclic compound 20).

Present Condensed Heterocyclic Compound 20

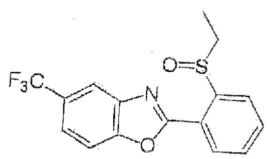

1H-NMR (CDCl3) S: 8.19-8.15 (2H, m), 7.71-7.67 (1H, m), 7.66-7.63 (1H, m), 7.52-7.47 (1H, m), 7.45-7.42 (1H, m), 7.31-7.27 (1H, m), 3.06 (2H, q), 1.44 (3H, t)

Production Example 21

0.27 g of 2-(2-ethyl-sulfinyl phenyl)-5-trifluoromethyl-benzoxazole (hereinafter, referred to as the present condensed heterocyclic compound 21) was obtained based on the method described in Production Example 2 using 2-(2-ethylsulfanylphenyl)-5-trifluoromethyl-benzoxazole instead of 2-(2-ethyl-sulfanyl-phenyl)-3-methyl-6-trifluoromethyl-3H-imidazo[4,5-b]pyridine.

Present Condensed Heterocyclic Compound 21

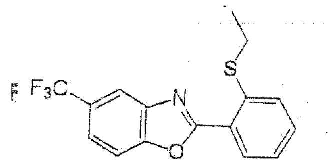

1H-NMR (CDCl3) δ: 8.35-8.30 (2H, m), 8.12-8.10 (1H, m), 7.85-7.79 (1H, m), 7.75-7.66 (3H, m), 3.48-3.38 (1H, m), 3.00-2.90 (1H, m), 1.41 (3H, t)

Production Example 22

0.24 g of 2-(2-ethyl-sulfonyl-phenyl)-5-trifluoromethyl benzoxazole (hereinafter, referred to as the present condensed heterocyclic compound 22) was obtained based on the method described in Production Example 3 using 2-(2-ethyl-sulfanylphenyl)-5-trifluoromethyl-benzoxazole instead of 2-(2-ethyl-sulfanyl-phenyl)-3-methyl-6-trifluoromethyl-3H-imidazo[4,5-b]pyridine.

Present Condensed Heterocyclic Compound 22

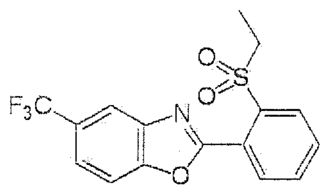

1H-NMR (CDCl3) S: 8.28-8.24 (1H, m), 8.11-8.09 (1H, m), 7.99-7.95 (1H, m), 7.85-7.77 (2H, m), 7.72-7.70 (2H, m), 3.82 (2H, q), 1.40 (3H, t)

Production Example 23

The temperature of a mixture of 0.92 g of 2-ethylsulfanyl-phenyl-N-(2-hydroxy-5-trifluoromethylpyridine-3-yl)-benzamide and 5 ml of phosphorus oxychloride was elevated to 120°C and the mixture was stirred under heating and refluxing. The reaction mixture, which was cooled to room temperature, was allowed to stand overnight. The temperature of the reaction mixture was elevated to 120°C again and the reaction mixture was stirred for 2 hours under heating and refluxing. Water was added to the reaction mixture, which was cooled to room temperature, to filter the deposited solid which was subsequently washed with water and hexane. Then, the solid was dried to obtain 0.53 g of 2-(2-ethy-sulfanyl-phenyl)-6-trifluoromethyl-oxazolo[5,4-b]pyridine (hereinafter, referred to as the present condensed heterocyclic compound 23).

Present Condensed Heterocyclic Compound 23

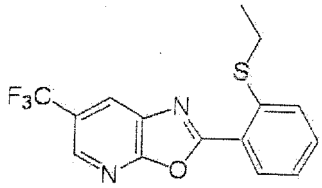

1H-NMR (CDC13) δ: 8.67 (1H, s), 8.40 (1H, s), 8.25 (1H, d), 7.53 (1H, t), 7.45 (1H, d), 7.32 (1H, t), 3.08 (2H, q), 1.45 (3H, t)

Production Example 24

0.17 g of 2-(2-ethyl-sulfinyl-phenyl)-6-trifluoromethyl-oxazolo[5,4-b]pyridine (hereinafter, referred to as the present condensed heterocyclic compound 24) was obtained based on the method described in Production Example 2 using 2-(2-ethyl-sulfanyl-phenyl)-6-trifluoromethyl-oxazolo[5,4-b]pyridine instead of 2-(2-ethyl-sulfanyl-phenyl)-3-methyl-6-trifluoromethyl-3H-imidazo[4,5-b]pyridine.

Present Condensed Heterocyclic Compound 24

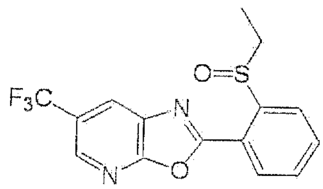

1H-NMR (CDC13) δ: 8.73-8.72 (1H, m), 8.41-8.38 (2H, m), 8.36-8.33 (1H, m), 7.90-7.84 (1H, m), 7.74-7.69 (1H, m), 3.45-3.35 (1H, m), 3.00-2.90 (1H, m), 1.40 (3H, t)

Production Example 25

0.19 g of 2-(2-ethyl-sulfonyl phenyl)-6-trifluoromethyl-oxazolo[5,4-b]pyridine (hereinafter, referred to as the present condensed heterocyclic compound 25) was obtained based on the method described in Production Example 3 using 2-(2-ethyl-sulfanyl-phenyl)-6-trifluoromethyl-oxazolo[5,4-b]pyridine instead of 2-(2-ethyl-sulfanyl-phenyl)-3-methyl-6-trifluoromethyl-3H-imidazo[4,5-b]pyridine.

Present Condensed Heterocyclic Compound 25

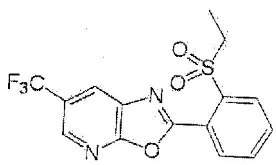

1H-NMR (CDC13) δ: 8.75-8.73 (1H, m), 8.40-8.37 (1H, m), 8.29-8.26 (1H, m), 8.05-8.02 (1H, m), 7.89-7.81 (2H, m), 3.81 (2H, q), 1.43 (3H, t)

Production Example 26

A mixture of 1.08 g of 2-amino-4-trifluoromethyl-benzenethiol hydrochloride, 1.04 g of 2-ethylsulfanyl benzoic acid chloride, and 10 mL of THF was stirred for 3 hours at room temperature. 0.43 g of sodium hydrogen carbonate was added to the reaction mixture, which was then stirred for 8 hours at room temperature. A saturated aqueous sodium

hydrogen carbonate solution was added to the reaction mixture, which was then subjected to extraction using ethyl acetate. An organic layer thereof was washed with water and was dried using sodium sulfate, and then, was condensed under reduced pressure. The obtained residue was subjected to silicagel column chromatography to obtain 0.50 g of 2-(2-ethyl-sulfanyl-phenyl)-5-trifluoromethyl-benzothiazole (hereinafter, referred to as the present condensed heterocyclic compound 26).

Present Condensed Heterocyclic Compound 26

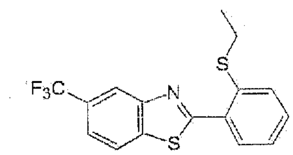

1H-NMR (CDC13) δ: 8.41-8.39 (1H, m), 8.06-8.00 (2H, m), 7.66-7.62 (1H, m), 7.55-7.51 (1H, m), 7.48-7.42 (1H, m), 7.37-7.32 (1H, m), 2.96 (2H, q), 1.33 (3H, t)

Production Example 27

0.25 g of 2-(2-ethyl-sulfinyl phenyl)-5-trifluoromethyl-benzothiazole (hereinafter, referred to as the present condensed heterocyclic compound 27) was obtained based on the method described in Production Example 2 using 2-(2-ethyl-sulfanylphenyl)-5-trifluoromethyl-benzothiazole instead of 2-(2-ethyl-sulfanyl-phenyl)-3-methyl-6-trifluoromethyl-3H-imidazo[4,5-b]pyridine.

Present Condensed Heterocyclic Compound 27

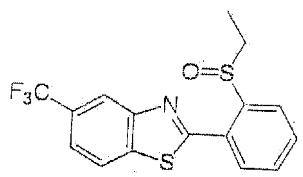

1H-NMR (CDC13) δ: 8.36-8.32 (1H, m), 8.32-8.30 (1H, m), 8.08-8.05 (1H, m), 7.97-7.94 (1H, m), 7.80-7.75 (1H, m), 7.71-7.68 (1H, m), 7.66-7.61 (1H, m), 3.56-3.45 (1H, m), 3.02-2.93 (1H, m), 1.46 (3H, t)

Production Example 28

0.30 g of 2-(2-ethyl-sulfonyl phenyl)-5-trifluoromethyl-benzothiazole (hereinafter, referred to as the present condensed heterocyclic compound 28) was obtained based on the method described in Production Example 3 using 2-(2-ethyl-sulfanyl-phenyl)-5-trifluoromethyl-benzothiazole instead of 2-(2-ethylsulfanyl-phenyl)-3-methyl-6-trifluoromethyl-3H-imidazo[4,5-b]pyridine.

Present Condensed Heterocyclic Compound 28

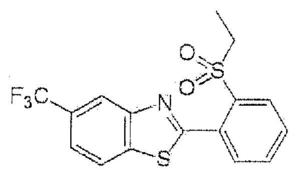

1H-NMR (CDCl3) δ: 8.33-8.31 (1H, m), 8.26-8.23 (1H, m), 8.10-8.06 (1H, m), 7.81-7.69 (4H, m), 3.75 (2H, q), 1.36 (3H, t)

Production Examples 29 and 30

27 mg of 2-(2-ethylsulfinyl-phenyl)-3-methyl-6-pentafluoroethyl-3H-imidazo[4,5-b]pyridine (hereinafter, referred to as the present condensed heterocyclic compound 29) and 31 mg of 2-(2-ethyl-sulfonyl phenyl)-3-methyl-6-pentafluoroethyl-3H-imidazo[4,5-b]pyridine (hereinafter, referred to as the present condensed heterocyclic compound 30) were obtained based on the methods described in Production Examples 32 and 33 using 2-(2-ethylsulfanyl-phenyl)-3-methyl-6-pentafluoroethyl-3H-imidazo[4,5-b]pyridine instead of 2-(2-ethylsulfanyl-4-fluorophenyl)-3-methyl-6-trifluoromethyl-3H-imidazo[4,5-b]pyridine. Present Condensed Heterocyclic Compound 29

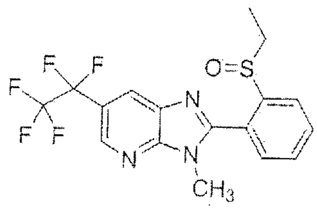

1H-NMR (CDCl3) δ: 8.70 (1H, d), 8.29 (1H, d), 8.27 (1H, d), 7.84 (1H, t), 7.71 (1H, t), 7.60 (1H, d), 3.90 (3H, s), 3.43-3.33 (1H, m), 3.04-2.94 (1H, m), 1.31 (3H, t)

Present Condensed Heterocyclic Compound 30

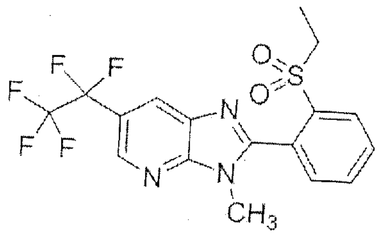

1H-NMR (CDCl3) δ: 8.69 (1H, s), 8.25 (1H, s), 8.23-8.22 (1H, m), 7.88-7.79 (2H, m), 7.59-7.52 (1H, m), 3.72 (3H, s), 3.43 (2H, q), 1.26 (3H, t)

Production Example 31

A mixture of 1.14 g of N2-methyl-5-trifluoromethyl-pyridine-2,3-diamine, 1.44 g of 2-ethylsulfanyl-4-fluoro-benzoic acid, 1.02 g of WSC, and 12 ml of pyridine was heated and stirred for 1.5 hours at 120°C. Water was added to the reaction mixture, which was cooled to room temperature, to subject the reaction mixture to extraction using ethyl acetate. An organic layer thereof was dried using magnesium sulfate, and then, was condensed under reduced pressure.

3.42 g of p-toluenesulfonic acid, 10 ml of xylene, and 2 ml of NMP were added to the obtained residue and the mixture was stirred for 4.5 hours at 150°C under heating and refluxing while removing water using a Dean-Stark apparatus. Water was added to the reaction mixture, which was cooled to room temperature, to subject the reaction mixture to extraction using ethyl acetate. The organic layer was dried using magnesium sulfate, and then, was condensed under reduced pressure. The obtained residue was subjected to silicagel column chromatography to obtain 1.05 g of 2-(2-ethylsulfanyl-4-fluorophenyl)-3-methyl-6-trifluoromethyl-3H-imidazo[4,5-b]pyridine (hereinafter, referred to as the present condensed heterocyclic compound 31).

Present Condensed Heterocyclic Compound 31

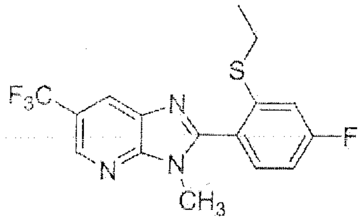

1H-NMR (CDC13) δ: 8.70-8.68 (1H, m), 8.31-8.28 (1H, m), 7.43-7.38 (1H, m), 7.17-7.13 (1H, m), 7.04-6.98 (1H, m), 3.75 (3H, s), 2.89 (2H, q), 1.26 (3H, t)

Production Examples 32 and 33

0.81 g of 3-chloroperbenzoic acid (with a purity greater than or equal to 65%) was added to a mixture of 0.85 g of 2-(2-ethylsulfanyl-4-fluorophenyl)-3-methyl-6-trifluoromethyl-3H-imidazo[4,5-b]pyridine and 12 ml of chloroform under ice-cooling. Then, the temperature was elevated to room temperature and the mixture was stirred for 30 minutes. A saturated aqueous sodium hydrogen carbonate solution and saturated aqueous sodium thiosulfate solution were added to the reaction mixture, which was then subjected to extraction using chloroform. An organic layer thereof was dried using magnesium sulfate, and then, was condensed under reduced pressure. The obtained residue was subjected to silicagel column chromatography to obtain 0.33 g of 2-(2-ethyl-sulfinyl-4-fluorophenyl)-3-methyl-6-trifluoromethyl-3H-imidazo[4,5-b]pyridine (hereinafter, referred to as the present condensed heterocyclic compound 32) and 0.52 g of 2-(2-ethyl-sulfonyl-4-fluorophenyl)-3-methyl-6-trifluoromethyl-3H-imidazo[4,5-b]pyridine (hereinafter, referred to as the present condensed heterocyclic compound 33).

Present Condensed Heterocyclic Compound 32

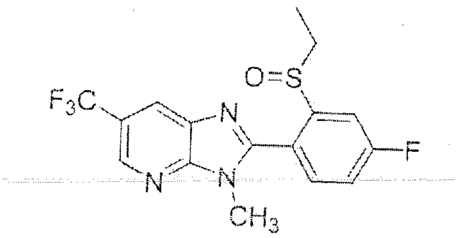

1H-NMR (CDC13) δ: 8.76-8.75 (1H, m), 8.31-8.30 (1H, m), 8.01-7.98 (1H, m), 7.65-7.61 (1H, m), 7.41-7.36 (1H, m), 3.90 (3H, s), 3.47-3.37 (1H, m), 3.04-2.94 (1H, m), 1.33

(3H, t)

Present Condensed Heterocyclic Compound 33

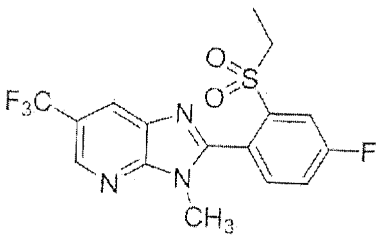

1H-NMR (CDC13) δ: 8.76-8.74 (1H, m), 8.29-8.27 (1H, m), 7.97-7.94 (1H, m), 7.60-7.51 (2H, m), 3.72 (3H, s), 3.44 (2H, q), 1.28 (3H, t)

Production Example 34

1.0 g of a DMF solution of 2-(2-fluoro-4-trifluoromethyl-phenyl)-3-methyl-6-trifluoromethyl-3H-imidazo[4,5-b]pyridine was added dropwise to a mixture of 0.35 g of ethyl mercaptan sodium salt (80%) and 9 ml of DMF under ice-cooling, and then, the temperature was elevated to room temperature and the mixture was stirred for 30 minutes at room temperature. A saturated aqueous sodium hydrogen carbonate solution was added to the reaction mixture, which was then subjected to extraction using ethyl acetate. An organic layer thereof was dried using magnesium sulfate, and then, was condensed under reduced pressure. The obtained residue was subjected to silicagel column chromatography to obtain 1.10 g of 2-(2-ethyl-sulfanyl-4-trifluoromethylphenyl)-3-methyl-6-trifluoromethyl-3H-imidazo[4,5-b]pyridine (hereinafter, referred to as the present condensed heterocyclic compound 34).

Present Condensed Heterocyclic Compound 34

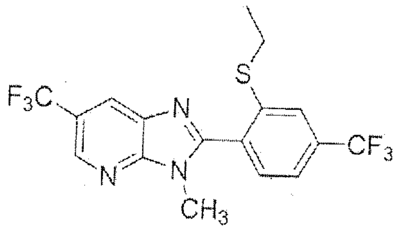

1H-NMR (CDC13) δ: 8.75-8.73 (1H, m), 8.35-8.33 (1H, m), 7.70-7.68 (1H, m), 7.62-7.56 (2H, m), 3.79 (3H, s), 2.95 (2H, q), 1.28 (3H, t)

Production Example 35

A mixture of 311 mg of 2-(2-ethylsulfanyl-phenyl)-6-iodo-3-methyl-3H-imidazo[4,5-b]pyridine, 1.5 g of copper iodide, 1.8 g of sodium heptafluorobutyrate, 5 mL of NMP, and 25 mL of xylene was heated and stirred for 12 hours at 150°C. A saturated aqueous sodium hydrogen carbonate solution and 28% ammonia water were added to the reaction mixture, which was cooled to room temperature, to subject the reaction mixture to extraction using tert-butyl methyl ether. A combined organic layer was dried using sodium sulfate, and then, was condensed under reduced pressure. The residue was subjected to silicagel column chromatography to obtain 118 mg of 2-(2-ethylsulfanyl-phenyl)-6-heptafluoropropyl-3-methyl-3H-imidazo[4,5-b]pyridine (hereinafter, referred to as the present condensed heterocyclic compound 35).

Present Condensed Heterocyclic Compound 35

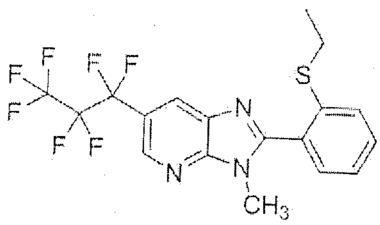

1H-NMR (CDC13) δ: 8.65 (1H, d), 8.29 (1H, d), 7.56-7.51 (2H, m), 7.48-7.43 (1H, m), 7.38-7.34 (1H, m), 3.78 (3H, s), 2.89 (2H, q), 1.25 (3H, t)

Production Examples 36 and 37

2-(2-ethylsulfinyl-phenyl)-6-heptafluoropropyl-3-methyl-3H-imidazo[4,5-b]pyridine (hereinafter, referred to as the present condensed heterocyclic compound 36) and 2-(2-ethyl sulfonyl-phenyl)-6-heptafluoropropyl-3-methyl-3H-imidazo[4,5-b]pyridine (hereinafter, referred to as the present condensed heterocyclic compound 37) were obtained based on the methods described in Production Examples 32 and 33 using 2-(2-ethylsulfanyl-phenyl)-6-heptafluoropropyl-3-methyl-3H-imidaze[4,5-b]pyridine instead of 2-(2-ethylsulfanyl-4-fluorophenyl)-3-methyl-6-trifluoromethyl-3H-imidazo[4,5-b]pyridine.

Present Condensed Heterocyclic Compound 36

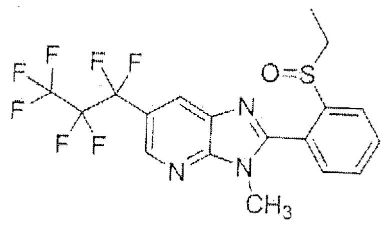

1H-NMR (CDC13) δ: 8.68 (1H, d), 8.29-8.24 (2H, m), 7.87-7.81 (1H, m), 7.74-7.68 (1H, m), 7.61 (1H, dd), 3.91 (3H, s), 3.43-3.32 (1H, m), 3.05-2.94 (1H, m), 1.31 (3H, t) Present Condensed Heterocyclic Compound 37

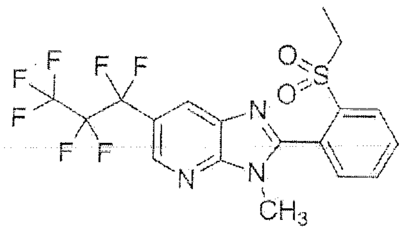

1H-NMR (CDCl3) 5: 8.67 (1H, d), 8.26-8.22 (2H, m), 7.87-7.81 (2H, m), 7.59-7.55 (1H, m), 3.73 (3H, s), 3.43 (2H, q), 1.26 (3H, t).

Production Examples 38 and 39

0.51 g of 2-(2-ethylsulfinyl-4-trifluoromethylphenyl)-3-methyl-6-trifluoromethyl-3H-imidazo[4,5-b]pyridine (hereinafter, referred to as the present condensed heterocyclic compound 38) and 0.26 g of 2-(2-ethyl-sulfonyl-4-trifluoromethylphenyl)-3-methyl-6-trifluoromethyl-3H-imidazo[4,5-b]pyridine (hereinafter, referred to as the present condensed heterocyclic compound 39) were obtained based on the methods described in Production Examples 32 and 33 using 2-(2-ethylsulfanyl-4-trifluoromethyl-phenyl)-3-methyl-6-trifluoromethyl-3H-imidazo[4,5-b]pyridine instead of 2-(2-ethylsulfanyl-4-fluorophenyl)-3-methyl-6-trifluoromethyl-3H-imidazo[4,5-b]pyridine.

Present Condensed Heterocyclic Compound 38

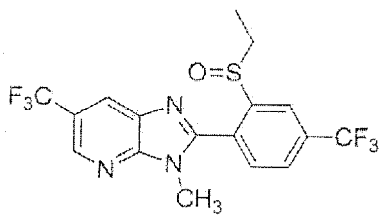

1H-NMR (CDC13) δ: 8.79-8.78 (1H, m), 8.57-8.55 (1H, m), 8.35-8.34 (1H, m), 7.97-7.94 (1H, m), 7.77 (1H, d), 3.94 (3H, s), 3.53-3.43 (1H, m), 3.07-2.98 (1H, m), 1.36 (3H, t)

Present Condensed Heterocyclic Compound 39

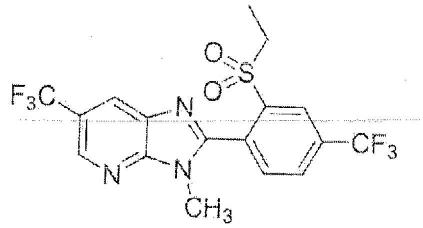

1H-NMR (CDCl3) δ: 8.78-8.76 (1H, m), 8.51-8.49 (1H, m), 8.31-8.30 (1H, m), 8.12-8.09 (1H, m), 7.74 (1H, d), 3.74 (3H, s), 3.48 (2H, q), 1.29 (3H, t)

Production Example 40

A mixture of 0.56 g of 3-amino-5-(trifluoromethyl)pyridine-2-thiol, 0.52 g of 2-ethylsulfanyl benzoic acid, 0.80 g of WSC, 39 mg of HOBt, and 6 ml of pyridine was stirred for 2 hours at 60°C. Water was added to the reaction mixture, which was allowed to cool, to subject the reaction mixture to extraction using ethyl acetate. An organic layer thereof was washed with water, and then, was dried using anhydrous magnesium sulfate and condensed under reduced pressure.

A mixture of the obtained residue, 0.65 g of p-toluenesulfonic acid monohydrate and 5 mL of N-methylpyrrolidinone was heated and stirred for 2 hours at 150°C. Water was added to the reaction mixture, which was allowed to cool, to subject the reaction mixture to extraction using ethyl acetate. An organic layer thereof was washed with water, and then, was dried using anhydrous magnesium sulfate and condensed under reduced pressure. The obtained residue was subjected to silicagel column chromatography to obtain 0.38 g of 2-(2-ethylsulfanylphenyl)-6-(trifluoromethyl)thiazolo[5,4-b]pyridine (hereinafter, referred to as the present condensed heterocyclic compound 40).

Present Condensed Heterocyclic Compound 40

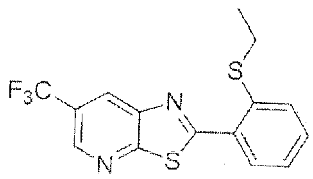

1H-NMR (CDCl3) δ: 8.86 (1H, d), 8.57 (1H, d), 8.03 (1H, dd), 7.55 (1H, dd), 7.48 (1H, td), 7.36 (1H, td), 2.98 (2H, q), 1.34 (3H, t).

Production Examples 41 and 42

0.13 g of 2-(2-ethyl-sulfinyl-phenyl)-6-(trifluoromethyl)thiazolo[5,4-b]pyridine (hereinafter, referred to as the present condensed heterocyclic compound 41) and 0.14 g of 2-(2-ethylsulfonylphenyl)-6-(trifluoromethyl)thiazolo[5,4-b]pyridine (hereinafter, referred to as the present condensed heterocyclic compound 42) were obtained based on the methods described in Production Examples 32 and 33 using 2-(2-ethylsulfanyl-phenyl)-6-(trifluoromethyl)thiazolo[5,4-b]pyridine instead of 2-(2-ethylsulfanyl-4-fluorophenyl)-3-methyl-6-trifluoromethyl-3H-imidazo[4,5-b]pyridine.

Present Condensed Heterocyclic Compound 41

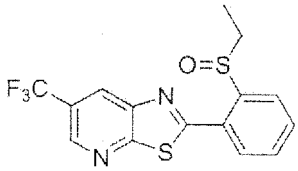

1H-NMR (CDC13) δ: 8.90 (1H, d), 8.49 (1H, d), 8.37 (1H, dd), 7.99 (1H, dd), 7.81 (1H, td), 7.67 (1H, td), 3.52-3.42 (1H, m), 3.01-2.92 (1H, m), 1.45 (3H, t).

Present Condensed Heterocyclic Compound 42

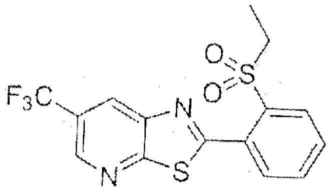

1H-NMR (CDCl3) δ: 8.92 (1H, d), 8.52 (1H, d), 8.25 (1H, dd), 7.84-7.71 (3H, m), 3.73 (2H, q), 1.37 (3H, t).

Production Examples 43 and 44

2-(2-ethylsulfonylphenyl)-6-trifluoromethylsulfanyl-3-methyl-3H-imidazo[4,5-b]pyridine (hereinafter, referred to as the present condensed heterocyclic compound 43) and 2-(2-ethylsulfinyl-phenyl)-6-trifluoromethylsulfanyl-3-methyl-3H-imidazo[4,5-b]pyridine (hereinafter, referred to as the present condensed heterocyclic compound 44) were obtained based on the methods described in Production Examples 32 and 33 using 2-(2-ethylsulfanyl-phenyl)-3-methyl-6-trifluoromethylsulfanyl-3H-imidazo[4,5-b]pyridine instead of 2-(2-ethylsulfanyl-4-fluorophenyl)-3-methyl-6-trifluoromethyl-3H-imidazo[4,5-b]pyridine.

Present Condensed Heterocyclic Compound 43

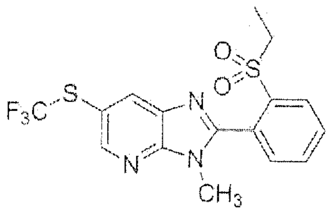

1H-NMR (CDCl3) δ: 8.68 (1H, d), 8.36 (1H, d), 8.21 (1H, dd), 7.87-7.77 (2H, m), 7.59 (1H, dd), 3.71 (3H, s), 3.44 (2H, q), 1.24 (3H, t)

Present Condensed Heterocyclic Compound 44

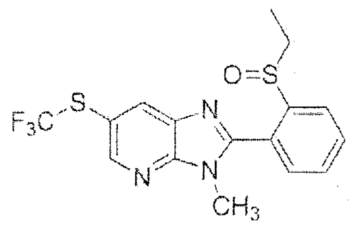

1H-NMR (CDC13) δ: 8.69 (1H, d), 8.38 (1H, d), 8.25 (1H, dd), 7.86-7.80 (1H, m), 7.72-7.67 (1H, m), 7.60 (1H, dd), 3.88 (3H, s), 3.43-3.31 (1H, m), 3.03-2.92 (1H, m), 1.31 (3H, t)

Production Examples 45 and 46

2-(2-ethylsulfonylphenyl)-6-trifluoromethyl-sulfinyl-3-methyl-3H-imidazo[4,5-b]pyridine (hereinafter, referred to as the present condensed heterocyclic compound 45) and 2-(2-ethylsulfonylphenyl)-6-trifluoromethylsulfonyl-3-methyl-3H-imidazo[4,5-b]pyridine (hereinafter, referred to as the present condensed heterocyclic compound 46) were obtained based on the methods described in Production Examples 32 and 33 using 2-(2-ethyl-sulfonylphenyl)-6-trifluoromethyl-sulfanyl-3-methyl-3H-imidazo[4,5-b]pyridine instead of 2-(2-ethylsulfanyl-4-fluorophenyl)-3-methyl-6-trifluoromethyl-3H-imidazo[4,5-b]pyridine.

Present Condensed Heterocyclic Compound 45

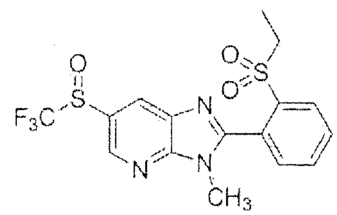

1H-NMR (CDCl3) δ: 8.77 (1H, d), 8.55 (1H, d), 8.24 (1H, dd), 7.90-7.83 (2H, m), 7.61 (1H, dd), 3.75 (3H, s), 3.43 (2H, q), 1.26 (3H, t)

Present Condensed Heterocyclic Compound 46

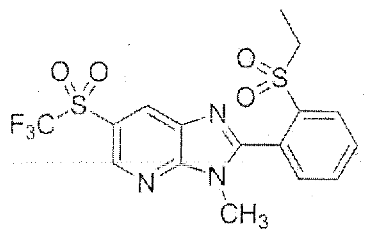

1H-NMR (CDCl3) δ: 9.05 (1H, d), 8.65 (1H, d), 8.26-8.23 (1H, m), 7.90-7.85 (2H, m), 7.61-7.57 (1H, m), 3.77 (3H, s), 3.41 (2H, q), 1.27 (3H, t)

Production Example 47

63 mg of sodium thiomethoxide was added to a mixture of 0.18 g of 2-[2-fluoro-4-(trifluoromethyl)phenyl]-6-(trifluoromethyl)thiazolo[5,4-b]pyridine and 2 ml of DMF under ice-cooling and the mixture was stirred for 4 hours at room temperature. Water was added to the reaction mixture, which was then subjected to extraction using ethyl acetate. An organic layer thereof was washed with water, and then, was dried using anhydrous magnesium sulfate and condensed

under reduced pressure. The obtained residue was subjected to silicagel column chromatography to obtain 0.11 g of 2-[2-ethyl-sulfanyl-4-[trifluoromethyl)phenyl]-6-(trifluoromethyl)thiazolo[5,4-b]pyridine (hereinafter, referred to as the present condensed heterocyclic compound 47).

Present Condensed Heterocyclic Compound 47

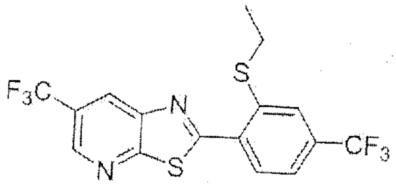

1H-NMR (CDCl3) δ: 8.90 (1H, d), 8.61 (1H, d), 8.14 (1H, d), 7.75 (1H, s), 7.58 (1H, d), 3.04 (2H, q), 1.38 (3H, t).

Production Example 48

0.13 g of 3-chloroperbenzoic acid (with a purity greater than or equal to 65%) was added to a mixture of 0.11 g of 2-[2-ethylsulfanyl-4-(trifluoromethyl)phenyl]-6-(trifluoromethyl)thiazolo[5,4-b]pyridine and 3 ml of chloroform, and then, the mixture was stirred for 12 hours at room temperature. The reaction mixture was diluted with chloroform and washed successively with a 10% aqueous sodium thiosulfate solution and a saturated aqueous sodium hydrogen carbonate solution, and then, was dried using anhydrous magnesium sulfate and condensed under reduced pressure. The obtained residue was subjected to silicagel column chromatography to obtain 0.11 g of 2-[2-ethylsulfonyl-4-(trifluoromethyl)phenyl]-6-(trifluoromethyl)thiazolo[5,4-b]pyridine (hereinafter, referred to as the present condensed heterocyclic compound 48).

Present condensed heterocyclic compound 48

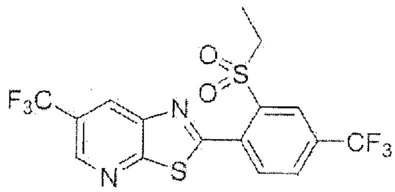

1H-NMR (CDC13) δ: 8.95 (1H, s), 8.55 (1H, s), 8.52 (1H, s), 8.07 (1H, d), 7.88 (1H, d), 3.77 (2H, q), 1.40 (3H, t).

Production Example 49

200 mg of potassium hydroxide was added to a mixture of 535 mg of 2-(2-ethylsulfanyl-phenyl)-3-methyl-3H-imidazo[4,5-b]pyridine-6-thiol, 166 μL of iodomethane, and 5 mL of ethanol, and the mixture was stirred for 5 hours at room temperature. A saturated aqueous ammonium chloride solution was added to the reaction mixture, which was then subjected to extraction using ethyl acetate. A combined organic layer was dried using sodium sulfate, and then, was condensed under reduced pressure. The residue was subjected to silicagel column chromatography to obtain 515 mg of 2-(2-ethylsulfanyl-phenyl)-3-methyl-6-methylsulfanyl-3H-imidazo[4,5-b]pyridine (hereinafter, referred to as the present condensed heterocyclic compound 49).

Present Condensed Heterocyclic Compound 49

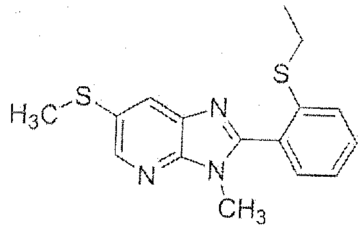

1H-NMR (CDCl3) δ: 8.46 (1H, d), 8.10 (1H, d), 7.52-7.42 (3H, m), 7.37-7.26 (1H, m), 3.73 (3H, s), 2.86 (2H, q), 2.54 (3H, s), 1.22 (3H, t)

Production Example 50

1.13 g of 69% to 75% 3-chloroperbenzoic acid was added to a mixture of 363 mg of 2-(2-ethylsulfanyl-phenyl)-3-methyl-6-methylsulfanyl-3H-imidazo[4,5-b]pyridine and 5 mL of chloroform under ice-cooling. The temperature of the mixture was elevated to room temperature and the mixture was stirred for 5 hours. Then, a saturated aqueous sodium hydrogen carbonate solution and a saturated aqueous sodium thiosulfate solution were added to the mixture, which was then subjected to extraction using chloroform. A combined organic layer was dried using sodium sulfate, and then, was condensed under reduced pressure. The residue was subjected to silicagel column chromatography to obtain 356 mg of 2-(2-ethylsulfonylphenyl)-6-methylsulfonyl-3-methyl-3H-imidazo[4,5-b]pyridine (hereinafter, referred to as the present condensed heterocyclic compound 50).

Present Condensed Heterocyclic Compound 50

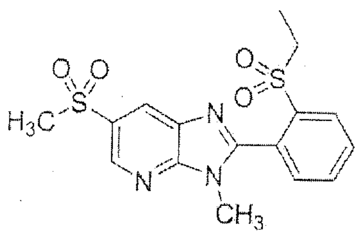

1H-NMR (CDC13) δ: 9.02 (1H, d), 8.58 (1H, d), 8.23 (1H, dd), 7.90-7.81 (2H, m), 7.59 (1H, dd), 3.74 (3H, s), 3.42 (2H, q), 3.19 (3H, s), 1.26 (3H, t).

Production Example 51

Compound 301A was obtained based on the method described in Production Example 103 using 2-(2-ethylsulfanyl-4-trifluoromethylphenyl)-6-iodo-3-methyl-3H-imidazo[4,5-b]pyridine instead of 2-(2-ethylsulfanyl-phenyl)-6-iodo-3-methyl-3H-imidazo[4,5-b]pyridine.

A mixture, of 0.94 g of the compound 301A and 13 ml of DMF was cooled to -50°C, and an excessive amount of CF3I gas was bubbled and was dissolved in DMF. 1.2 ml of tetrakis dimethylamino ethylene diamine was added dropwise to the mixture at a rate at which the internal temperature does not exceed -40°C. Thereafter, the temperature was elevated to -10°C over 1 hour and the mixture was stirred for 1 hour at -10°C. Water was added to the reaction mixture, and the temperature was elevated to room temperature, and then, the reaction mixture was subjected to extraction using ethyl acetate. A combined organic layer was dried using magnesium sulfate, and then, was condensed under reduced pressure. The obtained residue was subjected to silicagel column chromatography to obtain 0.68 g of 2-(2-ethyl-sulfanyl-4-trifluoromethylphenyl)-3-methyl-6-trifluoromethylsulfanyl-3H-imidazo[4,5-b]pyridine (hereinafter, referred to as the present condensed heterocyclic compound 51).

Present Condensed Heterocyclic Compound 51

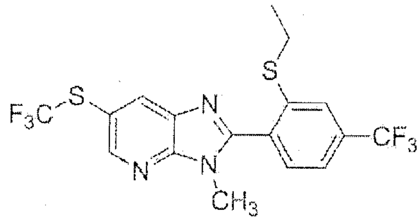

1H-NMR (CDCl3) δ: 8.66 (1H, d) 8.39 (1H, d), 7.67-7.64 (1H, m), 7.59-7.52 (2H, m), 3.75 (3H, s), 2.94 (2H, q), 1.27 (3H, t).

Production Example 52

1.05 g of 69% 3-chloroperbenzoic acid was added to a mixture of 2-(2-ethylsulfanyl-4-trifluoromethylphenyl)-3-methyl-6-trifluoromethylaulfanyl-3H-imidazo[4,5-b]pyridine and 5 ml of chloroform under ice-cooling, and then, the temperature was elevated to room temperature and the mixture was stirred for 1.5 hours. Thereafter, a saturated aqueous sodium hydrogen carbonate solution and a saturated aqueous sodium thiosulfate solution were added to the reaction mixture, which was then subjected to extraction using chloroform. A combined organic layer was dried using magnesium sulfate, and then, was condensed under reduced pressure. The obtained residue was subjected to silicagel column chromatography to obtain 0.20 g of 2-(2-ethyl-sulfonyl-4-trifluoromethyl-phenyl)-3-methyl-6-trifluoromethylsulfanyl-3H-imidazo[4,5-b]pyridine (hereinafter, referred to as the present condensed heterocyclic compound 52).

Present Condensed Heterocyclic Compound 52

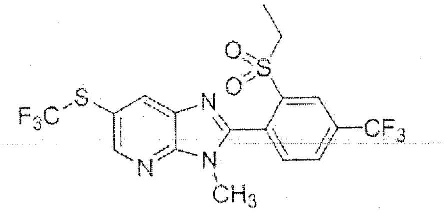

1H-NMR (CDCl3) δ: 8.71-8.70 (1H, m), 8.50-8.49 (1Hm), 8.38-8.36 (1H, m), 8.12-8.08 (1H, m), 7.74-7.71 (1H, m), 3.72 (3H, s), 3.49 (2H, q), 1.29 (3H, t).

Production Example 53

A mixture of 0.26 g of 2-(2-ethylsulfonyl-4-trifluoromethylphenyl)-3-methyl-6-trifluoromethylaulfinyl-3H-imidazo[4,5-b]pyridine, 36 mg of sodium tungstate dihydrate, 1 ml of a 30% hydrogen peroxide solution, and 5 ml of acetonitrile was stirred for 4.5 hours under heating and refluxing. Water was added to the reaction mixture, which was cooled to room temperature, to subject the reaction mixture to extraction using ethyl acetate. A combined organic layer was dried using magnesium sulfate, and then, was condensed under reduced pressure. The obtained residue was subjected to silicagel column chromatography to obtain 0.24 g of 2-(2-ethylsulfonyl-4-trifluoromethylphenyl)-3-methyl-6-trifluoromethyl-sulfonyl-3H-imidazo[4,5-b]pyridine (hereinafter, referred to as the present condensed heterocyclic compound 53).
Present Condensed Heterocyclic Compound 53

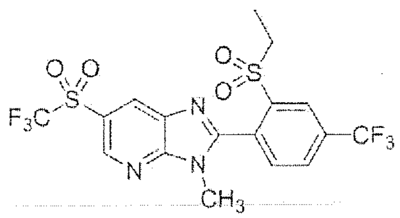

1H-NMR (CDC13) 8: 9.08-9.07 (1H, m), 8.68-8.66 (1H, m), 8.52-8.50 (1H, m), 8.16-8.12 (1H, m), 7.76-7.73 (1H, m), 3.78 (3H, s), 3.46 (2H, q), 1.30 (3H, t)

Production Example 54

422 mg of 1-ethyl-3-(3-dimethylaminopropyl)carbodiimide hydrochloride and 27 mg of 1-hydroxybenzotriazole were added to a mixture of 552 mg of 4-(1,2,2,2-tetrafluoro-1-trifluoromethyl-ethyl)-benzene-1,2-diamine, 401 mg of 2-ethylsulfanyl benzoic acid, and 27 mL of pyridine at room temperature. After stirring the mixture for 5 hours at room temperature, the reaction mixture was diluted with water and was subjected to extraction using ethyl acetate. A combined organic layer was dried using sodium sulfate, and then, was condensed under reduced pressure. The obtained residue was dissolved in a mixed solution of 7.5 mL of DMF and 30 mL of toluene and 837 mg of p-toluenesulfonic acid was added to the mixture at room temperature. The mixture was heated and stirred for 8 hours at 130°C, and was allowed to cool to room temperature. A saturated aqueous sodium hydrogen carbonate solution was added to the reaction mixture, which was then subjected to extraction using ethyl acetate. A combined organic layer was dried using sodium sulfate, and then, was condensed under reduced pressure. The residue was subjected to silicagel column chromatography to obtain 97 mg of 2-(2-ethylsulfanyl-phenyl)-5-(1,2,2,2-tetrafluoro-1-trifluoromethyl-ethyl)-1H-benzimidazole (hereinafter, referred to as the present condensed heterocyclic compound 54).

Present Condensed Heterocyclic Compound 54

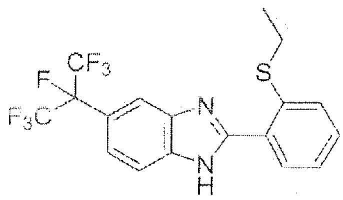

1H-NMR (CDC13) δ: 12.08-11.87 (1H, m), 8.31 (1H, s), 8.12-7.44 (4H, m), 7.42-7.30 (2H, m), 2.86 (2H, q), 1.22 (3H, t).

Production Example 55

2.08 g of a DMF solution of 2-(4-bromo-2-fluorophenyl)-3-methyl-6-trifluoromethyl-3H-imidazo[4,5-b]pyridine was added dropwise to a mixture of 0.63 g of ethyl mercaptan sodium salt (80%) and 10 ml of DMF under ice-cooling, and then, the temperature was elevated to room temperature and the mixture was stirred for 30 minutes. A saturated aqueous sodium hydrogen carbonate solution was added to the reaction mixture, which was then subjected to extraction using ethyl acetate. A combined organic layer was dried using magnesium sulfate, and then, was condensed under reduced pressure. The obtained residue was subjected to silicagel column chromatography to obtain 1.57 g of 2-(4-bromo-2-ethylsulfanylphenyl)-3-methyl-6-trifluoromethyl-3H-imidazo[4,5-b]pyridine (hereinafter, referred to as the present condensed heterocyclic compound 55).

Present Condensed Heterocyclic Compound 55

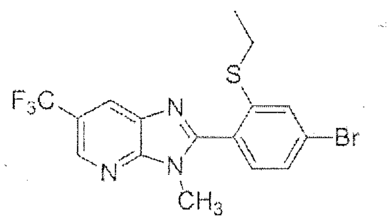

1H-NMR (CDCl3) δ: 8.73-8.71 (1H, m), 8.33-8.32 (1H, m), 7.59 (1H, d), 7.50-7.47 (1H, m), 7.30 (1H, d), 3.77 (3H, s), 2.91 (2H, q), 1.27 (3H, t).

Production Examples 56 and 57

0.29 g of 3-chloroperbenzoic acid (with a purity greater than or equal to 65%) was added to a mixture of 0.40

g of 2-(4-bromo-2-ethylsulfanyl-phenyl)-3-methyl-6-trifluoromethyl-3H-imidazo[4,5-b]pyridine and 5 ml of chloroform under ice-cooling, and then, the temperature was elevated to room temperature and the mixture was stirred for 2 hours. A saturated aqueous sodium hydrogen carbonate solution and a saturated aqueous sodium thiosulfate solution were added to the reaction mixture, which was then subjected to extraction using chloroform. A combined organic layer was dried using magnesium sulfate, and then, was condensed under reduced pressure. The obtained residue was subjected to silicagel column chromatography to obtain 0.26 g of 2-(4-bromo-2-ethylsulfinylphenyl)-3-methyl-6-trifluoromethyl-3H-imidazo[4,5-b]pyridine (hereinafter, referred to as the present condensed heterocyclic compound 56) and 0.17 g of 2-(4-bromo-2-ethylsulfonylphenyl)-3-methyl-6-trifluoromethyl-3H-imidazo[4,5-b]pyridine (hereinafter, referred to as the present condensed heterocyclic compound 57).

Present Condensed Heterocyclic Compound 56

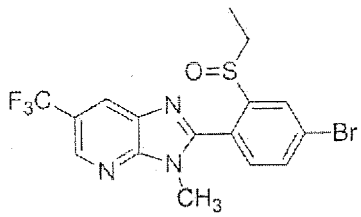

1H-NMR (CDC13) δ: 8.77-8.75 (1H, m), 8.39 (1H, d), 8.32-8.31 (1H, m), 7.84-7.81 (1H, m), 7.49 (1H, d), 3.91 (3H, s), 3.50-3.40 (1H, m), 3.06-2.96 (1H, m), 1.35 (3H, t). Present Condensed Heterocyclic Compound 57

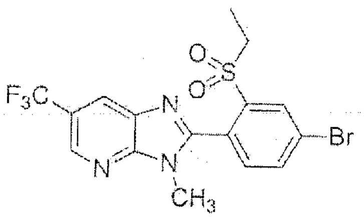

1H-NMR (CDC13) δ: 8.77-8.75 (1H, m), 8.37 (1H, d), 8.31-8.29 (1H, m), 7.99-7.96 (1H, m), 7.44 (1H, d), 3.72 (3H, s), 3.44 (2H, q), 1.28 (3H, t).

Production Example 58

A mixture of 0.20 g of 2-(4-bromo-2-ethylsulfonylphenyl)-3-methyl-6-trifluoromethyl-3H-imidazo[4,5-b]pyridine, 0.17 g of 2-tributylstannyl pyrimidine, 27 mg of tetrakistriphenylphosphinepalladium, and 5 ml of toluene was heated and refluxed for 5.5 hours under a nitrogen atmosphere. After cooling the mixture to room temperature, 0.17 g of 2-tributylstannyl pyrimidine, and 27 mg of tetrakistriphenylphosphinepalladium were added to the mixture, which was then stirred for 8 hours under heating and refluxing. The temperature was cooled to room temperature, and then, water was added to the mixture, which was then subjected to extraction using ethyl acetate. A combined organic layer was dried using magnesium sulfate, and then, was condensed under reduced pressure. The obtained residue was subjected to silicagel column chromatography to obtain 0.20 g of 2-[2-ethylsulfonyl-4-(pyrimidin-2-yl)-phenyl]-3-methyl-6-trifluoromethyl-3H-imidazo[d,5-b]pyridine (hereinafter, referred to as the present condensed heterocyclic compound 58).

Present Condensed Heterocyclic Compound 58

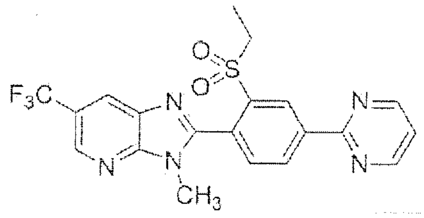

1H-NMR (CDC13) δ: 8.77-8.74 (1H, m), 8.38-8.36 (1H, m), 8.30-8.27 (1H, m), 8.00-7.95 (1H, m), 7.63-7.55 (1H, m), 7.43 (1H, d), 7.41-7.30 (2H, m), 3.72 (3H, s), 3.44 (2H, q), 1.28 (3H, t).

Production Example 59-1

71 g of N-bromosuccinimide was added to a mixture of 65 g of 5-trifluoromethyl-pyridine-2-ylamine and 100 mL of chloroform by being divided into 5 portions under ice water-cooling, The temperature was elevated to room temperature and the mixture was stirred for 1 hour. Then, the mixture was heated to 80°C and was heated and stirred for 3C minutes. After allowing the mixture to cool to room temperature, a saturated aqueous sodium thiosulfate solution and a saturated aqueous sodium hydrogen carbonate solution were added to the mixture, which was then subjected to extraction using chloroform. A combined organic layer was dried using sodium sulfate, and then, was condensed under reduced pressure. The residue was subjected to silicagel column chromatography to obtain 96 g of 3-bromo-5-trifluoromethyl-pyridin-2-ylamine. 3-bromo-5-trifluoromethyl-pyridin-2-ylamine

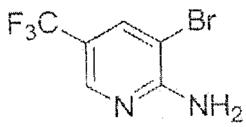

1H-NMR (CDCl3) δ: 8.27 (1H, d), 7.86 (1H, d), 5.38 (2H, brs).

Production Example 59-2

40 g of 3-bromo-5-trifluoromethyl-pyridin-2-ylamine, 2.2 g of copper (II) acetylacetone, 6.6 g of acetylacetone, 59 g cf cesium carbonate, and 105 mL of NMP were added to an autoclave reactor, and 25 mL of a 28% ammonia water solution was added thereto under ice-cooling. After sealing the reactor, the temperature was elevated to 110°C and the mixture was heated and stirred for 12 hours. After ice-cooling to room temperature, the reaction mixture was diluted with water and was subjected to extraction using ethyl acetate. A combined organic layer was dried using sodium sulfate, and then, was condensed under reduced pressure. The residue was subjected to silicagel column chromatography to obtain 15 g of 5-trifluoromethyl-pyridine-2,3-diamine. 5-trifluoromethyl-pyridine-2,3-diamine

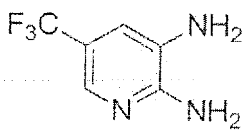

1H-NMR (CDC13) δ: 7.93 (1H, d), 7.04 (1H, d), 4.71 (2H, brs), 3.46 (2H, brs).

Present Example 59-3

7.35 g of sodium ethane thiolate (90%) was added to a mixture of 15.1 g of 2-fluoro-4-trifluoromethyl benzaldehyde and 61 ml of DMF under ice-cooling and the mixture was stirred for 6 hours at room temperature. The reaction mixture was added to water and was subjected to extraction using ethyl acetate. A combined organic layer was dried using magnesium sulfate, and then, was condensed under reduced pressure. The residue was subjected to silicagel column chromatography to obtain 11.8 g of 2-formyl-5-trifluoromethyl-phenyl ethyl sulfide.

2-formyl-5-trifluoromethylphenyl ethyl sulfide

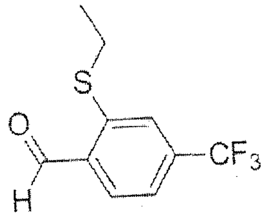

1H-NMR (CDC13) δ: 10.41 (1H, s), 7.94 (1H, d), 7.63 (1H, s), 7.53 (1H, d), 3.04 (2H, q), 1.41 (3H, t).

Production Example 59-4

6.1 g of sodium hydrogen sulfite was added to a mixture of 8.6 g of 5-trifluoromethyl-pyridine-2,3-diamine, 11 g of 2-formyl-5-trifluoromethylphenyl ethyl sulfide, and 67 mL of DMF at room temperature. After heating and stirring the mixture for 3 hours at 100°C. 1 g of copper (II) chloride dihydrate was added thereto, and the mixture was heated and stirred for 1 hour at 100°C. After allowing the mixture to cool to room temperature, the reaction mixture was added to water and was subjected to extraction using ethyl acetate. A combined organic layer was dried using sodium sulfate, and then, was condensed under reduced pressure. The residue was subjected to silicagel column chromatography to obtain yellow solid powder. The powder was washed with hexane to obtain 12 g of 2-(2-ethylsulfanyl-4-trifluoromethyl-phenyl)-6-trifluoromethyl-3H-imidazo[4,5-b]pyridine (hereinafter, referred to as the present condensed heterocyclic compound 59).

Present Condensed Heterocyclic Compound 59

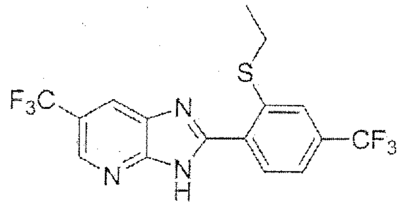

1H-NMR (CDC13) δ: 12.79 (1H, brs), 8.72 (1H, brs), 8.49-8.34 (2H, m), 7.79 (1H, s), 7.64 (1H, d), 3.00 (2H, q), 1.31 (3H, t).

Production Example 60

8.0 g of 69% to 75% 3-chloroperbenzoic acid was added to a mixture of 12 g of 2-(2-ethylsulfanyl-4-trifluoromethyl-phenyl)-6-trifluoromethyl-3H-imidazo[4,5-b]pyridine and 111 mL of chloroform under ice-cooling. The temperature of the mixture was elevated to room temperature and the mixture was stirred for 0.5 hours. Then, a saturated aqueous sodium hydrogen carbonate solution and a saturated aqueous sodium thiosulfate solution were added to the mixture, which was then subjected to extraction using chloroform. A combined organic layer was dried using magnesium sulfate, and then, was condensed under reduced pressure. The residue was subjected to silicagel column chromatography to obtain 9.1 g of 2-(2-ethylsulfonyl-4-trifluoromethyl-phenyl)-6-trifluoromethyl-3H-imidazo[4,5-b]pyridine (hereinafter, referred to as the present condensed heterocyclic compound 60).

Present Condensed Heterocyclic Compound 60

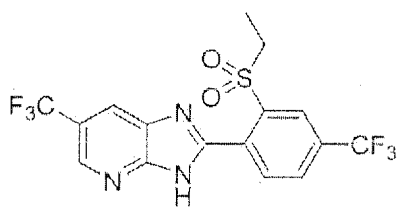

1H-NMR (DMSO-D6) δ: 14.15 (1H, brs), 8.83 (1H, s), 8.58 (1H, a), 8.41 (1H, d), 8.37 (1H, s), 8.19 (1H, d), 3.97 (2H, q), 1.23 (3H, t).

Production Example 61

A mixture of 200 mg of N-(2-amino-5-trifluoromethylpyridin-3-yl)-2-ethylsulfanyl-benzamide, 1 ml of tert-butyl alcohol, and 9 ml of THF was heated and stirred at 80°C, and 56 mg of 60% oil-based sodium hydride was added thereto. After heating and stirring the mixture for 2 hours at 80°C, 56 mg of 60% oil-based sodium hydride was added thereto. After further heating and stirring the mixture for 2 hours at the same temperature, 56 mg of 60% oil-based sodium hydride was added thereto and the mixture was heated and stirred for 2 hours at the same temperature. After cooling the reaction mixture to room temperature, a solvent was distilled, a saturated aqueous ammonium chloride solution was added to the distilled solvent to subject the distilled solvent to extraction using ethyl acetate. An organic layer thereof was dried using sodium sulfate, and then, was condensed under reduced pressure. The obtained residue was subjected to silicagel column chromatography to obtain 132 mg of 2-(2-ethylsulfanylphenyl)-6-trifluoromethyl-3H-imidazo[4,5-b]pyridine (hereinafter, referred to as the present condensed heterocyclic compound 61).

Present Condensed Heterocyclic Compound 61

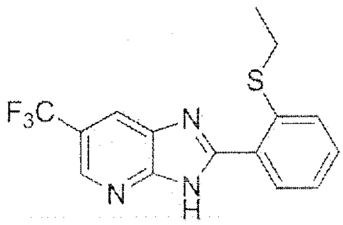

1H-NMR (CDC13) δ: 8.67 (1H, d), 8.51-8.48 (1H, m), 8.33 (1H, d), 7.66-7.61 (1H, m), 7.51-7.46 (2H, m), 2.93 (2H, q), 1.27 (3H, t)

Production Example 62

8.0 g of 69% to 75% 3-chloroperbenzoic acid was added to a mixture of 2.28 g of 2-(2-ethylsulfanylphenyl)-6-trifluoromethyl-3H-imidazo[4,5-b]pyridine and 20 ml of chloroform under ice-cooling. The temperature of the mixture was elevated to room temperature and the mixture was stirred for 0.5 hours. Then, a saturated aqueous sodium hydrogen carbonate solution and a saturated aqueous sodium thiosulfate solution were added to the mixture, which was then subjected to extraction using chloroform. A combined organic layer was dried using magnesium sulfate, and then, was condensed under reduced pressure. The residue was subjected to silicagel column chromatography and the obtained crystals were washed with hexane to obtain 2.5 g of 2-(2-ethylsulfonyl-phenyl)-6-trifluoromethyl-3H-imidazo[4,5-b]pyridine (hereinafter, referred to as the present condensed heterocyclic compound 62).

Present Condensed Heterocyclic Compound 62

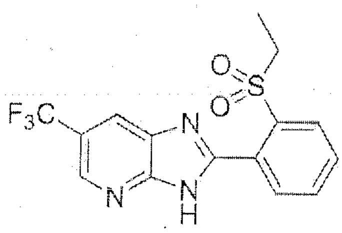

1H-NMR (CDC13) δ: 8.63 (1H, s), 8.35 (1H, s), 8.24 (1H, d), 8.09 (1H, d), 7.83 (1H, t), 7.76 (1H, t), 3.33 (2H, q), 0.88 (3H, t).

Production Examples 63 and 64

1.02 g of 2-(2-fluoro-4-trifluoromethoxyphenyl)-3-methyl-6-trifluoromethyl-3H-imidazo[4,5-b]pyridine was added to a mixture of 0.31 g of ethyl mercaptan sodium salt (80%) and 9 ml of DMF under ice-cooling, and then, the temperature was elevated to room temperature and the mixture was stirred for 2 hours. A saturated aqueous sodium hydrogen carbonate solution was added to the reaction mixture, which was then subjected to extraction using ethyl acetate. An organic layer thereof was dried using magnesium sulfate, and then, was condensed under reduced pressure. The obtained residue was subjected to silicagel column chromatography to obtain 0.82 g of a mixture (about 3:1) of 2-(2-ethylsulfanyl-4-trifluoromethoxyphenyl)-3-methyl-6-trifluoromethyl-3H-imidazo[4,5-b]pyridine and 2-(2-fluoro-4-trifluoromethoxyphenyl)-3-methyl-6-trifluoromethyl-3H-imidazo[4,5-b]pyridine.

0.45 g of 3-chloroperbenzoic acid (with a purity greater than or equal to 65%) was added to a mixture of the obtained mixture and 4 ml of chloroform under ice-cooling, and then, the temperature was elevated to room temperature and the mixture was stirred for 2 hours. A saturated aqueous sodium hydrogen carbonate solution and a saturated aqueous sodium thiosulfate solution were added to the reaction mixture, which was then subjected to extraction using chloroform. A combined organic layer was dried using magnesium sulfate, and then, was condensed under reduced pressure. The obtained residue was subjected to silicagel column chromatography to obtain 0.47 g of 2-(2-ethylsulfinyl-4-trifluoromethoxyphenyl)-3-methyl-6-trifluoromethyl-3H-imidazo[4,5-b]pyridine (hereinafter, referred to as the present condensed heterocyclic compound 63) and 0.14 g of 2-(2-ethanesulfonyl-4-trifluoromethoxyphenyl)-3-methyl-6-trifluoromethyl-3H-imidazo[4,5-b]pyridine (hereinafter, referred to as the present condensed heterocyclic compound 64).

Present Condensed Heterocyclic Compound 63

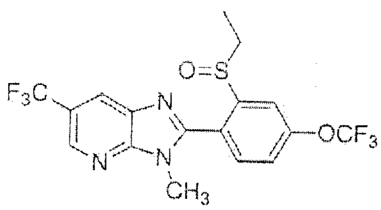

1H-NMR (CDC13) δ: 8.78-8.76 (1H, m), 8.33-8.31 (1H, m), 8.14-8.12 (1H, m), 7.68 (1H, d), 7.54-7.50 (1H, m), 3.93 (3H, s), 3.49-3.39 (1H, m), 3.06-2.96 (1H, m), 1.33 (3H, t).

Present Condensed Heterocyclic Compound 64

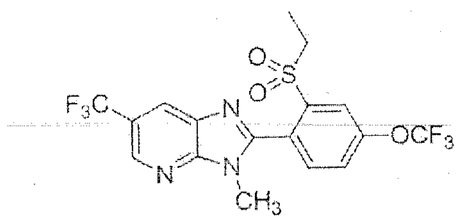

1H-NMR (CDC13) δ: 8.77-8.75 (1H, m), 8.30-8.28 (1H, m), 8.09-8.07 (1H, m), 7.70-7.66 (1H, m), 7.63 (1H, d), 3.74 (3H, s), 3.46 (2H, q), 1.28 (3H, t).

Production Example 65

A mixture of 0.34 g of 2-(2-ethyl-sulfinyl-4-trifluoromethoxyphenyl)-3-methyl-6-trifluoromethyl-3H-imidazo[4,5-b]pyridine, 0.36 g of zirconium chloride, 0.47 g of sodium iodide, and 8 ml of acetonitrile was stirred for 2 hours under heating and refluxing. After cooling the mixture to room temperature, a saturated aqueous sodium hydrogen carbonate solution was added to the reaction mixture, which was then subjected to extraction using ethyl acetate. An organic layer thereof was dried using magnesium sulfate, and then, was condensed under reduced pressure. The obtained residue was subjected to silicagel column chromatography to obtain 0.29 g of 2-(2-ethylsulfanyl-4-trifluoromethoxyphenyl)-3-methyl-6-trifluoromethyl-3H-imidazo[4,5-b]pyridine (hereinafter, referred to as the present condensed heterocyclic compound 65).

Present Condensed Heterocyclic Compound 65

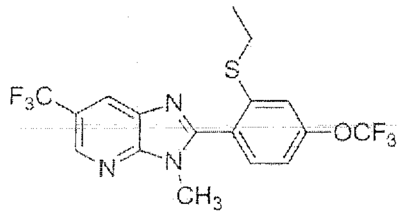

1H-NMR (CDCl3) δ: 8.74-8.72 (1H, m), 8.33-8.32 (1H, m), 7.48 (1H, d), 7.29-7.27 (1H, m), 7.21-7.17 (1H, m), 3.79 (3H, s), 2.92 (2H, q), 1.29 (3H, t).

Production Example 66-1

A mixture of 0.50 g of 2-amino-4-trifluoromethylphenol, 0.71 g of 2-ethylsulfanyl-4-trifluoromethyl benzoic acid, 0.65 g of WSC, and 6 ml of chloroform was stirred for 3.5 hours at room temperature. A saturated aqueous ammonium chloride solution was added to the reaction mixture, which was then subjected to extraction using chloroform. An organic layer thereof was washed with a saturated aqueous sodium hydrogen carbonate solution and saturated brine, and then, was dried using anhydrous sodium sulfate and condensed under reduced pressure. The obtained residue was subjected to silicagel column chromatography to obtain 0.57 g of 2-ethylsulfanyl-4-trifluoromethyl-N-[2-hydroxy-5-trifluoromethylphenyl]benzamide.

2-ethylsulfanyl-4-trifluoromethyl-N-[2-hydroxy-5-trifluoromethylphenyl]benzamide

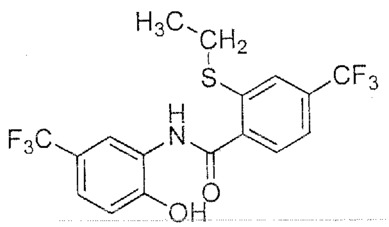

$^1$H-NMR ($CDCl_3$) δ: 9.33 (1H, brs), 8.87 (1H, s), 8.03 (1H, d), 7.75 (1H, s), 7.62 (1H, d), 7.57 (1H, s), 7.44 (1H, d), 7.14 (1H, d), 3.05 (2H, q), 1.36 (3H, t)

Production Example 66-2

A mixture of 0.56 g of 2-ethylsulfanyl-4-trifluoromethyl-N-[2-hydroxy-5-trifluoromethylphenyl]benzamide, 0.40 g of di-2-methoxyethyl azodicarboxylate (hereinafter, referred to as DMEAD), 0.39 g of triphenylphosphine, and 15 ml of THF was stirred for 30 minutes at room temperature and for 1 hour at 50°C. After condensing the reaction mixture, which was allowed to cool to room temperature, under reduced pressure, water was added to the reaction mixture, which was then subjected to extraction using ethyl acetate. An organic layer thereof was washed with saturated brine, and then, was dried using anhydrous sodium sulfate and condensed under reduced pressure. The obtained residue was subjected to silicagel column chromatography to obtain 0.52 g of 2-(2-ethylsulfanyl-4-trifluoromethylphenyl)-5-trifluoromethylbenzoxazole.

2-(2-ethylsulfanyl-4-trifluoromethylphenyl)-5-trifluoromethylbenzoxazole

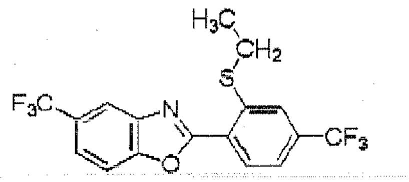

$^1$H-NMR (CDCl3) δ: 8.29 (1H, d), 8.19 (1H, d), 7.75-7.66 (2H, m), 7.63 (1H s), 7.51 (1H, dd), 3.10 (2H, q), 1.47 (3H, t).

Production Example 66-3

0.46 g of m-chloroperbenzoic acid (with a purity greater than or equal to 65%) was added to a mixture of 0.35 g of 2-(2-ethylsulfanyl-4-trifluoromethylphenyl)-5-trifluoromethylbenzoxazole and 10 ml of chloroform under ice-cooling, and then, the mixture was stirred for 1.5 hours at room temperature. A 10% aqueous sodium sulfite solution was added to the reaction mixture, which was then subjected to extraction using chloroform. An organic layer thereof was washed with a saturated aqueous sodium hydrogen carbonate solution, and then, was dried using anhydrous sodium sulfate and condensed under reduced pressure. The obtained residue was subjected to silicagel column chromatography to obtain 0.34 g of 2-(2-ethylsulfonyl-4-trifluoromethylphenyl)-5-trifluoromethylbenzoxazole (hereinafter, referred to as the present condensed heterocyclic compound 130).

Present Condensed Heterocyclic Compound 130

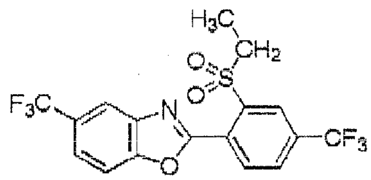

$^1$H-NMR ($CDCl_3$) δ: 8.54 (1H, s), 8.18-8.12 (2H, m), 8.08 (1H, dd), 7.77-7.74 (2H, m), 3.90 (2H, q), 1.44 (3H, t). Production Example 67-1

1.1 ml of oxalyl chloride was added to a mixture of 1.2 g of 2-ethylsulfanyl benzoic acid, 10 ml of chloroform, and 0.1 ml of DMF, and the mixture was stirred for 1 hour at room temperature. The reaction mixture was condensed under reduced pressure and 10 ml of THF was added to the reaction mixture. The reaction mixture was added to a mixture of 1.38 g of 2-amino-4-(trifluoromethylsulfanyl)phenol and 15 ml of THF under ice-cooling, and was stirred for 2 hours at room temperature. Water was added to the reaction mixture, which was then subjected to extraction using chloroform. An organic layer thereof was washed with a saturated aqueous sodium hydrogen carbonate solution and saturated brine, and then, was dried using anhydrous sodium sulfate and condensed under reduced pressure, thereby obtaining 1.78 g of 2-ethylsulfanyl-N-[2-hydroxy-5-(trifluoromethylsulfanyl)phenyl]benzamide.

2-ethylsulfanyl-N-[2-hydroxy-5-(trifluoromethylsulfanyl)phenyl]benzamide

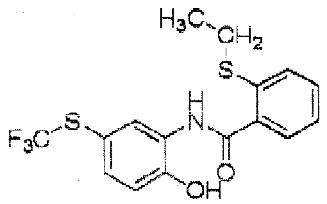

$^1$H-NMR ($CDCl_3$) δ: 9.89 (1H, s), 9.71 (1H, s), 8.05 (1H, dd), 7.58 (1H, dd), 7.51 (1H, ddd), 7.48-7.41 (3H, m), 7.12 (1H, d), 2.99 (2H, q), 1.31 (3H, t).

Production Example 67-2

A mixture of 1.78 g of 2-ethylsulfanyl-N-[2-hydroxy-5-(trifluoromethylsulfanyl)phenyl]benzamide, 1.79 g of DMEAD, 1.88 g of triphenylphosphine, and 20 ml of THF was stirred for 30 minutes at room temperature and for 1 hour at 50°C. After condensing the reaction mixture, which was allowed to cool to room temperature, under reduced pressure, water was added to the reaction mixture, which was then subjected to extraction using ethyl acetate. An organic layer thereof was washed with saturated brine, and then, was dried using anhydrous sodium sulfate and condensed under reduced pressure. The obtained residue was subjected to silicagel column chromatography to obtain 1.33 g of 2-(2-ethyl-sulfanylphenyl)-5(trifluoromethylsulfanyl)benzoxazole.

2-(2-ethyl-sulfanylphenyl)-5(trifluoromethylsulfanyl)benzoxazole

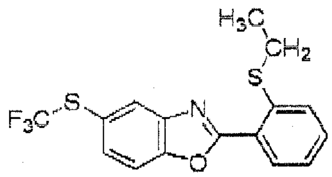

$^1$H-NMR (CDCl3) δ: 8.21 (1H, d), 8.17 (1H, dd), 7.67 (1H, dd), 7.54 (1H, d), 7.52-7.46 (1H, m), 7.43 (1H, dd), 7.31-7.27 (1H, m), 3.07 (2H, q), 1.45 (3H, t).

Production Example 67-3

2.04 g of of m-chloroperbenzoic acid (with a purity greater than or equal to 65%) was added to a mixture of 1.15 g of 2-(2-ethylsulfanylphenyl)-5-(trifluoromethylsulfanyl)benzoxazole and 25 ml of chloroform under ice-cooling, and then, the mixture was stirred for' 1.5 hour at room temperature. A 10% aqueous sodium sulfite solution was added to the reaction mixture, which was then subjected to extraction using chloroform. An organic layer thereof was washed with saturated aqueous sodium hydrogen carbonate solution, and then, was dried using anhydrous sodium sulfate and condensed under reduced pressure. The obtained residue was subjected to silicagel column chromatography to obtain 0.38 g of 2-(2-ethylsulfonylphenyl)-5-(trifluoromethylsulfanyl) benzoxazole and 0.55 g of 2-(2-ethylsulfonylphenyl)-5-(trifluoromethylsulfinyl) benzoxazole (hereinafter, referred to as the present condensed heterocyclic compound 131).

2-(2-ethylsulfonylphenyl)-5-(trifluoromethylsulfanyl) benzoxazole

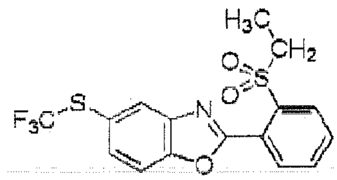

$^1$H-NMR ($CDCl_3$) δ: 8.28-8.24 (1H, m), 8.16-8.13 (1H, m), 7.99-7.95 (1H, m), 7.85-7.76 (2H, m), 7.73 (1H, dd), 7.66 (1H, d), 3.83 (2H, q), 1.40 (3H, t).

Present Condensed Heterocyclic Compound 131

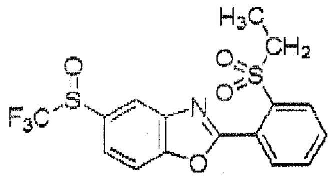

1H-NMR (CDCl3) δ: 8.30-8.25 (2H, m), 8.01-7.97 (1H, m), 7.87-7.79 (4H, m), 3.82 (2H, q), 1.41 (3H, t).

Hereinafter, 1H-NMR data of the present condensed heterocyclic compounds described in Tables 3 and 4 will be shown.

Present Condensed Heterocyclic Compound 66

1H-NMR (CDC13) δ: 8.51 (1H, a), 8.22 (1H, s), 7.55-7.50 (2H, m), 7.47-7.43 (1H, m), 7.38-7.32 (1H, m), 5.81 (1H, dq), 3.76 (3H, s), 2.88 (2H, q), 1.24 (3H, t).

Present Condensed Heterocyclic Compound 67

1H-NMR (CDC13) δ: 8.55 (1H, d), 8.26 (1H, d), 8.21 (1H, d), 7.83 (1H, t), 7.69 (1H, t), 7.60 (1H, d), 5.84 (1H, dq), 3.88 (3H, s), 3.42-3.30 (1H, m), 3.03-2.91 (1H, m), 1.33-1.25 (3H, m).

Present Condensed Heterocyclic Compound 68

1H-NMR (CDC13) δ: 8.53 (1H, s), 8.26-8.16 (2H, m), 7.88-7.78 (2H, m), 7.59-7.53 (1H, m), 5.82 (1H, dq), 3.70 (3H, s), 3.44 (2H, q), 1.26 (3H, t).

Present Condensed Heterocyclic Compound 69

1H-NMR (CDCl3) δ: 8.68 (1H, d), 8.29 (1H, d), 7.74-7.68 (1H, m), 7.49-7.40 (2H, m), 3.73 (3H, s), 2.74 (2H, q), 1.06 (3H, t).

Present Condensed Heterocyclic Compound 70

1H-NMR (CDC13) δ: 8.64 (1H, a), 8.24 (1H, s), 7.65 (1H, d), 7.58 (1H, t), 7.39 (1H, d), 3.72 (3H, a), 3.63-3.47 (1H, m), 3.37-3.22 (1H, m), 1.38-1.30 (3H, m).

Present Condensed Heterocyclic Compound 71

1H-NMR (CDCl3) δ: 8.74-8.72 (1H, m), 8.33-8.32 (1H, m), 7.52-7.49 (1H, m), 7.47-7.43 (2H, m), 3.79 (3H, s), 2.85 (2H, q), 1.23 (3H, t).

Present Condensed Heterocyclic Compound 72

1H-NMR (CDC13) δ: 8.78-8.76 (1H, m), 8.33-8.31 (1H, m), 8.20 (1H, d), 7.81-7.78 (1H, m), 7.60 (1H, d), 3.93 (3H, s), 3.42-3.32 (1H, m), 3.02-2.92(1H, m), 1.31 (3H, t).

Present Condensed Heterocyclic Compound 73

1H-NMR (CDC13) δ: 8.76-8.75 (1H, m), 8.29-8.28 (1H, m), 8.16 (1H, d), 7.80-7.77 (1H, m), 7.57 (1H, d), 3.75 (3H, s), 3.40 (2H, q), 1.26 (3H, t).

Present Condensed Heterocyclic Compound 74

1H-NMR (CDC13) δ: 8.74-8.72 (1H, m), 8.33-8.31 (1H, m), 7.51-7.45 (1H, m), 7.36-7.31 (2H, m), 3.75 (3H, s), 2.78 (2H, q), 1.10 (3H, t).

Present Condensed Heterocyclic Compound 75

1H-NMR (CDC13) δ: 8.73-8.71 (1H, m), 8.29-8.27 (1H, m), 7.70-7.64 (1H, m), 7.44-7.38 (1H, m), 7.33 (1H, d), 3.81 (3H, s), 3.53-3.43 (1H, m), 3.42-3.31 (1H, m), 1.33 (3H, t).

Present Condensed Heterocyclic Compound 76

1H-NMR (CDC13) δ: 8.73-8.72 (1H, m), 8.28-8.26 (1H, m), 7.84-7.78 (1H, m), 7.55-7.49 (1H, m), 7.38-7.35 (1H, m), 3.77 (3H, s), 3.50-3.34 (2H, m), 1.34 (3H, t).

Present Condensed Heterocyclic Compound 77

1H-NMR (CDCl3) δ: 8.74-8.72 (1H, m), 8.37-8.35 (1H, m), 7.54-7.48 (1H, m), 7.27-7.24 (1H, m), 7.09-7.03 (1H, m), 3.77 (3H, d), 2.93 (2H, q), 1.28 (3H, t).

Present Condensed Heterocyclic Compound 78

1H-NMR (CDC13) δ: 8.79-8.76 (1H, m), 8.35-8.32 (1H, m), 8.03 (1H, d), 7.87-7.80 (1H, m), 7.43 (1H, t), 3.84 (3H, d), 3.51-3.40 (1H, m), 3.13-3.02 (1H, m), 1.33 (3H, t).

Present Condensed Heterocyclic Compound 79

$^1$H-NMR (CDC13) δ: 8.77-8.75 (1H, m), 8.32-8.30 (1H, m), 8.07-8.04 (1H, m), 7.86-7.80 (1H, m), 7.62-7.57 (1H, m), 3.76 (3H, a), 3.69-3.58 (1H, m), 3.41-3.31 (1H, m), 1.29 (3H, t).

Present Condensed Heterocyclic Compound 80

1H-NMR (CDC13) δ: 8.71-8.69 (1H, m), 8.31-8.30 (1H, m), 7.35-7.31 (2H, m), 7.19-7.15 (1H, m, 3.76 (3H, s), 2.85 (2H, q), 2.46 (3H, s), 1.22 (3H, t).

Present Condensed Heterocyclic Compound 81

1H-NMR (CDC13) δ: 8.74-8.73 (1H, m), 8.30-8.28 (1H, m), 8.06-8.05 (1H, m), 7.49-7.48 (2H, m), 3.88 (3H, s), 3.42-3.32 (1H, m), 3.00-2.90 (1H, m), 2.58 (3H, s), 1.32 (3H, t).

Present Condensed Heterocyclic Compound 82

1H-NMR (CDC13) δ: 8.74-8.72 (1H, m), 8.27-8.26 (1H, m), 8.03-8.02 (1H, m), 7.64-7.61 (1H, m), 7.44 (1H, d), 3.70 (3H, s), 3.41 (2H, q), 2.59 (3H, s), 1.26 (3H, t).

Present Condensed Heterocyclic Compound 83

$^1$H-NMR (CDC13) δ: 8.75-8.74 (1H, m), 8.35-8.34 (1H, m), 7.66-7.65 (1H, m), 7.61-7.55 (2H, m), 3.80 (3H, s), 2.95 (2H, q), 1.28 (3H, t).

Present Condensed Heterocyclic Compound 84

1H-NMR (CDC13) δ: 8.80-8.78 (1H, m), 8.54-8.53 (1H, m), 8.35-8.34 (1H, m), 7.95-7.91 (1H, m), 7.79 (1H, d), 3.95 (3H, s), 3.52-3.41 (1H, m), 3.09-2.99 (1H, m), 1.33 (3H, t).

Present Condensed Heterocyclic Compound 85

1H-NMR (CDC13) δ: 8.78-8.76 (1H, m), 8.47-8.46 (1H, m), 8.31-8.30 (1H, m), 8.10-8.07 (1H, m), 7.76 (1H, d), 3.76 (3H, s), 3.48 (2H, q), 1.28 (3H, t).

Present Condensed Heterocyclic Compound 86

1H-NMR (CDCl3) δ**:** 8.51 (1H, d), 8.19 (1H, d), 7.53-7.42 (3H, m), 7.38-7.29 (1H, m), 3.73 (3H, s), 2.97-2.83

(4H, m), 1.33-1.19 (6H, m).

Present Condensed Heterocyclic Compound 87

1H-NMR (CDC13) δ: 8.96 (1H, d), 8.54 (1H, d), 8.28-8.18 (1H, m), 7.91-7.80 (2H, m), 7.63-7.55 (1H, m), 3.74 (3H, s), 3.43 (2H, q), 3.24 (2H, q), 1.38 (3H, t), 1.26 (3H, t).

Present Condensed Heterocyclic Compound 88

$^1$H-NMR (CDC13) δ; 8.53 (1H, d), 8.23 (1H, d), 7.54-7.42 (3H, m), 7.38-7.29 (1H, m), 3.74 (3H, s), 3.33-3.22 (1H, m), 2.87 (2H, q), 1.30 (6H, d), 1.24 (3H, t).

Present Condensed Heterocyclic Compound 89

1H-NMR (CDC13) δ: 8.92 (1H, d), 8.51 (1H, d), 8.22 (1H, dd), 7.92-7.81 (2H, m), 7.62 (1H, dd), 3.75 (3H, s), 3.43 (2H, q), 3.36-3.26 (1H, m), 1.38 (6H, d), 1.26 (3H, t).

Present Condensed Heterocyclic Compound 90

$^1$H-NMR (CDC13) δ: 8.69-8.68 (1H, m), 8.33-8.31 (1H, m), 7.70-7.68 (1H, m), 7.62-7.55 (2H, m), 3.79 (3H, s), 2.97 (2H, q), 1.29 (3H, t).

Present Condensed Heterocyclic Compound 91

1H-NMR (CDCl3) δ: 8.93 (1H, d), 8.52 (1H, d), 8.25 (1H, d), 7.78 (1H, dd), 7.67(1H, d), 3.76 (2H, q), 1.40 (3H, t).

Present Condensed Heterocyclic Compound 92

1H-NMR (CDCl3) δ: 8.89 (1H, d), 8.56 (1H, d), 7.56-7.42 (3H, m), 7.39-7.33 (1H, m), 3.77 (3H, s), 2.89 (2H, q), 1.25 (3H, t).

Present. Condensed Heterocyclic Compound 93

1H-NMR (CDCl3) δ: 8.93 (1H, d), 8.53 (1H, d), 8.26 (1H, dd), 7.84 (1H, td), 7.71 (1H, td), 7.60 (1H, dd), 3.89 (3H, s), 3.42-3.32 (1H, m), 3.05-2.96 (1H, m), 1.31 (3H, t).

Present Condensed Heterocyclic Compound 94

1H-NMR (CDCl3) δ: 8.91 (1H, d), 8.50 (1H, d), 8.23 (1H, dd), 7.87-7.80 (2H, m), 7.56 (1H, dd), 3.71 (3H, s), 3.45 (2H, q), 1.26 (3H, t).

Present Condensed Heterocyclic Compound 95

1H-NMR (CDC13) δ: 8.72-8.70 (1H, m), 8.51-8.49 (1H, m), 8.29-8.27 (1H, m), 8.13-8.09 (1H, m), 7.74-7.71 (1H, m), 3.74 (3H, s), 3.49 (2H, q), 1.29 (3H, t).

Present Condensed Heterocyclic Compound 96

1H-NMR (CDC13) δ: 8.09 (1H, a), 7.59-7.42 (5H, m), 7.37-7.31 (1H, m), 3.69 (3H, s), 2.85 (2H, q), 1.23 (3H, t).

Present Condensed Heterocyclic Compound 97

1H-NMR (CDC13) δ: 8.22 (1H, d), 8.07 (1H, s), 7.80 (1H, t), 7.67 (1H, t), 7.62-7.52 (3H, m), 3.79 (3H, s), 3.37-3.26 (1H, m), 3.01-2.89 (1H, m), 1.27 (3H, t).

Present Condensed Heterocyclic Compound 98

1H-NMR (CDC13) δ: 8.20-8.18 (1H, m), 8.06 (1H, s), 7.82-7.74 (2H, m), 7.59-7.52 (3H, m), 3.61 (3H, s), 3.43

(2H, brs), 1.22 (3H, t).

Present Condensed Heterocyclic Compound 99

1H-NMR($CDCl_3$) δ 8.65-8.64(1H, m), 8.29-8.28(1H, m). 7.35-7.31 (2H, m), 7.18-7.15 (1H, m), 3.76 (3H, s), 2.86 (2H, q), 2.46 (3H, s), 1.23 (3H, t).

Present Condensed Heterocyclic Compound 100

1H-NMR (CDC13) δ: 8.69-8.68 (1H, m), 8.28-8.26 (1H, m), 8.07-8.05 (1H, m), 7.50-7.48 (2H, m), 3.89 (3H, s), 3.44-3.33 (1H, m), 3.01-2.92 (1H, m), 2.58 (3H, s), 1.32 (3H, t).

Present Condensed Heterocyclic Compound 101

1H-NMR (CDC13) δ: 8.68-8.66 (1H, m), 8.25-8.23 (1H, m), 8.04-8.02 (1H, m), 7.65-7.61 (1H, m), 7.45-7.42 (1H, m), 3.71 (3H, s), 3.47-3.38 (2H, m), 2.59 (3H, s), 1.26 (3H, t).

Present Condensed Heterocyclic Compound 102

1H-NMR (CDC13) δ: 8.81 (1H, d), 8.55 (1H, d), 8.07-8.00 (1H, m), 7.59-7.53 (1H, m), 7.52-7.45 (1H, m), 7.41-7.33 (1H, m), 2.99 (2H, q), 1.35 (3H, t).

Present Condensed Heterocyclic Compound 103

1H-NMR (CDC13) δ: 8.85 (1H, d), 8.59 (1H, .d), 8.15 (1H, d), 7.75 (1H, s), 7.59 (1H, d), 3.05 (2H, q), 1.38 (3H, t).

Present Condensed Heterocyclic Compound 104

1H-NMR (CDC13) δ: 8.87 (1H, d), 8.50 (1H, d), 8.28-8.22 (1H, m), 7.85-7.76 (2H, m), 7.74-7.70 (1H, m), 3.74 (2H, q), 1.37 (3H, t).

Present Condensed Heterocyclic Compound 105

1H-NMR (CDCl3) δ: 8.90 (1H, s), 8.57-8.49 (2H, m), 8,07 (1H, d), 7.88 (1H, d), 3.77 (2H, q), 1.40 (3H, t).

Present Condensed Heterocyclic Compound 106

$^1$H-NMR (CDC13) δ: 8.93 (1H, d), 8.61 (1H, d), 7.45-7.34 (3H, m), 2.91 (2H, q), 1.27 (3H, t).

Present Condensed Heterocyclic Compound 107

1H-NMR (CDC13) δ: 8.95 (1H, d), B.55 (1H, d), 8.14 (1H, dd), 7.86 (1H, dd), 7.73 (1H, t), 3.40 (2H, q), 1.28 (3H, t).

Present Condensed Heterocyclic Compound 108

$^1$H-NMR (CDC13) δ: 8.74-8.72 (1H, m), 8.28-6.26 (1H, m), 8.05-8.03 (1H, m), 7.67-7.64 (1H, m), 7.46 (1H, d), 3.71 (3H, s), 3.41 (2H, q), 2.88 (2H, q), 1.37 (3H, t), 1.26 (3H, t).

Present Condensed Heterocyclic Compound 109

1H-NMR (CDCl3) δ: 8.78-8.76 (1H, m), 8.46-8.44 (1H, m), 8.31-8.30 (1H, m), 8.09-8.05 (1H, m), 7.76 (1H, d), 3.75 (3H, s), 3.48 (2H, q), 1.27 (3H, t).

Present Condensed Heterocyclic Compound 110

$^1$H-NMR (CDC13) δ: 8.69 (1H, s), 8.33 (1H, s), 8.25 (1H, dd), 8.21 (1H, dd), 7.73-7.58 (2H, m), 3.47-3.36 (1H, m), 3.13-3.01 (1H, m), 1.57-0.71 (3H, m).

Present Condensed Heterocyclic Compound 111

1H-NMR (CDC13) δ: 8.76 (1H, d), 8.51 (1H, d), 8.29 (1H, d), 8.10 (1H, dd), 7.74 (1H, d), 4.22 (2H, q), 3.55 (2H, q), 1.42 (3H, t), 1.30 (3H, t).

Present Condensed Heterocyclic Compound 112

1H-NMR (CDC13) δ: 8.13 (1H, s), 7.67 (1H, s), 7.62 (1H, d), 7.59-7.57 (2H, m), 7.52 (1H, d), 3.70 (3H, s), 2.92 (2H, q), 1.27 (3H, t).

Present Condensed Heterocyclic Compound 113

1H-NMR (CDCl3) δ: 8.53 (1H, s), 8.10 (1H, s), 7.93 (1H, d), 7.75 (1H, d), 7.65 (1H, d), 7.57 (1H, d), 3.85 (3H, s), 3.52-3.41 (1H, m), 3.05-2.95 (1H, m), 1.32 (3H, t).

Present Condensed Heterocyclic Compound 114

1H-NMR (CDCl3) δ: 8.48 (1H, s), 8.10-8.05 (2H, m), 7.74 (1H, d), 7.62 (1H, d), 7.53 (1H, d), 3.63 (3H, s), 3.47 (2H, q), 1.25 (3H, t).

Present Condensed Heterocyclic Compound 115

$^1$H-NMR (CDC13) δ: 8.83-8.74 (3H, m), 8.54 (1H, dd), 8.31-8.29 (1H, m), 7.95-7.86 (2H, m), 7.68 (1H, d), 7.41-7.37 (1H, m), 3.75 (3H, s), 3.49 (2H, q), 1.30 (3H, t).

Present Condensed Heterocyclic Compound 116

$^1$H-NMR (CDC13) δ: 8.74-8.72 (1H, m), 8.28-8.25 (1H, m), 8.07-8.05 (1H, m), 7.69-7.66 (1H, m), 7.47 (1H, d), 3.72 (3H, a), 3.41 (2H, q), 3.18-3.10 (1H, m), 1.37 (6H, d), 1.26 (3H, t).

Present Condensed Heterocyclic Compound 117

1H-NMR (CDC13) δ: 8.77-8.75 (1H, m), 8.38-8.36 (1H, m), 8.31-8.29 (1H, m), 8.02-7.99 (1H, m), 7.69 (1H, d), 6.85 (1H, t), 3.73 (3H, s), 3.54-3.33 (2H, m), 1.28 (3H, t).

Present Condensed Heterocyclic Compound 118

$^1$H-NMR (CDC13) δ: 8.68-8.66 (1H, m), 8.31-8.30 (1H, m), 7.48 (1H, d), 7.29-7.26 (1H, m), 7.21-7.17 (1H, m), 3.80 (3H, s), 2.93 (2H, q), 1.29 (3H, t).

Present Condensed Heterocyclic Compound 119

1H-NMR (CDCl3) δ: 8.72-8.71 (1H, m), 8.30-8.29 (1H, m), 8.14-8.12 (1H, m), 7.68 (1H, d), 7.55-7.51 (1H, m), 3.93 (3H, s), 3.50-3.40 (1H, m), 3.08-2.98 (1H, m), 1.33 (3H, t).

Present Condensed Heterocyclic Compound 120

$^1$H-NMR (CDC13) δ: 8.71-8.69 (1H, m), 8.27-8.26 (1H, m), 8.09-8.07 (1H, m), 7.70-7.66 (1H, m), 7.62 (1H, d), 3.74 (3H, s), 3.47 (2H, q), 1.28 (3H, t).

Present Condensed Heterocyclic Compound 121

1H-NMR (CDC13) δ: 8.11 (1H, a), 7.67 (1H, s), 7.61-7.56 (3H, m), 7.53 (1H, d), 3.70 (3H, s), 2.93 (2H, q), 1.28 (3H, t).

Present Condensed Heterocyclic Compound 122

1H-NMR (CDC13) δ: 8.53 (1H, d), 8.09 (1H, s), 7.93 (1H, dd), 7.74 (1H, d), 7.64 (1H, d), 7.59 (1H, d), 3.85 (3H,

s), 3.51-3.40 (1H, m), 3.06-2.97 (1H, m), 1.32 (3H, t).

Present Condensed Heterocyclic Compound 123

1H-NMR (CDCl3) δ: 8.49 (1H, d), 8.10-8.04 (2H, m), 7.73 (1H, d), 7.60 (1H, d), 7.55 (1H, d), 3.64 (3H, s), 3.47 (2H, q), 1.25 (3H, t).

Present Condensed Heterocyclic Compound 124

$^1$H-NMR (CDCl3) δ: 9.06-9.04 (1H, m), 8.87 (1H, d), 8.77-8.75 (1H, m), 8.59 (1H, dd), 8.31-8.30 (1H, m), 8.14-8.11 (1H, m), 8.07-8.04 (1H, m), 7.72 (1H, d), 3.76 (3H, s), 3.51 (2H, q), 1.31 (3H, t).

Present Condensed Heterocyclic Compound 125

$^1$H-NMR (CDC13) δ: 8.67-8.66 (1H, m), 8.40-8.39 (1H, m), 7.47 (1H, d), 7.29-7.26 (1H, m), 7.21-7.16 (1H, m), 3.78 (3H, s), 2.93 (2H, q), 1.29 (3H, t).

Present Condensed Heterocyclic Compound 126

1H-NMR (CDC13) δ: 8.71 (1H, d), 8.39 (1H, d), 8.13-8.12 (1H, m), 7.67 (1H, d), 7.54-7.50 (1H, m), 3.91 (3H, s), 3.49-3.39 (1H, m), 3.06-2.96 (1H, m), 1.33 (3H, t).

Present Condensed Heterocyclic Compound 127

1H-NMR (CDC13) δ: 8.70 (1H, d), 8.36 (1H, d), 8.09-8.07 (1H, m), 7.70-7.66 (1H, m), 7.62 (1H, d), 3.72 (3H, s), 3.46 (2H, q), 1.28 (3H, t).

Present Condensed Heterocyclic Compound 128

$^1$H-NMR ($CDCl_3$) δ: 8.53 (1H, s), 8.19-8.13 (2H, m), 8.07 (1H, dd), 7.77 (1H, dd), 7.69 (1H, d), 3.91 (2H, q), 1.44 (3H, t).

Present Condensed Heterocyclic Compound 129

$^1$H-NMR ($CDCl_3$) δ: 8.54 (1H, s), 8.33 (1H, s), 8.17 (1H, d), 8.10 (1H, d), 7.89 (2H, s), 3.91 (2H, q), 1.45 (3H, t).

First, formulation examples will be shown.

Formulation Example 1

5 parts of any one of the present condensed heterocyclic compounds 1 to 131, 10 parts of tebuconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 2

5 parts of any one of the present condensed heterocyclic compounds 1 to 131, 10 parts of metconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 3

5 parts of any one of the present condensed Heterocyclic compounds 1 to 131, 10 parts of difenoconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 4

5 parts of any one of the present condensed heterocyclic compounds 1 to 131, 10 parts of triticonazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 5

5 parts of any one of the present condensed heterocyclic compounds 1 to 131, 10 parts of triadimenol, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 6

5 parts of any one of the present condensed heterocyclic compounds 1 to 131, 10 parts of fluquinconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 7

5 parts of any one of the present condensed heterocyclic compounds 1 to 131, 10 parts of prothioconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 8

5 parts of any one of the present condensed heterocyclic compounds 1 to 131, 10 parts of cyproconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 9

5 parts of any one of the present condensed heterocyclic compounds 1 to 131, 10 parts of tetraconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 10

5 parts of any one of the present condensed heterocyclic compounds 1 to 131, 10 parts of ipconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 11

5 parts of any one of the present condensed heterocyclic compounds 1 to 131, 10 parts of epoxiconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 12

5 parts of any one of the present condensed heterocyclic compounds 1 to 131, 10 parts of hexaconazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 13

10 parts of any one of present condensed heterocyclic compounds 1 to 131, 0.1 parts of tebuconazole, 1.5 parts of sorbitan trioleate, and 28 parts' of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 14

10 parts of any one of present condensed heterocyclic compounds 1 to 131, 0.1 parts of metconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 15

10 parts of any one of present condensed heterocyclic compounds 1 to 131, 0.1 parts of difenoconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 16

10 parts of any one of present condensed Heterocyclic compounds 1 to 131, 0.1 parts of triticonazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 17

10 parts of any one of present condensed heterocyclic compounds 1 to 131, 0.1 parts of triadimenol, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 18

10 parts of any one of present condensed heterocyclic compounds 1 to 131, 0.1 parts of fluquinconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume

of the mixture 90 parts, and 10 parts of propylene glycol is further added to the mixture, which is then stirred and mixed to obtain each formulation.

Formulation Example 19

10 parts of any one of present condensed heterocyclic compounds 1 to 131, 0.1 parts of prothioconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene' glycol is further added to the mixture, which is then stirred and mixed to obtain each formulation.

Formulation Example 20

10 parts of any one of present condensed heterocyclic compounds 1 to 131, 0.1 parts of cyproconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 21

10 parts of any one of present condensed heterocyclic compounds 1 to 131, 0.1 parts of tetraconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 22

1o parts of any one of present condensed heterocyclic compounds 1 to 131, 0.1 parts of ipconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parte, and 10 parts of propylene glycol is further added to the mixture, which is then stirred and mixed to obtain each formulation.

Formulation Example 23

10 parts of any one of present condensed heterocyclic compounds 1 to 131, 0.1 parts of epoxiconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 24

10 parts of any one of present condensed heterocyclic compounds 1 to 131, 0.1 parts of hexaconazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 25

10 parts of any one of present condensed heterocyclic compounds 1 to 131, 10 parts of tebuconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 26

10 parts of any one of present condensed heterocyclic compounds 1 to 131, 10 parts of metconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 27

10 parts of any one of present condensed heterocyclic compounds 1 to 131, 10 parts of difenoconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 28

10 parts of any one of present condensed heterocyclic compounds 1 to 131, 10 parts of triticonazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 29

10 parts of any one of present condensed heterocyclic compounds 1 to 131, 10 parts of triadimenol, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 30

10 parts of any one of present condensed heterocyclic compounds 1 to 131, 10 parts of fluquinconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 31

10 parts of any one of present condensed heterocyclic compounds 1 to 131, 10 parts of prothioconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 32

10 parts of any one of present condensed heterocyclic compounds 1 to 131, 10 parts of cyproconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 33

10 parts of any one of present condensed heterocyclic compounds 1 to 131, 10 parts of tetraconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 34

10 parts of any one of present condensed heterocyclic compounds 1 to 131, 10 parts of ipconazole, 3 parts of lignin calcium sulfonate, 2 parts of sodium lauryl sulfate, and a remainder of synthetic hydrated silicon oxide are well

pulverized and mixed to obtain 100 parts of each water dispersible powder.

Formulation Example 35

10 parts of any one of present condensed heterocyclic compounds 1 to 131, 10 parts of epoxiconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formuation Example 36

10 parts of any one of present condensed heterocyclic compounds 1 to 131, 10 parts of hexaconazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 37

1 part of any one of present condensed heterocyclic compounds 1 to 131, 0.5 parts of tebuconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 38

1 part of any one of present condensed heterocyclic compounds 1 to 131, 0.5 parts of metconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 39

1 part of any one of present condensed heterocyclic compounds 1 to 131, 0.5 parts of difenoconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 40

1 part of any one of present condensed heterocyclic compounds 1 to 131, 0.5 parts of triticonazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 41

1 part of any one of present condensed heterocyclic compounds 1 to 131, 0.5 parts of triadimenol, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 42

1 part of any one of present condensed heterocyclic compounds 1 to 131, 0.5 parts of fluquinconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 43

1 part of any one of present condensed heterocyclic compounds 1 to 131, 0.5 parts of prothioconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 44

1 part of any one of present condensed heterocyclic compounds 1 to 131, 0.5 parts of cyproconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 45

1 part of any one of present condensed heterocyclic compounds 1 to 131, 0.5 parts of tetraconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 46

1 part of any one of present condensed heterocyclic compounds 1 to 131, 0.5 parts of ipconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 47

1 part of any one of present condensed heterocyclic compounds 1 to 131, 0.5 parts of epoxiconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 48

1 part of any one of present condensed heterocyclic compounds 1 to 131, 0.5 parts of hexaconazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 49

5 parts of any one of the present condensed heterocyclic compounds 1 to 131, 10 parts of azoxystrobin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 50

5 parts of any one of the present condensed heterocyclic compounds 1 to 131, 10 parts of pyraclostrobin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 51

5 parts of any one of the present condensed heterocyclic compounds 1 to 131, 10 parts of picoxystrobin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 52

5 parts of any one of the present condensed heterocyclic compounds 1 to 131, 10 parts of trifloxystrobin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 53

5 parts of any one of the present condensed heterocyclic compounds 1 to 131, 10 parts of fluoxastrobin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 54

5 parts of any one of the present condensed heterocyclic compounds 1 to 131, 10 parts of orysastrobin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 55

5 parts of any one of the present condensed heterocyclic compounds 1 to 131, 10 parts of the present amide compound (compound represented by Formula (2): the same applies hereinafter), 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 56

10 parts of any one of present condensed heterocyclic compounds 1 to 131, 0.1 parts of azoxystrobin, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminium magnesium silicate, is added to the mixture to make the total volume of the mixture 90 parts, and 10 parts of propylene glycol is further added to the mixture, which is then stirred and mixed to obtain each formulation.

Formulation Example 57

10 parts of any one of present condensed heterocyclic compounds 1 to 131, 0.1 parts of pyraclostrobin, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 58

10 parts of any one of present condensed heterocyclic compounds 1 to 131, 0.1 parts of picoxystrobin, 1.5 parts of sorbitan trioleate, and 28 parts of an aqueous solution, which contains 2 parts of polyvinyl alcohol, are mixed

and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 59

10 parts of any one of present condensed heterocyclic compounds 1 to 131, 0.1 parts of trifloxystrobin, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 60

10 parts of any one of present condensed heterocyclic compounds 1 to 131, 0.1 parts of fluoxastrobin, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 61

10 parts of any one of present condensed heterocyclic compounds 1 to 131, 0.1 parts of orysastrobin, 1.5 parts of sorbitan trioleate, and 28 parts of an aqueous solution, which contains 2 parts of polyvinyl alcohol, are mixed and the mixture, is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 62

10 parts of any one of present condensed heterocyclic compounds 1 to 131, 0.1 parts of the present amide compound, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 63

10 parts of any one of present condensed heterocyclic compounds 1 to 131, 10 parts of azoxystrobin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 64

10 parts of any one of present condensed heterocyclic compounds 1 to 131, 10 parts of pyraclostrobin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 65

10 parts of any one of present condensed heterocyclic compounds 1 to 131, 10 parts of picoxystrobin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 66

10 parts of any one of present condensed heterocyclic compounds 1 to 131, 10 parts of trifloxystrobin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 67

10 parts of any one of present condensed heterocyclic compounds 1 to 131, 10 parts of fluoxastrobin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 68

10 parts of any one of present condensed heterocyclic compounds 1 to 131, 10 parts of orysastrobin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 69

10 parts of any one of present condensed heterocyclic compounds 1 to 131, 10 parts of the present amide compound, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 70

1 part of any one of present condensed heterocyclic compounds 1 to 131, 0.5 parts of azoxystrobin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 71

1 part of any one of present condensed heterocyclic compounds 1 to 131, 0.5 parts of pyraclostrobin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 72

1 part of any one of present condensed heterocyclic compounds 1 to 131, 0.5 parts of picoxystrobin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 73

1 part of any one of present condensed heterocyclic compounds 1 to 131, 0.5 parts of trifloxystrobin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 74

1 part of any one of present condensed heterocyclic compounds 1 to 131, 0.5 parts of fluoxastrobin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 75

1 part of any one of present condensed heterocyclic compounds 1 to 131, 0.5 parts of orysastrobin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 76

1 part of any one of present condensed heterocyclic compounds 1 to 131, 0.5 parts of the present amide compound, 1 part of synthetic hydrated silicon oxide fine powder, 2 parts of lignin calcium sulfonate, 30 parts of bantonite, and a remainder of kaolin clay are mixed. Next, an appropriate amount of water is added to the mixture, which is then stirred, granulated by a granulator, and dried with air to obtain each granule.

Formulation Example 77

5 parts of any one of the present condensed heterocyclic compounds 1 to 131, 10 parts of metalaxyl, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 78

5 parts of any one of the present condensed heterocyclic compounds 1 to 131, 10 parts of metalaxyl-M, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 79

10 parts of any one of present condensed heterocyclic compounds 1 to 131, 0.1 parts of metalaxyl, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 80

10 parts of any one of present condensed heterocyclic compounds 1 to 131, 0.1 parts of metalaxyl-M, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 81

10 parts of any one of present condensed heterocyclic compounds 1 to 131, 10 parts of metalaxyl, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 82

10 parts of any one of present condensed heterocyclic compounds 1 to 131, 10 parts of metalaxyl-M, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 83

1 part of any one of present condensed heterocyclic compounds 1 to 131, 0.5 parts of metalaxyl, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 84

1 part of any one of present condensed heterocyclic compounds 1 to 131, 0.5 parts of metalaxyl-M, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 85

5 parts of any one of the present condensed heterocyclic compounds 1 to 131, 10 parts of probenazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 86

5 parts of any one of the present condensed heterocyclic compounds 1 to 131, 10 parts of tricyclazole, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 87

5 parts of any one of the present condensed heterocyclic compounds 1 to 131, 10 parts of pyroquilon, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 88

5 parts of any one of the present condensed heterocyclic compounds 1 to 131, 10 parts of kasugamycin hydrochloride, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 89

5 parts of any one of the present condensed heterocyclic compounds 1 to 131, 10 parts of ferimzone, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 90

5 parts of any one of the present condensed heterocyclic compounds 1 to 131, 10 parts of isotianil, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 91

5 parts of any one of the present condensed heterocyclic compounds 1 to 131, 10 parts of fthalide, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 92

5 parts of any one of the present condensed heterocyclic compounds 1 to 131, 10 parts of tebufloquin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 93

10 parts of any one of present condensed heterocyclic compounds 1 to 131, 0.1 parts of probenazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 94

10 parts of any one of present condensed heterocyclic compounds 1 to 131, 0.1 parts of tricyclazole, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 95

10 parts of any one of present condensed heterocyclic compounds 1 to 131, 0.1 parts of pyroquilon, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 96

10 parts of any one of present condensed heterocyclic compounds 1 to 131, 0.1 parts of kasugamycin hydrochloride, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 97

10 parts of any one of present condensed heterocyclic compounds 1 to 131, 0.1 parts of ferimzone, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.0.5 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 98

10 parts of any one of present condensed heterocyclic compounds 1 to 131, 0.1 parts of isotianil, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0,1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 99

10 parts of any one of present condensed heterocyclic compounds 1 to 131, 0.1 parts of fthalide, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 100

10 parts of any one of present condensed heterocyclic compounds 1 to 131, 0.1 parts of tebufloquin, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 101

10 parts of any one of present condensed heterocyclic compounds 1 to 131, 10 parts of probenazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 102

1C parts of any one of present condensed heterocyclic compounds 1 to 131, 10 parts of tricyclazole, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 103

10 parts of any one of present condensed heterocyclic compounds 1 to 131, 10 parts of pyroquilon, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 104

10 parts of any one of present condensed heterocyclic compounds 1 to 131, 10 parts of kasugamycin hydrochloride, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 105

10 parts of any one of present condensed heterocyclic compounds 1 to 131, 10 parts of ferimzone, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 106

10 parts of any one of present condensed heterocyclic compounds 1 to 131, 10 parts of isotianil, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 107

10 parts of any one of present condensed heterocyclic compounds 1 to 131, 10 parts of fthalide, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 108

10 parts of any one of present condensed heterocyclic compounds 1 to 131, 10 parts of tebufloquin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 109

1 part of any one of present condensed heterocyclic compounds 1 to 131, 0.5 parts of probenazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 110

1 part of any one of present condensed heterocyclic compounds 1 to 131, 0.5 parts of tricyclazole, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 111

1 part of any one of present condensed heterocyclic compounds 1 to 131, 0.5 parts of pyroquilon, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 112

1 part of any one of present condensed heterocyclic compounds 1 to 131, 0.5 parts of kasugamycin hydrochloride, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 113

1 part of any one of present condensed heterocyclic compounds 1 to 131, C.5 parts of ferimzone, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 114

1 part of any one of present condensed heterocyclic compounds 1 to 131, 0.5 parts of isotianil, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a

granulator, and dried with air to obtain each granule.

Formulation Example 115

1 part of any one of present condensed heterocyclic compounds 1 to 131, 0.5 parts of fthalide, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 116

1 part of any one of present condensed heterocyclic compounds 1 to 131, 0.5 parts of tebufloquin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 117

5 parts of any one of the present condensed heterocyclic compounds 1 to 131, 10 parts of pencycuron, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 118

5 parts of any one of the present condensed heterocyclic compounds 1 to 131, 10 parts of furametpyr, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 119

5 parts of any one of the present condensed heterocyclic compounds 1 to 131, 10 parts of validamycin, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 120

10 parts of any one of present condensed heterocyclic compounds 1 to 131, 0.1 parts of pencycuron, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 121

10 parts of any one of present condensed heterocyclic compounds 1 to 131, 0.1 parts of furametpyr, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 122

10 parts of any one of present condensed heterocyclic compounds 1 to 131, 0.1 parts of validamycin, 1.5 parts

of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 123

10 parts of any one of present condensed heterocyclic compounds 1 to 131, 10 parts of pencycuron, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 124

10 parts of any one of present condensed heterocyclic compounds 1 to 131, 10 parts of furametpyr, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 125

10 parts of any one of present condensed heterocyclic compounds 1 to 131, 10 parts of validamycin, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 126

1 part of any one of present condensed heterocyclic compounds 1 to 131, 0.5 parts of pencycuron, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 127

1 part of any one of present condensed heterocyclic compounds 1 to 131, 0.5 parts of furametpyr, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 128

1 part of any one of present condensed heterocyclic compounds 1 to 131, 0.5 parts of validamycin, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 129

5 parts of any one of the present condensed heterocyclic compounds 1 to 131, 10 parts of flutolanil, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 130

5 parts of any one of the present condensed heterocyclic compounds 1 to 131, 10 parts of fluopyram, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 131

5 parts of any one of the present condensed heterocyclic compounds 1 to 131, 10 parts of sedaxane, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 132

5 parts of any one of the present condensed heterocyclic compounds 1 to 131, 10 parts of penflufen, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 133

5 parts of any one of the present condensed heterocyclic compounds 1 to 131, 10 parts of fluxapyroxad, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 134

10 parts of any one of present condensed heterocyclic compounds 1 to 131, 0.1 parts of flutolanil, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 135

10 parts of any one of present condensed heterocyclic compounds 1 to 131, 0.1 parts of fluopyram, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 136

10 parts of any one of present condensed heterocyclic compounds 1 to 131, 0.1 parts of sedaxane, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 137

10 parts of any one of present condensed heterocyclic compounds 1 to 131, 0.1 parts of penflufen, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts cf aluminum magnesium silicate, is added to the mixture to make the total volume of the mixture 90 parts, and 10 parts of propylene glycol is further added to the mixture, which is then stirred and mixed to obtain each formulation.

Formulation Example 138

10 parts of any one of present condensed heterocyclic compounds 1 to 131, 0.1 parts of fluxapyroxad, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 139

10 parts of any one of present condensed heterocyclic compounds 1 to 131, 10 parts of flutolanil, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 140

10 parts of any one of present condensed heterocyclic compounds 1 to 131, 10 parts of fluopyram, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 141

10 parts of any one of present condensed heterocyclic compounds 1 to 131, 10 parts of sedaxane, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 142

10 parts of any one of present condensed heterocyclic compounds 1 to 131, 10 parts of penflufen, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 143

10 parts of any one of present condensed heterocyclic compounds 1 to 131, 10 parts of fluxapyroxad, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 144

1 part of any one of present condensed heterocyclic compounds 1 to 131, 0.5 parts of flutolanil, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 145

1 part of any one of present condensed heterocyclic compounds 1 to 131, 0.5 parts of fluopyram, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 146

1 part of any one of present condensed heterocyclic compounds 1 to 131, 0.5 parts of sedaxane, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated

by a granulator, and dried with air to obtain each granule.

Formulation Example 147

1 part of any one of present condensed heterocyclic compounds 1 to 131, 0.5 parts of penflufen, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 148

1 part of any one of present condensed heterocyclic compounds 1 to 131, 0.5 parts of fluxapyroxad, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 149

5 parts of any one of the present condensed heterocyclic compounds 1 to 131, 10 parts of fludioxonil, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 150

5 parts of any one cf the present condensed heterocyclic compounds 1 to 131, 10 parts of ethaboxam, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 151

5 parts of any one of the present condensed heterocyclic compounds 1 to 131, 10 parts of tolclofos-methyl, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 152

5 parts of any one of the present condensed heterocyclic compounds 1 to 131, 10 parts of captan, 35 parts of a mixture (weight ratio of 1:1) of white carbon and polyoxyethylene alkyl ether sulfate ammonium salt, and water are mixed to make the total volume of the mixture 100 parts, and the mixture is finely pulverized through a wet pulverization method to obtain each formulation.

Formulation Example 153

10 parts of any one of present condensed heterocyclic compounds 1 to 131, 0.1 parts of fludioxonil, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 154

10 parts of any one of present condensed heterocyclic compounds 1 to 131, 0.1 parts of ethaboxam, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts

of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 155

10 parts of any one of present condensed heterocyclic compounds 1 to 131, 0.1 parts of tolclofosmethyl, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 156

10 parts of any one of present condensed heterocyclic compounds 1 to 131, 0.1 parts of captan, 1.5 parts of sorbitan trioleate, and 28 parts of an aqueous solution, which contains 2 parts of polyvinyl alcohol, are mixed and the mixture is finely pulverized through a wet pulverization method. Then, an aqueous solution, which contains 0.05 parts of xanthan gum and 0.1 parts of aluminum magnesium silicate, is added to the mixture to make the total volume of the mixture 90 parts, and 10 parts of propylene glycol is further added to the mixture, which is then stirred and mixed to obtain each formulation.

Formulation Example 157

10 parts of any one of present condensed heterocyclic compounds 1 to 131, 10 parts of fludioxonil, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 158

10 parts of any one of present condensed heterocyclic compounds 1 to 131, 10 parts of ethaboxam, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 159

10 parts of any one of present condensed heterocyclic compounds 1 to 131, 10 parts of tolclofos-methyl, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 160

10 parts of any one of present condensed heterocyclic compounds 1 to 131, 10 parts of captan, 3 parts of lignin calcium sulfonate, 2 parts of sodium lauryl sulfate, and a remainder of synthetic hydrated silicon oxide are well pulverized and mixed to obtain 100 parts of each water dispersible powder.

Formulation Example 161

1 part of any one of present condensed heterocyclic compounds 1 to 131, 0.5 parts of fludioxonil, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Formulation Example 162

1 part of any one of present condensed heterocyclic compounds 1 to 131, 0.5 parts of ethaboxam, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated

by a granulator, and dried with air to obtain each granule.

Formulation Example 163

1 part of any one of present condensed heterocyclic compounds 1 to 131, 0.5 parts of tolclofos-methyl, 1 part of synthetic hydrated silicon oxide fine powder, 2 parts of lignin calcium sulfonate, 30' parts of bentonite, and a remainder of kaolin clay are mixed. Next, an appropriate amount of water is added to the mixture, which is then stirred, granulated by a granulator, and dried with air to obtain each granule.

Formulation Example 164

1 part of any one of present condensed heterocyclic compounds 1 to 131, 0.5 parts of captan, 1 part of synthetic hydrated silicon oxide fine powder, 2 parts of lignin calcium sulfonate, 30 parts of bentonite, and a remainder of kaolin clay are mixed. Next, an appropriate amount of water is added to the mixture, which is then stirred, granulated by a granulator, and dried with air to obtain each granule.

Next, the effect of the compounds of the present invention in controlling harmful arthropod is shown with reference to test examples.

Test Example 1

1 mg of each of the present condensed heterocyclic compounds 3, 23, 27, 31, 39, 41, 42, 46, 48, 51, 56, 58, 64, 69, 72, 74, 78, 83, 91, 97, 104, 105, 107, 108, 109, 118, 119, 120, 127, 130, and 131 was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

Each of commercial formulations, such as tebuconazole (trade name: Horizon EW, manufactured by Bayer CorpScience), prothioconazole (trade name: Joao, manufactured by Bayer CorpScience), metconazole (trade name: Sunorg Pro, manufactured by BASF Japan Ltd.), difenoconazole (trade name: Score granular hydration agent, manufactured by Syngenta Japan K.K.), tetraconazole (trade name: Salvatore ME, manufactured by Arysta Life Science Corporation), and hexaconazole (trade name: Anvil flowable, manufactured by Sumitomo Chemical Co., Ltd.), was diluted with water which contains 0.02 volume% of a spreading agent (trade name: Shindain, manufactured by Sumitomo Chemical Co., Ltd.) so as to have a predetermined concentration.

1 mg of each of ipconazole (manufactured by Waco Pure Chemical Industries, Ltd.) and triticonazole (Waco Pure Chemical Industries, Ltd.) was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A water-diluted solution of the above-described present condensed heterocyclic compounds and a water-diluted solution of tebuconazole, prothioconazole, metconazole, difenoconazole, tetraconazole, hexaconazole, ipconazole, or triticonazole were mixed together to prepare a liquid medicine for a test.

Each leaf disc (diameter of 1.5 cm) of a cabbage (Brassicae oleracea) was accommodated in each well of a 24 hole microplate (manufactured by Becton Dickinson) and 40 μL of liquid medicine for a test was scattered to each well. Wells to which 40 μL of water containing 0.02 volume% of a spreading agent (trade name: Shindain, manufactured by Sumitomo Chemical Co., Ltd.) was scattered was set to a non-treatment section.

After air-drying, 5 second-instar larvae of diamondback moths (Plutella xylostella) were released in each well, which was then covered with paper towel and closed with a lid. 2 days after the releasing, the number of surviving larvae in each well was observed.

The mortality rates of the treatment section and the non-treatment section were calculated using the following Expression 1). The test was repeated once.

Expression 1) Mortality rate (%) = (the number of larvae provided for a test - the number of surviving larvae) / the number of larvae provided for a test x 100

Table 5

| Compounds provided for test | Concentration (ppm) | Mortality rate |
|---|---|---|
| Present condensed heterocyclic compound 3 + tebuconazole | 200+2000 | 100 |
| Present condensed heterocyclic compound 3 + tebuconazole | 500+50 | 100 |
| Present condensed heterocyclic compound 3 + tebuconazole | 500+5 | 100 |
| Present condensed heterocyclic compound 3 + prothioconazole | 200+2000 | 100 |
| Present condensed heterocyclic compound 3 + prothioconazole | 500+5 | 100 |
| Present condensed heterocyclic compound 3 + metconazole | 200+2000 | 100 |
| Present condensed heterocyclic compound 3 + metconazole | 500+50 | 100 |
| Present condensed heterocyclic compound 3 + metconazole | 500+5 | 100 |
| Present condensed heterocyclic compound 39 + tebuconazole | 200+2000 | 100 |
| Present condensed heterocyclic compound 39 + tebuconazole | 500+50 | 100 |
| Present condensed heterocyclic compound 39 + tebuconazole | 500+5 | 100 |
| Present condensed heterocyclic compound 39 + prothioconazole | 200+2000 | 100 |
| Present condensed heterocyclic compound 39 + prothioconazole | 500+5 | 100 |
| Present condensed heterocyclic compound 39 + metconazole | 200+2000 | 100 |
| Present condensed heterocyclic compound 39 + metconazole | 500+50 | 100 |
| Present condensed heterocyclic compound 39 + metconazole | 500+5 | 100 |
| Present condensed heterocyclic compound 23 + tebuconazole | 200+2000 | 100 |
| Present condensed heterocyclic compound 23 + tebuconazole | 500+50 | 100 |
| Present condensed heterocyclic compound 23 + tebuconazole | 500+5 | 100 |
| Present condensed heterocyclic compound 23 + prothioconazole | 200+2000 | 100 |
| Present condensed heterocyclic compound 23 + prothioconazole | 500+5 | 100 |
| Present condensed heterocyclic compound 23 + metconazole, | 200+2000 | 100 |
| Present condensed heterocyclic compound 23 + metconazole | 500+50 | 100 |
| Present condensed heterocyclic compound 23 + metconazole | 500+5 | 100 |
| Present condensed heterocyclic compound 3 + ipconazole | 200+2000 | 100 |
| Present condensed heterocyclic compound 3 + ipconazole | 500+5 | 100 |
| Present condensed heterocyclic compound 3 + triticonazole | 200+2000 | 100 |
| Present condensed heterocyclic compound 3 + triticonazole | 500+5 | 100 |
| Present condensed heterocyclic compound 3 + difenoconazole | 200+2000 | 100 |
| Present condensed heterocyclic compound 3 + difenoconazole | 500+5 | 100 |
| Present condensed heterocyclic compound 3 + tetraconazole | 200+2000 | 100 |
| Present condensed heterocyclic compound 3 + tetraconazole | 500+5 | 100 |
| Present condensed heterocyclic compound 3 + hexaconazole | 200+2000 | 100 |
| Present condensed heterocyclic compound 3 + hexaconazole | 500+50 | 100 |
| Present condensed heterocyclic compound 3 + hexaconazole | 500+5 | 100 |
| Present condensed heterocyclic compound 39 + ipconazole | 200+2000 | 100 |
| Present condensed heterocyclic compound 39 + ipconazole | 500+5 | 100 |

(continued)

| Compounds provided for test | Concentration (ppm) | Mortality rate |
|---|---|---|
| Present condensed heterocyclic compound 39 + triticonazole | 200+2000 | 100 |
| Present condensed heterocyclic compound 39 + triticonazole | 500+5 | 100 |
| Non-treatment section | - | 0 |

Table 6

| Compounds provided for test | Concentration (ppm) | Mortality rate |
|---|---|---|
| Present condensed heterocyclic compound 39 + difenoconazole | 200+2000 | 100 |
| Present condensed heterocyclic compound 39 + difenoconazole | 500+5 | 100 |
| Present condensed heterocyclic compound 39 + tetraconazole | 200+2000 | 100 |
| Present condensed heterocyclic compound 39 + tetraconazole | 500+5 | 100 |
| Present condensed heterocyclic compound 39 + hexaconazole | 200+2000 | 100 |
| Present condensed heterocyclic compound 39 + hexaconazole | 500+50 | 100 |
| Present condensed heterocyclic compound 39 + hexaconazole | 500+5 | 100 |
| Present condensed heterocyclic compound 23 + ipconazole | 200+2000 | 100 |
| Present condensed heterocyclic compound 23 + ipconazole | 500+5 | 100 |
| Present condensed heterocyclic compound 23 + triticonazole | 200+2000 | 100 |
| Present condensed heterocyclic compound 23 + triticonazole | 500+5 | 100 |
| Present condensed heterocyclic compound 23 + difenoconazole | 200+2000 | 100 |
| Present condensed heterocyclic compound 23 + difenoconazole | 500+5 | 100 |
| Present condensed heterocyclic compound 23 + tetraconazole | 200+2000 | 100 |
| Present condensed heterocyclic compound 23 + tetraconazole | 500+5 | 100 |
| Present condensed heterocyclic compound 23 + hexaconazole | 200+2000 | 100 |
| Present condensed heterocyclic compound 23 + hexaconazole | 500+50 | 100 |
| Present condensed heterocyclic compound 23 + hexaconazole | 500+5 | 100 |
| Present condensed heterocyclic compound 74 + tebuconazole | 200+2000 | 100 |
| Present condensed heterocyclic compound 74 + tebuconazole | 500+50 | 100 |
| Present condensed heterocyclic compound 74 + tebuconazole | 500+5 | 100 |
| Present condensed heterocyclic compound 74 + prothioconazole | 200+2000 | 100 |
| Present condensed heterocyclic compound 74 + prothioconazole | 500+5 | 100 |
| Present condensed heterocyclic compound 74 + metconazole, | 200+2000 | 100 |
| Present condensed heterocyclic compound 74 + metconazole | 500+50 | 100 |
| Present condensed heterocyclic compound 74 + metconazole | 500+5 | 100 |
| Present condensed heterocyclic compound 74 + ipconazole | 200+2000 | 100 |
| Present condensed heterocyclic compound 74 + ipconazole | 500+5 | 100 |
| Present condensed heterocyclic compound 74 + triticcnazole | 200+2000 | 100 |
| Present condensed heterocyclic compound 74 + triticonazole | 500+5 | 100 |
| Present condensed heterocyclic compound 74 + difenoconazole | 200+2000 | 100 |

(continued)

| Compounds provided for test | Concentration (ppm) | Mortality rate |
|---|---|---|
| Present condensed heterocyclic compound 74 + difenoconazole | | 100 |
| Present condensed heterocyclic compound 74 + tetraconazole | 200+2000 | 100 |
| Present condensed heterocyclic compound 74 + tetraconazole | 500+5 | 100 |
| Present condensed heterocyclic compound 74 + hexaconazole | 200+2000 | 100 |
| Present condensed heterocyclic compound 74 + hexaconazole | 500+50 | 100 |
| Present condensed heterocyclic compound 74 + hexaconazole | 500+5 | 100 |
| Non-treatment section | - | 0 |

Table 7

| Compounds provided for test | Concentration (ppm) | Mortality rate |
|---|---|---|
| Present condensed heterocyclic compound 83 + tebuconazole | 200+2000 | 100 |
| Present condensed heterocyclic compound 83 + tebuconazole | 500+50 | 100 |
| Present condensed heterocyclic compound 83 + tebuconazole | 500+5 | 100 |
| Present condensed heterocyclic compound 83 + prothioconazole | 200+2000 | 100 |
| Present condensed heterocyclic compound 83 + prothioconazole | 500+5 | 100 |
| Present condensed heterocyclic compound 83 + metconazole | 200+2000 | 100 |
| Present condensed heterocyclic compound 83 + metconazole | 500+50 | 100 |
| Present condensed heterocyclic compound 83 + metconazole | 500+5 | 100 |
| Present condensed heterocyclic compound 83 + ipconazole | 200+2000 | 100 |
| Present condensed heterocyclic compound 83 + ipconazole | 500+5 | 100 |
| Present condensed heterocyclic compound 83 + triticonazole | 200+2000 | 100 |
| Present condensed heterocyclic compound 83 + triticonazole | 500+5 | 100 |
| Present condensed heterocyclic compound 83 + difenoconazole | 200+2000 | 100 |
| Present condensed heterocyclic compound 83 + difenoconazole | 500+5 | 100 |
| Present condensed heterocyclic compound 83 + tetraconazole | 200+2000 | 100 |
| Present condensed heterocyclic compound 83 + tetraconazole | 500+5 | 100 |
| Present condensed heterocyclic compound 83 + hexaconazole | 200+2000 | 100 |
| Present condensed heterocyclic compound 83 + hexaconazole | 500+50 | 100 |
| Present condensed heterocyclic compound 83 + hexaconazole | 500+5 | 100 |
| Present condensed heterocyclic compound 58 + tebuconazole | 200+2000 | 100 |
| Present condensed heterocyclic compound 58 + tebuconazole | 500+50 | 100 |
| Present condensed heterocyclic compound 58 + tebuconazole | 500+5 | 100 |
| Present condensed heterocyclic compound 58 + prothioconazole | 200+2000 | 100 |
| Present condensed heterocyclic compound 58 + prothioconazole | 500+5 | 100 |
| Present condensed heterocyclic compound 58 + metconazcle | 200+2000 | 100 |
| Present condensed heterocyclic compound 58 + metconazole | 500+50 | 100 |
| Present condensed heterocyclic compound 58 + metconazole | 500+5 | 100 |

(continued)

| Compounds provided for test | Concentration (ppm) | Mortality rate |
|---|---|---|
| Present condensed heterocyclic compound 58 + ipconazole | 200+2000 | 100 |
| Present condensed heterocyclic compound 58 + ipconazole | 500+5 | 100 |
| Present condensed heterocyclic compound 58 + triticonazole | 200+2000 | 100 |
| Present condensed heterocyclic compound 58 + triticonazole | 500+5 | 100 |
| Present condensed heterocyclic compound 58 + difenoconazole | 200+2000 | 100 |
| Present condensed heterocyclic compound 58 + difenoconazole | 500-5 | 100 |
| Present condensed heterocyclic compound 58 + tetraconazole | 200+2000 | 100 |
| Present condensed heterocyclic compound 58 + tetraconazole | 500+5 | 100 |
| Present condensed heterocyclic compound 58 + hexaconazole | 200+2000 | 100 |
| Present condensed heterocyclic compound 58 + hexaconazole | 500+50 | 100 |
| Present condensed heterocyclic compound 58 + hexaconazole | 500+5 | 100 |
| Non-treatment section | - | 0 |

Table 8

| Compounds provided for test | Concentration (ppm) | Mortality rate |
|---|---|---|
| Present condensed heterocyclic compound 31 + tebuconazole | 200+2000 | 100 |
| Present condensed heterocyclic compound 31 + tebuconazole | 500+50 | 100 |
| Present condensed heterocyclic compound 31 + tebuconazole | 500+5 | 100 |
| Present condensed heterocyclic compound 31 + prothioconazole | 200+2000 | 100 |
| Present condensed heterocyclic compound 31 + prothioconazole | 500+5 | 100 |
| Present condensed heterocyclic compound 31 + metconazole | 200+2000 | 100 |
| Present condensed heterocyclic compound 31 + metconazole | 500+50 | 100 |
| Present condensed heterocyclic compound 31 + metconazole, | 500+5 | 100 |
| Present condensed heterocyclic compound 31 + ipconazole | 200+2000 | 100 |
| Present condensed heterocyclic compound 31 + ipconazole | 500+5 | 100 |
| Present condensed heterocyclic compound 31 + triticonazole | 200+2000 | 100 |
| Present condensed heterocyclic compound 31 + triticonazole | 500+5 | 100 |
| Present condensed heterocyclic compound 31 + difenoconazole | 200+2000 | 100 |
| Present condensed heterocyclic compound 31 + difenoconazole | 500+5 | 100 |
| Present condensed heterocyclic compound 31 + tetraconazole | 200+2000 | 100 |
| Present condensed heterocyclic compound 31 + tetraconazole | 500+5 | 100 |
| Present condensed heterocyclic compound 31 + hexaconazole | 200+2000 | 100 |
| Present condensed heterocyclic compound 31 + hexaconazole | 500+50 | 100 |
| Present condensed heterocyclic compound 31 + hexaconazole | 500+5 | 100 |
| Present condensed heterocyclic compound 56 + tebuconazole | 200+2000 | 100 |
| Present condensed heterocyclic compound 56 + tebuconazole | 500+50 | 100 |
| Present condensed heterocyclic compound 56 + tebuconazole | 500+5 | 100 |

(continued)

| Compounds provided for test | Concentration (ppm) | Mortality rate |
|---|---|---|
| Present condensed heterocyclic compound 56 + prothioconazole | 200+2000 | 100 |
| Present condensed heterocyclic compound 56 + prothioconazole | 500+5 | 100 |
| Present condensed heterocyclic compound 56 + metconazole | 200+2000 | 100 |
| Present condensed heterocyclic compound 56 + metconazole, | 500+50 | 100 |
| Present condensed heterocyclic compound 56 + metconazole | 500+5 | 100 |
| Present condensed heterocyclic compound 56 + ipconazole | 200+2000 | 100 |
| Present condensed heterocyclic compound 56 + ipconazole | 500+5 | 100 |
| Present condensed heterocyclic compound 56 + triticonazole | 200+2000 | 100 |
| Present condensed 56 + heterocyclic compound 56 + triticonazole | 500+5 | 100 |
| Present condensed heterocyclic compound 56 + difenoconazole | 200+2000 | 100 |
| Present condensed heterocyclic compound 56 + difenoconazole | 500+5 | 100 |
| Present condensed heterocyclic compound 56 + tetraconazole | 200+2000 | 100 |
| Present condensed heterocyclic compound 56 + tetraconazole | 500+5 | 100 |
| Present condensed heterocyclic compound 56 + hexaconazole | 200+2000 | 100 |
| Present condensed heterocyclic compound 56 + hexaconazole | 500+50 | 100 |
| Present condensed heterocyclic compound 56 + hexaconazole | 500+5 | 100 |
| Non-treatment section | - | 0 |

Table 9

| Compounds provided for test | Concentration (ppm) | Mortality rate |
|---|---|---|
| Present condensed heterocyclic compound 46 + tebuconazole | 200+2000 | 100 |
| Present condensed heterocyclic compound 46 + tebuconazole | 500+50 | 100 |
| Present condensed heterocyclic compound 46 + tebuconazole | 500+5 | 100 |
| Present condensed heterocyclic compound 46 + prothioconazole | 200+2000 | 100 |
| Present condensed heterocyclic compound 46 + prothioconazole | 500+5 | 100 |
| Present condensed heterocyclic compound 46 + metconazole | 200+2000 | 100 |
| Present condensed heterocyclic compound 46 + metconazole | 500+50 | 100 |
| Present condensed heterocyclic compound 46 + metconazole | 500+5 | 100 |
| Present condensed heterocyclic compound 46 + ipconazole | 200+2000 | 100 |
| Present condensed heterocyclic compound 46 + ipconazole | 500+5 | 100 |
| Present condensed heterocyclic compound 46 + triticonazole | 200+2000 | 100 |
| Present condensed heterocyclic compound 46 + triticonazole | 500+5 | 100 |
| Present condensed heterocyclic compound 46 + difenoconazole | 200+2000 | 100 |
| Present condensed heterocyclic compound 46 + difenoconazole | 500+5 | 100 |
| Present condensed heterocyclic compound 46 + tetraconazole | 200+2000 | 100 |
| Present condensed heterocyclic compound 46 + tetraconazole | 500+5 | 100 |
| Present condensed heterocyclic compound 46 + hexaconazole | 200+2000 | 100 |

(continued)

| Compounds provided for test | Concentration (ppm) | Mortality rate |
|---|---|---|
| Present condensed heterocyclic compound 46 + hexaconazole, | 500+50 | 100 |
| Present condensed heterocyclic compound 46 + hexaconazole | 500+5 | 100 |
| Present oondensed heterocyclic compound 105 + tebuconazole | 200+2000 | 100 |
| Present condensed heterocyclic compound 105 + tebuconazole | 500+50 | 100 |
| Present condensed heterocyclic compound 105 + tebuconazole | 500+5 | 100 |
| Present condensed heterocyclic compound 105 + prothioconazole | 200+2000 | 100 |
| Present condensed heterocyclic compound 105 + prothioconazole | 500+5 | 100 |
| Present condensed heterocyclic compound 105 + metconazole | 200+2000 | 100 |
| Present condensed heterocyclic compound 105 + metconazole | 500+50 | 100 |
| Present condensed heterocyclic compound 105 + metconazole | 500+5 | 100 |
| Present condensed heterocyclic compound 105 + ipconazole | 200+2000 | 100 |
| Present condensed heterocyclic compound 105 + ipconazole | 500+5 | 100 |
| Present condensed heterocyclic compound 105 + triticonazole | 200+2000 | 100 |
| Present condensed heterocyclic compound 105 + triticonazole | 500+5 | 100 |
| Present condensed heterocyclic compound 105 + difenoconazole | 200+2000 | 100 |
| Present condensed heterocyclic compound 105 + difenoconazole | 500+5 | 100 |
| Present condensed heterocyclic compound 105 + tetraconazole | 200+2000 | 100 |
| Present condensed heterocyclic compound 105 + tetraconazole | 500+5 | 100 |
| Present condensed heterocyclic compound 105 + hexaconazole | 200+2000 | 100 |
| Present condensed heterocyclic compound 105 + hexaconazole | 500+50 | 100 |
| Present condensed heterocyclic compound 105 + hexaconazole, | 500+5 | 100 |
| Non-treatment section | - | 0 |

Table 10

| Compounds provided for test | Concentration (ppm) | Mortality rate |
|---|---|---|
| Present condensed heterocyclic compound 91 + tebuconazole | 200+2000 | 100 |
| Present condensed heterocyclic compound 91 + tebuconazole | 500+50 | 100 |
| Present condensed heterocyclic compound 91 + tebuconazole | 500+5 | 100 |
| Present condensed heterocyclic compound 91 + prothioconazole | 200+2000 | 100 |
| Present condensed heterocyclic compound 91 + prothioconazole | 500+5 | 100 |
| Present condensed heterocyclic compound 91 + metconazole | 200+2000 | 100 |
| Present condensed heterocyclic compound 91 + metconazole | 500+50 | 100 |
| Present condensed heterocyclic compound 91 + metconazole | 500+5 | 100 |
| Present condensed heterocyclic compound 91 + ipconazole | 200+2000 | 100 |
| Present condensed heterocyclic compound 91 + ipconazole | 500+5 | 100 |
| Present condensed heterocyclic compound 91 + triticonazole | 200+2000 | 100 |
| Present condensed heterocyclic compound 91 + triticonazole | 500+5 | 100 |

(continued)

| Compounds provided for test | Concentration (ppm) | Mortality rate |
|---|---|---|
| Present condensed heterocyclic compound 91 + difenoconazole | 200+2000 | 100 |
| Present condensed heterocyclic compound 91 + difenoconazole | 500+5 | 100 |
| Present condensed heterocyclic compound 91 + tetraconazole | 200+2000 | 100 |
| Present condensed heterocyclic compound 91 + tetraconazole | 500+5 | 100 |
| Present condensed heterocyclic compound 91 + hexaconazole | 200+2000 | 100 |
| Present condensed heterocyclic compound 91 + hexaconazole | 500+50 | 100 |
| Present condensed heterocyclic compound 91 + hexaconazole | 500+5 | 100 |
| Present condensed heterocyclic compound 107 + tebuconazole | 200+2000 | 100 |
| Present condensed heterocyclic compound 107 + tebuconazole | 500+50 | 100 |
| Present condensed heterocyclic compound 107 + tebuconazole | 500+5 | 100 |
| Present condensed heterocyclic compound 107 + prothioconazole | 200+2000 | 100 |
| Present condensed heterocyclic compound 107 + prothioconazole | 500+5 | 100 |
| Present condensed heterocyclic compound 107 + metconazole | 200+2000 | 100 |
| Present condensed heterocyclic compound 107 + metconazole | 500+50 | 100 |
| Present condensed heterocyclic compound 107 + metconazole | 500+5 | 100 |
| Present condensed heterocyclic compound 107 + ipconazole | 200+2000 | 100 |
| Present condensed heterocyclic compound 107 + ipconazole | 500+5 | 100 |
| Present condensed heterocyclic compound 107 + triticonazole | 200+2000 | 100 |
| Present condensed heterocyclic compound 107 + triticonazole | 500+5 | 100 |
| Present condensed heterocyclic compound 107 + difenoconazole | 200+2000 | 100 |
| Present condensed heterocyclic compound 107 + difenoconazole | 500+5 | 100 |
| Present condensed heterocyclic compound 107 + tetraconazole | 200+2000 | 100 |
| Present condensed heterocyclic compound 107 + tetraconazole | 500+5 | 100 |
| Present condensed heterocyclic compound 107 + hexaconazole | 200+2000 | 100 |
| Present condensed heterocyclic compound 107 + hexaconazole | 500+50 | 100 |
| Present condensed heterocyclic compound 107 + hexaconazole | 500+5 | 100 |
| Non-treatment section | - | 0 |

Table 11

| compounds provided for test | Concentration (ppm) | Mortality rate |
|---|---|---|
| Present condensed heterocyclic compound 51 + tebuconazole | 200+2000 | 100 |
| Present condensed heterocyclic compound 51 + tebuconazole | 500+50 | 100 |
| Present condensed heterocyclic compound 51 + tebuconazole | 500+5 | 100 |
| Present condensed heterocyclic compound 51 + prothioconazole | 200+2000 | 100 |
| Present condensed heterocyclic compound 51 + prothioconazole | 500+5 | 100 |
| Present condensed heterocyclic compound 51 + metconazole | 200+2000 | 100 |
| Present condensed heterocyclic compound 51 + metconazole | 500+50 | 100 |

(continued)

| compounds provided for test | Concentration (ppm) | Mortality rate |
|---|---|---|
| Present condensed heterocyclic compound 51 + metconazole | 500+5 | 100 |
| Present condensed heterocyclic compound 51 + ipconazole | 200+2000 | 100 |
| Present condensed heterocyclic compound 51 + ipconazole | 500+5 | 100 |
| Present condensed heterocyclic compound 51 + triticonazole | 200+2000 | 100 |
| Present condensed heterocyclic compound 51 + triticonazole | 500+5 | 100 |
| Present condensed heterocyclic compound 51 + difenoconazole | 200+2000 | 100 |
| Present condensed heterocyclic compound 51 + difenoconazole | 500+5 | 100 |
| present condensed heterocyclic compound 51 + tetraconazole | 200+2000 | 100 |
| Present condensed heterocyclic compound 51 + tetraconazole | 500+5 | 100 |
| Present condensed heterocyclic compound 51 + hexaconazole | 200+2000 | 100 |
| Present condensed heterocyclic compound 51 + hexaconazole | 500+50 | 100 |
| Present condensed heterocyclic compound 51 + hexaconazole | 500+5 | 100 |
| Present condensed heterocyclic compound 27 + tebuconazole | 200+2000 | 100 |
| Present condensed heterocyclic compound 27 + tebuconazole | 500+50 | 100 |
| Present condensed heterocyclic compound 27 + tebuconazole | 500+5 | 100 |
| Present condensed heterocyclic compound 27 + prothioconazole | 200+2000 | 100 |
| Present condensed heterocyclic compound 27 + prothioconazole | 500+5 | 100 |
| Present condensed heterocyclic compound 27 + metconazole | 200+2000 | 100 |
| Present condensed heterocyclic compound 27 + metconazole | 500+50 | 100 |
| Present condensed heterocyclic compound 27 + metconazole | 500+5 | 100 |
| Present condensed heterocyclic compound 27 + ipconazole | 200+2000 | 100 |
| Present condensed heterocyclic compound 27 + ipconazole | 500+5 | 100 |
| Present condensed heterocyclic compound 27 + triticonazole | 200+2000 | 100 |
| Present condensed heterocyclic compound 27 + triticonazole | 500+5 | 100 |
| Present condensed heterocyclic compound 27 + difenoconazole | 200+2000 | 100 |
| Present condensed heterocyclic compound 27 + difenoconazole | 500+5 | 100 |
| Present condensed heterocyclic compound 27 + tetraconazole | 200+2000 | 100 |
| Present condensed heterocyclic compound 27 + tetraconazole | 500+5 | 100 |
| Present condensed heterocyclic compound 27 + hexaconazole | 200+2000 | 100 |
| Present condensed heterocyclic compound 27 + hexaconazole | 500+50 | 100 |
| Present condensed heterocyclic compound 27 + hexaconazole | 500+5 | 100 |
| Non-treatment section | - | 0 |

Table 12

| Compounds provided for test | Concentration (ppm) | Mortality rate |
|---|---|---|
| Present condensed heterocyclic compound 108 + tebuconazole | 200+2000 | 100 |
| Present condensed heterocyclic compound 108 + tebuconazole | 500+50 | 100 |

(continued)

| Compounds provided for test | Concentration (ppm) | Mortality rate |
|---|---|---|
| Present condensed heterocyclic compound 108 + tebuconazole | 500+5 | 100 |
| Present condensed heterocyclic compound 108 + prothioconazole | 200+2000 | 100 |
| Present condensed heterocyclic compound 108 + prothioconazole | 500+5 | 100 |
| Present condensed heterocyclic compound 108 + metconazole | 200+2000 | 100 |
| Present condensed heterocyclic compound 108 + metconazole | 500+50 | .100 |
| Present condensed heterocyclic compound 108 + metconazole | 500+5 | 100 |
| Present condensed heterocyclic compound 108 + ipconazole | 200+2000 | 100 |
| Present condensed heterocyclic compound 108 + ipconazole | 500+5 | 100 |
| Present condensed heterocyclic compound 108 + triticonazole | 200+2000 | 100 |
| Present condensed heterocyclic compound 108 + triticonazole | 500+5 | 100 |
| Present condensed heterocyclic compound 108 + difenoconazole | 200+2000 | 100 |
| Present condensed heterocyclic compound 108 + difenoconazole | 500+5 | 100 |
| Present condensed heterocyclic compound 108 + tetraconazole | 200+2000 | 100 |
| Present condensed heterocyclic compound 108 + tetraconazole | 500+5 | 100 |
| Present condensed heterocyclic compound 108 + hexaconazole | 200+2000 | 100 |
| Present condensed heterocyclic compound 108 + hexaconazole | 500+50 | 100 |
| Present condensed heterocyclic compound 108 + hexaconazole | 500+5 | 100 |
| Present condensed heterocyclic compound 48 + tebuconazole | 200+2000 | 100 |
| Present condensed heterocyclic compound 48 + tebuconazole | 500+50 | 100 |
| Present condensed heterocyclic compound 48 + tebuconazole | 500+5 | 100 |
| Present condensed heterocyclic compound 48 + prothioconazole | 200+2000 | 100 |
| Present condensed heterocyclic compound 48 + prothioconazole | 500+5 | 100 |
| Present condensed heterocyclic compound 48 + metconazole | 200+2000 | 100 |
| Present condensed heterocyclic compound 48 + metconazole | 500+50 | 100 |
| Present condensed heterocyclic compound 48 + metconazole | 500+5 | 100 |
| Present condensed heterocyclic compound 48 + ipconazole | 200+2000 | 100 |
| Present condensed heterocyclic compound 48 + ipconazole | 500+5 | 100 |
| Present condensed heterocyclic compound 48 + triticonazole | 200+2000 | 100 |
| Present condensed heterocyclic compound 48 + triticonazole | 500+5 | 100 |
| Present condensed heterocyclic compound 48 + difenoconazole | 200+2000 | 100 |
| Present condensed heterocyclic compound 48 + difenoconazole | 500+5 | 100 |
| Present condensed heterocyclic compound 48 + tetraconazole | 200+2000 | 100 |
| Present condensed heterocyclic compound 48 + tetraconazole | 500+5 | 100 |
| Present condensed heterocyclic compound 48 + hexaconazole | 200+2000 | 100 |
| Present condensed heterocyclic compound 48 + hexaconazole | 500+50 | 100 |
| Present condensed heterocyclic compound 48 + hexaconazole | 500+5 | 100 |
| Non-treatment section | - | 0 |

Table 13

| Compounds provided for test | Concentration (ppm) | Mortality rate |
|---|---|---|
| Present condensed heterocyclic compound 97 + tebuconazole | 200+2000 | 100 |
| Present condensed heterocyclic compound 97 + tebuconazole | 500+50 | 100 |
| Present condensed heterocyclic compound 97 + tebuconazole | 500+5 | 100 |
| Present condensed heterocyclic compound 97 + prothioconazole | 200+2000 | 100 |
| Present condensed heterocyclic compound 97 + prothioconazole | 500+5 | 100 |
| Present condensed heterocyclic compound 97 + metconazole | 200+2000 | 100 |
| Present condensed heterocyclic compound 97 + metconazole | 500+50 | 100 |
| Present condensed heterocyclic compound 97 + metconazole | 500+5 | 100 |
| Present condensed heterocyclic compound 97 + ipconazole | 200+2000 | 100 |
| Present condensed heterocyclic compound 97 + ipconazole | 500+5 | 100 |
| Present condensed heterocyclic compound 97 + triticonazole | 200+2000 | 100 |
| Present condensed heterocyclic compound 97 + triticonazole | 500+5 | 100 |
| Present condensed heterocyclic compound 97 + difenoconazole | 200+2000 | 100 |
| Present condensed heterocyclic compound 97 + difenoconazcle | 500+5 | 100 |
| Present condensed heterocyclic compound 97 + tetraconazole | 200+2000 | 100 |
| Present condensed heterocyclic compound 97 + tetraconazole | 500+5 | 100 |
| Present condensed heterocyclic compound 97 + hexaconazole | 200+2000 | 100 |
| Present condensed heterocyclic compound 97 + hexaconazole | 500+50 | 100 |
| Present condensed heterocyclic compound 97 + hexaconazole | 500+5 | 100 |
| Present condensed heterocyclic compound 69 + tebuconazole | 200+2000 | 100 |
| Present condensed heterocyclic compound 69 + tebuconazole | 500+50 | 100 |
| Present condensed heterocyclic compound 69 + tebuconazole | 500+5 | 100 |
| Present condensed heterocyclic compound 69 + prothioconazole | 200+2000 | 100 |
| Present condensed heterocyclic compound 69 + prothioconazole | 500+5 | 100 |
| Present condensed heterocyclic compound 69 + metconazole | 200+2000 | 100 |
| Present condensed heterocyclic compound 69 + metconazole | 500+50 | 100 |
| Present condensed heterocyclic compound 69 + metconazole | 500+5 | 100 |
| Present condensed heterocyclic compound 69 + ipconazole | 200+2000 | 100 |
| Present condensed heterocyclic compound 69 + ipconazole | 500+5 | 100 |
| Present condensed heterocyclic compound 69 + triticonazole | 200+2000 | 100 |
| Present condensed heterocyclic compound 69 + triticonazole | 500+5 | 100 |
| Present condensed heterocyclic compound 69 + difenoconazole | 200+2000 | 100 |
| Present condensed heterocyclic compound 69 + difenoconazole | 500+5 | 100 |
| Present condensed heterocyclic compound 69 + tetraconazole | 200+2000 | 100 |
| Present condensed heterocyclic compound 69 + tetraconazole | 500+5 | 100 |
| Present condensed heterocyclic compound 69 + hexaconazole | 200+2000 | 100 |
| Present condensed heterocyclic compound 69 + hexaconazole | 500+50 | 100 |

(continued)

| Compounds provided for test | Concentration (ppm) | Mortality rate |
|---|---|---|
| Present condensed heterocyclic compound 69 + hexaconazole | 500+5 | 100 |
| Non-treatment section | - | 0 |

Table 14

| Compounds provided for test | Concentration (ppm) | Mortality rate |
|---|---|---|
| Present condensed heterocyclic compound 109 + tebuconazole | 200+2000 | 100 |
| Present condensed heterocyclic compound 109 + tebuconazole | 500+50 | 100 |
| Present condensed heterocyclic compound 109 + tebuconazole | 500+5 | 100 |
| Present condensed heterocyclic compound 109 + prothioconazole | 200+2000 | 100 |
| Present condensed heterocyclic compound 109 + prothioconazole | 500+5 | 100 |
| Present condensed heterocyclic compound 109 + metconazole | 200+2000 | 100 |
| Present condensed heterocyclic compound 109 + metconazole | 500+50 | 100 |
| Present condensed heterocyclic compound 109 + metconazole | 500+5 | 100 |
| Present condensed heterocyclic compound 109 + ipconazole | 200+2000 | 100 |
| Present condensed heterocyclic compound 109 + ipconazole | 500+5 | 100 |
| Present condensed heterocyclic compound 109 + triticonazole | 200+2000 | 100 |
| Present condensed heterocyclic compound 109 + triticonazole | 500+5 | 100 |
| Present condensed heterocyclic compound 109 + difenoconazole | 200+2000 | 100 |
| Present condensed heterocyclic compound 109 + difenoccnazole | 500+5 | 100 |
| Present condensed heterocyclic compound 109 + tetraconazole | 200+2000 | 100 |
| Present condensed heterocyclic compound 109 + tetraconazole | 500+5 | 100 |
| Present condensed heterocyclic compound 109 + hexaconazole | 200+2000 | 100 |
| Present condensed heterocyclic compound 109 + hexaconazole | 500+50 | 100 |
| Present condensed heterocyclic compound 109 + hexaconazole | 500+5 | 100 |
| Present condensed heterocyclic compound 72 + tebuconazole | 200+2000 | 100 |
| Present condensed heterocyclic compound 72 + tebuconazole | 500+50 | 100 |
| Present condensed heterocyclic compound 72 + tebuconazole | 500+5 | 100 |
| Present condensed heterocyclic compound 72 + prothioconazole | 200+2000 | 100 |
| Present condensed heterocyclic compound 72 + prothioconazole | 500+5 | 100 |
| Present condensed heterocyclic compound 72 + metconazole | 200+2000 | 100 |
| Present condensed heterocyclic compound 72 + metconazole | 500+50 | 100 |
| Present condensed heterocyclic compound 72 + metconazole | 500+5 | 100 |
| Present condensed heterocyclic compound 72 + ipconazole | 200+2000 | 100 |
| Present condensed heterocyclic compound 72 + ipconazole | 500+5 | 100 |
| Present condensed heterocyclic compound 72 + triticonazole | 200+2000 | 100 |
| Present condensed heterocyclic compound 72 + triticonazole | 500+5 | 100 |
| Present condensed heterocyclic compound 72 + difenoconazole | 200+2000 | 100 |

(continued)

| Compounds provided for test | Concentration (ppm) | Mortality rate |
|---|---|---|
| Present condensed heterocyclic compound 72 + difenoconazole | 500+5 | 100 |
| Present condensed heterocyclic compound 72 + tetraconazole | 200+2000 | 100 |
| Present condensed heterocyclic compound 72 + tetraconazole | 500+5 | 100 |
| Present condensed heterocyclic compound 72 + hexaconazole | 200+2000 | 100 |
| Present condensed heterocyclic compound 72 + hexaconazole | 500+50 | 100 |
| Present condensed heterocyclic compound 72 + hexaconazole | 500+5 | 100 |
| Non-treatment section | - | 0 |

Table 15

| Compounds provided for test | Concentration (ppm) | Mortality rate |
|---|---|---|
| Present condensed heterocyclic compound 78 + tebuconazole | 200+2000 | 100 |
| Present condensed heterocyclic compound 78 + tebuconazole | 500+50 | 100 |
| Present condensed heterocyclic compound 78 + tebuconazole | 500+5 | 100 |
| Present condensed heterocyclic compound 78 + prothioconazole | 200+2000 | 100 |
| Present condensed heterocyclic compound 78 + prothioconazole | 500+5 | 100 |
| Present condensed heterocyclic compound 78 + metconazole | 200+2000 | 100 |
| Present condensed heterocyclic compound 78 + metconazole | 500+50 | 100 |
| Present condensed heterocyclic compound 78 + metconazole | 500+5 | 100 |
| Present condensed heterocyclic compound 78 + ipconazole | 200+2000 | 100 |
| Present condensed heterocyclic compound 78 + ipconazole | 500+5 | 100 |
| Present condensed heterocyclic compound 78 + triticonazole | 200+2000 | 100 |
| Present condensed heterocyclic compound 78 + triticonazole | 500+5 | 100 |
| Present condensed heterocyclic compound 78 + difenoconazole | 200+2000 | 100 |
| Present condensed heterocyclic compound 78 + difenoconazole | 500+5 | 100 |
| Present condensed heterocyclic compound 78 + tetraconazole | 200+2000 | 100 |
| Present condensed' heterocyclic compound 78 + tetraccnazole | 500+5 | 100 |
| Present condensed heterocyclic compound 78 + hexaconazole | 200+2000 | 100 |
| Present condensed heterocyclic compound 78 + hexaconazole | 500+50 | 100 |
| Present condensed heterocyclic compound 78 + hexaconazole | 500+5 | 100 |
| Present condensed heterocyclic compound 64 + tebuconazole | 200+2000 | 100 |
| Present condensed heterocyclic compound 64 + tebuconazole | 500+50 | 100 |
| Present condensed heterocyclic compound 64 + tebuconazole | 500+5 | 100 |
| Present condensed heterocyclic compound 64 + prothioconazole | 200+2000 | 100 |
| Present condensed heterocyclic compound 64 + prothioconazole | 500+5 | 100 |
| Present condensed heterocyclic compound 64 + metconazole | 200+2000 | 100 |
| Present condensed heterocyclic compound 64 + metconazole | 500+50 | 100 |
| Present condensed heterocyclic compound 64 + metconazole | 500+5 | 100 |

(continued)

| Compounds provided for test | Concentration (ppm) | Mortality rate |
|---|---|---|
| Present condensed heterocyclic compound 64 + ipconazole | 200+2000 | 100 |
| Present condensed heterocyclic compound 64 + ipconazole | 500+5 | 100 |
| Present condensed heterocyclic compound 64 + triticonazole | 200+2000 | 100 |
| Present condensed heterocyclic compound 64 + triticonazole | 500+5 | 100 |
| Present condensed heterocyclic compound 64 + difenoconazole | 200+2000 | 100 |
| Present condensed heterocyclic compound 64 + difenoconazole | 500+5 | 100 |
| Present condensed heterocyclic compound 64 + tetraconazole | 200+2000 | 100 |
| Present condensed heterocyclic compound 64 + tetraconazole | 500+5 | 100 |
| Present condensed heterocyclic compound 64 + hexaconazole | 200+2000 | 100 |
| Present condensed heterocyclic compound 64 + hexaconazole | 500+50 | 100 |
| Present condensed heterocyclic compound 64 + hexaconazole | 500+5 | 100 |
| Non-treatment section | - | 0 |

Table 16

| Compounds provided for test | Concentration (ppm) | Mortality rate |
|---|---|---|
| Present condensed heterocyclic compound 120 + tebuconazole | 200+2000 | 100 |
| Present condensed heterocyclic compound 120 + tebuconazole | 500+50 | 100 |
| Present condensed heterocyclic compound 120 + tebuconazole | 500+5 | 100 |
| Present condensed heterocyclic compound 120 + prothioconazole | 200+2000 | 100 |
| Present condensed heterocyclic compound 120 + prothioconazole | 500+5 | 100 |
| Present condensed heterocyclic compound 120 + metconazole | 200+2000 | 100 |
| Present condensed heterocyclic compound 120 + metconazole | 500+50 | 100 |
| Present condensed heterocyclic compound 120 + metconazole | 500+5 | 100 |
| Present condensed heterocyclic compound 120 + ipconazole | 200+2000 | 100 |
| Present condensed heterocyclic compound 120 + ipconazole | 500+5 | 100 |
| Present condensed heterocyclic compound 120 + triticonazole | 200+2000 | 100 |
| Present condensed heterocyclic compound 120 + triticonazole | 500+5 | 100 |
| Present condensed heterocyclic compound 120 + difenoconazole | 200+2000 | 100 |
| Present condensed heterocyclic compound 120 + difenoconazole | 500+5 | 100 |
| Present condensed heterocyclic compound 120 + tetraconazole | 200+2000 | 100 |
| Present condensed heterocyclic compound 120 + tetraconazole | 500+5 | 100 |
| Present condensed heterocyclic compound 120 + hexaconazole | 200+2000 | 100 |
| Present condensed heterocyclic compound 120 + hexaconazole | 500+50 | 100 |
| Present condensed heterocyclic compound 120 + hexaconazole | 500+5 | 100 |
| Present condensed heterocyclic compound 127 + tebuconazole | 200+2000 | 100 |
| Present condensed heterocyclic compound 127 + tebuconazole | 500+50 | 100 |
| Present condensed heterocyclic compound 127 + tebuconazole | 500+5 | 100 |

(continued)

| Compounds provided for test | Concentration (ppm) | Mortality rate |
|---|---|---|
| Present condensed heterocyclic compound 127 + prothioconazole | 200+2000 | 100 |
| Present condensed heterocyclic compound 127 + prothioconazole | 500+5 | 100 |
| Present condensed heterocyclic compound 127 + metconazole | 200+2000 | 100 |
| Present condensed heterocyclic compound 127 + metconazole | 500+50 | 100 |
| Present condensed heterocyclic compound 127 + metconazole | 500+5 | 100 |
| Present condensed heterocyclic compound 127 + ipconazole | 200+2000 | 100 |
| Present condensed heterocyclic compound 127 + ipconazole | 500+5 | 100 |
| Present condensed heterocyclic compound 127 + triticonazole | 200+2000 | 100 |
| Present condensed heterocyclic compound 127 + triticonazole | 500+5 | 100 |
| Present condensed heterocyclic compound 127 + difenoconazole | 200+2000 | 100 |
| Present condensed heterocyclic compound 127 + difenoconazole | 500+5 | 100 |
| Present condensed heterocyclic compound 127 + tetraconazole | 200+2000 | 100 |
| Present condensed heterocyclic compound 127 + tetraconazole | 500+5 | 100 |
| Present condensed heterocyclic compound 127 + hexaconazole | 200+2000 | 100 |
| Present condensed heterocyclic compound 127 + hexaconazole | 500+50 | 100 |
| Present condensed heterocyclic compound 127 + hexaconazole | 500+5 | 100 |
| Non-treatment section | - | 0 |

Table 17

| Compounds provided for test | Concentration (ppm) | Mortality rate |
|---|---|---|
| Present condensed heterocyclic compound 42 + tebuconazole | 200+2000 | 100 |
| Present condensed heterocyclic compound 42 + tebuconazole | 500+50 | 100 |
| Present condensed heterocyclic compound 42 + tebuconazole | 500+5 | 100 |
| Present condensed heterocyclic compound 42 + prothioconazole | 200+2000 | 100 |
| Present condensed heterocyclic compound 42 + prothioconazole | 500+5 | 100 |
| Present condensed heterocyclic compound 42 + metconazole | 200+2000 | 100 |
| Present condensed heterocyclic compound 42 + metconazole | 500+50 | 100 |
| Present condensed heterocyclic compound 42 + metconazole | 500+5 | 100 |
| Present condensed heterocyclic compound 42 + ipconazole | 200+2000 | 100 |
| Present condensed heterocyclic compound 42 + ipconazole | 500+5 | 100 |
| Present condensed heterocyclic compound 42 + triticonazole | 200+2000 | 100 |
| Present condensed heterocyclic compound 42 + triticonazole | 500+5 | 100 |
| Present condensed heterocyclic compound 42 + difenoconazole | 200+2000 | 100 |
| Present condensed heterocyclic compound 42 + difenoconazole | 500+5 | 100 |
| Present condensed heterocyclic compound 42 + tetraconazole | 200+2000 | 100 |
| Present condensed heterocyclic compound 42 + tetraconazole | 500+5 | 100 |
| Present condensed heterocyclic compound 42 + hexaconazole | 200+2000 | 100 |

(continued)

| Compounds provided for test | Concentration (ppm) | Mortality rate |
|---|---|---|
| Present condensed heterocyclic compound 42 + hexaconazole | 500+50 | 100 |
| Present condensed heterocyclic compound 42 + hexaconazole | 500+5 | 100 |
| Present condensed heterocyclic compound 41 + tebuconazole | 200+2000 | 100 |
| Present condensed heterocyclic compound 41 + tebuconazole | 500+50 | 100 |
| Present condensed heterocyclic compound 41 + tebuconazole | 500+5 | 100 |
| Present condensed heterocyclic compound 41 + prothioconazole | 200+2000 | 100 |
| Present condensed heterocyclic compound 41 + prothioconazole | 500+5 | 100 |
| Present condensed heterocyclic compound 41 + metconazole | 200+2000 | 100 |
| Present condensed heterocyclic compound 41 + metconazole | 500+50 | 100 |
| Present condensed heterocyclic compound 41 + metconazole | 500+5 | 100 |
| Present condensed heterocyclic compound 41 + ipconazole | 200+2000 | 100 |
| Present condensed heterocyclic compound 41 + ipconazole | 500+5 | 100 |
| Present condensed heterocyclic compound 41 + triticonazole | 200+2000 | 100 |
| Present condensed heterocyclic compound 41 + triticonazole | 500+5 | 100 |
| Present condensed heterocyclic compound 41 + difenoconazole | 200+2000 | 100 |
| Present condensed heterocyclic compound 41 + difenoconazole | 500+5 | 100 |
| Present condensed heterocyclic compound 41 + tetraconazole | 200+2000 | 100 |
| Present condensed heterocyclic compound 41 + tetraconazole | 500+5 | 100 |
| Present condensed heterocyclic compound 41 + hexaconazole | 200+2000 | 100 |
| Present condensed heterocyclic compound 41 + hexaconazole | 500+50 | 100 |
| Present condensed heterocyclic compound 41 + hexaconazole | 500+5 | 100 |
| Non-treatment section | - | 0 |

Table 18

| Compounds provided for test | Concentration (ppm) | Mortality rate |
|---|---|---|
| Present condensed heterocyclic compound 104 + tebuconazole | 200+2000 | 100 |
| Present condensed heterocyclic compound 104 + tebuconazole | 500+50 | 100 |
| Present condensed heterocyclic compound 104 + tebuconazole | 500+5 | 100 |
| Present condensed heterocyclic compound 104 + prothioconazole | 200+2000 | 100 |
| Present condensed heterocyclic compound 104 + prothioconazole | 500+5 | 100 |
| Present condensed heterocyclic compound 104 + metconazole | 200+2000 | 100 |
| Present condensed heterocyclic compound 104 + metconazole | 500+50 | 100 |
| Present condensed heterocyclic compound 104 + metconazole | 500+5 | 100 |
| Present condensed heterocyclic compound 104 + ipconazole | 200+2000 | 100 |
| Present condensed heterocyclic compound 104 + ipconazole | 500+5 | 100 |
| Present condensed heterocyclic compound 104 + triticonazole | 200+2000 | 100 |
| Present condensed heterocyclic compound 104 + triticonazole | 500+5 | 100 |

(continued)

| Compounds provided for test | Concentration (ppm) | Mortality rate |
|---|---|---|
| Present condensed heterocyclic compound 104 + difenoconazole | 200+2000 | 100 |
| Present condensed heterocyclic compound 104 + difenoconazole | 500+5 | 100 |
| Present condensed heterocyclic compound 104 + tetraconazole | 200+2000 | 100 |
| Present condensed heterocyclic compound 104 + tetraconazole | 500+5 | 100 |
| Present condensed heterocyclic compound 104 + hexaconazole | 200+2000 | 100 |
| Present condensed heterocyclic compound 104 + hexaconazole | 500+50 | 100 |
| Present condensed heterocyclic compound 104 + hexaconazole | 500+5 | 100 |
| Present condensed heterocyclic compound 118 + tebuconazole | 200+2000 | 100 |
| Present condensed heterocyclic compound 118 + tebuconazole | 500+50 | 100 |
| Present condensed heterocyclic compound 118 + tebuconazole | 500+5 | 100 |
| Present condensed heterocyclic compound 118 + prothioconazole | 200+2000 | 100 |
| Present condensed heterocyclic compound 118 + prothioconazole | 500+5 | 100 |
| Present condensed heterocyclic compound 118 + metconazole | 200+2000 | 100 |
| Present condensed heterocyclic compound 118 + metconazole | 500+50 | 100 |
| Present condensed heterocyclic compound 118 + metconazole | 500+5 | 100 |
| Present condensed heterocyclic compound 118 + ipconazole | 200+2000 | 100 |
| Present condensed heterocyclic compound 118 + ipconazole | 500+5 | 100 |
| Present condensed heterocyclic compound 118 + triticonazole | 200+2000 | 100 |
| Present condensed heterocyclic compound 118 + triticonazole | 500+5 | 100 |
| Present condensed heterocyclic compound 118 + difenoconazole | 200+2000 | 100 |
| Present condensed heterocyclic compound 118 + difenoconazole | 500+5 | 100 |
| Present condensed heterocyclic compound 118 + tetraconazole | 200+2000 | 100 |
| Present condensed heterocyclic compound 118 + tetraconazole | 500+5 | 100 |
| Present condensed heterocyclic compound 118 + hexaconazole | 200+2000 | 100 |
| Present condensed heterocyclic compound 118 + hexaconazole | 500+50 | 100 |
| Present condensed heterocyclic compound 118 + hexaconazole | 500+5 | 100 |
| Non-treatment section | - | 0 |

Table 19

| Compounds provided for test | Concentration (ppm) | Mortality rate |
|---|---|---|
| Present condensed heterocyclic compound 119 + tebuconazole | 200+2000 | 100 |
| Present condensed heterocyclic compound 119 + tebuconazole | 500+50 | 100 |
| Present condensed heterocyclic compound 119 + tebuconazole | 500+5 | 100 |
| Present condensed heterocyclic compound 119 + prothioconazole | 200+2000 | 100 |
| Present condensed heterocyclic compound 119 + prothioconazole | 500+5 | 100 |
| Present condensed heterocyclic compound 119 + metconazole | 200+2000 | 100 |
| Present condensed heterocyclic compound 119 + metconazole | 500+50 | 100 |

(continued)

| Compounds provided for test | Concentration (ppm) | Mortality rate |
|---|---|---|
| Present condensed heterocyclic compound 119 + metconazole | 500+5 | 100 |
| Present condensed heterocyclic compound 119 + ipconazole | 200+2000 | 100 |
| Present condensed heterocyclic compound 119 + ipconazole | 500+5 | 100 |
| Present condensed heterocyclic compound 119 + triticonazole | 200+2000 | 100 |
| Present condensed heterocyclic compound 119 + triticonazole | 500+5 | 100 |
| Present condensed heterocyclic compound 119 + difenoconazole | 200+2000 | 100 |
| Present condensed heterocyclic compound 119 + difenoconazole | 500+5 | 100 |
| Present condensed heterocyclic compound 119 + tetraconazole | 200+2000 | 100 |
| Present condensed heterocyclic compound 119 + tetraconazole | 500+5 | 100 |
| Present condensed heterocyclic compound 119 + hexaconazole | 200+2000 | 100 |
| Present condensed heterocyclic compound' 119 + hexaconazole | 500+50 | 100 |
| Present condensed heterocyclic compound 119 + hexaconazole | 500+5 | 100 |
| Present condensed heterocyclic compound 130 + tebuconazole | 200+2000 | 100 |
| Present condensed heterocyclic compound 130 + tebuconazole | 500+50 | 100 |
| Present condensed heterocyclic compound 130 + tebuconazole | 500+5 | 100 |
| Present condensed heterocyclic compound 130 + prothioconazole | 200+2000 | 100 |
| Present condensed heterocyclic compound 130 + prothioconazole | 500+5 | 100 |
| Present condensed heterocyclic compound 130 + metconazole | 200+2000 | 100 |
| Present condensed haterocyclic compound 130 + metconazole | 500+50 | 100 |
| Present condensed heterocyclic compound 130 + metconazole | 500+5 | 100 |
| Present condensed heterocyclic compound 130 + ipconazole | 200+2000 | 100 |
| Present condensed heterocyclic compound 130 + ipconazole | 500+5 | 100 |
| Present condensed heterocyclic compound 130 + triticonazole | 200+2000 | 100 |
| Present condensed heterocyclic compound 130 + triticonazole | 500+5 | 100 |
| Present condensed heterocyclic compound 130 + difenoconazole | 200+2000 | 100 |
| Present condensed heterocyclic compound 130 + difenoconazole | 500+5 | 100 |
| Present condensed heterocyclic compound 130 + tetraconazole | 200+2000 | 100 |
| Present condensed heterocyclic compound 130 + tetraconazole | 500+5 | 100 |
| Present condensed heterocyclic compound 130 + hexaconazole | 200+2000 | 100 |
| Present condensed heterocyclic compound 130 + hexaconazole | 500+50 | 100 |
| Present condensed heterocyclic compound 130 + hexaconazole | 500+5 | 100 |
| Present condensed heterocyclic compound 131 + tebuconazole | 200+2000 | 100 |
| Present condensed heterocyclic compound 131 + tebuconazole | 500+50 | 100 |
| Present condensed heterocyclic compound 131 + tebuconazole | 500+5 | 100 |
| Present condensed heterocyclic compound 131 + prothioconazole | 200+2000 | 100 |
| Present condensed heterocyclic compound 131 + prothioconazole | 500+5 | 100 |
| Present condensed heterocyclic compound 131 + metconazole | 200+2000 | 100 |
| Present condensed heterocyclic compound 131 + metconazole | 500+50 | 100 |

(continued)

| Compounds provided for test | Concentration (ppm) | Mortality rate |
|---|---|---|
| Present condensed heterocyclic compound 131 + metconazole | 500+5 | 100 |
| Present condensed heterocyclic compound 131 + ipconazole | 200+2000 | 100 |
| Present condensed heterocyclic compound 131 + ipconazole | 500+5 | 100 |
| Present condensed heterocyclic compound 131 + triticonazole | 200+2000 | 100 |
| Present condensed heterocyclic compound 131 + triticonazole | 500+5 | 100 |
| Present condensed heterocyclic compound 131 + difenoconazole | 200+2000 | 100 |
| Present condensed heterocyclic compound 131 + difenoconazole | 500+5 | 100 |
| Present condensed heterocyclic compound 131 + tetraconazole | 200+2000 | 100 |
| Present condensed heterocyclic compound 131 + tetraconazole | 500+5 | 100 |
| Present condensed heterocyclic compound 131 + hexaconazole | 200+2000 | 100 |
| Present condensed heterocyclic compound 131 + hexaconazole | 500+50 | 100 |
| Present condensed heterocyclic compound 131 + hexaconazole | 500+5 | 100 |
| Non-treatment section | - | 0 |

Test Example 2

1 ng of each of the present condensed heterocyclic compounds 3, 23, 27, 31, 39, 41, 42, 46, 48, 51, 56, 58, 64, 69, 72, 74, 78, 83, 91, 97, 104, 105, 107, 108, 109, 118, 119, 120, 127, 130, and 131 was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

Each of commercial formulations, such as azoxystrobin (trade name: Amistar, manufactured by Syngenta Japan K.K.), pyraclostrobin (trade name: Comet, manufactured by BASF Japan Ltd.), and trifloxystrobin (trade name: Flint, manufactured by Bayer CorpScience), was diluted with water which contains 0.02 volume% of a spreading agent (trade name: Shindain, manufactured by Sumitomo Chemical Co., Ltd.) so as to have a predetermined concentration.

1 mg of each of fluoxastrobin and the present amide compound (compound represented by Formula (2) : the same applies hereinafter), and orysastrobin (manufactured by Waco Pure Chemical Industries, Ltd.) was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A water-diluted solution of the above-described present condensed heterocyclic compounds and a water-diluted solution of azoxystrobin, pyraclostrobin, trifluoxastrobin, flucxastrobin, the present amide compound, or orysastrobin were mixed together to prepare a liquid medicine for a test.

Each leaf disc (diameter of 1.5 cm) of a cabbage (Brassicae oleracea) was accommodated in each well of a 24 hole microplate (manufactured by Becton Dickinson) and 40 μL of liquid medicine for a test was scattered to each well. Wells to which 40 μL of water containing 0.02 volume% of a spreading agent (trade name: Shindain, manufactured by Sumitomo Chemical Co., Ltd.) was scattered was set to a non-treatment section.

After air-drying, 5 second-instar larvae of diamondback moths (Plutella xylostella) were released in each well, which was then covered with paper towel and closed with a lid. 2 days after the releasing, the number of surviving larvae in each well was observed.

The mortality rates of the treatment section and the non-treatment section were calculated using the following Formula 1). The test was repeated once.

Formula 1) Mortality rate (%) = (the number of larvae provided for a test - the number of surviving larvae) / the number of larvae provided for a test x 100

Table 20

| Compounds provided for test | Concentration (ppm) | Mortality rate |
|---|---|---|
| Present condensed heterocyclic compound 3 + azoxystrobin | 200+2000 | 100 |
| Present condensed heterocyclic compound 3 + azoxystrobin | 500+50 | 100 |
| Present condensed heterocyclic compound 3 + azoxystrobin | 500+5 | 100 |
| Present condensed heterocyclic compound 3 + pyraclostrobin | 200+2000 | 100 |
| Present condensed heterocyclic compound 3 + pyraclostrobin | 500+5 | 100 |
| Present condensed heterocyclic compound 3 + trifloxystrobin | 200+2000 | 100 |
| Present condensed heterocyclic compound 3 + trifloxystrobin | 500+5 | 100 |
| Present condensed heterocyclic compound 3 + fluoxastrobin | 200+2000 | 100 |
| Present condensed heterocyclic compound 3 + fluoxaatrobin | 500+5 | 100 |
| Present condensed heterocyclic compound 3 + present amide compound | 200+2000 | 100 |
| Present condensed heterocyclic compound 3 + present amide compound | 500+50 | 100 |
| Present condensed heterocyclic compound 3 + present amide compound | 500+5 | 100 |
| Present condensed heterocyclic compound 3 + orysastrobin | 200+5000 | 100 |
| Present condensed heterocyclic compound 3 + orysastrobin | 500+50 | 100 |
| Present condensed heterocyclic compound 3 + orysastrobin | 500+5 | 100 |
| Present condensed heterocyclic compound 39 + azoxystrobin | 200+2000 | 100 |
| Present condensed heterocyclic compound 39 + azoxystrobin | 500+50 | 100 |
| Present condensed heterocyclic compound 39 + azoxystrobin | 500+5 | 100 |
| Present condensed heterocyclic compound 39 + pyraclostrobin | 200+2000 | 100 |
| Present condensed heterocyclic compound 39 + pyraclostrobin | 500+5 | 100 |
| Present condensed heterocyclic compound 39 + trifloxystrobin | 200+2000 | 100 |
| Present condensed heterooyolio compound 39 + trifloxystrobin | 500+5 | 100 |
| Present condensed heterocyclic compound 39 + fluoxastrobin | 200+2000 | 100 |
| Present condensed heterocyclic compound 39 + fluoxastrobin | 500+5 | 100 |
| Present condensed heterocyclic compound 39 + present amide compound | 200+2000 | 100 |
| Present condensed heterocyclic compound 39 + present amide compound | 500+50 | 100 |
| Present condensed heterocyclic compound 39 + present amide compound | 500+5 | 100 |
| Present condensed heterocyclic compound 39 + orysastrobin | 200+5000 | 100 |
| Present condensed heterocyclic compound 39 + orysastrobin | 500+50 | 100 |
| Present condensed heterocyclic compound 39 + orysastrobin | 500+5 | 100 |
| Present condensed heterocyclic compound 23 + azoxystrobin | 200+2000 | 100 |
| Present condensed heterocyclic compound 23 + azoxystrobin | 500+50 | 100 |

(continued)

| Compounds provided for test | Concentration (ppm) | Mortality rate |
|---|---|---|
| Present condensed heterocyclic compound 23 + azoxystrobin | 500+5 | 100 |
| Present condensed heterocyclic compound 23 + pyraclostrobin | 200+2000 | 100 |
| Present condensed heterocyclic compound 23 + pyraclostrobin | 500+5 | 100 |
| Present condensed heterocyclic compound 23 + trifloxystrobin | 200+2000 | 100 |
| Present condensed heterocyclic compound 23 + trifloxystrobin | 500+5 | 100 |
| Non-treatment section | - | 0 |

Table 21

| Compounds provided for test | Concentration (ppm) | Mortality rate |
|---|---|---|
| Present condensed heterocyclic compound 23 + fluoxastrobin | 200+2000 | 100 |
| Present condensed heterocyclic compound 23 + fluoxastrobin | 500+5 | 100 |
| Present condensed heterocyclic compound 23 + present amide compound | 200+2000 | 100 |
| Present condensed heterocyclic compound 23 + present amide compound | 500+50 | 100 |
| Present condensed heterocyclic compound 23 + present amide compound | 500+5 | 100 |
| Present condensed heterocyclic compound 23 + orysastrobin | 200+5000 | 100 |
| Present condensed heterocyclic compound 23 + orysastrobin | 500+50 | 100 |
| Present condensed heterocyclic compound 23 + orysastrobin | 500+5 | 100 |
| Present condensed heterocyclic compound 74 + azoxystrobin | 200+2000 | 100 |
| Present condensed heterocyclic compound 74 + azoxystrobin | 500+50 | 100 |
| Present condensed heterocyclic compound 74 + azoxystrobin | 500+5 | 100 |
| Present condensed heterocyclic compound 74 + pyraclostrobin | 200+2000 | 100 |
| Present condensed heterocyclic compound 74 + pyraclostrobin | 500+5 | 100 |
| Present condensed heterocyclic compound 74 + trifloxystrobin | 200+2000 | 100 |
| Present condensed heterocyclic compound 74 + trifloxystrobin | 500+5 | 100 |
| Present condensed heterocyclic compound 74 + fluoxasrtrobin | 200+2000 | 100 |
| Present condensed heterocyclic compound 74 + fluoxastrobin | 500+5 | 100 |
| Present condensed heterocyclic compound 74 + present amide compound | 200+2000 | 100 |
| Present condensed heterocyclic compound 74 + present amide compound | 500+5C | 100 |
| Present condensed heterocyclic compound 74 + present amide compound | 500+5 | 100 |
| Present condensed heterocyclic compound 74 + orysastrobin | 200+5000 | 100 |
| Present condensed heterocyclic compound 74 + orysastrobin | 500+50 | 100 |
| Present condensed heterocyclic compound 74 + orysastrobin | 500+5 | 100 |
| Present condensed heterocyclic compound 83 + azoxystrobin | 200+2000 | 100 |
| Present condensed heterocyclic compound 83 + azoxystrobin | 500+50 | 100 |
| Present condensed heterocyclic compound 83 + azoxystrobin | 500+5 | 100 |
| Present condensed heterocyclic compound 83 + pyraclostrobin | 200+2000 | 100 |
| Present condensed heterocyclic compound 83 + pyraclostrobin | 500+5 | 100 |

(continued)

| Compounds provided for test | Concentration (ppm) | Mortality rate |
|---|---|---|
| Present condensed heterocyclic compound 83 + trifloxystrobin | 200+2000 | 100 |
| Present condensed heterocyclic compound 83 + trifloxystrobin | 500+5 | 100 |
| Present condensed heterocyclic compound 83 + fluoxastrobin | 200+2000 | 100 |
| Present condensed heterocyclic compound 83 + flucxastrobin | 500+5 | 100 |
| Present condensed heterocyclic compound 83 + present amide compound | 200+2000 | 100 |
| Present condensed heterocyclic compound 83 + present amide compound | 500+50 | 100 |
| Present condensed heterocyclic compound 83 + present amide compound | 500+5 | 100 |
| Present condensed heterocyclic compound 83 + orysastrobin | 200+5000 | 100 |
| Present condensed heterocyclic compound 83 + orysastrobin | 500+50 | 100 |
| Present condensed heterocyclic compound 83 + orysastrobin | 500+5 | 100 |
| Non-treatment section | - | 0 |

Table 22

| Compounds provided for test | Concentration (ppm) | Mortality rate |
|---|---|---|
| Present condensed heterocyclic compound 58 + azoxystrobin | 200+2000 | 100 |
| Present condensed heterocyclic compound 58 + azoxystrobin | 500+50 | 100 |
| Present condensed heterocyclic compound 58 + | 500+5 | 100 |
| Present condensed heterocyclic compound 58 + pyraclostrobin | 200+2000 | 100 |
| Present condensed heterocyclic compound 58 + pyraclostrobin | 500+5 | 100 |
| Present condensed heterocyclic compound 58 + trifloxystrobin | 200+2000 | 100 |
| Present condensed heterocyclic compound 58 + trifloxystrobin | 500+5 | 100 |
| Present condensed heterocyclic compound 58 + fluoxastrobin | 200+2000 | 100 |
| Present condensed heterocyclic compound 58 + fluoxastrobin | 500+5 | 100 |
| Present condensed heterocyclic compound 5B + present amide compound | 200+2000 | 100 |
| Present condensed heterocyclic compound 58 + present amide compound | 500+50 | 100 |
| Present condensed heterocyclic compound 58 + present amide compound | 500+5 | 100 |
| Present condensed heterocyclic compound 58 + orysastrobin | 200+5000 | 100 |
| Present condensed heterocyclic compound 58 + orysastrobin | 500+50 | 100 |
| Present condensed heterocyclic compound 58 + orysastrobin | 500+5 | 100 |
| Present condensed heterocyclic compound 31 + azoxystrobin | 200+2000 | 100 |
| Present condensed heterocyclic compound 31 + azoxystrobin | 500+50 | 100 |
| Present condensed heterocyclic compound 31 + azoxystrobin | 500+5 | 100 |
| Present condensed heterocyclic compound 31 + pyraclostrobin | 200+2000 | 100 |
| Present condensed heterocyclic compound 31 + pyraclostrobin | 500+5 | 100 |
| Present condensed heterocyclic compound 31 + trifloxystrobin | 200+2000 | 100 |
| Present condensed heterocyclic compound 31 + trifloxystrobin | 500+5 | 100 |
| Present condensed 31 heterocyclic compound 31 + fluoxastrobin | 200+2000 | 100 |

(continued)

| Compounds provided for test | Concentration (ppm) | Mortality rate |
|---|---|---|
| Present condensed heterocyclic compound 31 + fluoxastrobin | 500+5 | 100 |
| Present condensed heterocyclic compound 31 + present amide compound | 200+2000 | 100 |
| Present condensed heterocyclic compound 31 + present amide compound | 500+50 | 100 |
| Present condensed heterocyclic compound 31 + present amide compound | 500+5 | 100 |
| Present condensed heterocyclic compound 31 + orysastrobin | 200+5000 | 100 |
| Present condensed heterocyclic compound 31 + orysastrobin | 500+50 | 100 |
| Present condensed heterocyclic compound 31 + orysastrobin | 500+5 | 100 |
| Present condensed heterocyclic compound 56 + azoxystrobin | 200+2000 | 100 |
| Present condensed heterocyclic compound 56 + azoxystrobin | 500+50 | 100 |
| Present condensed heterocyclic compound 56 + azoxystrobin | 500+5 | 100 |
| Present condensed heterocyclic compound 56 + pyraclostrobin | 200+2000 | 100 |
| Present condensed heterocyclic compound 56 + pyraclostrobin | 500+5 | 100 |
| Present condensed heterocyclic compound 56 + trifloxystrobin | 200+2000 | 100 |
| Present condensed heterocyclic compound 56 + trifloxystrobin | 500+5 | 100 |
| Non-treatment section | - | 0 |

Table 23

| Compounds provided for test | Concentration (ppm) | Mortality rate |
|---|---|---|
| Present condensed heterocyclic compound 56 + fluoxastrobin | 200+2000 | 100 |
| Present condensed heterocyclic compound 56 + fluoxastrobin | 500+5 | 100 |
| Present condensed heterocyclic compound 56 + present amide compound | 200+2000 | 100 |
| Present condensed heterocyclic compound 56 + present amide compound | 500+50 | 100 |
| Present condensed heterocyclic compound 56 + present amide compound | 500+5 | 100 |
| Present condensed heterocyclic compound 56 + orysastrobin | 200+5000 | 100 |
| Present condensed heterocyclic compound 56 + orysastrobin | 500+50 | 100 |
| Present condensed heterocyclic compound 56 + orysastrobin | 500+5 | 100 |
| Present condensed heterocyclic compound 46 + azoxystrobin | 200+2000 | 100 |
| Present condensed heterocyclic compound 46 + azoxystrobin | 500+50 | 100 |
| Present condensed heterocyclic compound 46 + azoxystrobin | 500+5 | 100 |
| Present condensed heterocyclic compound 46 + pyraclostrobin | 200+2000 | 100 |
| Present condensed heterocyclic compound 46 + pyraclostrobin | 500+5 | 100 |
| Present condensed heterocyclic compound 46 + trifloxystrobin | 200+2000 | 100 |
| Present condensed heterocyclic compound 46 + trifloxystrobin | 500+5 | 100 |
| Present condensed heterocyclic compound 46 + fluoxastrobin | 200+2000 | 100 |
| Present condensed heterocyclic compound 46 + fluoxastrobin | 500+5 | 100 |
| Present condensed heterocyclic compound 46 + present amide compound | 200+2000 | 100 |
| Present condensed heterocyclic compound 46 + present amide compound | 500+50 | 100 |

(continued)

| Compounds provided for test | Concentration (ppm) | Mortality rate |
|---|---|---|
| Present condensed heterocyclic compound 46 + present amide compound | 500+5 | 100 |
| Present condensed heterocyclic compound 46 + orysastrobin | 200+5000 | 100 |
| Present condensed heterocyclic compound 46 + orysastrobin | 500+50 | 100 |
| Present condensed heterocyclic compound 46 + crysastrobin | 500+5 | 100 |
| Present condensed heterocyclic compound 105 + azoxystrobin | 200+2000 | 100 |
| Present condensed heterocyclic compound 105 + azoxystrobin | 500+50 | 100 |
| Present condensed heterocyclic compound 105 + azoxystrobin | 500+5 | 100 |
| Present condensed heterocyclic compound 105 + pyraclostrobin | 200+2000 | 100 |
| Present condensed heterocyclic compound 105 + pyraclostrobin | 500+5 | 100 |
| Present condensed heterocyclic compound 105 + trifloxystrobin | 200+2000 | 100 |
| Present condensed heterocyclic compound 105 + trifloxystrobin | 500+5 | 100 |
| Present condensed heterocyclic compound 105 + fluoxastrobin | 200+2000 | 100 |
| Present condensed heterocyclic compound 105 + fluoxastrobin | 500+5 | 100 |
| Present condensed heterocyclic compound 105 + present amide compound | 200+2000 | 100 |
| Present condensed heterocyclic compound 105 + present amide compound | 500+50 | 100 |
| Present condensed heterocyclic compound 105 + present amide compound | 500+5 | 100 |
| Present condensed heterocyclic compound 105 + orysastrobin | 200+5000 | 100 |
| Present condensed heterocyclic compound 105 + orysastrobin | 500+50 | 100 |
| Present condensed heterocyclic compound 105 + orysastrobin | 500+5 | 100 |
| Non-treatment section | - | 0 |

Table 24

| Compounds provided for test | Concentration (ppm) | Mortality rate |
|---|---|---|
| Present condensed heterocyclic compound 91 + azoxystrobin | 200+2000 | 100 |
| Present condensed heterocyclic compound 91 + azoxystrobin | 500+50 | 100 |
| Present condensed heterocyclic compound 91 + azoxystrobin | 500+5 | 100 |
| Present condensed heterocyclic compound 91 + pyraclostrobin | 200+2000 | 100 |
| Present condensed heterocyclic compound 91 + pyraclostrobin | 500+5 | 100 |
| Present condensed heterocyclic compound 91 + trifloxystrobin | 200+2000 | 100 |
| Present condensed heterocyclic compound 91 + trifloxystrobin | 500+5 | 100 |
| Present condensed heterocyclic compound 91 + fluoxastrobin | 200+2000 | 100 |
| Present condensed heterocyclic compound 91 + fluoxastrobin | 500+5 | 100 |
| Present condensed heterocyclic compound 91 + present amide compound | 200+2000 | 100 |
| Present condensed heterocyclic compound 91 + present amide compound | 500+50 | 100 |
| Present condensed heterocyclic compound 91 + present amide compound | 500+5 | 100 |
| Present condensed heterocyclic compound 91 + orysastrobin | 200+5000 | 100 |
| Present condensed heterocyclic compound 91 + orysastrobin | 500+50 | 100 |

(continued)

| Compounds provided for test | Concentration (ppm) | Mortality rate |
|---|---|---|
| Present condensed heterocyclic compound 91 + orysastrobin | 500+5 | 100 |
| Present condensed heterocyclic compound 107 + azoxystrobin | 200+2000 | 100 |
| Present condensed heterocyclic compound 107 + azoxystrobin | 500+50 | 100 |
| Present condensed heterocyclic compound 107 + azoxystrobin | 500+5 | 100 |
| Present condensed heterocyclic compound 107 + pyraclostrobin | 200+2000 | 100 |
| Present condensed heterocyclic compound 107 + pyraclostrobin | 500+5 | 100 |
| Present condensed heterocyclic compound 107 + trifloxystrobin | 200+2000 | 100 |
| Present condensed heterocyclic compound 107 + trifloxystrobin | 500+5 | 100 |
| Present condensed heterocyclic compound 107 + fluoxastrobin | 200+2000 | 100 |
| Present condensed heterocyclic compound 107 + fluoxastrobin | 500+5 | 100 |
| Present condensed heterocyclic compound 107 + present amide compound | 200+2000 | 100 |
| Present condensed heterocyclic compound 107 + present amide compound | 500+50 | 100 |
| Present condensed heterocyclic compound 107 + present amide compound | 500+5 | 100 |
| Present condensed heterocyclic compound 107 + orysastrobin | 200+5000 | 100 |
| Present condensed heterocyclic compound 107 + orysastrobin | 500+50 | 100 |
| Present condensed heterocyclic compound 107 + orysastrobin | 500+5 | 100 |
| Present condensed heterocyclic compound 51 + azoxystrobin | 200+2000 | 100 |
| Present condensed heterocyclic compound 51 + azoxystrobin | 500+50 | 100 |
| Present condensed heterocyclic compound 51 + azoxystrobin | 500+5 | 100 |
| Present condensed heterocyclic compound 51 + pyraclostrobin | 200+2000 | 100 |
| Present condensed heterooyolio compound 51 + pyraclostrobin | 500+5 | 100 |
| Present condensed heterocyclic compound 51 + trifloxystrobin | 200+2000 | 100 |
| Present condensed heterocyclic compound 51 + trifloxystrobin | 500+5 | 100 |
| Non-treatment section | - | 0 |

Table 25

| Compounds provided for test | Concentration (ppm) | Mortality rate |
|---|---|---|
| Present condensed heterocyclic compound 51 + fluoxastrobin | 200+2000 | 100 |
| Present condensed heterocyclic compound 51 + fluoxastrobin | 500+5 | 100 |
| Present condensed heterocyclic compound 51 + present amide compound | 200+2000 | 100 |
| Present condensed heterocyclic compound 51 + present amide compound | 500+50 | 100 |
| Present condensed heterocyclic compound 51 + present amide compound | 500+5 | 100 |
| Present condensed heterocyclic compound 51 + orysastrobin | 200+5000 | 100 |
| Present condensed heterocyclic compound 51 + orysastrobin | 500+50 | 100 |
| Present condensed heterocyclic compound 51 + orysastrobin | 500+5 | 100 |
| Present condensed heterocyclic compound 27 + azoxystrobin | 200+2000 | 100 |
| Present condensed heterocyclic compound 27 + azoxystrobin | 500+50 | 100 |

(continued)

| Compounds provided for test | Concentration (ppm) | Mortality rate |
|---|---|---|
| Present condensed heterocyclic compound 27 + azoxystrobin | 500+5 | 100 |
| Present condensed heterocyclic compound 27 + pyraclostrobin | 200+2000 | 100 |
| Present condensed heterocyclic compound 27 + pyraclostrobin | 500+5 | 100 |
| Present condensed heterocyclic compound 27 + trifloxystrobin | 200+2000 | 100 |
| Present condensed heterocyclic compound 27 + trifloxystrobin | 500+5 | 100 |
| Present condensed heterocyclic compound 27 + fluoxastrobin | 200+2000 | 100 |
| Present condensed heterocyclic compound 27 + fluoxastrobin | 500+5 | 100 |
| Present condensed heterocyclic compound 27 + present amide compound | 200+2000 | 100 |
| Present condensed heterocyclic compound 27 + present amide compound | 500+50 | 100 |
| Present condensed heterocyclic compound 27 + present amide compound | 500+5 | 100 |
| Present condensed heterocyclic compound 27 + orysastrobin | 200+5000 | 100 |
| Present condensed heterocyclic compound 27 + orysastrobin | 500+50 | 100 |
| Present condensed heterocyclic compound 27 + orysastrobin | 500+5 | 100 |
| Present condensed heterocyclic compound 108 + azoxystrobin | 200+2000 | 100 |
| Present condensed heterocyclic compound 108 + azoxystrobin | 500+50 | 100 |
| Present condensed heterocyclic compound 108 + azoxystrobin | 500+5 | 100 |
| Present condensed heterocyclic compound 108 + pyraclostrobin | 200+2000 | 100 |
| Present condensed heterocyclic compound 108 + pyraclostrobin | 500+5 | 100 |
| Present condensed heterocyclic compound 108 + trifloxystrobin | 200+2000 | 100 |
| Present condensed heterocyclic compound 108 + trifloxystrobin | 500+5 | 100 |
| Present condensed heterocyclic compound 108 + fluoxastrobin | 200+2000 | 100 |
| Present condensed heterocyclic compound 108 + fluoxastrobin | 500+5 | 100 |
| Present condensed heterocyclic compound 108 + present amide compound | 200+2000 | 100 |
| Present condensed heterocyclic compound 108 + present amide compound | 500+50 | 100 |
| Present condensed heterocyclic compound 108 + present amide compound | 500+5 | 100 |
| Present condensed heterocyclic compound 108 + orysastrobin | 200+5000 | 100 |
| Present condensed heterocyclic compound 108 + orysastrobin | 500+50 | 100 |
| Present condensed heterocyclic compound 108 + orysastrobin | 500+5 | 100 |
| Non-treatment section | - | 0 |

Table 26

| Compounds provided for test | Concentration (ppm) | Mortality rate |
|---|---|---|
| Present condensed heterocyclic compound 48 + azoxystrobin | 200+2000 | 100 |
| Present condensed heterocyclic compound 48 + azoxystrobin | 500+50 | 100 |
| Present condensed heterocyclic compound 48 + azoxystrobin | 500+5 | 100 |
| Present condensed heterocyclic compound 48 + pyraclostrobin | 200+2000 | 100 |
| Present condensed heterocyclic compound 48 + pyraclostrobin | 500+5 | 100 |

(continued)

| Compounds provided for test | Concentration (ppm) | Mortality rate |
|---|---|---|
| Present condensed heterocyclic compound 48 + trifloxystrobin | 200+2000 | 100 |
| Present condensed heterocyclic compound 48 trifloxystrobin | 500+5 | 100 |
| Present condensed heterocyclic compound 48 + fluoxastrobin | 200+2000 | 100 |
| Present condensed heterocyclic compound 48 + fluoxastrobin | 500+5 | 100 |
| Present condensed heterocyclic compound 48 + present amide compound | 200+2000 | 100 |
| Present condensed heterocyclic compound 48 + present amide compound | 500+50 | 100 |
| Present condensed heterocyclic compound 48 + present amide compound | 500+5 | 100 |
| Present condensed heterocyclic compound 48 + orysastrobin | 200+5000 | 100 |
| Present condensed heterocyclic compound 48 + orysastrobin | 500+50 | 100 |
| Present condensed heterocyclic compound 48 + orysastrobin | 500+5 | 100 |
| Present condensed heterocyclic compound 97 + azoxystrobin | 200+2000 | 100 |
| Present condensed heterocyclic compound 97 + azoxystrobin | 500+50 | 100 |
| Present condensed heterocyclic compound 97 + azoxystrobin | 500+5 | 100 |
| Present condensed heterocyclic compound 97 + pyraclostrobin | 200+2000 | 100 |
| Present condensed heterocyclic compound 97 + pyraclostrobin | 500+5 | 100 |
| Present condensed heterocyclic compound 97 + trifloxystrobin | 200+2000 | 100 |
| Present condensed heterocyclic compound 97 + trifloxystrobin | 500+5 | 100 |
| Present condensed heterocyclic compound 97 + fluoxastrobin | 200+2000 | 100 |
| Present condensed heterocyclic compound 97 + fluoxastrobin | 500+5 | 100 |
| Present condensed heterocyclic compound 97 + present amide compound | 200+2000 | 100 |
| Present condensed heterocyclic compound 97 + present amide compound | 500+50 | 100 |
| Present condensed heterocyclic compound 97 + present amide compound | 500+5 | 100 |
| Present condensed heterocyclic compound 97 + orysastrobin | 200+5000 | 100 |
| Present condensed heterocyclic compound 97 + orysastrobin | 500+50 | 100 |
| Present condensed heterocyclic compound 97 + orysastrobin | 500+5 | 100 |
| Present condensed heterocyclic compound 69 + azoxystrobin | 200+2000 | 100 |
| Present condensed heterocyclic compound 69 + azoxystrobin | 500+50 | 100 |
| Present condensed heterocyclic compound 69 + azoxystrobin | 500+5 | 100 |
| Present condensed heterocyclic compound 69 + pyraclostrobin | 200+2000 | 100 |
| Present condensed heterocyclic compound 69 + pyraclostrobin | 500+5 | 100 |
| Present condensed heterocyclic compound 69 + trifloxystrobin | 200+2000 | 100 |
| Present condensed heterocyclic compound 69 + trifloxystrobin | 500+5 | 100 |
| Present condensed heterocyclic compound 69 + fluoxastrobin | 200+2000 | 100 |
| Present condensed heterocyclic compound 69 + fluoxastrobin | 500+5 | 100 |
| Non-treatment section | - | 0 |

Table 27

| Compounds provided for test | Concentration (ppm) | Mortality rate |
|---|---|---|
| Present condensed heterocyclic compound 69 + present amide compound | 200+2000 | 100 |
| Present condensed heterocyclic compound 69 + present amide compound | 500+50 | 100 |
| Present condensed heterocyclic compound 69 + present amide compound | 500+5 | 100 |
| Present condensed heterocyclic compound 69 + orysastrobin | 200+5000 | 100 |
| Present condensed heterocyclic compound 69 + orysastrobin | 500+50 | 100 |
| Present condensed heterocyclic compound 69 + orysastrobin | 500+5 | 100 |
| Present condensed heterocyclic compound 109 + azoxystrobin | 200+2000 | 100 |
| Present condensed heterocyclic compound 109 + azoxvstrobin | 500+50 | 100 |
| Present condensed heterocyclic compound 109 + azoxystrobin | 500+5 | 100 |
| Present condensed heterocyclic compound 109 + pyraclostrobin | 200+2000 | 100 |
| Present condensed heterocyclic compound 109 + pyraclostrobin | 500+5 | 100 |
| Present condensed heterocyclic compound 109 + trifloxystrobin | 200+2000 | 100 |
| Present condensed heterocyclic compound 109 + trifloxystrobin | 500+5 | 100 |
| Present condensed heterocyclic compound 109 + fluoxastrobin | 200+2000 | 100 |
| Present condensed heterocyclic compound 109 + fluoxastrobin | 500+5 | 100 |
| Present condensed heterocyclic compound 109 + present amide compound | 200+2000 | 100 |
| Present condensed heterocyclic compound 109 + present amide compound | 500+50 | 100 |
| Present condensed heterocyclic compound 109 + present amide compound | 500+5 | 100 |
| Present condensed heterocyclic compound 109 + orysastrobin | 200+5000 | 100 |
| Present condensed heterocyclic compound 109 + orysastrobin | 500+50 | 100 |
| Present condensed heterocyclic compound 109 + orysastrobin | 500+5 | 100 |
| Present condensed heterocyclic compound 72 + azoxvstrobin | 200+2000 | 100 |
| Present condensed heterocyclic compound 72 + azoxystrobin | 500+50 | 100 |
| Present condensed heterocyclic compound 72 + azoxystrobin | 500+5 | 100 |
| Present condensed heterocyclic compound 72 + pyraclostrobin | 200+2000 | 100 |
| Present condensed heterocyclic compound 72 + pyraclcstrobin | 500+5 | 100 |
| Present condensed heterocyclic compound 72 + trifloxystrobin | 200+2000 | 100 |
| Present condensed heterocyclic compound 72 + trifloxystrobin | 500+5 | 100 |
| Present condensed heterocyclic compound 72 + fluoxastrobin | 200+2000 | 100 |
| Present condensed heterocyclic compound 72 + fluoxastrobin | 500+5 | 100 |
| Present condensed heterocyclic compound 72 + present amide compound | 200+2000 | 100 |
| Present condensed heterocyclic compound 72 + present amide compound | 500+50 | 100 |
| Present condensed heterocyclic compound 72 + present amide compound | 500+5 | 100 |
| Present condensed heterocyclic compound 72 + orysastrobin | 200+5000 | 100 |
| Present condensed heterocyclic compound 72 + orysastrobin | 500+50 | 100 |
| Present condensed heterocyclic compound 72 + orysastrobin | 500+5 | 100 |
| Present condensed heterocyclic compound 78 + azoxystrobin | 200+2000 | 100 |
| Present condensed heterocyclic compound 78 + azoxystrobin | 500+50 | 100 |

(continued)

| Compounds provided for test | Concentration (ppm) | Mortality rate |
|---|---|---|
| Present condensed heterocyclic compound 78 + azoxystrobin | 500+5 | 100 |
| Non-treatment section | - | 0 |

Table 28

| Compounds provided for test | Concentration (ppm) | Mortality rate |
|---|---|---|
| Present condensed heterocyclic compound 78 + pyraclostrobin | 200+2000 | 100 |
| Present condensed heterocyclic compound 78 + pyraclostrobin | 500+5 | 100 |
| Present condensed heterocyclic compound 78 + trifloxystrobin | 200+2000 | 100 |
| Present condensed heterocyclic compound 78 + trifloxystrobin | 500+5 | 100 |
| Present condensed heterocyclic compound 78 + fluoxastrobin | 200+2000 | 100 |
| Present condensed heterocyclic compound 78 + fluoxastrobin | 500+5 | 100 |
| Present condensed heterocyclic compound 78 + present amide compound | 200+2000 | 100 |
| Present condensed heterocyclic compound 78 + present amide compound | 500+50 | 100 |
| Present condensed heterocyclic compound 78 + present amide compound | 500+5 | 100 |
| Present condensed heterocyclic compound 78 + orysastrobin | 200+5000 | 100 |
| Present condensed heterocyclic compound 78 + orysastrobin | 500+50 | 100 |
| Present condensed heterocyclic compound 78 + orysastrobin | 500+5 | 100 |
| Present condensed heterocyclic compound 64 + azoxystrobin | 200+2000 | 100 |
| Present condensed heterocyclic compound 64 + azoxystrobin | 500+50 | 100 |
| Present condensed heterocyclic compound 64 + azoxystrobin | 500+5 | 100 |
| Present condensed heterocyclic compound 64 + pyraclostrobin | 200+2000 | 100 |
| Present condensed heterocyclic compound 64 + pyraclostrobin | 500+5 | 100 |
| Present condensed heterocyclic compound 64 + trifloxystrobin | 200+2000 | 100 |
| Present condensed heterocyclic compound 64 + trifloxystrobin | 500+5 | 100 |
| Present condensed heterocyclic compound 64 + fluoxastrobin | 200+2000 | 100 |
| Present condensed heterocyclic compound 64 + fluoxastrobin | 500+5 | 100 |
| Present condensed heterocyclic compound 64 + present amide compound | 200+2000 | 100 |
| Present condensed heterocyclic compound 64 + present amide compound | 500+50 | 100 |
| Present condensed heterocyclic compound 64 + present amide compound | 500+5 | 100 |
| Present condensed heterocyclic compound 64 + orysastrobin | 200+5000 | 100 |
| Present condensed heterocyclic compound 64 + orysastrobin | 500+50 | 100 |
| Present condensed heterocyclic compound 64 + orysastrobin | 500+5 | 100 |
| Present condensed heterocyclic compound 120 + azoxystrobin | 200+2000 | 100 |
| Present condensed heterocyclic compound 120 + azoxystrobin | 500+50 | 100 |
| Present condensed heterocyclic compound 120 + azoxystrobin | 500+5 | 100 |
| Present condensed heterocyclic compound 120 + pyraclostrobin | 200+2000 | 100 |
| Present condensed heterocyclic compound 120 + pyraclostrobin | 500+5 | 100 |

(continued)

| Compounds provided for test | Concentration (ppm) | Mortality rate |
|---|---|---|
| Present condensed heterocyclic compound 120 + trifloxystrobin | 200+2000 | 100 |
| Present condensed heterocyclic compound 120 + trifloxystrobin | 500+5 | 100 |
| Present condensed heterocyclic compound 120 + fluoxastrobin | 200+2000 | 100 |
| Present condensed heterocyclic compound 120 + fluoxastrobin | 500+5 | 100 |
| Present condensed heterocyclic compound 120 + present amide compound | 200+2000 | 100 |
| Present condensed heterocyclic compound 120 + present amide compound | 500+50 | 100 |
| Present condensed heterocyclic compound 120 + present amide compound | 500+5 | 100 |
| Non-treatment section | - | 0 |

Table 29

| Compounds provided for test | Concentration (ppm) | Mortality rate |
|---|---|---|
| Present condensed heterocyclic compound 120 + orysastrobin | 200+5000 | 100 |
| Present condensed heterocyclic compound 120 + orysastrobin | 500+50 | 100 |
| Present condensed heterocyclic compound 120 + orysastrobin | 500+5 | 100 |
| Present condensed heterocyclic compound 127 + azoxystrobin | 200+2000 | 100 |
| Present condensed heterocyclic compound 127 + azoxystrobin | 500+50 | 100 |
| Present condensed heterocyclic compound 127 + azoxystrobin | 500+5 | 100 |
| Present condensed heterocyclic compound 127 + pyraclostrobin | 200+2000 | 100 |
| Present condensed heterocyclic compound 127 + pyraclostrobin | 500+5 | 100 |
| Present condensed heterocyclic compound 127 + trifloxystrobin | 200+2000 | 100 |
| Present condensed heterocyclic compound 127 + trifloxystrobin | 500+5 | 100 |
| Present condensed heterocyclic compound 127 + fluoxastrobin | 200+2000 | 100 |
| Present condensed heterocyclic compound 127 + fluoxastrobin | 500+5 | 100 |
| Present condensed heterocyclic compound 127 + present amide compound | 200+2000 | 100 |
| Present condensed heterocyclic compound 127 + present amide compound | 500+50 | 100 |
| Present condensed heterocyclic compound 127 + present amide compound | 500+5 | 100 |
| Present condensed heterocyclic compound 127 + orvsastrobin | 200+5000 | 100 |
| Present condensed heterocyclic compound 127 + orysastrobin | 500+50 | 100 |
| Present condensed heterocyclic compound 127 + orysastrobin | 500+5 | 100 |
| Present condensed heterocyclic compound 42 + azoxystrobin | 200+2000 | 100 |
| Present condensed heterocyclic compound 42 + azoxystrobin | 500+50 | 100 |
| Present condensed heterocyclic compound 42 + azoxystrobin | 500+5 | 100 |
| Present condensed heterocyclic compound 42 + pyraclostrobin | 200+2000 | 100 |
| Present condensed heterocyclic compound 42 + pyraclostrobin | 500+5 | 100 |
| Present condensed heterocyclic compound 42 + trifloxystrobin | 200+2000 | 100 |
| Present condensed heterocyclic compound 42 + trifloxystrobin | 500+5 | 100 |
| Present condensed heterocyclic compound 42 + fluoxastrobin | 200+2000 | 100 |

(continued)

| Compounds provided for test | Concentration (ppm) | Mortality rate |
|---|---|---|
| Present condensed heterocyclic compound 42 + fluoxastrobin | 500+5 | 100 |
| Present condensed heterocyclic compound 42 + present amide compound | 200+2000 | 100 |
| Present condensed heterocyclic compound 42 + present amide compound | 500+50 | 100 |
| Present condensed heterocyclic compound 42 + present amide compound | 500+5 | 100 |
| Present condensed heterocyclic compound 42 + orysastrobin | 200+5000 | 100 |
| Present condensed heterocyclic compound 42 + orysastrobin | 500+50 | 100 |
| Present condensed heterocyclic compound 42 + orysastrobin | 500+5 | 100 |
| Present condensed heterocyclic compound 41 + azoxystrobin | 200+2000 | 100 |
| Present condensed heterocyclic compound 41 + azoxystrobin | 500+50 | 100 |
| Present condensed heterocyclic compound 41 + azoxystrobin | 500+5 | 100 |
| Present condensed heterocyclic compound 41 + pyraclostrobin | 200+2000 | 100 |
| Present condensed heterocyclic compound 41 + pyraclostrobin | 500+5 | 100 |
| Present condensed heterocyclic compound 41 + trifloxystrobin | 200+2000 | 100 |
| Present condensed heterocyclic compound 41 + trifloxystrobin | 500+5 | 100 |
| Non-treatment section | - | 0 |

Table 30

| Compounds provided for test | Concentration (ppm) | Mortality rate |
|---|---|---|
| Present condensed heterocyclic compound 41 + fluoxastrobin | 200+2000 | 100 |
| Present condensed heterocyclic compound 41 + fluoxastrobin | 500+5 | 100 |
| Present condensed heterocyclic compound 41 + present amide compound | 200+2000 | 100 |
| Present condensed heterocyclic compound 41 + Present amide compound | 500+50 | 100 |
| Present condensed heterocyclic compound 41 + present amide compound | 500+5 | 100 |
| Present condensed heterocyclic compound 41 + orvsastrobin | 200+5000 | 100 |
| Present condensed heterocyclic compound 41 + orysastrobin | 500+50 | 100 |
| Present condensed heterocyclic compound 41 + orysastrobin | 500+5 | 100 |
| Present condensed heterocyclic compound 104 + azoxystrobin | 200-2000 | 100 |
| Present condensed heterocyclic compound 104 + azoxystrobin | 500+50 | 100 |
| Present condensed heterocyclic compound 104 + azoxystrobin | 500+5 | 100 |
| Present condensed heterocyclic compound 104 + pyraclostrobin | 200+2000 | 100 |
| Present condensed heterocyclic compound 104 + pyraclostrobin | 500+5 | 100 |
| Present condensed heterocyclic compound 104 + trifloxystrobin | 200+2000 | 100 |
| Present condensed heterocyclic compound 104 + trifloxystrobin | 500+5 | 100 |
| Present condensed heterocyclic compound 104 + fluoxastrobin | 200+2000 | 100 |
| Present condensed heterocyclic compound 104 + fluoxastrobin | 500+5 | 100 |
| Present condensed heterocyclic compound 104 + present amide compound | 200+2000 | 100 |
| Present condensed heterocyclic compound 104 + present amide compound | 500+50 | 100 |

(continued)

| Compounds provided for test | Concentration (ppm) | Mortality rate |
|---|---|---|
| Present condensed heterocyclic compound 104 + present amide compound | 500+5 | 100 |
| Present condensed heterocyclic compound 104 + orysastrobin | 200+5000 | 100 |
| Present condensed heterocyclic compound 104 + orysastrobin | 500+50 | 100 |
| Present condensed heterocyclic compound 104 + orysastrobin | 500+5 | 100 |
| Present condensed heterocyclic compound 118 + azoxystrobin | 200+2000 | 100 |
| Present condensed heterocyclic compound 118 + azoxystrobin | 500+50 | 100 |
| Present condensed heterocyclic compound 118 + azoxystrobin | 500+5 | 100 |
| Present condensed heterocyclic compound 118 + pyraclostrobin | 200+2000 | 100 |
| Present condensed heterocyclic compound 118 + pyraclostrobin | 500+5 | 100 |
| Present condensed heterocyclic compound 118 + trifloxystrobin | 200+2000 | 100 |
| Present condensed heterocyclic compound 118 + trifloxystrobin | 500+5 | 100 |
| Present condensed heterocyclic compound 118 + fluoxastrobin | 200+2000 | 100 |
| Present condensed heterocyclic compound 118 + fluoxastrobin | 500+5 | 100 |
| Present condensed heterocyclic compound 118 + present amide compound | 200+2000 | 100 |
| Present condensed heterocyclic compound 118 + present amide compound | 500+50 | 100 |
| Present condensed heterocyclic compound 118 + present amide compound | 500+5 | 100 |
| Present condensed heterocyclic compound 118 + orysastrobin | 200+5000 | 100 |
| Present condensed heterocyclic compound 118 + orysastrobin | 500+50 | 100 |
| Present condensed heterocyclic compound 118 + orysastrobin | 500+5 | 100 |
| Present condensed heterocyclic compound 119 + azoxystrobin | 200+2000 | 100 |
| Present condensed heterocyclic compound 119 + azoxvstrobin | 500+50 | 100 |
| Present condensed heterocyclic compound 119 + azoxystrobin | 500+5 | 100 |
| Non-treatment section | - | 0 |

Table 31

| Compounds provided for test | Concentration (ppm) | Mortality rate |
|---|---|---|
| Present condensed heterocyclic compound 119 + pyraclostrobin | 200+2000 | 100 |
| Present condensed heterocyclic compound 119 + pyraclostrobin | 500+5 | 100 |
| Present condensed heterocyclic compound 119 + trifloxystrobin | 200+2000 | 100 |
| Present condensed heterocyclic compound 119 + trifloxystrobin | 500+5 | 100 |
| Present condensed heterocyclic compound 119 + fluoxastrobin | 200+2000 | 100 |
| Present condensed heterocyclic compound 119 + fluoxastrobin | 500+5 | 100 |
| Present condensed heterocyclic compound 119 + present amide compound | 200+2000 | 100 |
| Present condensed heterocyclic compound 119 + present amide compound | 500+50 | 100 |
| Present condensed heterocyclic compound 119 + present amide compound | 500+5 | 100 |
| Present condensed heterocyclic compound 119 + orysastrobin | 200+5000 | 100 |
| Present condensed heterocyclic compound 119 + orysastrobin | 500+50 | 100 |

(continued)

| Compounds provided for test | Concentration (ppm) | Mortality rate |
|---|---|---|
| Present condensed heterocyclic compound 119 + orysastrobin | 500+5 | 100 |
| Present condensed heterocyclic compound 130 + azoxystrobin | 200+2000 | 100 |
| Present condensed heterocyclic compound 130 + azoxystrobin | 500+50 | 100 |
| Present condensed heterocyclic compound 130 + azoxystrobin | 500+5 | 100 |
| Present condensed heterocyclic compound 130 + pyraclostrobin | 200+2000 | 100 |
| Present condensed heterocyclic compound 130 + pyraclostrobin | 500+5 | 100 |
| Present condensed heterocyclic compound 130 + trifloxystrobin | 200+2000 | 100 |
| Present condensed heterocyclic compound 130 + trifloxystrobin | 500+5 | 100 |
| Present condensed heterocyclic compound 130 + fluoxastrobin | 200+2000 | 100 |
| Present condensed heterocyclic compound 130 + fluoxastrobin | 500+5 | 100 |
| Present condensed heterocyclic compound 130 + present amide compound | 200-2000 | 100 |
| Present condensed heterocyclic compound 130 present amide compound | 500+50 | 100 |
| Present condensed heterocyclic compound 130 + present amide compound | 500+5 | 100 |
| Present condensed heterocyclic compound 130 + orysastrobin | 200+2000 | 100 |
| Present condensed heterocyclic compound 130 + orysastrobin | 500+50 | 100 |
| Present condensed heterocyclic compound 130 + orysastrobin | 500+5 | 100 |
| Present condensed heterocyclic compound 131 + azoxystrobin | 200+2000 | 100 |
| Present condensed heterocyclic compound 131 + azoxystrobin | 500+50 | 100 |
| Present condensend heterocyclic compound 131 + azoxystrobin | 500+5 | 100 |
| Present condensed heterocyclic compound 131 + pyraclostrobin | 200+2000 | 100 |
| Present condensed heterocyclic compound 131 + pyraclostrobin | 500+5 | 100 |
| Present condensed heterocyclic compound 131 + trifloxystrobin | 200+2000 | 100 |
| Present condensed heterocyclic compound 131 + trifloxvstrobin | 500+5 | 100 |
| Present condensed heterocyclic compound 131 + fluoxastrobin | 200+2000 | 100 |
| Present condensed heterocyclic compound 131 + fluoxastrobin | 500+5 | 100 |
| Present condensed heterocyclic compound 131 + present amide compound | 200+2000 | 100 |
| Present condensed heterocyclic compound 131 + present amide compound | 500+50 | 100 |
| Present condensed heterocyclic compound 131 + present amide compound | 500+5 | 100 |
| Present condensed heterocyclic compound 131 + orysastrobin | 200+2000 | 100 |
| Present condensed heterocyclic compound 131 + orysastrobin | 500+50 | 100 |
| Present condensed heterocyclic compound 131 + orysastrobin | 500+5 | 100 |
| Non-treatment section | - | 0 |

Test Example 3

1 mg of each of the present condensed heterocyclic compounds 3, 23, 27, 31, 39, 41, 42, 46, 48, 51, 56, 58, 64, 69, 72, 74, 78, 83, 91, 97, 104, 105, 107, 108, 109, 118, 119, 120, 127, 130, and 131 was dissolved in 10 $\mu$L of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a

predetermined concentration.

1 mg of metalaxyl (manufactured by Waco Pure Chemical Industries, Ltd.) was dissolved in 10 μL of a mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A commercial formulation of metalaxyl-M (trade name: Ridmil Gold EC, Syngenta Japan K.K.) was diluted with water which contains 0.02 volume% of a spreading agent (trade name: Shindain, manufactured by Sumitomo Chemical Co., Ltd.) so as to have a predetermined concentration.

A water-diluted solution of the above-described present condensed heterocyclic compound and a water-diluted solution of metalaxyl or metalaxyl-M were mixed together to prepare a liquid medicine for a test.

Each leaf disc (diameter of 1.5 cm) of a cabbage (Brassicae oleracea) was accommodated in each well of a 24 hole microplate (manufactured by Becton Dickinson) and 40 μL of liquid medicine for a test was scattered to each well. Wells to which 40 μL of water containing 0.02 volume% of a spreading agent (trade name: Shindain, manufactured by Sumitomo Chemical Co., Ltd.) was scattered was set to a non-treatment section.

After air-drying, 5 second-instar larvae of diamondback moths (Plutella xylostella) were released in each well, which was then covered with paper towel and closed with a lid. 2 days after the releasing, the number of surviving larvae in each well was observed.

The mortality rates of the treatment section and the non-treatment section were calculated using the following Formula 1). The test was repeated once.

Formula 1) Mortality rate (%) = (the number of larvae provided for a test - the number of surviving larvae) / the number of larvae provided for a test x 100

Table 32

| Compounds provided for test | Concentration (ppm) | Mortality rate |
|---|---|---|
| Present condensed heterocyclic compound 3 + metalaxyl-M | 200+2000 | 100 |
| Present condensed heterocyclic compound 3 + metalaxyl-M | 500+50 | 100 |
| Present condensed heterocyclic compound 3 + metalaxyl-M | 500+5 | 100 |
| Present condensed heterocyclic compound 39 + metalaxyl-M | 200+2000 | 100 |
| Present condensed heterocyclic compound 39 + metalaxyl-M | 500+50 | 100 |
| Present condensed heterocyclic compound 39 + metalaxyl-M | 500+5 | 100 |
| Present condensed heterocyclic compound 23 + metalaxyl-M | 200+2000 | 100 |
| Present condensed heterocyclic compound 23 + metalaxyl-M | 500+50 | 100 |
| Present condensed heterocyclic compound 23 + metalaxyl-M | 500+5 | 100 |
| Present condensed heterocyclic compound 3 + metalaxyl | 200+2000 | 100 |
| Present condensed heterocyclic compound 3 + metalaxyl | 500+5 | 100 |
| Present condensed heterocyclic compound 39 + metalaxyl | 200+2000 | 100 |
| Present condensed heterocyclic compound 39 + metalaxyl | 500+5 | 100 |
| Present condensed heterocyclic compound 23 + metalaxyl | 200+2000 | 100 |
| Present condensed heterocyclic compound 23 + netalaxyl | 500+5 | 100 |
| Present condensed heterocyclic compound 74 + metalaxyl | 200+2000 | 100 |
| Present condensed heterocyclic compound 74 + metalaxyl | 500+5 | 100 |
| Present condensed heterocyclic compound 74 + metalaxyl-M | 200+2000 | 100 |

(continued)

| Compounds provided for test | Concentration (ppm) | Mortality rate |
|---|---|---|
| Present condensed heterocyclic compound 74 + metalaxyl-M | 500+50 | 100 |
| Present condensed heterocyclic compound 74 + metalaxyl-M | 500+5 | 100 |
| Present condensed heterocyclic compound 83 + metalaxyl | 200+2000 | 100 |
| Present condensed heterocyclic compound 83 + metalaxyl | 500+5 | 100 |
| Present condensed heterocyclic compound 83 + metalaxyl-M | 200+2000 | 100 |
| Present condensed heterocyclic compound 83 + metalaxyl-M | 500+50 | 100 |
| Present condensed heterocyclic compound 83 + metalaxyl-M | 500+5 | 100 |
| Present condensed heterocyclic compound 58 + metalaxvl | 200+2000 | 100 |
| Present condensed heterocyclic compound 58 + metalaxyl | 500+5 | 100 |
| Present condensed heterocyclic compound 58 + metalaxyl-M | 200+2000 | 100 |
| Present condensed heterocyclic compound 58 + metalaxyl-M | 500+50 | 100 |
| Present condensed heterocyclic compound 58 + metalaxyl-M | 500+5 | 100 |
| Present condensed heterocyclic compound 31 + metalaxyl | 200+2000 | 100 |
| Present condensed heterocyclic compound 31 + metalaxyl | 500+5 | 100 |
| Present condensed heterocyclic compound 31 + metalaxyl-M | 200+2000 | 100 |
| Present condensed heterocyclic compound 31 + metalaxyl-M | 500+50 | 100 |
| Present condensed heterocyclic compound 31 + metalaxyl-M | 500+5 | 100 |
| Present condensed heterocyclic compound 56 + metalaxyl | 200+2000 | 100 |
| Present condensed heterocyclic compound 56 + metalaxyl | 500+5 | 100 |
| Present condensed heterocyclic compound 56 + metalaxyl-M | 200+2000 | 100 |
| Present condensed heterocyclic compound 56 + metalaxyl-M | 500+50 | 100 |
| Present condensed heterocyclic compound 56 + metalaxyl-M | 500+5 | 100 |
| Non-treatment section | - | 0 |

Table 33

| Compounds provided for test | Concentration (ppm) | Mortality rate |
|---|---|---|
| Present condensed heterocyclic compound 46 + metalaxyl | 200+2000 | 100 |
| Present condensed heterocyclic compound 46 + metalaxyl | 500+5 | 100 |
| Present condensed heterocyclic compound 46 + metalaxyl-M | 200+2000 | 100 |
| Present condensed heterocyclic compound 46 + metalaxyl-M | 500+50 | 100 |
| Present condensed heterocyclic compound 46 + metalaxyl-M | 500+5 | 100 |
| Present condensed heterocyclic compound 105 + metalaxyl | 200+2000 | 100 |
| Present condensed heterocyclic compound 105 + metalaxyl | 500+5 | 100 |
| Present condensed heterocyclic compound 105 + metalaxyl-M | 200+2000 | 100 |
| Present condensed heterocyclic compound 105 + metalaxyl-M | 500+50 | 100 |
| Present condensed heterocyclic compound 105 + metalaxyl-M | 500+5 | 100 |
| Present condensed heterocyclic compound 91 + metalaxyl | 200+2000 | 100 |

(continued)

| Compounds provided for test | Concentration (ppm) | Mortality rate |
|---|---|---|
| Present condensed heterocyclic compound 91 + metalaxyl | 500+5 | 100 |
| Present condensed heterocyclic compound 91 + metalaxyl-M | 200+2000 | 100 |
| Present condensed heterocyclic compound 91 metalaxyl-M | 500+50 | 100 |
| Present condensed heterocyclic compound 91 + metalaxyl-M | 500+5 | 100 |
| Present condensed heterocyclic compound 107 + metalaxyl | 200+2000 | 100 |
| Present condensed heterocyclic compound 107 + metalaxvl | 500+5 | 100 |
| Present condensed heterocyclic compound 107 + metalaxyl-M | 200+2000 | 100 |
| Present condensed heterocyclic compound 107 + metalaxyl-M | 500+50 | 100 |
| Present condensed heterocyclic compound 107 + metalaxyl-M | 500+5 | 100 |
| Present condensed heterocyclic compound 51 + metalaxyl | 200+2000 | 100 |
| Present condensed heterocyclic compound 51 + metalaxyl | 500+5 | 100 |
| Present condensed heterocyclic compound 51 + metalaxyl-M | 200+2000 | 100 |
| Present condensed heterocyclic compound 51 + metalaxyl-M | 500+50 | 100 |
| Present condensed heterocyclic compound 51 + metalaxyl-M | 500+5 | 100 |
| Present condensed heterocyclic compound 27 + metalaxyl | 200+2000 | 100 |
| Present condensed heterocyclic compound 27 + metalaxyl | 500+5 | 100 |
| Present condensed heterocyclic compound 27 + metalaxyl-M | 200+2000 | 100 |
| Present condensed heterocyclic compound 27 + metalaxyl-M | 500+50 | 100 |
| Present condensed heterocyclic compound 27 + metalaxyl-M | 500+5 | 100 |
| Present condensed heterocyclic compound 108 + metalaxyl | 200+2000 | 100 |
| Present condensed heterocyclic compound 108 + metalaxyl | 500+5 | 100 |
| Present condensed heterocyclic compound 108 + metalaxyl-M | 200+2000 | 100 |
| Present condensed heterocyclic compound 108 + metalaxyl-M | 500+50 | 100 |
| Present condensed heterocyclic compound 108 + metalaxyl-M | 500+5 | 100 |
| Present condensed heterocyclic compound 48 + metalaxyl | 200+2000 | 100 |
| Present condensed heterocyclic compound 48 + metalaxyl | 500+5 | 100 |
| Present condensed heterocyclic compound 48 + metalaxyl-M | 200+2000 | 100 |
| Present condensed heterocyclic compound 48 + metalaxyl-M | 500+50 | 100 |
| Present condensed heterocyclic compound 48 + metalaxyl-M | 500+5 | 100 |
| Non-treatment section | - | 0 |

Table 34

| Compounds provided for test | Concentration (ppm) | Mortality rate |
|---|---|---|
| Present condensed heterocyclic compound 97 + metalaxyl | 200+2000 | 100 |
| Present condensed heterocyclic compound 97 + metalaxyl | 500+5 | 100 |
| Present condensed heterocyclic compound 97 + metalaxyl-M | 200+2000 | 100 |
| Present condensed heterocyclic compound 97 + metalaxyl-M | 500+50 | 100 |

(continued)

| Compounds provided for test | Concentration (ppm) | Mortality rate |
|---|---|---|
| Present condensed heterocyclic compound 97 + metalaxyl-M | 500+5 | 100 |
| Present condensed heterocyclic compound 69 + metalaxyl | 200+2000 | 100 |
| Present condensed heterocyclic compound 69 + metalaxyl | 500+5 | 100 |
| Present condensed heterocyclic compound 69 + metalaxyl-M | 200+2000 | 100 |
| Present condensed heterocyclic compound 69 + metalaxyl-M | 500+50 | 100 |
| Present condensed heterocyclic compound 69 + metalaxyl-M | 500+5 | 100 |
| Present condensed heterocyclic compound 109 + metalaxyl | 200+2000 | 100 |
| Present condensed heterocyclic compound 109 + metalaxyl | 500+5 | 100 |
| Present condensed heterocyclic compound 109 + metalaxyl-M | 200+2000 | 100 |
| Present condensed heterocyclic compound 109 + metalaxyl-M | 500+50 | 100 |
| Present condensed heterocyclic compound 109 + metalaxyl-M | 500+5 | 100 |
| Present condensed heterocyclic compound 72 + metalaxyl | 200+2000 | 100 |
| Present condensed heterocyclic compound 72 + metalaxyl | 500+5 | 100 |
| Present condensed heterocyclic compound 72 + metalaxyl-M | 200+2000 | 100 |
| Present condensed heterocyclic compound 72 + metalaxyl-M | 500+50 | 100 |
| Present condensed heterocyclic compound 72 + metalaxyl-M | 500+5 | 100 |
| Present condensed heterocyclic compound 78 + metalaxyl | 200+2000 | 100 |
| Present condensed heterocyclic compound 78 + metalaxyl | 500+5 | 100 |
| Present condensed heterocyclic compound 78 + metalaxyl-M | 200+2000 | 100 |
| Present condensed heterocyclic compound 78 + metalaxyl-M | 500+50 | 100 |
| Present condensed heterocyclic compound 78 + metalaxyl-M | 500+5 | 100 |
| Present condensed heterocyclic compound 64 + metalaxyl | 200+2000 | 100 |
| Present condensed heterocyclic compound 64 + metalaxyl | 500+5 | 100 |
| Present condensed heterocyclic compound 64 + metalaxyl-M | 200+2000 | 100 |
| Present condensed heterocyclic compound 64 + metalaxyl-M | 500+50 | 100 |
| Present condensed heterocyclic compound 64 + metalaxyl-M | 500+5 | 100 |
| Present condensed heterocyclic compound 120 + metalaxyl | 200+2000 | 100 |
| Present condensed heterocyclic compound 120 + metalaxyl | 500+5 | 100 |
| Present condensed heterocyclic compound 120 + metalaxyl-M | 200+2000 | 100 |
| Present condensed heterocyclic compound 120 + metalaxyl-M | 500+50 | 100 |
| Present condensed heterocyclic compound 120 + metalaxyl-M | 500+5 | 100 |
| Present condensed heterocyclic compound 127 + metalaxyl | 200+2000 | 100 |
| Present condensed heterocyclic compound 127 + metalaxyl | 500+5 | 100 |
| Present condensed heterocyclic compound 127 + metalaxyl-M | 200+2000 | 100 |
| Present condensed heterocyclic compound 127 + metalaxyl-M | 500+50 | 100 |
| Present condensed heterocyclic compound 127 + metalaxyl-M | 500+5 | 100 |
| Non-treatment section | - | 0 |

Table 35

| Compounds provided for test | Concentration (ppm) | Mortality rate |
|---|---|---|
| Present condensed heterocyclic compound 42 + metalaxyl | 200+2000 | 100 |
| Present condensed heterocyclic compound 42 + metalaxyl | 500+5 | 100 |
| Present condensed heterocyclic compound 42 + metalaxyl-M | 200+2000 | 100 |
| Present condensed heterocyclic compound 42 + metalaxyl-M | 500+50 | 100 |
| Present condensed heterocyclic compound 42 + metalaxyl-M | 500+5 | 100 |
| Present condensed heterocyclic compound 41 + metalaxyl | 200+2000 | 100 |
| Present condensed heterocyclic compound 41 + metalaxyl | 500+5 | 100 |
| Present condensed heterocyclic compound 41 + metalaxyl-M | 200+2000 | 100 |
| Present condensed heterocyclic compound 41 + metalaxyl-M | 500+50 | 100 |
| Present condensed heterocyclic compound 41 + metalaxyl-M | 500+5 | 100 |
| Present condensed heterocyclic compound 104 + metalaxyl | 200+2000 | 100 |
| Present condensed heterocyclic compound 104 + metalaxyl | 500+5 | 100 |
| Present condensed heterocyclic compound 104 + metalaxyl-M | 200+2000 | 100 |
| Present condensed heterocyclic compound 104 + metalaxyl-M | 500+50 | 100 |
| Present condensed heterocyclic compound 104 + metalaxyl-M | 500+5 | 100 |
| Present condensed heterocyclic compound 118 + metalaxyl | 200+2000 | 100 |
| Present condensed heterocyclic compound 118 + metalaxyl | 500+5 | 100 |
| Present condensed heterocyclic compound 118 + metalaxyl-M | 200+2000 | 100 |
| Present condensed heterocyclic compound 118 + metalaxyl-M | 500+50 | 100 |
| Present condensed heterocyclic compound 118 + metalaxyl-M | 500+5 | 100 |
| Present condensed heterocyclic compound 119 + metalaxyl | 200+2000 | 100 |
| Present condensed heterocyclic compound 119 + metalaxyl | 500+5 | 100 |
| Present condensed heterocyclic compound 119 + metalaxyl-M | 200+2000 | 100 |
| Present condensed heterocyclic compound 119 + metalaxyl-M | 500+50 | 100 |
| Present condensed heterocyclic compound 119 + metalaxyl-M | 500+5 | 100 |
| Present condensed heterocyclic compound 130 + metalaxyl | 200+2000 | 100 |
| Present condensed heterocyclic compound 130 + metalaxyl | 500+5 | 100 |
| Present condensed heterocyclic compound 130 + metalaxyl-M | 200+2000 | 100 |
| Present condensed heterocyclic compound 130 + metalaxyl-M | 500+50 | 100 |
| Present condensed heterocyclic compound 130 + metalaxyl-M | 500+5 | 100 |
| Present condensed heterocyclic compound 131 + metalaxyl | 200+2000 | 100 |
| Present condensed heterocyclic compound 131 + metalaxyl | 500+5 | 100 |
| Present condensed heterocyclic compound 131 + metalaxyl-M | 200+2000 | 100 |
| Present condensed heterocyclic compound 131 + metalaxyl-M | 500+50 | 100 |
| Present condensed heterocyclic compound 131 + metalaxyl-M | 500+5 | 100 |
| Non-treatment section | - | 0 |

Test Example 4

1 mg of each of the present condensed heterocyclic compounds 3, 23, 27, 31, 39, 41, 42, 46, 48, 51, 56, 58, 64, 69, 72, 74, 78, 83, 91, 97, 104, 105, 107, 108, 109, 118, 119, 120, 127, 130, and 131 was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

1 mg of each of tricyclazole (manufactured by Waco Pure Chemical Industries, Ltd.), isotianil and probenazole (manufactured by Waco Pure Chemical Industries, Ltd.), fthalide, (manufactured by Waco Pure Chemical Industries, Ltd.), kasugamycin hydrochloride (manufactured by Waco Pure Chemical Industries, Ltd.), ferimzone, tebufloquin, and pyroquilon (manufactured by Waco Pure Chemical Industries, Ltd.) was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A water-diluted solution of the above-described present condensed heterocyclic compounds and a water-diluted solution of tricyclazole, isotianil, probenazole, fthalide, kasugamycin hydrochloride, ferimzone, tebufloquin, and pyroquilon were mixed together to prepare a liquid medicine for a test.

Each leaf disc (diameter of 1.5 cm) of a cabbage (Brassicae oleracea) was accommodated in each well of a 24 hole microplate (manufactured by Becton Dickinson) and 40 μL of liquid medicine for a test was scattered to each well. Wells to which 40 μL of water containing 0.02 volume% of a spreading agent (trade name: Shindain, manufactured by Sumitomo Chemical Co., Ltd.) was scattered was set to a non-treatment section.

After air-drying, 5 second-instar larvae of diamondback moths (Plutella xylostella) were released in each well, which was then covered with paper towel and closed with a lid. 2 days after the releasing, the number of surviving larvae in each well was observed.

The mortality rates of the treatment section and the non-treatment section were calculated using the following Formula 1). The test was repeated once.

Formula 1) Mortality rate (%) = (the number of larvae provided for a test - the number of surviving larvae) / the number of larvae provided for a test x 100

Table 36

| Compounds provided for test | Concentration (ppm) | Mortality rate |
|---|---|---|
| Present condensed heterocyclic compound 3 + tricyclazole | 200+2000 | 100 |
| Present condensed heterocyclic compound 3 + tricyclazole | 500+50 | 100 |
| Present condensed heterocyclic compound 3 + isotianil | 200+2000 | 100 |
| Present condensed heterocyclic compound 3 + isotianil | 500+500 | 100 |
| Present condensed heterocyclic compound 3 + isotianil | 500+50 | 100 |
| Present condensed heterocyclic compound 3 + probenazole | 200+5000 | 100 |
| Present condensed heterocyclic compound 3 + probenazole | 500+500 | 100 |
| Present condensed heterocyclic compound 3 + fthalide | 200+5000 | 100 |
| Present condensed heterocyclic compound 3 + fthalide | 500+500 | 100 |
| Present condensed heterocyclic compound 3 + kasugamycin hydrochloride | 200+2000 | 100 |
| Present condensed heterocyclic compound 3 + kasugamycin hydrochloride | 500+50 | 100 |
| Present condensed heterocyclic compound 3 + ferimzone | 200+5000 | 100 |

(continued)

| Compounds provided for test | Concentration (ppm) | Mortality rate |
|---|---|---|
| Present condensed heterocyclic compound 3 + ferimzone | 500+500 | 100 |
| Present condensed heterocyclic compound 3 + tebufloquin | 200+2000 | 100 |
| Present condensed heterocyclic compound 3 + tebufloquin | 500+50 | 100 |
| Present condensed heterocyclic compound 3 + pyroquilon | 200+5000 | 100 |
| Present condensed heterocyclic compound 3 + pyroquilon | 500+500 | 100 |
| Present condensed heterocyclic compound 39 + tricyclazole | 200+2000 | 100 |
| Present condensed heterocyclic compound 39 + tricyclazole | 500+50 | 100 |
| Present condensed heterocyclic compound 39 + isotianil | 200+2000 | 100 |
| Present condensed heterocyclic compound 39 + isotianil | 500+500 | 100 |
| Present condensed heterocyclic compound 39 + isotianil | 500+50 | 100 |
| Present condensed heterocyclic compound 39 + fthalide | 200+5000 | 100 |
| Present condensed heterocyclic compound 39 + fthalide | 500+500 | 100 |
| Present condensed heterocyclic compound 39 + kasugamycin hydrochloride | 200+2000 | 100 |
| Present condensed heterocyclic compound 39 + kasugamycin hydrochloride | 500+50 | 100 |
| Non-treatment section | - | 0 |

Table 37

| Compounds provided for test | Concentration (ppm) | Mortality rate |
|---|---|---|
| Present condensed heterocyclic compound 39 + ferimzone, | 200-5000 | 100 |
| Present condensed heterocyclic compound 39 + ferimzone | 500+500 | 100 |
| Present condensed heterocyclic compound 39 + tebufloquin | 200+2000 | 100 |
| Present condensed heterocyclic compound 39 + tebufloquin | 500+50 | 100 |
| Present condensed heterocyclic compound 39 + pyroquilon | 200+5000 | 100 |
| Present condensed heterocyclic compound 39 + pyroquilon | 500+500 | 100 |
| Present condensed heterocyclic compound 23 + tricyclazole | 200+2000 | 80 |
| Present condensed heterocyclic compound 23 + tricyclazole | 500+50 | 100 |
| Present condensed heterocyclic compound 23 + isotianil | 200+2000 | 100 |
| Present condensed heterocyclic compound 23 + isotianil | 200+5000 | 100 |
| Present condensed heterocyclic compound 23 + isotianil | 500+50 | 100 |
| Present condensed heterocyclic compound 23 + probenazole | 200+5000 | 100 |
| Present condensed heterocyclic compound 23 + probenazole | 500+500 | 100 |
| Present condensed heterocyclic compound 23 + fthalide | 200+5000 | 100 |
| Present condensed heterocyclic compound 23 + fthalide | 500+500 | 100 |
| Present condensed heterocyclic compound 23 + kasugamycin hydrochloride | 200+2000 | 80 |
| Present condensed heterocyclic compound 23 + kasugamycin hydrochloride | 500+50 | 100 |
| Present condensed heterocyclic compound 23 + ferimzone | 200+5000 | 100 |
| Present condensed heterocyclic compound 23 + ferimzone | 500+500 | 100 |

(continued)

| Compounds provided for test | Concentration (ppm) | Mortality rate |
|---|---|---|
| Present condensed heterocyclic compound 23 + tebufloquin | 200+2000 | 100 |
| Present condensed heterocyclic compound 23 + tebufloquin | 500+50 | 100 |
| Present condensed heterocyclic compound 23 + pyroquilon | 200+5000 | 100 |
| Present condensed heterocyclic compound 23 + pyroquilon | 500+500 | 100 |
| Present condensed heterocyclic compound 74 + tricyclazole | 200+2000 | 100 |
| Present condensed heterocyclic compound 74 + tricyclazole | 500+50 | 100 |
| Present condensed heterocyclic compound 74 + isotianil | 200+2000 | 100 |
| Present condensed heterocyclic compound 74 + isotianil | 500+500 | 100 |
| Present condensed heterocyclic compound 74 + isotianil | 500+50 | 100 |
| Present condensed heterocyclic compound 74 + probenazole | 200+5000 | 100 |
| Present condensed heterocyclic compound 74 + probenazole | 500+500 | 100 |
| Present condensed heterocyclic compound 74 + fthalide | 200+5000 | 100 |
| Present condensed heterocyclic compound 74 + fthalide | 500+500 | 100 |
| Present condensed heterocyclic compound 74 + kasugamycin hydrochloride | 200+2000 | 100 |
| Present condensed heterocyclic compound 74 + kasugamycin hydrochloride | 500+50 | 100 |
| Present condensed heterocyclic compound 74 + ferimzone | 200+5000 | 100 |
| Present condensed heterocyclic compound 74 + ferimzone | 500+500 | 100 |
| Present condensed heterocyclic compound 74 + tebufloquin | 200+2000 | 100 |
| Present condensed heterocyclic compound 74 + tebufloquin | 500+50 | 100 |
| Present condensed heterocyclic compound 74 + pyroquilon | 200+5000 | 100 |
| Present condensed heterocyclic compound 74 + pyroquilon | 500+500 | 100 |
| Non-treatment section | - | 0 |

Table 38

| Compounds provided for test | Concentration (ppm) | Mortality rate |
|---|---|---|
| Present condensed heterocyclic compound 83 + tricyclazole | 200+2000 | 100 |
| Present condensed heterocyclic compound 83 + tricyclazole | 500+50 | 100 |
| Present condensed heterocyclic compound 83 + isotianil | 200+2000 | 100 |
| Present condensed heterocyclic compound 83 + isotianil | 500+500 | 100 |
| Present condensed heterocyclic compound 83 + isotianil | 500+50 | 100 |
| Present condensed heterocyclic compound 83 + probenazole | 200+5000 | 100 |
| Present condensed heterocyclic compound 83 + probenazole | 500+500 | 100 |
| Present condensed heterocyclic compound 83 + fthalide | 200+5000 | 100 |
| Present condensed heterocyclic compound 83 + fthalide | 500+500 | 100 |
| Present condensed heterocyclic compound 83 + kasugamycin hydrochloride | 200+2000 | 100 |
| Present condensed heterocyclic compound 83 + kasugamycin hydrochloride | 500+50 | 100 |
| Present condensed heterocyclic compound 83 + ferimzone | 200+5000 | 100 |

(continued)

| Compounds provided for test | Concentration (ppm) | Mortality rate |
|---|---|---|
| Present condensed heterocyclic compound 83 + ferimzone | 500+500 | 100 |
| Present condensed heterocyclic compound 83 + tebufloquin | 200+20000 | 100 |
| Present condensed heterocyclic compound 83 + tebufloquin | 500+50 | 100 |
| Present condensed heterocyclic compound 83 + pyroquilon | 200+5000 | 100 |
| Present condensed heterocyclic compound 83 + pyroquilon | 500+500 | 100 |
| Present condensed heterocyclic compound 58 + tricyclazole | 200+2000 | 100 |
| Present condensed heterocyclic compound 58 + tricyclazole | 500+50 | 100 |
| Present condensed heterocyclic compound 58 + isotianil | 200+2000 | 100 |
| Present condensed heterocyclic compound 58 + isotianil | 500+500 | 100 |
| Present condensed heterocyclic compound 58 + isotianil | 500+50 | 100 |
| Present condensed heterocyclic compound 58 + probenazole | 200+5000 | 100 |
| Present condensed heterocyclic compound 58 + probenazole | 500+500 | 100 |
| Present condensed heterocyclic compound 58 + fthalide | 200+5000 | 100 |
| Present condensed heterocyclic compound 58 + fthalide, | 500+500 | 100 |
| Present condensed heterocyclic compound 58 + kasugamycin hydrochloride | 200+2000 | 100 |
| Present condensed heterocyclic compound 58 + kasugamycin hydrochloride | 500+50 | 100 |
| Present condensed heterocyclic compound 58 + ferimzone | 200+5000 | 100 |
| Present condensed heterocyclic compound 58 + ferimzone | 500+500 | 100 |
| Present condensed heterocyclic compound 58 + tebufloquin | 200+20000 | 100 |
| Present condensed heterocyclic compound 58 - tebufloquin | 500+50 | 100 |
| Present condensed heterocyclic compound 58 + pyroquilon | 200+5000 | 100 |
| Present condensed heterocyclic compound 58 + pyroquilon | 500+500 | 100 |
| Present condensed heterocyclic compound 31 + tricyclazole | 200+2000 | 100 |
| Present condensed heterocyclic compound 31 + tricyclazole | 500+50 | 100 |
| Present condensed heterocyclic compound 31 + isotianil | 200+2000 | 100 |
| Present condensed heterocyclic compound 31 - + isotianil | 500+500 | 100 |
| Present condensed heterocyclic compound 31 + isotianil | 500+50 | 100 |
| Present condensed heterocyclic compound 31 + probenazole | 200+5000 | 100 |
| Present condensed heterocyclic compound 31 + probenazole | 500+500 | 100 |
| Non-treatment section | - | 0 |

Table 39

| Compounds provided for test | Concentration (ppm) | Mortality rate |
|---|---|---|
| Present condensed heterocyclic compound 31 + fthalide | 200+5000 | 100 |
| Present condensed heterocyclic compound 31 + fthalide | 500+500 | 100 |
| Present condensed heterocyclic compound 31 + kasugamycin hydrochloride | 200+2000 | 100 |
| Present condensed heterocyclic compound 31 + kasugamycin hydrochloride | 500+50 | 100 |

(continued)

| Compounds provided for test | Concentration (ppm) | Mortality rate |
|---|---|---|
| Present condensed heterocyclic compound 31 + ferimzone | 200+5000 | 100 |
| Present condensed heterocyclic compound 31 + ferimzone | 500+500 | 100 |
| Present condensed heterocyclic compound 31 + tebufloquin | 200+2000 | 100 |
| Present condensed heterocyclic compound 31 + tebufloquin | 500+50 | 100 |
| Present condensed heterocyclic compound 31 + pyroquilon | 200+5000 | 100 |
| Present condensed heterocyclic compound 31 + pyroquilon | 500+500 | 100 |
| Present condensed heterocyclic compound 56 + tricyclazole | 200+2000 | 100 |
| Present condensed heterocyclic compound 56 + tricyclazole | 500+50 | 100 |
| Present condensed heterocyclic compound 56 + isotianil | 200+2000 | 100 |
| Present condensed heterocyclic compound 56 + isotianil | 500+500 | 100 |
| Present condensed heterocyclic compound 56 + isotianil | 500+50 | 100 |
| Present condensed heterocyclic compound 56 + probenazole | 200+5000 | 100 |
| Present condensed heterocyclic compound 56 + probenazole | 500+500 | 100 |
| Present condensed heterocyclic compound 56 + fthalide | 200+5000 | 100 |
| Present condensed heterocyclic compound 56 + fthalide | 500+500 | 100 |
| Present condensed heterocyclic compound 56 + kasugamycin hydrochloride | 200+2000 | 100 |
| Present condensed heterocyclic compound 56 + kasugamycin hydrochloride | 500+50 | 100 |
| Present condensed heterocyclic compound 56 + ferimzone | 200+5000 | 100 |
| Present condensed heterocyclic compound 56 + ferimzone | 500+500 | 100 |
| Present condensed heterocyclic compound 56 + tebufloquin | 200+2000 | 100 |
| Present condensed heterocyclic compound 56 + tebufloquin | 500+50 | 100 |
| Present condensed heterocyclic compound 56 + pyroquilon | 200+5000 | 100 |
| Present condensed heterocyclic compound 56 + pyroquilon | 500+500 | 100 |
| Present condensed heterocyclic compound 46 + tricyclazole | 200+2000 | 100 |
| Present condensed heterocyclic compound 46 + tricyclazole | 500+50 | 100 |
| Present condensed haterocyclic compound 46 + isotianil | 200+2000 | 100 |
| Present condensed heterocyclic compound 46 + isotianil | 500+500 | 100 |
| Present condensed heterocyclic compound 46 + isotianil | 500+50 | 100 |
| Present condensed heterocyclic compound 46 + probenazole | 200+5000 | 100 |
| Present condensed heterocyclic compound 46 + probenazole | 500+500 | 100 |
| Present condensed heterocyclic compound 46 + fthalide | 200+5000 | 100 |
| Present condensed heterocyclic compound 46 + fthalide | 500+500 | 100 |
| Present condensed heterocyclic compound 46 + kasugamycin hydrochloride | 200+2000 | 100 |
| Present condensed heterocyclic compound 46 + kasugamycin hydrochloride | 500+50 | 100 |
| Present condensed heterocyclic compound 46 + ferimzone | 200+5000 | 100 |
| Present condensed heterocyclic compound 46 + ferimzone | 500+500 | 100 |
| Non-treatment section | - | 0 |

Table 40

| Compounds provided for test | Concentration (ppm) | Mortality rate |
|---|---|---|
| Present condensed heterocyclic compound 46 + tebufloquin | 200+2000 | 100 |
| Present condensed heterocyclic compound 46 + tebufloquin | 500+50 | 100 |
| Present condensed heterocyclic compound 46 + pyroquilon | 200+5000 | 100 |
| Present condensed heterocyclic compound 46 + pyroquilon | 500+500 | 100 |
| Present condensed heterocyclic compound 105 + tricyclazole | 200+2000 | 100 |
| Present condensed heterocyclic compound 105 + tricyclazole | 500+50 | 100 |
| Present condensed heterocyclic compound 105 + isotianil | 200+2000 | 100 |
| Present condensed heterocyclic compound 105 + isotianil | 500+500 | 100 |
| Present condensed heterocyclic compound 105 + isotianil | 500+50 | 100 |
| Present condensed heterocyclic compound 105 + probenazole | 200+5000 | 100 |
| Present condensed heterocyclic compound 105 + probenazole | 500+500 | 100 |
| Present condensed heterocyclic compound 105 + fthalide | 200+5000 | 100 |
| Present condensed heterocyclic compound 105 + fthalide | 500+500 | 100 |
| Present condensed heterocyclic compound 105 + kasugamycin hydrochloride | 200+2000 | 100 |
| Present condensed heterocyclic compound 105 + kasugamycin hydrochloride | 500+50 | 100 |
| Present condensed heterocyclic compound 105 + ferimzone | 200+5000 | 100 |
| Present condensed heterocyclic compound 105 + ferimzone | 500+500 | 100 |
| Present condensed heterocyclic compound 105 + tebufloquin | 200+2000 | 100 |
| Present condensed heterocyclic compound 105 + tebufloquin | 500+50 | 100 |
| Present condensed heterocyclic compound 105 + pyroquilon | 200+5000 | 100 |
| Present condensed heterocyclic compound 105 + pyroquilon | 500+500 | 100 |
| Present condensed heterocyclic compound 91 + tricyclazole | 200+2000 | 100 |
| Present condensed heterocyclic compound 91 + tricyclazole | 500+50 | 100 |
| Present condensed heterocyclic compound 91 + isotianil | 200+2000 | 100 |
| Present condensed heterocyclic compound 91 + isotianil | 500+500 | 100 |
| Present condensed heterocyclic compound 91 + isotianil | 500+50 | 100 |
| Present condensed heterocyclic compound 91 + probenazole | 200+5000 | 100 |
| Present condensed heterocyclic compound 91 + probenazole | 500+500 | 100 |
| Present condensed heterocyclic compound 91 + fthalide | 200+5000 | 100 |
| Present condensed heterocyclic compound 91 + fthalide | 500+500 | 100 |
| Present condensed heterocyclic compound 91 + kasugamycin hydrochloride | 200+2000 | 100 |
| Present condensed heterocyclic compound 91 + kasugamycin hydrochloride | 500+50 | 100 |
| Present condensed heterocyclic compound 91 + ferimzone | 200+5000 | 100 |
| Present condensed heterocyclic compound 91 + ferimzone | 500+500 | 100 |
| Present condensed heterocyclic compound 91 + tebufloquin | 200+2000 | 100 |
| Present condensed heterocyclic compound 91 + tebufloquin | 500+50 | 100 |
| Present condensed heterocyclic compound 91 + pyroquilon | 200+5000 | 100 |

(continued)

| Compounds provided for test | Concentration (ppm) | Mortality rate |
|---|---|---|
| Present condensed heterocyclic compound 91 + pyroquilon | 500+500 | 100 |
| Present condensed heterocyclic compound 107 + tricyclazole | 200+2000 | 100 |
| Present condensed heterocyclic compound 107 + tricyclazole | 500+50 | 100 |
| Non-treatment section | - | 0 |

Table 41

| Compounds provided for test | Concentration (ppm) | Mortality rate |
|---|---|---|
| Present condensed heterocyclic compound 107 + isotianil | 200+2000 | 100 |
| Present condensed heterocyclic compound 107 + isotianil | 500+500 | 100 |
| Present condensed heterocyclic compound 107 + isotianil | 500+50 | 100 |
| Present condensed heterocyclic compound 107 + probenazole | 200+5000 | 100 |
| Present condensed heterocyclic compound 107 + probenazole | 500+500 | 100 |
| Present condensed heterocyclic compound 107 + fthalide | 200+500 | 100 |
| Present condensed heterocyclic compound 107 + fthalide | 500+500 | 100 |
| Present condensed heterocyclic compound 107 + kasugamycin hydrochloride | 200+2000 | 100 |
| Present condensed heterocyclic compound 107 + kasugamycin hydrochloride | 500+50 | 100 |
| Present condensed heterocyclic compound 107 + ferimzone | 200+5000 | 100 |
| Present condensed heterocyclic compound 107 + ferimzone | 500+500 | 100 |
| Present condensed heterocyclic compound 107 + tebufloquin | 200+2000 | 100 |
| Present condensed heterocyclic compound 107 + tebufloquin | 500+50 | 100 |
| Present condensed heterocyclic compound 107 + pyroquilon | 200+5000 | 100 |
| Present condensed heterocyclic compound 107 + pyroquilon | 500+500 | 100 |
| Present condensed heterocyclic compound 51 + tricyclazole | 200+2000 | 100 |
| Present condensed heterocyclic compound 51 + tricyclazole | 500+50 | 100 |
| Present condensed heterocyclic compound 51 + isotianil | 200+2000 | 100 |
| Present condensed heterocyclic compound 51 + isotianil | 500+500 | 100 |
| Present condensed heterocyclic compound 51 + isotianil | 500+50 | 100 |
| Present condensed hetrerocyclic compound 51 + probenazole | 200+5000 | 100 |
| Present condensed heterocyclic compound 51 + probenazole | 500+500 | 100 |
| Present condensed heterocyclic compound 51 + fthalide | 200+5000 | 100 |
| Present condensed heterocyclic compound 51 + fthalide | 500+500 | 100 |
| Present condensed heterocyclic compound 51 + kasugamycin hydrochloride | 200+2000 | 100 |
| Present condensed heterocyclic compound 51 + kasugamycin hydrochloride | 500+50 | 100 |
| Present condensed heterocyclic compound 51 + ferimzone | 200+5000 | 100 |
| Present condensed heterocyclic compound 51 + ferimzone | 500+500 | 100 |
| Present condensed heterocyclic compound 51 + tebufloquin | 200+2000 | 100 |
| Present condensed heterocyclic compound 51 + tebufloquin | 500+50 | 100 |

(continued)

| Compounds provided for test | Concentration (ppm) | Mortality rate |
|---|---|---|
| Present condensed heterocyclic compound 51 + pyroquilon | 200+5000 | 100 |
| Present condensed heterocyclic compound 51 + pyroquilon | 500+500 | 100 |
| Present condensed heterocyclic compound 27 + tricyclazole | 200+2000 | 100 |
| Present condensed heterocyclic compound 27 + tricyclazole | 500+50 | 100 |
| Present condensed heterocyclic compound 27 + isotianil | 200+2000 | 100 |
| Present condensed heterocyclic compound 27 + isotianil | 500+500 | 100 |
| Present condensed heterocyclic compound 27 + isotianil | 500+50 | 100 |
| Present condensed heterocyclic compound 27 + probenazole | 200+5000 | 100 |
| Present condensed heterocyclic compound 27 + probenazole | 500+500 | 100 |
| Present condensed heterocyclic compound 27 + fthalide | 200+5000 | 100 |
| Present condensed heterocyclic compound 27 + fthalide | 500+500 | 100 |
| Non-treatment section | - | 0 |

Table 42

| Compounds provided for test | Concentration (ppm) | Mortality rate |
|---|---|---|
| Present condensed heterocyclic compound 27 + kasugamycin hydrochloride | 200+2000 | 100 |
| Present condensed heterocyclic compound 27 + kasugamycin hydrochloride | 500+50 | 100 |
| Present condensed heterocyclic compound 27 + ferimzone | 200+5000 | 100 |
| Present condensed heterocyclic compound 27 + ferimzone | 500+500 | 100 |
| Present condensed heterocyclic compound 27 + tebufloquin | 200+2000 | 100 |
| Present condensed heterocyclic compound 27 + tebufloquin | 500+50 | 100 |
| Present condensed heterocyclic compound 27 + pyroquilon | 200+5000 | 100 |
| Present condensed heterocyclic compound 27 + pyroquilon | 500+500 | 100 |
| Present condensed heterocyclic compound 108 + tricyclazole | 200+2000 | 100 |
| Present condensed heterocyclic compound 108 + tricyclazole | 500+50 | 100 |
| Present condensed heterocyclic compound 108 + isotianil | 200+2000 | 100 |
| Present condensed heterocyclic compound 108 + isotianil | 500+500 | 100 |
| Present condensed heterocyclic compound 108 + isotianil | 500+50 | 100 |
| Present condensed heterocyclic compound 108 + probenazole | 200+5000 | 100 |
| Present condensed heterocyclic compound 108 + probenazole | 500+500 | 100 |
| Present condensed heterocyclic compound 108 + fthalide | 200+5000 | 100 |
| Present condensed heterocyclic compound 108 + fthalide | 500+500 | 100 |
| Present condensed heterocyclic compound 108 + kasugamycin hydrochloride | 200+2000 | 100 |
| Present condensed heterocyclic compound 108 + kasugamycin hydrochloride | 500+50 | 100 |
| Present condensed heterocyclic compound 108 + ferimzone | 200+5000 | 100 |
| Present condensed heterocyclic compound 108 + ferimzone | 500+500 | 100 |
| Present condensed heterocyclic compound 108 + tebufloquin | 200+2000 | 100 |

(continued)

| Compounds provided for test | Concentration (ppm) | Mortality rate |
|---|---|---|
| Present condensed heterocyclic compound 108 + tebufloquin | 500+50 | 100 |
| Present condensed heterocyclic compound 108 + pyroquilon | 200+5000 | 100 |
| Present condensed heterocyclic compound 108 + pyroquilon | 500+500 | 100 |
| Present condensed heterocyclic compound 48 + tricyclazole | 200+2000 | 100 |
| Present condensed heterocyclic compound 48 + tricyclazole | 500+50 | 100 |
| Present condensed heterocyclic compound 48 + isotianil | 200+2000 | 100 |
| Present condensed heterocyclic compound 48 + isotianil | 500+500 | 100 |
| Present condensed heterocyclic compound 48 + isotianil | 500+50 | 100 |
| Present condensed heterocyclic compound 48 + probenazole | 200+5000 | 100 |
| Present condensed heterocyclic compound 48 + probenazole | 500+500 | 100 |
| Present condensed heterocyclic compound 48 + fthalide | 200+5000 | 100 |
| Present condensed heterocyclic compound 48 + fthalide | 500+500 | 100 |
| Present condensed heterocyclic compound 48 + kasugamycin hydrochloride | 200+2000 | 100 |
| Present condensed heterocyclic compound 48 + kasugamycin hydrochloride | 500+50 | 100 |
| Present condensed heterocyclic compound 48 + ferimzone | 200+5000 | 100 |
| Present condensed heterocyclic compound 48 + ferimzone | 500+500 | 100 |
| Present condensed heterocyclic compound 48 + tebufloquin | 200+2000 | 100 |
| Present condensed heterocyclic compound 48 + tebufloquin | 500+50 | 100 |
| Non-treatment section | - | 0 |

Table 43

| Compounds provided for test | Concentration (ppm) | Mortality rate |
|---|---|---|
| Present condensed heterocyclic compound 48 + pyroquilon | 200+5000 | 100 |
| Present condensed heterocyclic compound 48 + pyroquilon | 500+500 | 100 |
| Present condensed heterocyclic compound 97 + tricyclazole | 200+2000 | 100 |
| Present condensed heterocyclic compound 97 + tricyclazole | 500+50 | 100 |
| Present condensed heterocyclic compound 97 + isotianil | 200+2000 | 100 |
| Present condensed heterocyclic compound 97 + isotianil | 500+500 | 100 |
| Present condensed heterocyclic compound 97 + isotianil | 500+50 | 100 |
| Present condensed heterocyclic compound 97 + probenazole | 200+5000 | 100 |
| Present condensed heterocyclic compound 97 + probenazole | 500+500 | 100 |
| Present condensed heterocyclic compound 97 + fthalide | 200+5000 | 100 |
| Present condensed heterocyclic compound 97 + fthalide | 500+500 | 100 |
| Present condensed heterocyclic compound 97 + kasugamycin hydrochloride | 200+2000 | 100 |
| Present condensed heterocyclic compound 97 + kasugamycin hydrochloride | 500+50 | 100 |
| Present condensed heterocyclic compound 97 + ferimzone | 200+5000 | 100 |
| Present condensed heterocyclic compound 97 + ferimzone | 500+500 | 100 |

(continued)

| Compounds provided for test | Concentration (ppm) | Mortality rate |
|---|---|---|
| Present condensed heterocyclic compound 97 + tebufloquin | 200+2000 | 100 |
| Present condensed heterocyclic compound 97 + tebufloquin | 500+50 | 100 |
| Present condensed heterocyclic compound 97 + pyroquilon | 200+5000 | 100 |
| Present condensed heterocyclic compound 97 + pyroquilon | 500+500 | 100 |
| Present condensed heterocyclic compound 69 + tricyclazole | 200+2000 | 100 |
| Present condensed heterocyclic compound 69 + tricyclazole | 500+50 | 100 |
| Present condensed heterocyclic compound 69 + isotianil | 200+2000 | 100 |
| Present condensed heterocyclic compound 69 + isotianil | 500+500 | 100 |
| Present condensed heterocyclic compound 69 + isotianil | 500+50 | 100 |
| Present condensed heterocyclic compound 69 + probenazole | 200+5000 | 100 |
| Present condensed heterocyclic compound 69 + probenazole | 500+500 | 100 |
| Present condensed heterocyclic compound 69 + fthalide | 200+5000 | 100 |
| Present condensed heterocyclic compound 69 + fthalide | 500+500 | 100 |
| Present condensed heterocyclic compound 69 + kasugamycin hydrochloride | 200+2000 | 100 |
| Present condensed heterocyclic compound 69 + kasugamycin hydrochloride | 500+50 | 100 |
| Present condensed heterocyclic compound 69 + ferimzone | 200+5000 | 100 |
| Present condensed heterocyclic compound 69 + ferimzone | 500+500 | 100 |
| Present condensed heterocyclic compound 69 + tebufloquin | 200+2000 | 100 |
| Present condensed heterocyclic compound 69 + tebufloquin | 500+50 | 100 |
| Present condensed heterocyclic compound 69 + pyroquilon | 200+5000 | 100 |
| Present condensed heterocyclic compound 69 + pyroquilon | 500+500 | 100 |
| Present condensed heterocyclic compound 109 + tricyclazole | 200+2000 | 100 |
| Present condensed heterocyclic compound 109 + tricyclazole | 500+50 | 100 |
| Present condensed heterocyclic compound 109 + isotianil | 200+2000 | 100 |
| Present condensed heterocyclic compound 109 + isotianil | 500+500 | 100 |
| Present condensed heterocyclic compound 109 + isotianil | 500+50 | 100 |
| Non-treatment section | - | 0 |

Table 44

| Compounds provided for test | Concentration (ppm) | Mortality rate |
|---|---|---|
| Present condensed heterocyclic compound 109 + probenazole | 200+5000 | 100 |
| Present condensed heterocyclic compound 109 + probenazole | 500+500 | 100 |
| Present condensed heterocyclic compound 109 + fthalide | 200+5000 | 100 |
| Present condensed heterocyclic compound 109 + fthalide | 500+500 | 100 |
| Present condensed heterocyclic compound 109 + kasugamycin hydrochloride | 200+2000 | 100 |
| Present condensed heterocyclic compound 109 + kasugamycin hydrochloride | 500+50 | 100 |

(continued)

| Compounds provided for test | Concentration (ppm) | Mortality rate |
|---|---|---|
| Present condensed heterocyclic compound 109 + ferimzone | 200+5000 | 100 |
| Present condensed heterocyclic compound 109 + ferimzone | 500+500 | 100 |
| Present condensed heterocyclic compound 109 + tebufloquin | 200+2000 | 100 |
| Present condensed heterocyclic compound 109 + tebufloquin | 500+50 | 100 |
| Present condensed heterocyclic compound 109 + pyroquilon | 200+5000 | 100 |
| Present condensed heterocyclic compound 109 + pyroquilon | 500+500 | 100 |
| Present condensed heterocyclic compound 72 + tricyclazole | 200+2000 | 100 |
| Present condensed heterocyclic compound 72 + tricyclazole | 500+50 | 100 |
| Present condensed heterocyclic compound 72 + isotianil | 200+2000 | 100 |
| Present condensed heterocyclic compound 72 + isotianil | 500+50 | 100 |
| Present condensed heterocyclic compound 72 + isotianil | 500+50 | 100 |
| Present condensed heterocyclic compound 72 + probenazole | 200+5000 | 100 |
| Present condensed heterocyclic compound 72 + probenazole | 500+500 | 100 |
| Present condensed heterocyclic compound 72 fthalide | 200+5000 | 100 |
| Present condensed heterocyclic compound 72 + fthalide | 500+500 | 100 |
| Present condensed heterocyclic compound 72 + kasugamycin hydrochloride | 200+2000 | 100 |
| Present condensed Present heterocyclic compound 72 + kasugamycin hydrochloride | 500+50 | 100 |
| Present condensed heterocyclic compound 72 + ferimzone | 200+5000 | 100 |
| Present condensed heterocyclic compound 72 + ferimzone | 500+500 | 100 |
| Present condensed heterocyclic compound 72 + tebufloquin | 200+2000 | 100 |
| Present condensed heterocyclic compound 72 + tebufloquin | 500+50 | 100 |
| Present condensed heterocyclic compound 72 + pyroquilon | 200+5000 | 100 |
| Present condensed heterocyclic compound 72 + pyroquilon | 500+500 | 100 |
| Present condensed heterocyclic compound 78 + tricyclazole | 200+2000 | 100 |
| Present condensed heterocyclic compound 78 + tricyclazole | 500+50 | 100 |
| Present condensed heterocyclic compound 78 + isotianil | 200+2000 | 100 |
| Present condensed heterocyclic compound 78 + isotianil | 500+500 | 100 |
| Present condensed heterocyclic compound 78 + isotianil | 500+50 | 100 |
| Present condensed heterocyclic compound 78 + probenazole | 200+5000 | 100 |
| Present condensed heterocyclic compound 78 + probenazole | 500+500 | 100 |
| Present condensed heterocyclic compound 78 + fthalide | 200+5000 | 100 |
| Present condensed heterocyclic compound 78 + fthalide | 500+500 | 100 |
| Present condensed heterocyclic compound 78 + kasugamycin hydrochloride | 200+2000 | 100 |
| Present condensed heterocyclic compound 78 + kasugamycin hydrochloride | 500+50 | 100 |
| Non-treatment section | - | 0 |

Table 45

| Compounds provided for test | Concentration (ppm) | Mortality rate |
|---|---|---|
| Present condensed heterocyclic compound 78 + ferimzone | 200+5000 | 100 |
| Present condensed heterocyclic compound 78 + ferimzone | 500+500 | 100 |
| Present condensed heterocyclic compound 78 + tebufloquin | 200+2000 | 100 |
| Present condensed heterocyclic compound 78 + tebufloquin | 500+50 | 100 |
| Present condensed heterocyclic compound 78 + pyroquilon | 200+5000 | 100 |
| Present condensed heterocyclic compound 78 + pyroquilon | 500+500 | 100 |
| Present condensed heterocyclic compound 64 + tricyclazole | 200+2000 | 100 |
| Present condensed heterocyclic compound 64 + tricyclazcle | 500+50 | 100 |
| Present condensed heterocyclic compound 64 + isotianil | 200+2000 | 100 |
| Present condensed heterocyclic compound 64 + isotianil | 500+500 | 100 |
| Present condensed heterocyclic compound 64 + isotianil | 500+50 | 100 |
| Present condensed heterocyclic compound 64 + probenazole | 200+5000 | 100 |
| Present condensed heterocyclic compound 64 + probenazole | 500+500 | 100 |
| Present condensed heterocyclic compound 64 + fthalide | 200+5000 | 100 |
| Present condensed heterocyclic compound 64 + fthalide | 500+500 | 100 |
| Present condensed heterocyclic compound 64 + kasugamycin hydrochloride | 200+2000 | 100 |
| Present condensed heterocyclic compound 64 + kasugamycin hydrochloride | 500+50 | 100 |
| Present condensed heterocyclic compound 64 + ferimzone | 200+5000 | 100 |
| Present condensed heterocyclic compound 64 + ferimzone | 500+500 | 100 |
| Present condensed heterocyclic compound 64 + tebufloquin | 200+2000 | 100 |
| Present condensed heterocyclic compound 64 + tebufloquin | 500+50 | 100 |
| Present condensed heterocyclic compound 64 + pyroquilon | 200+5000 | 100 |
| Present condensed heterocyclic compound 64 + pyroquilon | 500+500 | 100 |
| Present condensed heterocyclic compound 120 + tricyclazole | 200+2000 | 100 |
| Present condensed heterocyclic compound 120 + tricyclazole | 500+50 | 100 |
| Present condensed heterocyclic compound 120 + isotianil | 200+2000 | 100 |
| Present condensed heterocyclic compound 120 + isotianil | 500+500 | 100 |
| Present condensed heterocyclic compound 120 + isotianil | 500+50 | 100 |
| Present condensed heterocyclic compound 120 + probenazole | 200+5000 | 100 |
| Present condensed heterocyclic compound 120 + probenazole | 500+500 | 100 |
| Present condensed heterocyclic compound 120 + fthalide | 200+5000 | 100 |
| Present condensed heterocyclic compound 120 + fthalide | 500+500 | 100 |
| Present condensed heterocyclic compound 120 + kasugamycin hydrochloride | 200+2000 | 100 |
| Present condensed heterocyclic compound 120 + kasugamycin hydrochloride | 500+50 | 100 |
| Present condensed heterocyclic compound 120 + ferimzone | 200+5000 | 100 |
| Present condensed heterocyclic compound 120 + ferimzone | 500+500 | 100 |
| Present condensed heterocyclic compound 120 + tebufloquin | 200+2000 | 100 |
| Present condensed heterocyclic compound 120 + tebufloquin | 500+50 | 100 |

(continued)

| Compounds provided for test | Concentration (ppm) | Mortality rate |
|---|---|---|
| Present condensed heterocyclic compound 120 + pyroquilon | 200+5000 | 100 |
| Present condensed heterocyclic compound 120 + pyroquilon | 500+500 | 100 |
| Non-treatment section | - | 0 |

Table 46

| Compounds provided for test | Concentration (ppm) | Mortality rate |
|---|---|---|
| Present condensed heterocyclic compound 127 + tricyclazole | 200+2000 | 100 |
| Present condensed heterocyclic compound 127 + tricyclazole | 500+50 | 100 |
| Present condensed heterocyclic compound 127 + isotianil | 200+2000 | 100 |
| Present condensed heterocyclic compound 127 + isotianil | 500+500 | 100 |
| Present condensed heterocyclic compound 127 + isotianil | 500+50 | 100 |
| Present condensed heterocyclic compound 127 + probenazole | 200+5000 | 100 |
| Present condensed heterocyclic compound 127 + probenazole | 500+500 | 100 |
| Present condensed heterocyclic compound 127 + fthalide | 200+5000 | 100 |
| Present condensed heterocyclic compound 127 + fthalide | 500+500 | 100 |
| Present condensed heterocyclic compound 127 + kasugamycin hydrochloride | 200+2000 | 100 |
| Present condensed heterocyclic compound 127 + kasugamycin hydrochloride | 500+50 | 100 |
| Present condensed heterocyclic compound 127 + ferimzone | 200+5000 | 100 |
| Present condensed heterocyclic compound 127 + ferimzone | 500+500 | 100 |
| Present condensed heterocyclic compound 127 + tebufloquin | 200+2000 | 100 |
| Present condensed heterocyclic compound 127 + tebufloquin | 500+50 | 100 |
| Present condensed heterocyclic compound 127 + pyroquilon | 200+5000 | 100 |
| Present condensed heterocyclic compound 127 + pyroquilon | 500+500 | 100 |
| Present condensed heterocyclic compound 42 + tricyclazole | 200+2000 | 100 |
| Present condensed heterocyclic compound 42 + tricyclazole | 500+50 | 100 |
| Present condensed heterocyclic compound 42 + isotianil | 200+2000 | 100 |
| Present condensed heterocyclic compound 42 + isotianil | 500+500 | 100 |
| Present condensed heterocyclic compound 42 + isotianil | 500+50 | 100 |
| Present condensed heterocyclic compound 42 + probenazole | 200+5000 | 100 |
| Present condensed heterocyclic compound 42 + probenazole | 500+500 | 100 |
| Present condensed heterocyclic compound 42 + fthalide | 200+5000 | 100 |
| Present condensed heterocyclic compound 42 + fthalide | 500+500 | 100 |
| Present condensed heterocyclic compound 42 + kasugamycin hydrochloride | 200+2000 | 100 |
| Present condensed heterocyclic compound 42 + kasugamycin hydrochloride | 500+50 | 100 |
| Present condensed heterocyclic compound 42 + ferimzone | 200+5000 | 100 |
| Present condensed heterocyclic compound 42 + ferimzone | 500+500 | 100 |
| Present condensed heterocyclic compound 42 + tebufloquin | 200+2000 | 100 |

(continued)

| Compounds provided for test | Concentration (ppm) | Mortality rate |
|---|---|---|
| Present condensed heterocyclic compound 42 + tebufloquin | 500+50 | 100 |
| Present condensed heterocyclic compound 42 + pyroquilon | 200+5000 | 100 |
| Present condensed heterocyclic compound 42 + pyroquilon | 500+500 | 100 |
| Present condensed heterocyclic compound 41 + tricyclazole | 200+2000 | 100 |
| Present condensed heterocyclic compound 41 + tricyclazole | 500+50 | 100 |
| Present condensed heterocyclic compound 41 + isotianil | 200+2000 | 100 |
| Present condensed heterocyclic compound 41 + isotianil | 500+500 | 100 |
| Present condensed heterocyclic compound 41 + isotianil | 500+50 | 100 |
| Present condensed heterocyclic compound 41 + probenazole | 200+5000 | 100 |
| Present condensed heterocyclic compound 41 + probenazole | 500+500 | 100 |
| Non-treatment section | - | 0 |

Table 47

| Compounds provided for test | Concentration (ppm) | Mortality rate |
|---|---|---|
| Present condensed heterocyclic compound 41 + fthalide | 200+5000 | 100 |
| Present condensed heterocyclic compound 41 + fthalide | 500+500 | 100 |
| Present condensed heterocyclic compound 41 + kasugamycin hydrochloride | 200+2000 | 100 |
| Present condensed heterocyclic compound 41 + kasugamycin hydrochloride | 500+50 | 100 |
| Present condensed heterocyclic compound 41 + ferimzone | 200+5000 | 100 |
| Present condensed heterocyclic compound 41 + ferimzone | 500+500 | 100 |
| Present condensed heterocyclic compound 41 + tebufloquin | 200+2000 | 100 |
| Present condensed heterocyclic compound 41 + tebufloquin | 500+50 | 100 |
| Present condensed heterocyclic compound 41 + pyroquilon | 200+5000 | 100 |
| Present condensed heterocyclic compound 41 + pyroquilon | 500+500 | 100 |
| Present condensed heterocyclic compound 104 + tricyclazole | 200+2000 | 100 |
| Present condensed heterocyclic compound 104 + tricyclazole | 500+50 | 100 |
| Present condensed heterocyclic compound 104 + isotianil | 200+2000 | 100 |
| Present condensed heterocyclic compound 104 + isotianil | 500+500 | 100 |
| Present condensed heterocyclic compound 104 + isotianil | 500+50 | 100 |
| Present condensed heterocyclic compound 104 + probenazole | 200+5000 | 100 |
| Present condensed heterocyclic compound 104 + probenazole | 500+500 | 100 |
| Present condensed heterocyclic compound 104 + fthalide | 200+5000 | 100 |
| Present condensed heterocyclic compound 104 + fthalide | 500+500 | 100 |
| Present condensed heterocyclic compound 104 + kasugamycin hydrochloride | 200+2000 | 100 |
| Present condensed heterocyclic compound 104 + kasugamycin hydrochloride | 500+50 | 100 |
| Present condensed heterocyclic compound 104 + ferimzone | 200+5000 | 100 |
| Present condensed heterocyclic compound 104 + ferimzone | 500+500 | 100 |

(continued)

| Compounds provided for test | Concentration (ppm) | Mortality rate |
|---|---|---|
| Present condensed heterocyclic compound 104 + tebufloquin | 200+2000 | 100 |
| Present condensed heterocyclic compound 104 + tebufloquin | 500+50 | 100 |
| Present condensed heterocyclic compound 104 + pyroquilon | 200+5000 | 100 |
| Present condensed heterocyclic compound 104 + pyroquilon | 500+500 | 100 |
| Present condensed heterocyclic compound 118 + tricyclazole | 200+2000 | 100 |
| Present condensed heterocyclic compound 118 + tricyclazole | 500+50 | 100 |
| Present condensed heterocyclic compound 118 + isotianil | 200+2000 | 100 |
| Present condensed heterocyclic compound 118 + isotianil | 500+500 | 100 |
| Present condensed heterocyclic compound 118 + isotianil | 500+50 | 100 |
| Present condensed heterocyclic compound 118 + probenazole | 200+5000 | 100 |
| Present condensed heterocyclic compound 118 + probenazole | 500+500 | 100 |
| Present condensed heterocyclic compound 118 + fthalide | 200+5000 | 100 |
| Present condensed heterocyclic compound 118 + fthalide | 500+500 | 100 |
| Present condensed heterocyclic compound 118 + kasugamycin hydrochloride | 200+2000 | 100 |
| Present condensed heterocyclic compound 118 + kasugamycin hydrochloride | 500+50 | 100 |
| Present condensed heterocyclic compound 118 + ferimzone | 200+5000 | 100 |
| Present condensed heterocyclic compound 118 + ferimzone | 500+500 | 100 |
| Non-treatment section | - | 0 |

Table 48

| Compounds provided for test | Concentration (ppm) | Mortality rate |
|---|---|---|
| Present condensed heterocyclic compound 118 + tebufloquin | 200+2000 | 100 |
| Present condensed heterocyclic compound 118 + tebufloquin | 500+50 | 100 |
| Present condensed heterocyclic compound 118 + pyroquilon | 200+5000 | 100 |
| Present condensed heterocyclic compound 118 + pyroquilon | 500+500 | 100 |
| Present condensed heterocyclic compound 119 + tricyclazole | 200+2000 | 100 |
| Present condensed heterocyclic compound 119 + tricyclazole | 500+50 | 100 |
| Present condensed heterocyclic compound 119 + isotianil | 200+2000 | 100 |
| Present condensed heterocyclic compound 119 + isotianil | 500+500 | 100 |
| Present condensed heterocyclic compound 119 + isotianil | 500+50 | 100 |
| Present condensed heterocyclic compound 119 + probenazole | 200+5000 | 100 |
| Present condensed heterocyclic compound 119 + probenazole | 500+500 | 100 |
| Present condensed heterocyclic compound 119 + fthalide | 200+5000 | 100 |
| Present condensed heterocyclic compound 119 + fthalide | 500+500 | 100 |
| Present condensed heterocyclic compound 119 + kasugamycin hydrochloride | 200+2000 | 100 |
| Present condensed heterocyclic compound 119 + kasugamycin hydrochloride | 500+50 | 100 |
| Present condensed heterocyclic compound 119 + ferimzone | 200+5000 | 100 |

(continued)

| Compounds provided for test | Concentration (ppm) | Mortality rate |
|---|---|---|
| Present condensed heterocyclic compound 119 + ferimzone | 500+500 | 100 |
| Present condensed heterocyclic compound 119 + tebufloquin | 200+2000 | 100 |
| Present condensed heterocyclic compound 119 + tebufloquin | 500+50 | 100 |
| Present condensed heterocyclic compound 119 + pyroquilon | 200+5000 | 100 |
| Present condensed heterocyclic compound 119 + pyroquilon | 500+500 | 100 |
| Present condensed heterocyclic compound 130 + tricyclazole | 200+2000 | 100 |
| Present condensed heterocyclic compound 130 + tricyclazole | 500+50 | 100 |
| Present condensed heterocyclic compound 130 + isotianil | 200+2000 | 100 |
| Present condensed heterocyclic compound 130 + isotianil | 500+500 | 100 |
| Present condensed hetezocyclic compound 130 + isotianil | 500+50 | 100 |
| Present condensed heterocyclic compound 130 + probenazole | 200+5000 | 100 |
| Present condensed heterocyclic compound 130 + probenazole | 500+500 | 100 |
| Present condensed heterocyclic compound 130 + fthalide | 200+5000 | 100 |
| Present condensed heterocyclic compound 130 + fthalide | 500+500 | 100 |
| Present condensed heterocyclic compound 130 + kasugamycin hydrochloride | 200+2000 | 100 |
| Present condensed heterocyclic compound 130 + kasugamycin hydrochloride | 500+50 | 100 |
| Present condensed heterocyclic compound 130 + ferimzone | 200+5000 | 100 |
| Present condensed heterocyclic compound 130 + ferimzone | 500+500 | 100 |
| Present condensed heterocyclic compound 130 + tebufloquin | 200+2000 | 100 |
| Present condensed heterocyclic compound 130 + tebufloquin | 500+50 | 100 |
| Present condensed heterocyclic compound 130 + pyroquilon | 200+5000 | 100 |
| Present condensed heterocyclic compound 130 + pyroquilon | 500+500 | 100 |
| Present condensed heterocyclic compound 131 + tricyclazole | 200+2000 | 100 |
| Present condensed heterocyclic compound 131 + tricyclazole | 500+50 | 100 |
| Present condensed heterocyclic compound 131 + isotianil | 200+2000 | 100 |
| Present condensed heterocyclic compound 131 + isotianil | 500+500 | 100 |
| Present condensed heterocyclic compound 131 + isotianil | 500+50 | 100 |
| Present condensed heterocyclic compound 131 + probenazole | 200+5000 | 100 |
| Present condensed heterocyclic compound 131 + probenazole | 500+500 | 100 |
| Present condensed heterocyclic compound 131 + fthalide | 200+5000 | 100 |
| Present condensed heterocyclic compound 131 + fthalide | 500+500 | 100 |
| Present condensed heterocyclic compound 131 + kasugamycin hydrochloride | 200+2000 | 100 |
| Present condensed heterocyclic compound 131 + kasugamycin hydrochloride | 500+50 | 100 |
| Present condensed heterocyclic compound 131 + ferimzone | 200+5000 | 100 |
| Present condensed heterocyclic compound 131 + ferimzone | 500+500 | 100 |
| Present condensed heterocyclic compound 131 + tebufloquin | 200+2000 | 100 |
| Present condensed heterocyclic compound 131 + tebufloquin | 500+50 | 100 |
| Present condensed heterocyclic compound 131 + pyroquilon | 200+5000 | 100 |

(continued)

| Compounds provided for test | Concentration (ppm) | Mortality rate |
|---|---|---|
| Present condensed heterocyclic compound 131 + pyroquilon | 500+500 | 100 |
| Non-treatment section | - | 0 |

Test Example 5

1 mg of each of the present condensed heterocyclic compounds 3, 23, 27, 31, 39, 41, 42, 46, 48, 51, 56, 58, 64, 69, 72, 74, 78, 83, 91, 97, 104, 105, 107, 108, 109, 118, 119, 120, 127, 130, and 131 was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A commercial formulation of validamycin A (trade name: Validacin 5 liquid medicine, Sumitomo Chemical Co., Ltd.) was diluted with water which contains 0.02 volume% of a spreading agent (trade name: Shindain, manufactured by Sumitomo Chemical Co., Ltd.) so as to have a predetermined concentration.

1 mg of each of furametpyr (manufactured by Waco Pure Chemical Industries, Ltd.) and pencycuron (manufactured by Waco Pure Chemical Industries, Ltd.) was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A water-diluted solution of the above-described present condensed heterocyclic compounds and a water-diluted solution of validamycin A, furametpyr, or pencycuron were mixed together to prepare a liquid medicine for a test.

Each leaf disc (diameter of 1.5 cm) of a cabbage (Brassicae oleracea) was accommodated in each well of a 24 hole microplate (manufactured by Becton Dickinson) and 40 μL of liquid medicine for a test was scattered to each well. Wells to which 40 μL of water containing 0.02 volume% of a spreading agent (trade name: Shindain, manufactured by Sumitomo Chemical Co., Ltd.) was scattered was set to a non-treatment section.

After air-drying, 5 second-instar larvae of diamondback moths (Plutella xylostella) were released in each well, which was then covered with paper towel and closed with a lid. 2 days after the releasing, the number of surviving larvae in each well was observed.

The mortality rates of the treatment section and the non-treatment section were calculated using the following Formula 1). The test was repeated once.

Formula 1) Mortality rate (%) = (the number of larvae provided for a test - the number of surviving larvae) / the number of larvae provided for a test x 100

Table 49

| Compounds provided for test | Concentration (ppm) | Mortality rate |
|---|---|---|
| Present condensed heterocyclic compound 3 + validamycin A | 200+2000 | 100 |
| Present condensed heterocyclic compound 3 + validamycin A | 500+50 | 100 |
| Present condensed heterocyclic compound 3 + furametpyr | 200+2000 | 100 |
| Present condensed heterocyclic compound 3 + furametpyr | 500+50 | 100 |
| Present condensed heterocyclic compound 3 + pencycuron | 200+5000 | 100 |
| Present condensed heterocyclic compound 3 + pencycuron | 500+500 | 100 |
| Present condensed heterocyclic compound 39 + validamycin A | 200+2000 | 100 |

(continued)

| Compounds provided for test | Concentration (ppm) | Mortality rate |
|---|---|---|
| Present condensed heterocyclic compound 39 + validamycin A | 500+50 | 100 |
| Present condensed heterocyclic compound 39 + furametpyr | 200+2000 | 100 |
| Present condensed heterocyclic compound 39 + furametpyr, | 500+50 | 100 |
| Present condensed heterocyclic compound 39 + pencycuron | 200+5000 | 100 |
| Present condensed heterocyclic compound 39 + pencycuron | 500+500 | 100 |
| Present condensed heterocyclic compound 23 + validamycin A | 200+2000 | 100 |
| Present condensed heterocyclic compound 23 + validamycin A | 500+50 | 100 |
| Present condensed heterocyclic compound 23 + furametpyr | 200+2000 | 100 |
| Present condensed heterocyclic compound 23 + furametpyr | 500+50 | 100 |
| Present condensed heterocyclic compound 23 + pencycuron | 200+5000 | 100 |
| Present condensed heterocyclic compound 23 + pencycuron | 500+500 | 100 |
| Present condensed heterocyclic compound 74 + validamycin A | 200+2000 | 100 |
| Present condensed heterocyclic compound 74 + validamycin A | 500+50 | 100 |
| Present condensed heterocyclic compound 74 + furametpyr | 200+2000 | 100 |
| Present condensed heterocyclic compound 74 + furametpyr, | 500+50 | 100 |
| Present condensed heterocyclic compound 74 + pencycuron | 200+5000 | 100 |
| Present condensed heterocyclic compound 74 + pencycuron | 500+500 | 100 |
| Present condensed heterocyclic compound 83 + validamycin A | 200+2000 | 100 |
| Present condensed heterocyclic compound 83 + validamycin A | 500+50 | 100 |
| Present condensed heterocyclic compound 83 + furametpyr | 200+2000 | 100 |
| Present condensed heterocyclic compound 83 + furametpyr, | 500+50 | 100 |
| Present condensed heterocyclic compound 83 + pencycuron | 200+5000 | 100 |
| Present condensed heterocyclic compound 83 + pencycuron | 500+500 | 100 |
| Present condensed heterocyclic compound 58 + validamycin A | 200+2000 | 100 |
| Present condensed heterocyclic compound 58 + validamycin A | 500+50 | 100 |
| Non-treatment section | - | 0 |

Table 50

| Compounds provided for test | Concentration (ppm) | Mortality rate |
|---|---|---|
| Present condensed heterocyclic compound 58 + furametpyr | 200+2000 | 100 |
| Present condensed heterocyclic compound 58 + furametpyr | 500+50 | 100 |
| Present condensed heterocyclic compound 58 + pencycuron | 200+5000 | 100 |
| Present condensed heterocyclic compound 58 + pencvcuron | 500+500 | 100 |
| Present condensed heterocyclic compound 31 + validamycin A | 200+2000 | 100 |
| Present condensed heterocyclic compound 31 + valicamycin A | 500+50 | 100 |
| Present condensed heterocyclic compound 31 + furametpyr | 200+2000 | 100 |
| Present condensed heterocyclic compound 31 + furametpyr | 500+50 | 100 |

(continued)

| Compounds provided for test | Concentration (ppm) | Mortality rate |
|---|---|---|
| Present condensed heterocyclic compound 31 + pencycuron | 200+5000 | 100 |
| Present condensed heterocyclic compound 31 + pencycuron | 500+500 | 100 |
| Present condensed heterocyclic compound 56 + validamycin A | 200+2000 | 100 |
| Present condensed heterocyclic compound 56 + validamycin A | 500+50 | 100 |
| Present condensed heterocyclic compound 56 + furametpyr | 200+2000 | 100 |
| Present condensed heterocyclic compound 56 + furametpyr | 500+50 | 100 |
| Present condensed heterocyclic compound 56 + pencycuron | 200+5000 | 100 |
| Present condensed heterocyclic compound 56 + pencycuron | 500+500 | 100 |
| Present condensed heterocyclic compound 46 + validamycin A | 200+2000 | 100 |
| Present condensed heterocyclic compound 46 + validamycin A | 500+50 | 100 |
| Present condensed heterocyclic compound 46 + furametpyr | 200+2000 | 100 |
| Present condensed heterocyclic compound 46 + furametpyr | 500+50 | 100 |
| Present condensed heterocyclic compound 46 + pencycuron | 200+5000 | 100 |
| Present condensed heterocyclic compound 46 + pencycuron | 500+500 | 100 |
| Present condensed heterocyclic compound 105 + validamycin A | 200+2000 | 100 |
| Present condensed heterocyclic compound 105 + validamycin A | 500+50 | 100 |
| Present condensed heterocyclic compound 105 + furametpyr | 200+2000 | 100 |
| Present condensed heterocyclic compound 105 + furametpyr | 500+50 | 100 |
| Present condensed heterocyclic compound 105 + pencycuron | 200+5000 | 100 |
| Present condensed heterocyclic compound 105 + pencycuron | 500+500 | 100 |
| Present condensed heterocyclic compound 91 + validamycin A | 200+2000 | 100 |
| Present condensed heterocyclic compound 91 + validamycin A | 500+50 | 100 |
| Present condensed heterocyclic compound 91 + furametpyr, | 200+2000 | 100 |
| Present condensed heterocyclic compound 91 + furametpyr, | 500+50 | 100 |
| Present condensed heterocyclic compound 91 + pencycuron | 200+5000 | 100 |
| Present condensed heterocyclic compound 91 + pencycuron | 500+500 | 100 |
| Present condensed heterocyclic compound 107 + validamycin A | 200+2000 | 100 |
| Present condensed heterocyclic compound 107 + validamycin A | 500+50 | 100 |
| Present condensed heterocyclic compound 107 + furametpyr | 200+2000 | 100 |
| Present condensed heterocyclic compound 107 + furametpyr | 500+50 | 100 |
| Present condensed heterocyclic compound 107 + pencycuron | 200+5000 | 100 |
| Present condensed heterocyclic compound 107 + pencycuron | 500+500 | 100 |
| Non-treatment section | - | 0 |

Table 51

| Compounds provided for test | Concentration (ppm) | Mortality rate |
|---|---|---|
| Present condensed heterocyclic compound 51 + validamycin A | 200+2000 | 100 |

(continued)

| Compounds provided for test | Concentration (ppm) | Mortality rate |
|---|---|---|
| Present condensed heterocyclic compound 51 + validamycin A | 500+50 | 100 |
| Present condensed heterocyclic compound 51 + furametpyr | 200+2000 | 100 |
| Present condensed heterocyclic compound 51 + furametpyr, | 500+50 | 100 |
| Present condensed heterocyclic compound 51 + pencycuron | 200+5000 | 100 |
| Present condensed heterocyclic compound 51 + pencycuron | 500+500 | 100 |
| Present condensed heterocyclic compound 27 + validamycin A | 200+2000 | 100 |
| Present condensed heterocyclic compound 27 + validamycin A | 500+50 | 100 |
| Present condensed heterocyclic compound 27 + furametpyr | 200+2000 | 100 |
| Present condensed heterocyclic compound 27 + furametpyr | 500+50 | 100 |
| Present condensed heterocyclic compound 27 + pencycuron | 200+5000 | 100 |
| Present condensed heterocyclic compound 27 + pencycuron | 500+500 | 100 |
| Present condensed heterocyclic compound 108 + validamycin A | 200+2000 | 100 |
| Present condensed heterocyclic compound 108 + validamycin A | 500+50 | 100 |
| Present condensed heterocyclic compound 108 + furametpyr | 200+2000 | 100 |
| Present condensed heterocyclic compound 108 + furametpyr | 500+50 | 100 |
| Present condensed heterocyclic compound 108 + pencycuron | 200+5000 | 100 |
| Present condensed heterocyclic compound 108 + pencycuron | 500+500 | 100 |
| Present condensed heterocyclic compound 48 + validamycin A | 200+2000 | 100 |
| Present condensed heterocyclic compound 48 + validamycin A | 500+50 | 100 |
| Present condensed heterocyclic compound 48 + furametpyr | 200+2000 | 100 |
| Present condensed heterocyclic compound 48 + furametpyr | 500+50 | 100 |
| Present condensed heterocyclic compound 48 + pencycuron | 200+5000 | 100 |
| Present condensed heterocyclic compound 48 + pencycuron | 500+500 | 100 |
| Present condensed heterocyclic compound 97 + validamycin A | 200+2000 | 100 |
| Present condensed heterocyclic compound 97 + validamycin A | 500+50 | 100 |
| Present condensed heterocyclic compound 97 + furametpyr | 200+2000 | 100 |
| Present condensed heterocyclic compound 97 + furametpyr | 500+50 | 100 |
| Present condensed heterocyclic compound 97 + pencycuron | 200+5000 | 100 |
| Present condensed heterocyclic compound 97 + pencycuron | 500+500 | 100 |
| Present condensed heterocyclic compound 69 + validamycin A | 200+2000 | 100 |
| Present condensed heterocyclic compound 69 + validamycin A | 500+50 | 100 |
| Present condensed heterocyclic compound 69 + furametpyr | 200+2000 | 100 |
| Present condensed heterocyclic compound 69 + furametpyr | 500+50 | 100 |
| Present condensed heterocyclic compound 69 + pencycuron | 200+5000 | 100 |
| Present condensed heterocyclic compound 69 + pencycuron | 500+500 | 100 |
| Present condensed heterocyclic compound 109 + validamycin A | 200+2000 | 100 |
| Present condensed heterocyclic compound 109 + validamycin A | 500+50 | 100 |
| Present condensed heterocyclic compound 109 + furametpyr | 200+2000 | 100 |

(continued)

| Compounds provided for test | Concentration (ppm) | Mortality rate |
|---|---|---|
| Present condensed heterocyclic compound 109 + furametpyr | 500+50 | 100 |
| Non-treatment section | - | 0 |

Table 52

| Compounds provided for test | Concentration (ppm) | Mortality rate |
|---|---|---|
| Present condensed heterocyclic compound 109 + pencycuron | 200+5000 | 100 |
| Present condensed heterocyclic compound 109 + pencycuron | 500+500 | 100 |
| Present condensed heterocyclic compound 72 + validamycin A | 200+2000 | 100 |
| Present condensed heterocyclic compound 72 + validamycin A | 500+50 | 100 |
| Present condensed heterocyclic compound 72 + furametpyr | 200+2000 | 100 |
| Present condensed heterocyclic compound 72 + furametpyr | 500+50 | 100 |
| Present condensed heterocyclic compound 72 + pencycuron | 200+5000 | 100 |
| Present condensed heterocyclic compound 72 + pencycuron | 500+500 | 100 |
| Present condensed heterocyclic compound 78 + validamycin A | 200+2000 | 100 |
| Present condensed heterocyclic compound 78 + validamycin A | 500+50 | 100 |
| Present condensed heterocyclic compound 78 + furametpyr | 200+2000 | 100 |
| Present condensed heterocyclic compound 78 + furametpyr | 500+50 | 100 |
| Present condensed heterocyclic compound 78 + pencycuron | 200+5000 | 100 |
| Present condensed heterocyclic compound 78 + pencycuron | 500+500 | 100 |
| Present condensed heterocyclic compound 64 + validamycin A | 200+2000 | 100 I |
| Present condensed heterocyclic compound 64 + validamycin A | 500+50 | 100 |
| Present condensed heterocyclic compound 64 + furametpyr | 200+2000 | 100 |
| Present condensed heterocyclic compound 64 + furametpyr, | 500+50 | 100 |
| Present condensed heterocyclic compound 64 + pencycuron | 200+5000 | 100 |
| Present condensed heterocyclic compound 64 + pencycuron | 500+500 | 100 |
| Present condensed heterocyclic compound 120 + validamycin A | 200+2000 | 100 |
| Present condensed heterocyclic compound 120 + validamycin A | 500+50 | 100 |
| Present condensed heterocyclic compound 120 + furametpyr | 200+2000 | 100 |
| Present condensed heterocyclic compound 120 + furametpyr | 500+50 | 100 |
| Present condensed heterocyclic compound 120 + pencycuron | 200+5000 | 100 |
| Present condensed heterocyclic compound 120 + pencycuron | 500+500 | 100 |
| Present condensed heterocyclic compound 127 + validamycin A | 200+2000 | 100 |
| Present condensed heterocyclic compound 127 + validamycin A | 500+50 | 100 |
| Present condensed heterocyclic compound 127 + furametpyr | 200+2000 | 100 |
| Present condensed heterocyclic compound 127 + furametpyr | 500+50 | 100 |
| Present condensed heterocyclic compound 127 + pencycuron | 200+5000 | 100 |
| Present condensed heterocyclic compound 127 + pencycuron | 500+500 | 100 |

(continued)

| Compounds provided for test | Concentration (ppm) | Mortality rate |
|---|---|---|
| Present condensed heterocyclic compound 42 + validamycin A | 200+2000 | 100 |
| Present condensed heterocyclic compound 42 + validamycin A | 500+50 | 100 |
| Present condensed heterocyclic compound 42 + furametpyr | 200+2000 | 100 |
| Present condensed heterocyclic compound 42 + furametpyr, | 500+50 | 100 |
| Present condensed heterocyclic compound 42 + pencycuron | 200+5000 | 100 |
| Present condensed heterocyclic compound 42 + pencycuron | 500+500 | 100 |
| Present condensed heterocyclic compound 41 + validanycin A | 200+2000 | 100 |
| Present condensed heterocyclic compound 41 + validamycin A | 500+50 | 100 |
| Non-treatment section | - | 0 |

Table 53

| Compounds provided for test | Concentration (ppm) | Mortality rate |
|---|---|---|
| Present condensed heterocyclic compound 41 + furametpyr | 200+2000 | 100 |
| Present condensed heterocyclic compound 41 + furametpyr | 500+50 | 100 |
| Present condensed heterocyclic compound 41 + pencycuron | 200+5000 | 100 |
| Present condensed heterocyclic compound 41 + pencycuron | 500+500 | 100 |
| Present condensed heterocyclic compound 104 + validamycin A | 200+2000 | 100 |
| Present condensed heterocyclic compound 104 + validamycin A | 500+50 | 100 |
| Present condensed heterocyclic compound 104 + furametpyr | 200+2000 | 100 |
| Present condensed heterocyclic compound 104 + furametpyr | 500+50 | 100 |
| Present condensed heterocyclic compound 104 + pencycuron | 200+5000 | 100 |
| Present condensed heterocyclic compound 104 + pencycuron | 500+500 | 100 |
| Present condensed heterocyclic compound 118 + validamycin A | 200-2000 | 100 |
| Present condensed heterocyclic compound 118 + validamycin A | 500+50 | 100 |
| Present condensed heterocyclic compound 118 + furametpyr, | 200+2000 | 100 |
| Present condensed heterocyclic compound 118 + furametpyr | 500+50 | 100 |
| Present condensed heterocyclic compound 118 + pencycuron | 200+5000 | 100 |
| Present condensed heterocyclic compound 118 + pencycuron | 500+500 | 100 |
| Present condensed heterocyclic compound 119 + validamycin A | 200+2000 | 100 |
| Present condensed heterocyclic compound 119 + validamycin A | 500+50 | 100 |
| Present condensed heterocyclic compound 119 + furametpyr | 200+2000 | 100 |
| Present condensed heterocyclic compound 119 + furametpyr | 500+50 | 100 |
| Present condensed heterocyclic compound 119 + pencycuron | 200+5000 | 100 |
| Present condensed heterocyclic compound 119 + pencycuron | 500+500 | 100 |
| Present condensed heterocyclic compound 130 + validamycin | 200+2000 | 100 |
| Present condensed heterocyclic compound 130 + validamycin A | 500+50 | 100 |
| Present condensed heterocyclic compound 130 + furametpyr | 200+2000 | 100 |

(continued)

| Compounds provided for test | Concentration (ppm) | Mortality rate |
|---|---|---|
| Present condensed heterocyclic compound 130 + furametpyr | 500+50 | 100 |
| Present condensed heterocyclic compound 130 + pencycuron | 200+5000 | 100 |
| Present condensed heterocyclic compound 130 + pencycuron | 500+500 | 100 |
| Present condensed heterocyclic compound 131 + validamycin A | 200+2000 | 100 |
| Present condensed heterocyclic compound 131 + validamycin A | 500+50 | 100 |
| Present condensed heterocyclic compound 131 + furametpyr | 200+2000 | 100 |
| Present condensed heterocyclic compound 131 + furametpyr | 500+50 | 100 |
| Present condensed heterocyclic compound 131 + pencycuron | 200+5000 | 100 |
| Present condensed heterocyclic compound 131 + pencycuron | 500+500 | 100 |
| Non-treatment section | - | 0 |

Test Example 6

1 mg of each of the present condensed heterocyclic compounds 3, 23, 27, 31, 39, 41, 42, 46, 48, 51, 56, 58, 64, 69, 72, 74, 78, 83, 91, 97, 104, 105, 107, 108, 109, 118, 119, 120, 127, 130, and 131 was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

1 mg of each of flutolanil (manufactured by Waco Pure Chemical Industries, Ltd.) fluopyram, penflufen, sedaxane, and fluxapyroxad was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A water-diluted solution of the above-described present condensed heterocyclic compounds and a water-diluted solution of flutolanil, fluopyram, penflufen, sedaxane, or fluxapyroxad were mixed together to prepare a liquid medicine for a test. Each leaf disc (diameter of 1.5 cm) of a cabbage (Brassicae oleracea) was accommodated in each well of a 24 hole microplate (manufactured by Becton Dickinson) and 40 μL of liquid medicine for a test was scattered to each well. Wells to which 40 μL of water containing 0.02 volume% of a spreading agent (trade name: Shindain, manufactured by Sumitomo Chemical Co., Ltd.) was scattered was set to a non-treatment section.

After air-drying, 5 second-instar larvae of diamondbaok moths (Plutella xylostella) were released in each well, which was then covered with paper towel and closed with a lid. 2 days after the releasing, the number of surviving larvae in each well was observed.

The mortality rates of the treatment section and the non-treatment section were calculated using the following Formula 1). The test was repeated once.

Formula 1) Mortality rate (%) = (the number of larvae provided for a test - the number of surviving larvae) / the number of larvae provided for a test x 100

Table 54

| Compounds provided for test | Concentration (ppm) | Mortality rate |
|---|---|---|
| Present condensed heterocyclic compound 3 + flutolanil | 200+5000 | 100 |
| Present condensed heterocyclic compound 3 + flutolanil | 500+500 | 100 |

(continued)

| Compounds provided for test | Concentration (ppm) | Mortality rate |
|---|---|---|
| Present condensed heterocyclic compound 39 + flutolanil | 200+5000 | 100 |
| Present condensed heterocyclic compound 39 + flutolanil | 500+500 | 100 |
| Present condensed heterocyclic compound 23 + flutolanil | 200+5000 | 80 |
| Present condensed heterocyclic compound 23 + flutolanil | 500+500 | 100 |
| Present condensed heterocyclic compound 74 + flutolanil | 200+5000 | 100 |
| Present condensed heterocyclic compound 74 + flutolanil | 500+500 | 100 |
| Present condensed heterocyclic compound 83 + flutolanil | 200+5000 | 100 |
| Present condensed heterocyclic compound 83 + flutolanil | 500+500 | 100 |
| Present condensed heterocyclic compound 58 + flutolanil | 200+5000 | 100 |
| Present condensed heterocyclic compound 58 + flutolanil | 500+500 | 100 |
| Present condensed heterocyclic compound 31 + flutolanil | 200+5000 | 100 |
| Present condensed heterocyclic compound 31 + flutolanil | 500+500 | 100 |
| Present condensed heterocyclic compound 56 + flutolanil | 200+5000 | 100 |
| Present condensed heterocyclic compound 56 + flutolanil | 500+500 | 100 |
| Present condensed heterocyclic compound 46 + flutolanil | 200+5000 | 100 |
| Present condensed heterocyclic compound 46 + flutolanil | 500+500 | 100 |
| Present condensed heterocyclic compound 105 + flutolanil | 200+5000 | 100 |
| Present condensed heterocyclic compound 105 + flutolanil | 500+500 | 100 |
| Present condensed heterocyclic compound 91 + flutolanil | 200+5000 | 100 |
| Present condensed heterocyclic compound 91 + flutolanil | 500+500 | 100 |
| Present condensed heterocyclic compound 107 + flutolanil | 200+5000 | 100 |
| Present condensed heterocyclic compound 107 + flutolanil | 500+500 | 100 |
| Present condensed heterocyclic compound 51 + flutolanil | 200+5000 | 100 |
| Present condensed heterocyclic compound 51 + flutolanil | 500+500 | 100 |
| Present condensed heterocyclic compound 27 + flutolanil | 200+5000 | 100 |
| Present condensed heterocyclic compound 27 + flutolanil | 500+500 | 100 |
| Present condensed heterocyclic compound 108 + flutolanil | 200+5000 | 100 |
| Present condensed heterocyclic compound 108 + flutolanil | 500+500 | 100 |
| Present condensed heterocyclic compound 48 + flutolanil | 200+5000 | 100 |
| Present condensed heterocyclic compound 48 + flutolanil | 500+500 | 100 |
| Present condensed heterocyclic compound 97 + flutolanil | 200+5000 | 80 |
| Present condensed heterocyclic compound 97 + flutolanil | 500+500 | 100 |
| Non-treatment section | - | 0 |

Table 55

| Compounds provided for test | Concentration (ppm) | Mortality rate |
|---|---|---|
| Present condensed heterocyclic compound 69 + flutolanil | 200+5000 | 100 |

(continued)

| Compounds provided for test | Concentration (ppm) | Mortality rate |
|---|---|---|
| Present condensed heterocyclic compound 69 + flutolanil | 500+500 | 100 |
| Present condensed heterocyclic compound 109 + flutolanil | 200+5000 | 100 |
| Present condensed heterocyclic compound 109 + flutolanil | 500+500 | 100 |
| Present condensed heterocyclic compound 72 + flutolanil | 200+5000 | 80 |
| Present condensed heterocyclic compound 72 + flutolanil | 500+500 | 100 |
| Present condensed heterocyclic compound 78 + flutolanil | 200+5000 | 80 |
| Present condensed heterocyclic compound 78 + flutolanil | 500+500 | 100 |
| Present condensed heterocyclic compound 64 + flutolanil | 200+5000 | 100 |
| Present condensed heterocyclic compound 64 + flutolanil | 500+500 | 100 |
| Present condensed heterocyclic compound 120 + flutolanil | 200+5000 | 100 |
| Present condensed heterocyclic compound 120 + flutolanil | 500+500 | 100 |
| Present condensed heterocyclic compound 127 + flutolanil | 200+5000 | 100 |
| Present condensed heterocyclic compound 127 + flutolanil | 500+500 | 100 |
| Present condensed heterocyclic compound 42 + flutolanil | 200+5000 | 100 |
| Present condensed heterocyclic compound 42 + flutolanil | 500+500 | 100 |
| Present condensed heterocyclic compound 41 + flutolanil | 200+5000 | 80 |
| Present condensed heterocyclic compound 41 + flutolanil | 500+500 | 100 |
| Present condensed heterocyclic compound 104 + flutolanil | 200+5000 | 80 |
| Present condensed heterocyclic compound 104 + flutolanil | 500+500 | 100 |
| Present condensed heterocyclic compound 97 + fluopyram | 200+2000 | 100 |
| Present condensed heterocyclic compound 97 + fluopyram | 500+0.5 | 100 |
| Present condensed heterocyclic compound 97 + penflufen | 200+2000 | 100 |
| Present condensed heterocyclic compound 97 + penflufen | 500+500 | 100 |
| Present condensed heterocyclic compound 97 + penflufen | 500+0.5 | 100 |
| Present condensed heterocyclic compound 97 + sedaxane | 200+2000 | 100 |
| Present condensed heterocyclic compound 97 + sedaxane | 500+0.5 | 100 |
| Present condensed heterocyclic compound 97 + fluxapyroxad | 200+2000 | 100 |
| Present condensed heterocyclic compound 97 + fluxapyroxad | 500+0.5 | 100 |
| Present condensed heterocyclic compound 104 + fluopyram | 200+2000 | 100 |
| Present condensed heterocyclic compound 104 + fluopyram | 500+0.5 | 100 |
| Present condensed heterocyclic compound 104 + penflufen | 200+2000 | 100 |
| Present condensed heterocyclic compound 104 + penflufen | 500+500 | 100 |
| Present condensed heterocyclic compound 104 + penflufen | 500+0.5 | 100 |
| Present condensed heterocyclic compound 104 + sedaxane | 200+2000 | 100 |
| Present condensed heterocyclic compound 104 + sedaxane | 500+0.5 | 100 |
| Present condensed heterocyclic compound 104 + fluxapyroxad | 200+2000 | 100 |
| Present condensed heterocyclic compound 104 + fluxapyroxad | 500+0.5 | 100 |
| Present condensed heterocyclic compound 105 + fluopyram | 200+2000 | 100 |

(continued)

| Compounds provided for test | Concentration (ppm) | Mortality rate |
|---|---|---|
| Present condensed heterocyclic compound 105 + fluopyram | 500+0.5 | 100 |
| Non-treatment section | - | 0 |

Table 56

| Compounds provided for test | Concentration (ppm) | Mortality rate |
|---|---|---|
| Present condensed heterocyclic compound 105 + penflufen | 200+2000 | 100 |
| Present condensed heterocyclic compound 105 + penflufen | 500+500 | 100 |
| Present condensed heterocyclic compound 105 + penflufen | 500+0.5 | 100 |
| Present condensed heterocyclic compound 105 + sedaxane | 200+2000 | 100 |
| Present condensed heterocyclic compound 105 + sedaxane | 500+0.5 | 100 |
| Present condensed heterocyclic compound 105 + fluxapyroxad | 200-2000 | 100 |
| Present condensed heterocyclic compound 105 + fluxapyroxad | 500+0.5 | 100 |
| Present condensed heterocyclic compound 107 + fluopyram | 200+2000 | 100 |
| Present condensed heterocyclic compound 107 + fluopyram | 500+0.5 | 100 |
| Present condensed heterocyclic compound 107 + penflufen | 200+2000 | 100 |
| Present condensed heterocyclic compound 107 + penflufen | 500+500 | 100 |
| Present condensed heterocyclic compound 107 + penflufen | 500+0.5 | 100 |
| Present condensed heterocyclic compound 107 + sedaxane | 200+2000 | 100 |
| Present condensed heterocyclic compound 107 + sedaxane | 500+0.5 | 100 |
| Present condensed heterocyclic compound 107 + fluxapyroxad | 200+2000 | 100 |
| Present condensed heterocyclic compound 107 + fluxapyroxad | 500+0.5 | 100 |
| Present condensed heterocyclic compound 108 + fluopyram | 200+2000 | 100 |
| Present condensed heterocyclic compound 108 + fluopyram | 500+0.5 | 100 |
| Present condensed heterocyclic compound 108 + penflufen | 200+2000 | 100 |
| Present condensed heterocyclic compound 108 + penflufen | 500+500 | 100 |
| Present condensed heterocyclic compound 108 + penflufen | 500+0.5 | 100 |
| Present condensed heterocyclic compound 108 + sedaxane | 200+2000 | 100 |
| Present condensed heterocyclic compound 108 + sedaxane | 500+0.5 | 100 |
| Present condensed heterocyclic compound 108 + fluxapyroxad | 200+2000 | 100 |
| Present condensed heterocyclic compound 108 + fluxapyroxad | 500+0.5 | 100 |
| Present condensed heterocyclic compound 109 + fluopyram | 200+2000 | 100 |
| Present condensed heterocyclic compound 109 + fluopyram | 500+0.5 | 100 |
| Present condensed heterocyclic compound 109 + penflufen | 200+2000 | 100 |
| Present condensed heterocyclic compound 109 + penflufen | 500+500 | 100 |
| Present condensed heterocyclic compound 109 + penflufen | 500+0.5 | 100 |
| Present condensed heterocyclic compound 109 + sedaxane | 200+2000 | 100 |
| Present condensed heterocyclic compound 109 + sedaxane | 500+0.5 | 100 |

(continued)

| Compounds provided for test | Concentration (ppm) | Mortality rate |
|---|---|---|
| Present condensed heterocyclic compound 109 + fluxapyroxad | 200+2000 | 100 |
| Present condensed heterocyclic compound 109 + fluxapyroxad | 500+0.5 | 100 |
| Present condensed heterocyclic compound 58 + fluopyram | 200+2000 | 100 |
| Present condensed heterocyclic compound 58 + fluopyram | 500+0.5 | 100 |
| Present condensed heterocyclic compound 58 + penflufen | 200+2000 | 100 |
| Present condensed heterocyclic compound 58 + penflufen | 500+500 | 100 |
| Present condensed heterocyclic compound 58 + penflufen | 500+0.5 | 100 |
| Non-treatment section | - | 0 |

Table 57

| Compounds provided for test | Concentration (ppm) | Mortality rate |
|---|---|---|
| Present condensed heterocyclic compound 58 + sedaxane | 200+2000 | 100 |
| Present condensed heterocyclic compound 58 + sedaxane | 500+0.5 | 100 |
| Present condensed heterocyclic compound 58 + fluxapyroxad | 200+2000 | 100 |
| Present condensed heterocyclic compound 58 + fluxapyroxad | 500+0.5 | 100 |
| Present condensed heterocyclic compound 64 + fluopyram | 200+2000 | 100 |
| Present condensed heterocyclic compound 64 + fluopyram | 500+0.5 | 100 |
| Present condensed heterocyclic compound 64 + penflufen | 200+2000 | 100 |
| Present condensed heterocyclic compound 64 + penflufen | 500+500 | 100 |
| Present condensed heterocyclic compound 64 + penflufen | 500+0.5 | 100 |
| Present condensed heterocyclic compound 64 + sedaxane | 200+2000 | 100 |
| Present condensed heterocyclic compound 64 + sedaxane | 500+0.5 | 100 |
| Present condensed heterocyclic compound 64 + fluxapyroxad | 200+2000 | 100 |
| Present condensed heterocyclic compound 64 + fluxapyroxad | 500+0.5 | 100 |
| Present condensed heterocyclic compound 118 + flutolanil | 200+5000 | 100 |
| Present condensed heterocyclic compound 118 + flutolanil | 500+500 | 100 |
| Present condensed heterocyclic compound 118 + fluopyram | 200+2000 | 100 |
| Present condensed heterocyclic compound 118 + fluopyram | 500+0.5 | 100 |
| Present condensed heterocyclic compound 118 + penflufen | 200+2000 | 100 |
| Present condensed heterocyclic compound 118 + penflufen | 500+500 | 100 |
| Present condensed heterocyclic compound 118 + penflufen | 500+0.5 | 100 |
| Present condensed heterocyclic compound 118 + sedaxane | 200+2000 | 100 |
| Present condensed heterocyclic compound 118 + sedaxane | 500+0.5 | 100 |
| Present condensed heterocyclic compound 118 + fluxapyroxad | 200+2000 | 100 |
| Present condensed heterocyclic compound 118 + fluxapyroxad | 500+0.5 | 100 |
| Present condensed heterocyclic compound 119 + flutolanil | 200+5000 | 100 |
| Present condensed heterocyclic compound 119 + flutolanil | 500+500 | 100 |

(continued)

| Compounds provided for test | Concentration (ppm) | Mortality rate |
|---|---|---|
| Present condensed heterocyclic compound 119 + fluopyram | 200+2000 | 100 |
| Present condensed heterocyclic compound 119 + fluopyram | 500+0.5 | 100 |
| Present condensed heterocyclic compound 119 + penflufen | 200+2000 | 100 |
| Present condensed heterocyclic compound 119 + penflufen | 500+500 | 100 |
| Present condensed heterocyclic compound 119 + penflufen | 500+0.5 | 100 |
| Present condensed heterocyclic compound 119 + sedaxane | 200+2000 | 100 |
| Present condensed heterocyclic compound 119 + sedaxane | 500+0.5 | 100 |
| Present condensed heterocyclic compound 119 + fluxapyroxad | 200+2000 | 100 |
| Present condensed heterocyclic compound 119 + fluxapyroxad | 500+0.5 | 100 |
| Present condensed heterocyclic compound 120 + fluopyram | 200+2000 | 100 |
| Present condensed heterocyclic compound 120 + fluopyram | 500+0.5 | 100 |
| Present condensed heterocyclic compound 120 + penflufen | 200+2000 | 100 |
| Present condensed heterocyclic compound 120 + penflufen | 500+500 | 100 |
| Present condensed heterocyclic compound 120 + penflufen | 500+0.5 | 100 |
| Non-treatment section | - | 0 |

Table 58

| Compounds provided for test | Concentration (ppm) | Mortality rate |
|---|---|---|
| Present condensed heterocyclic compound 120 + sedaxane | 200+2000 | 100 |
| Present condensed heterocyclic compound 120 + sedaxane | 500+0.5 | 100 |
| Present condensed heterocyclic compound 120 + fluxapyroxad | 200+2000 | 100 |
| Present condensed heterocyclic compound 120 + fluxapyroxad | 500+0.5 | 100 |
| Present condensed heterocyclic compound 127 + fluopyram | 200+2000 | 100 |
| Present condensed heterocyclic compound 127 + fluopyram | 500+0.5 | 100 |
| Present condensed heterocyclic compound 127 + penflufen | 200+2000 | 100 |
| Present condensed heterocyclic compound 127 + penflufen | 500+500 | 100 |
| Present condensed heterocyclic compound 127 + penflufen | 500+0.5 | 100 |
| Present condensed heterocyclic compound 127 + sedaxane | 200+2000 | 100 |
| Present condensed heterocyclic compound 127 + sedaxane | 500+0.5 | 100 |
| Present condensed heterocyclic compound 127 + fluxapyroxad | 200+2000 | 100 |
| Present condensed heterocyclic compound 127 + fluxapyroxad | 500+0.5 | 100 |
| Present condensed heterocyclic compound 3 + fluopyram | 200+2000 | 100 |
| Present condensed heterocyclic compound 3 + fluopyram | 500+0.5 | 100 |
| Present condensed heterocyclic compound 3 + penflufen | 200+2000 | 100 |
| Present condensed heterocyclic compound 3 + penflufen | 500+500 | 100 |
| Present condensed heterocyclic compound 3 + penflufen | 500+0.5 | 100 |
| Present condensed heterocyclic compound 3 + sedaxane | 200+2000 | 100 |

(continued)

| Compounds provided for test | Concentration (ppm) | Mortality rate |
|---|---|---|
| Present condensed heterocyclic compound 3 + sedaxane | 500+0.5 | 100 |
| Present condensed heterocyclic compound 3 + fluxapyroxad | 200+2000 | 100 |
| Present condensed heterocyclic compound 3 + fluxapyroxad | 500+0.5 | 100 |
| Present condensed heterocyclic compound 23 + fluopyram | 200+2000 | 100 |
| Present condensed heterocyclic compound 23 + fluopyram | 500+0.5 | 100 |
| Present condensed heterocyclic compound 23 + penflufen | 200+2000 | 100 |
| Present condensed heterocyclic compound 23 + penflufen | 500+500 | 100 |
| Present condensed heterocyclic compound 23 + penflufen | 500+0.5 | 100 |
| Present condensed heterocyclic compound 23 + sedaxane | 200+2000 | 100 |
| Present condensed heterocyclic compound 23 + sedaxane | 500+0.5 | 100 |
| Present condensed heterocyclic compound 23 + fluxapyroxad | 200+2000 | 100 |
| Present condensed heterocyclic compound 23 + fluxapyroxad | 500+0.5 | 100 |
| Present condensed heterocyclic compound 27 + fluopyram | 200+2000 | 100 |
| Present condensed heterocyclic compound 27 + fluopyram | 500+0.5 | 100 |
| Present condensed heterocyclic compound 27 + penflufen | 200+2000 | 100 |
| Present condensed heterocyclic compound 27 + penflufen | 500+500 | 100 |
| Present condensed heterocyclic compound 27 + penflufen | 500+0.5 | 100 |
| Present condensed heterocyclic compound 27 sedaxane | 200+2000 | 100 |
| Present condensed heterocyclic compound 27 sedaxane | 500+0.5 | 100 |
| Present condensed heterocyclic compound 27 + fluxapyroxad | 200+2000 | 100 |
| Present condensed heterocyclic compound 27 + fluxapyroxad | 500+0.5 | 100 |
| Non-treatment section | - | 0 |

Table 59

| Compounds provided for test | Concentration (ppm) | Mortality rate |
|---|---|---|
| Present condensed heterocyclic compound 31 + fluopyram | 200+2000 | 100 |
| Present condensed heterocyclic compound 31 + fluopyram | 500+0.5 | 100 |
| Present condensed heterocyclic compound 31 + penflufen | 200+2000 | 100 |
| Present condensed heterocyclic compound 31 + penflufen | 500+500 | 100 |
| Present condensed heterocyclic compound 31 + penflufen | 500+0.5 | 100 |
| Present condensed heterocyclic compound 31 + sedaxane | 200+2000 | 100 |
| Present condensed heterocyclic compound 31 + sedaxane | 500+0.5 | 100 |
| Present condensed heterocyclic compound 31 + fluxapyroxad | 200+2000 | 100 |
| Present condensed heterocyclic compound 31 + fluxapyroxad | 500+0.5 | 100 |
| Present condensed heterocyclic compound 69 + fluopyram | 200+2000 | 100 |
| Present condensed heterocyclic compound 69 + fluopyram | 500+0.5 | 100 |
| Present condensed heterocyclic compound 69 + penflufen | 200+2000 | 100 |

(continued)

| Compounds provided for test | Concentration (ppm) | Mortality rate |
|---|---|---|
| Present condensed heterocyclic compound 69 .+ penflufen | 500+500 | 100 |
| Present condensed heterocyclic compound 69 + penflufen | 500+0.5 | 100 |
| Present condensed heterocyclic compound 69 + sedaxane | 200+2000 | 100 |
| Present condensed heterocyclic compound 69 + sedaxane | 500+0.5 | 100 |
| Present condensed heterocyclic compound 69 + fluxapyroxad | 200+2000 | 100 |
| Present condensed heterocyclic compound 69 + fluxapyroxad | 500+0.5 | 100 |
| Present condensed heterocyclic compound 72 + fluopyram | 200+2000 | 100 |
| Present condensed heterocyclic compound 72 + fluopyram | 500+0.5 | 100 |
| Present condensed heterocyclic compound 72 + penflufen | 200+2000 | 100 |
| Present condensed heterocyclic compound 72 + penflufen | 500+500 | 100 |
| Present condensed heterocyclic compound 72 + penflufen | 500+0.5 | 100 |
| Present condensed heterocyclic compound 72 + sedaxane | 200+2000 | 100 |
| Present condensed heterocyclic compound 72 + sedaxane | 500+0.5 | 100 |
| Present condensed heterocyclic compound 72 + fluxapyroxad | 200+2000 | 100 |
| Present condensed heterocyclic compound 72 + fluxapyroxad | 500+0.5 | 100 |
| Present condensed heterocyclic compound 74 + fluopyram | 200+2000 | 100 |
| Present condensed heterocyclic compound 74 + fluopyram | 500+0.5 | 100 |
| Present condensed heterocyclic compound 74 + penflufen | 200+2000 | 100 |
| Present condensed heterocyclic compound 74 + penflufen | 500+500 | 100 |
| Present condensed heterocyclic compound 74 + penflufen | 500-0.5 | 100 |
| Present condensed heterocyclic compound 74 + sedaxane | 200+2000 | 100 |
| Present condensed heterocyclic compound 74 + sedaxane | 500+0.5 | 100 |
| Present condensed heterocyclic compound 74 + fluxapyroxad | 200+2000 | 100 |
| Present condensed heterocyclic compound 74 + fluxapyroxad | 500+0.5 | 100 |
| Present condensed heterocyclic compound 78 + fluopyram | 200+2000 | 100 |
| Present condensed heterocyclic compound 78 + fluopyram | 500+0.5 | 100 |
| Non-treatment section | - | 0 |

Table 60

| Compounds provided for test | Concentration (ppm) | Mortality rate |
|---|---|---|
| Present condensed heterocyclic compound 78 + penflufen | 200+2000 | 100 |
| Present condensed heterocyclic compound 78 + penflufen | 500+500 | 100 |
| Present condensed heterocyclic compound 78 + penflufen | 500+0.5 | 100 |
| Present condensed heterocyclic compound 78 + sedaxane | 200+2000 | 100 |
| Present condensed heterocyclic compound 78 + sedaxane | 500+0.5 | 100 |
| Present condensed heterocyclic compound 78 + fluxapyroxad | 200+2000 | 100 |
| Present condensed heterocyclic compound 78 + fluxapyroxad | 500+0.5 | 100 |

(continued)

| Compounds provided for test | Concentration (ppm) | Mortality rate |
|---|---|---|
| Present condensed heterocyclic compound 39 + fluopyram | 200+2000 | 100 |
| Present condensed heterocyclic compound 39 + fluopyram | 500+0.5 | 100 |
| Present condensed heterocyclic compound 39 + penflufen | 200+2000 | 100 |
| Present condensed heterocyclic compound 39 + penflufen | 500+500 | 100 |
| Present condensed heterocyclic compound 39 + penflufen | 500+0.5 | 100 |
| Present condensed heterocyclic compound 39 + sedaxane | 200+2000 | 100 |
| Present condensed heterocyclic compound 39 + sedaxane | 500+0.5 | 100 |
| Present condensed heterocyclic compound 39 + fluxapyroxad | 200+2000 | 100 |
| Present condensed heterocyclic compound 39 + fluxapyroxad | 500+0.5 | 100 |
| Present condensed heterocyclic compound 41 + fluopyram | 200+2000 | 100 |
| Present condensed heterocyclic compound 41 + fluopyram | 500+0.5 | 100 |
| Present condensed heterocyclic compound 41 + penflufen | 200+2000 | 100 |
| Present condensed heterocyclic compound 41 + penflufen | 500+500 | 100 |
| Present condensed heterocyclic compound 41 + penflufen | 500+0.5 | 100 |
| Present condensed heterocyclic compound 41 + sedaxane | 200+2000 | 100 |
| Present condensed heterocyclic compound 41 + sedaxane | 500+0.5 | 100 |
| Present condensed heterocyclic compound 41 + fluxapyroxad | 200+2000 | 100 |
| Present condensed heterocyclic compound 41 + fluxapyroxad | 500+0.5 | 100 |
| Present condensed heterocyclic compound 42 + fluopyram | 200+2000 | 100 |
| Present condensed heterocyclic compound 42 + fluopyram | 500+0.5 | 100 |
| Present condensed heterocyclic compound 42 + penflufen | 200+2000 | 100 |
| Present condensed heterocyclic compound 42 + penflufen | 500+500 | 100 |
| Present condensed heterocyclic compound 42 + penflufen | 500+0.5 | 100 |
| Present condensed heterocyclic compound 42 + sedaxane | 200+2000 | 100 |
| Present condensed heterocyclic compound 42 + sedaxane | 500+0.5 | 100 |
| Present condensed heterocyclic compound 42 + fluxapyroxad | 200+2000 | 100 |
| Present condensed heterocyclic compound 42 + fluxapyroxad | 500+0.5 | 100 |
| Present condensed heterocyclic compound 46 + fluopyram | 200+2000 | 100 |
| Present condensed heterocyclic compound 46 + fluopyram | 500+0.5 | 100 |
| Present condensed heterocyclic compound 46 + penflufen | 200+2000 | 100 |
| Present condensed heterocyclic compound 46 + penflufen | 500+500 | 100 |
| Present condensed heterocyclic compound 46 + penflufen | 500+0.5 | 100 |
| Non-treatment section | - | 0 |

Table 61

| Compounds provided for test | Concentration (ppm) | Mortality rate |
|---|---|---|
| Present condensed heterocyclic compound 46 + sedaxane | 200+2000 | 100 |

(continued)

| Compounds provided for test | Concentration (ppm) | Mortality rate |
|---|---|---|
| Present condensed heterocyclic compound 46 + sedaxane | 500+0.5 | 100 |
| Present condensed heterocyclic compound 46 + fluxapyroxad | 200+2000 | 100 |
| Present condensed heterocyclic compound 46 + fluxapyroxad | 500+0.5 | 100 |
| Present condensed heterocyclic compound 56 + fluopyram | 200+2000 | 100 |
| Present condensed heterocyclic compound 56 + fluopyram | 500+0.5 | 100 |
| Present condensed heterocyclic compound 56 + penflufen | 200+2000 | 100 |
| Present condensed heterocyclic compound 56 + penflufen | 500+500 | 100 |
| Present condensed heterocyclic compound 56 + penflufen | 500+0.5 | 100 |
| Present condensed heterocyclic compound 56 + sedaxane | 200+2000 | 100 |
| Present condensed heterocyclic compound 56 + sedaxane | 500+0.5 | 100 |
| Present condensed heterocyclic compound 56 + fluxapyroxad | 200+2000 | 100 |
| Present condensed heterocyclic compound 56 + fluxapyroxad | 500+0.5 | 100 |
| Present condensed heterocyclic compound 83 + fluopyram | 200+2000 | 100 |
| Present condensed heterocyclic compound 83 + fluopyram | 500+0.5 | 100 |
| Present condensed heterocyclic compound 83 + penflufen | 200+2000 | 100 |
| Present condensed heterocyclic compound 83 + penflufen | 500+500 | 100 |
| Present condensed heterocyclic compound 83 + penflufen | 500+0.5 | 100 |
| Present condensed heterocyclic compound 83 + sedaxane | 200+2000 | 100 |
| Present condensed heterocyclic compound 83 + sedaxane | 500+0.5 | 100 |
| Present condensed heterocyclic compound 83 + fluxapyroxad | 200+2000 | 100 |
| Present condensed heterocyclic compound 83 + fluxapyroxad | 500+0.5 | 100 |
| Present condensed heterocyclic compound 48 + fluopyram | 200+2000 | 100 |
| Present condensed heterocyclic compound 48 + fluopyram | 500+0.5 | 100 |
| Present condensed heterocyclic compound 48 + penflufen | 200+2000 | 100 |
| Present condensed heterocyclic compound 48 + penflufen | 500+500 | 100 |
| Present condensed heterocyclic compound 48 + penflufen | 500+0.5 | 100 |
| Present condensed heterocyclic compound 48 + sedaxane | 200+2000 | 100 |
| Present condensed heterocyclic compound 48 + sedaxane | 500+0.5 | 100 |
| Present condensed heterocyclic compound 48 + fluxapyroxad | 200+2000 | 100 |
| Present condensed heterocyclic compound 48 + fluxapyroxad | 500+0.5 | 100 |
| Present condensed heterocyclic compound 91 + fluopyram | 200+2000 | 100 |
| Present condensed heterocyclic compound 91 + fluopyram | 500+0.5 | 100 |
| Present condensed heterocyclic compound 91 + penflufen | 200+2000 | 100 |
| Present condensed heterocyclic compound 91 + penflufen | 500+500 | 100 |
| Present condensed heterocyclic compound 91 + penflufen | 500+0.5 | 100 |
| Present condensed heterocyclic compound 91 + sedaxane | 200+2000 | 100 |
| Present condensed heterocyclic compound 91 + sedaxane | 500+0.5 | 100 |
| Present condensed heterocyclic compound 91 + fluxapyroxad | 200+2000 | 100 |

(continued)

| Compounds provided for test | Concentration (ppm) | Mortality rate |
|---|---|---|
| Present condensed heterocyclic compound 91 + fluxapyroxad | 500+0.5 | 100 |
| Non-treatment section | - | 0 |

Table 62

| Compounds provided for test | Concentration (ppm) | Mortality rate |
|---|---|---|
| Present condensed heterocyclic compound 51 + fluopyram | 200+2000 | 100 |
| Present condensed heterocyclic compound 51 + fluopyram | 500+0.5 | 100 |
| Present condensed heterocyclic compound 51 + penflufen | 200+2000 | 100 |
| Present condensed heterocyclic compound 51 + penflufen | 500+500 | 100 |
| Present condensed heterocyclic compound 51 + penflufen | 500+0.5 | 100 |
| Present condensed heterocyclic compound 51 + sedaxane | 200+2000 | 100 |
| Present condensed heterocyclic compound 51 + sedaxane | 500+0.5 | 100 |
| Present condensed heterocyclic compound 51 + fluxapyroxad | 200+2000 | 100 |
| Present condensed heterocyclic compound 51 + fluxapyroxad | 500+0.5 | 100 |
| Present condensed heterocyclic compound 130 + flutolanil | 200+5000 | 100 |
| Present condensed heterocyclic compound 130 + flutolanil | 500+500 | 100 |
| Present condensed heterocyclic compound 130 + fluopyram | 200+2000 | 100 |
| Present condensed heterocyclic compound 130 + fluopyram | 500+0.5 | 100 |
| Present condensed heterocyclic compound 130 + penflufen | 200+2000 | 100 |
| Present condensed heterocyclic compound 130 + penflufen | 500+500 | 100 |
| Present condensed heterocyclic compound 130 + penflufen | 500+0.5 | 100 |
| Present condensed heterocyclic compound 130 + sedaxane | 200+2000 | 100 |
| Present condensed heterocyclic compound 130 + sedaxane | 500+0.5 | 100 |
| Present condensed heterocyclic compound 130 + fluxapyroxad | 200+2000 | 100 |
| Present condensed heterocyclic compound 130 + fluxapyroxad | 500+0.5 | 100 |
| Present condensed heterocyclic compound 131 + flutolanil | 200+5000 | 100 |
| Present condensed heterocyclic compound 131 + flutolanil | 500+500 | 100 |
| Present condensed heterocyclic compound 131 + fluopyram | 200+2000 | 100 |
| Present condensed heterocyclic compound 131 + fluopyram | 500+0.5 | 100 |
| Present condensed heterocyclic compound 131 + penflufen | 200+2000 | 100 |
| Present condensed heterocyclic compound 131 + penflufen | 500+500 | 100 |
| Present condensed heterocyclic compound 131 + penflufen | 500+0.5 | 100 |
| Present condensed heterocyclic compound 131 + sedaxane | 200+2000 | 100 |
| Present condensed heterocyclic compound 131 + sedaxane | 500+0.5 | 100 |
| Present condensed heterocyclic compound 131 + fluxapyroxad | 200+2000 | 100 |
| Present condensed heterocyclic compound 131 + fluxapyroxad | 500+0.5 | 100 |
| Non-treatment section | - | 0 |

Test Example 7

1 mg of each of the present condensed heterocyclic compounds 3, 23, 27, 31, 39, 41, 42, 46, 48, 51, 56, 58, 64, 69, 72, 74, 78, 83, 91, 97, 104, 105, 107, 108, 109, 118, 119, 120, 127, 130, and 131 was dissolved in 10 μL of each mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

Each of commercial formulations, such as fludioxonil (trade name: GEOXE, Syngenta Japan K.K.), tolclofos-methyl (trade name: Rizolex water dispersible powder, manufactured by Sumitomo Chemical Co., Ltd.), and captan (trade name: Orthocide water dispersible powder, manufactured by Nissan Chemical Industries, Ltd.), was diluted with water which contains 0.02 volume% of a spreading agent (trade name: Shindain, manufactured by Sumitomo Chemical Co., Ltd.) so as to have a predetermined concentration.

1 mg of ethaboxam was dissolved in 10 μL of a mixed solvent of xylene, dimethylformamide, and a surfactant (trade name: Sorpol 3005X, manufactured by Toho Chemical Industry Co., Ltd.) at a volume ratio of 4:4:1, and then, the mixture was diluted with water which contains 0.02 volume% of a spreading agent (trade name: Shindain, manufactured by Sumitomo Chemical Co., Ltd.) so as to have a predetermined concentration.

A water-diluted solution of the above-described present condensed heterocyclic compounds and a water-diluted solution of fludioxonil, tolclofos-methyl, captan, or ethaboxam were mixed together to prepare a liquid medicine for a test.

Each leaf disc (diameter of 1.5 cm) of a cabbage (Brassicae oleracea) was accommodated in each well of a 24 hole microplate (manufactured by Becton Dickinson) and 40 μL of liquid medicine for a test was scattered to each well. Wells to which 40 μL of water containing 0.02 volume% of a spreading agent (trade name: Shindain, manufactured by Sumitomo Chemical Co., Ltd.) was scattered was set to a non-treatment section.

After air-drying, 5 second-instar larvae of diamondback moths (Plutella xylostella) were released in each well, which was then covered with paper towel and closed with a lid. 2 days after the releasing, the number of surviving larvae in each well was observed.

The mortality rates of the treatment section and the non-treatment section were calculated using the following Formula 1). The test was repeated once.

Formula 1) Mortality rate (%) = (the number of larvae provided for a test - the number of surviving larvae) / the number of larvae provided for a test x 100

Table 63

| Compounds provided for test | Concentration (ppm) | Mortality rate |
|---|---|---|
| Present condensed heterocyclic compound 3 + fludioxonil | 200+2000 | 100 |
| Present condensed heterocyclic compound 3 + fludioxonil | 500+50 | 100 |
| Present condensed heterocyclic compound 3 + fludioxonil | 500+5 | 100 |
| Present condensed heterocyclic compound 3 + ethaboxam | 200+2000 | 100 |
| Present condensed heterocyclic compound 3 + ethaboxam | 500+5 | 100 |
| Present condensed heterocyclic compound 3 + tolclofos-methyl | 200+2000 | 100 |
| Present condensed heterocyclic compound 3 + tolclofos-methyl | 500+5 | 100 |
| Present condensed heterocyclic compound 3 + captan | 200+2000 | 100 |
| Present condensed heterocyclic compound 3 + captan | 500+5 | 100 |
| Present condensed heterocyclic compound 39 + fludioxonil | 200+2000 | 100 |
| Present condensed heterocyclic compound 39 + fludioxonil | 500+50 | 100 |
| Present condensed heterocyclic compound 39 + fludioxonil | 500+5 | 100 |

(continued)

| Compounds provided for test | Concentration (ppm) | Mortality rate |
|---|---|---|
| Present condensed heterocyclic compound 39 + ethaboxam | 200+2000 | 100 |
| Present condensed heterocyclic compound 39 + ethaboxam | 500+5 | 100 |
| Present condensed heterocyclic compound 39 + tolclofos-methyl | 200+2000 | 100 |
| Present condensed heterocyclic compound 39 + tolclofos-methyl | 500+5 | 100 |
| Present condensed heterocyclic compound 39 + captan | 200+2000 | 100 |
| Present condensed heterocyclic compound 39 + captan | 500+5 | 100 |
| Present condensed heterocyclic compound 23 + fludioxonil | 200+2000 | 100 |
| Present condensed heterocyclic compound 23 + fludioxonil | 500+50 | 100 |
| Present condensed heterocyclic compound 23 + fludioxonil | 500+5 | 100 |
| Present condensed heterocyclic compound 23 + ethaboxam | 200+2000 | 100 |
| Present condensed heterocyclic compound 23 + ethaboxam | 500+5 | 100 |
| Present condensed heterocyclic compound 23 + tolclofos-methyl | 200+2000 | 100 |
| Present condensed heterocyclic compound 23 + tolclofos-methyl | 500+5 | 100 |
| Present condensed heterocyclic compound 23 + captan | 200+2000 | 100 |
| Present condensed heterocyclic compound 23 + captan | 500+5 | 100 |
| Present condensed heterocyclic compound 74 + fludioxonil | 200+2000 | 100 |
| Present condensed heterocyclic compound 74 + fludioxonil | 500+50 | 100 |
| Present condensed heterocyclic compound 74 + fludioxonil | 500+5 | 100 |
| Present condensed heterocyclic compound 74 + ethaboxam | 200+2000 | 100 |
| Present condensed heterocyclic compound 74 + ethaboxam | 500+5 | 100 |
| Present condensed heterocyclic compound 74 + tolclofos-methyl | 200+2000 | 100 |
| Present condensed heterocyclic compound 74 + tolclofos-methyl | 500+5 | 100 |
| Present condensed heterocyclic compound 74 + captan | 200+2000 | 100 |
| Present condensed heterocyclic compound 74 + captan | 500+5 | 100 |
| Present condensed heterocyclic compound 83 + fludioxonil | 200+2000 | 100 |
| Present condensed heterocyclic compound 83 + fludioxonil | 500+50 | 100 |
| Present condensed heterocyclic compound 83 + fludioxonil | 500+5 | 100 |
| Non-treatment section | - | 0 |

Table 64

| Compounds provided for test | Concentration (ppm) | Mortality rate |
|---|---|---|
| Present condensed heterocyclic compound 83 + ethaboxam | 200+2000 | 100 |
| Present condensed heterocyclic compound 83 + ethaboxam | 500+5 | 100 |
| Present condensed heterocyclic compound 83 + tolclofos-methyl | 200+2000 | 100 |
| Present condensed heterocyclic compound 83 + tolclofos-methyl | 500+5 | 100 |
| Present condensed heterocyclic compound 83 + captan | 200+2000 | 100 |
| Present condensed heterocyclic compound 83 + captan | 500+5 | 100 |

(continued)

| Compounds provided for test | Concentration (ppm) | Mortality rate |
|---|---|---|
| Present condensed heterocyclic compound 58 + fludioxonil | 200+2000 | 100 |
| Present condensed heterocyclic compound 58 + fludioxonil | 500+50 | 100 |
| Present condensed heterocyclic compound 58 + fludioxonil | 500+5 | 100 |
| Present condensed heterocyclic compound 58 + ethaboxam | 200+2000 | 100 |
| Present condensed heterocyclic compound 58 + ethaboxam | 500+5 | 100 |
| Present condensed heterocyclic compound 58 + tolclofos-methyl | 200+2000 | 100 |
| Present condensed heterocyclic compound 58 + tolclofos-methyl | 500+5 | 100 |
| Present condensed heterocyclic compound 58 + captan | 200+2000 | 100 |
| Present condensed heterocyclic compound 58 + captan | 500+5 | 100 |
| Present condensed heterocyclic compound 31 + fludioxonil | 200+2000 | 100 |
| Present condensed heterocyclic compound 31 + fludioxonil | 500+50 | 100 |
| Present condensed heterocyclic compound 31 + fludioxonil | 500+5 | 100 |
| Present condensed heterocyclic compound 31 + ethaboxam | 200+2000 | 100 |
| Present condensed heterocyclic compound 31 + ethaboxam | 500+5 | 100 |
| Present condensed heterocyclic compound 31 + tolclofos-methyl | 200+2000 | 100 |
| Present condensed heterocyclic compound 31 + tolclofos-methyl | 500+5 | 100 |
| Present condensed heterocyclic compound 31 + captan | 200+2000 | 100 |
| Present condensed heterocyclic compound 31 + captan | 500+5 | 100 |
| Present condensed heterocyclic compound 56 + fludioxonil | 200+2000 | 100 |
| Present condensed heterocyclic compound 56 + fludioxonil | 500+50 | 100 |
| Present condensed heterocyclic compound 56 + fludioxonil | 500+5 | 100 |
| Present condensed heterocyclic compound 56 + ethaboxam | 200+2000 | 100 |
| Present condensed heterocyclic compound 56 + ethaboxam | 500+5 | 100 |
| Present condensed heterocyclic compound 56 + tolclofos-methyl | 200+2000 | 100 |
| Present condensed heterocyclic compound 56 + tolclofos-methyl | 500+5 | 100 |
| Present condensed heterocyclic compound 56 + captan | 200+2000 | 100 |
| Present condensed heterocyclic compound 56 + captan | 500+5 | 100 |
| Present condensed heterocyclic compound 46 + fludioxonil | 200+2000 | 100 |
| Present condensed heterocyclic compound 46 + fludioxonil | 500+50 | 100 |
| Present condensed heterocyclic compound 46 + fludioxonil | 500+5 | 100 |
| Present condensed heterocyclic compound 46 + ethaboxam | 200+2000 | 100 |
| Present condensed heterocyclic compound 46 + ethaboxam | 500+5 | 100 |
| Present condensed heterocyclic compound 46 + tolclofos-methyl | 200+2000 | 100 |
| Present condensed heterocyclic compound 46 + tolclofos-methyl | 500+5 | 100 |
| Non-treatment section | - | 0 |

Table 65

| Compounds provided for test | Concentration (ppm) | Mortality rate |
|---|---|---|
| Present condensed heterocyclic compound 46 + captan | 200+2000 | 100 |
| Present condensed heterocyclic compound 46 + captan | 500+5 | 100 |
| Present condensed heterocyclic compound 105 + fludioxonil | 200+2000 | 100 |
| Present condensed heterocyclic compound 105 + fludioxonil | 500+50 | 100 |
| Present condensed heterocyclic compound 105 + fludioxonil | 500+5 | 100 |
| Present condensed heterocyclic compound 105 + ethabcxam | 200+2000 | 100 |
| Present condensed heterocyclic compound 105 + ethaboxam | 500+5 | 100 |
| Present condensed heterocyclic compound 105 + tolclofos-methyl | 200+2000 | 100 |
| Present condensed heterocyclic compound 105 + tolclofos-methyl | 500+5 | 100 |
| Present condensed heterocyclic compound 105 + captan | 200+2000 | 100 |
| Present condensed heterocyclic compound 105 + captan | 500+5 | 100 |
| Present condensed heterocyclic compound 91 + fludioxonil | 200+2000 | 100 |
| Present condensed heterocyclic compound 91 + fludioxonil | 500+50 | 100 |
| Present condensed heterocyclic compound 91 + fludioxonil | 500+5 | 100 |
| Present condensed heterocyclic compound 91 + ethaboxam | 200+2000 | 100 |
| Present condensed heterocyclic compound 91 + ethaboxam | 500+5 | 100 |
| Present condensed heterocyclic compound 91 + tolclofos-methyl | 200+2000 | 100 |
| Present condensed heterocyclic compound 91 + tolclofos-methyl | 500+5 | 100 |
| Present condensed heterocyclic compound 91 + captan | 200+2000 | 100 |
| Present condensed heterocyclic compound 91 + captan | 500+5 | 100 |
| Present condensed heterocyclic compound 107 + fludioxonil | 200+2000 | 100 |
| Present condensed heterocyclic compound 107 + fludioxonil | 500+50 | 100 |
| Present condensed heterocyclic compound 107 + fludioxonil | 500+5 | 100 |
| Present condensed heterocyclic compound 107 + ethaboxam | 200+2000 | 100 |
| Present condensed heterocyclic compound 107 + ethaboxam | 500+5 | 100 |
| Present condensed heterocyclic compound 107 + tolclofos-methyl | 200+2000 | 100 |
| Present condensed heterocyclic compound 107 + tolclofos-methyl | 500+5 | 100 |
| Present condensed heterocyclic compound 107 + captan | 200+2000 | 100 |
| Present condensed heterocyclic compound 107 + captan | 500+5 | 100 |
| Present condensed heterocyclic compound 51 + fludioxonil | 200+2000 | 100 |
| Present condensed heterocyclic compound 51 + fludioxonil | 500+50 | 100 |
| Present condensed heterocyclic compound 51 + fludioxonil | 500+5 | 100 |
| Present condensed heterocyclic compound 51 + ethaboxam | 200+2000 | 100 |
| Present condensed heterocyclic compound 51 + ethabcxam | 500+5 | 100 |
| Present condensed heterocyclic compound 51 + tolclofos-methyl | 200+2000 | 100 |
| Present condensed heterocyclic compound 51 + tolclofos-methyl | 500+5 | 100 |
| Present condensed heterocyclic compound 51 + captan | 200+2000 | 100 |
| Present condensed heterocyclic compound 51 + captan | 500+5 | 100 |

(continued)

| Compounds provided for test | Concentration (ppm) | Mortality rate |
|---|---|---|
| Present condensed heterocyclic compound 27 + fludioxonil | 200+2000 | 100 |
| Present condensed heterocyclic compound 27 + fludioxonil | 500+50 | 100 |
| Present condensed heterocyclic compound 27 + fludioxonil | 500+5 | 100 |
| Non-treatment section | - | 0 |

Table 66

| Compounds provided for test | Concentration (ppm) | Mortality rate |
|---|---|---|
| Present condensed heterocyclic compound 27 + ethaboxam | 200+2000 | 100 |
| Present condensed heterocyclic compound 27 + ethaboxam | 500+5 | 100 |
| Present condensed heterocyclic compound 27 + tolclofos-methyl | 200+2000 | 100 |
| Present condensed heterocyclic compound 27 + tolclofos-methyl | 500+5 | 100 |
| Present condensed heterocyclic compound 27 + captan | 200+2000 | 100 |
| Present condensed heterocyclic compound 27 + captan | 500+5 | 100 |
| Present condensed heterocyclic compound 108 + fludioxonil | 200+2000 | 100 |
| Present condensed heterocyclic compound 108 + fludioxonil | 500+50 | 100 |
| Present condensed heterocyclic compound 108 + fludioxonil | 500+5 | 100 |
| Present condensed heterocyclic compound 108 + ethaboxam | 200+2000 | 100 |
| Present condensed heterocyclic compound 108 + ethaboxam | 500+5 | 100 |
| Present condensed heterocyclic compound 108 + tolclofos-methyl | 200+2000 | 100 |
| Present condensed heterocyclic compound 108 + tolclofos-methyl | 500+5 | 100 |
| Present condensed heterocyclic compound 108 + captan | 200+2000 | 100 |
| Present condensed heterocyclic compound 108 + captan | 500+5 | 100 |
| Present condensed heterocyclic compound 48 + fludioxonil | 200+2000 | 100 |
| Present condensed heterocyclic compound 48 + fludioxonil | 500+50 | 100 |
| Present condensed heterocyclic compound 48 + fludioxonil | 500+5 | 100 |
| Present condensed heterocyclic compound 48 + ethaboxam | 200+2000 | 100 |
| Present condensed heterocyclic compound 48 + ethaboxam | 500+5 | 100 |
| Present condensed heterocyclic compound 48 + tolclofos-methyl | 200+2000 | 100 |
| Present condensed heterocyclic compound 48 + tolclofos-methyl | 500+5 | 100 |
| Present condensed heterocyclic compound 48 + captan | 200+2000 | 100 |
| Present condensed heterocyclic compound 48 + captan | 500+5 | 100 |
| Present condensed heterocyclic compound 97 + fludioxonil | 200+2000 | 100 |
| Present condensed heterocyclic compound 97 + fludioxonil | 500+50 | 100 |
| Present condensed heterocyclic compound 97 + fludioxonil | 500+5 | 100 |
| Present condensed heterocyclic compound 97 + ethaboxam | 200+2000 | 100 |
| Present condensed heterocyclic compound 97 + ethaboxam | 500+5 | 100 |
| Present condensed heterocyclic compound 97 + tolclofos-methyl | 200+2000 | 100 |

(continued)

| Compounds provided for test | Concentration (ppm) | Mortality rate |
|---|---|---|
| Present condensed heterocyclic compound 97 + tolclofos-methyl | 500+5 | 100 |
| Present condensed heterocyclic compound 97 + captan | 200+2000 | 100 |
| Present condensed heterocyclic compound 97 + captan | 500+5 | 100 |
| Present condensed heterocyclic compound 69 + fludioxonil | 200+2000 | 100 |
| Present condensed heterocyclic compound 69 + fludioxonil | 500+50 | 100 |
| Present condensed heterocyclic compound 69 + fludioxonil | 500+5 | 100 |
| Present condensed heterocyclic compound 69 + ethaboxam | 200+2000 | 100 |
| Present oondensed heterocyclic compound 69 + ethaboxam | 500+5 | 100 |
| Present condensed heterocyclic compound 69 + tolclofos-methyl | 200+2000 | 100 |
| Present condensed heterocyclic compound 69 + tolclofos-methyl | 500+5 | 100 |
| Non-treatment section | - | 0 |

Table 67

| Compounds provided for test | Concentration (ppm) | Mortality rate |
|---|---|---|
| Present condensed heterocyclic compound 69 + captan | 200+2000 | 100 |
| Present condensed heterocyclic compound 69 + captan | 500+5 | 100 |
| Present condensed heterocyclic compound 109 + fludioxonil | 200+2000 | 100 |
| Present condensed heterocyclic compound 109 + fludioxonil | 500+50 | 100 |
| Present condensed heterocyclic compound 109 + fludioxonil | 500+5 | 100 |
| Present condensed heterocyclic compound 109 + ethaboxam | 200+2000 | 100 |
| Present condensed heterocyclic compound 109 + ethabcxam | 500+5 | 100 |
| Present condensed heterocyclic compound 109 + tolclofos-methyl | 200+2000 | 100 |
| Present condensed heterocyclic compound 109 + tolclofos-methyl | 500+5 | 100 |
| Present condensed heterocyclic compound 109 + captan | 200+2000 | 100 |
| Present condensed heterocyclic compound 109 + captan | 500+5 | 100 |
| Present condensed heterocyclic compound 72 + fludioxonil | 200+2000 | 100 |
| Present condensed heterocyclic compound 72 + fludioxonil | 500+50 | 100 |
| Present condensed heterocyclic compound 72 + fludioxonil | 500+5 | 100 |
| Present condensed heterocyclic compound 72 + ethaboxam | 200+2000 | 100 |
| Present condensed heterocyclic compound 72 + ethaboxam | 500+5 | 100 |
| Present condensed heterocyclic compound 72 + tolclofos-methyl | 200+2000 | 100 |
| Present condensed heterocyclic compound 72 + tolclofos-methyl | 500+5 | 100 |
| Present condensed heterocyclic compound 72 + captan | 200+2000 | 100 |
| Present condensed heterocyclic compound 72 + captan | 500+5 | 100 |
| Present condensed heterocyclic compound 78 + fludioxonil | 200+2000 | 100 |
| Present condensed heterocyclic compound 78 + fludioxonil | 500+50 | 100 |
| Present condensed heterocyclic compound 78 + fludioxonil | 500+5 | 100 |

(continued)

| Compounds provided for test | Concentration (ppm) | Mortality rate |
|---|---|---|
| Present condensed heterocyclic compound 78 + ethaboxam | 200+2000 | 100 |
| Present condensed heterocyclic compound 78 + ethaboxam | 500+5 | 100 |
| Present condensed heterocyclic compound 78 + tolclofos-methyl | 200+2000 | 100 |
| Present condensed heterocyclic compound 78 + tolclofos-methyl | 500+5 | 100 |
| Present condensed heterocyclic compound 78 + captan | 200+2000 | 100 |
| Present condensed heterocyclic compound 78 + captan | 500+5 | 100 |
| Present condensed heterocyclic compound 64 + fludioxonil | 200+2000 | 100 |
| Present condensed heterocyclic compound 64 + fludioxonil | 500+50 | 100 |
| Present condensed heterocyclic compound 64 + fludioxonil | 500+5 | 100 |
| Present condensed heterocyclic compound 64 + ethaboxam | 200+2000 | 100 |
| Present condensed heterocyclic compound 64 + ethaboxam | 500+5 | 100 |
| Present condensed heterocyclic compound 64 + tolclofos-methyl | 200+2000 | 100 |
| Present condensed heterocyclic compound 64 + tolclofos-methyl | 500+5 | 100 |
| Present condensed heterocyclic compound 64 + captan | 200+2000 | 100 |
| Present condensed heterocyclic compound 64 + captan | 500+5 | 100 |
| Present condensed heterocyclic compound 120 + fludioxonil | 200+2000 | 100 |
| Present condensed heterocyclic compound 120 + fludioxonil | 500+50 | 100 |
| Present condensed heterocyclic compound 120 + fludioxonil | 500+5 | 100 |
| Non-treatment section | - | 0 |

Table 68

| Compounds provided for test | Concentration (ppm) | Mortality rate |
|---|---|---|
| Present condensed heterocyclic compound 120 + ethaboxam | 200+2000 | 100 |
| Present condensed heterocyclic compound 120 + ethaboxam | 500+5 | 100 |
| Present condensed heterocyclic compound 120 + tolclofos-methyl | 200+2000 | 100 |
| Present condensed heterocyclic compound 120 + tolclofos-methyl | 500+5 | 100 |
| Present condensed heterocyclic compound 120 + captan | 200+2000 | 100 |
| Present condensed heterocyclic compound 120 + captan | 500+5 | 100 |
| Present condensed heterocyclic compound 127 + fludioxonil | 200+2000 | 100 |
| Present condensed heterocyclic compound 127 + fludioxonil | 500+50 | 100 |
| Present condensed heterocyclic compound 127 + fludioxonil | 500+5 | 100 |
| Present condensed heterocyclic compound 127 + ethaboxam | 200+2000 | 100 |
| Present condensed heterocyclic compound 127 + ethaboxam | 500+5 | 100 |
| Present condensed heterocyclic compound 127 + tolclofos-methyl | 200+2000 | 100 |
| Present condensed heterocyclic compound 127 + tolclofos-methyl | 500+5 | 100 |
| Present condensed heterocyclic compound 127 + captan | 200+2000 | 100 |
| Present condensed heterocyclic compound 127 + captan | 500+5 | 100 |

(continued)

| Compounds provided for test | Concentration (ppm) | Mortality rate |
|---|---|---|
| Present condensed heterocyclic compound 42 + fludioxonil | 200+2000 | 100 |
| Present condensed heterocyclic compound 42 + fludioxonil | 500+50 | 100 |
| Present condensed heterocyclic compound 42 + fludioxonil | 500+5 | 100 |
| Present condensed heterocyclic compound 42 + ethaboxam | 200+2000 | 100 |
| Present condensed heterocyclic compound 42 + ethaboxam | 500+5 | 100 |
| Present condensed heterocyclic compound 42 + tolclofos-methyl | 200+2000 | 100 |
| Present condensed heterocyclic compound 42 + tolclofos-methyl | 500+5 | 100 |
| Present condensed heterocyclic compound 42 + captan | 200+2000 | 100 |
| Present condensed heterocyclic compound 42 + captan | 500+5 | 100 |
| Present condensed heterocyclic compound 41 + fludioxonil | 200+2000 | 100 |
| Present condensed heterocyclic compound 41 + fludioxonil | 500+50 | 100 |
| Present condensed heterocyclic compound 41 + fludioxonil | 500+5 | 100 |
| Present condensed heterocyclic compound 41 + ethaboxam | 200+2000 | 100 |
| Present condensed heterocyclic compound 41 + ethaboxam | 500+5 | 100 |
| Present condensed heterocyclic compound 41 + tolclocfos-methyl | 200+2000 | 100 |
| Present condensed heterocyclic compound 41 + tolclofos-methyl | 500+5 | 100 |
| Present condensed heterocyclic compound 41 + captan | 200+2000 | 100 |
| Present condensed heterocyclic compound 41 + captan | 500+5 | 100 |
| Present condensed heterocyclic compound 104 + fludioxonil | 200+2000 | 100 |
| Present condensed heterocyclic compound 104 + fludioxonil | 500+50 | 100 |
| Present condensed heterocyclic compound 104 + fludioxonil | 500+5 | 100 |
| Present condensed heterocyclic compound 104 + ethaboxam | 200+2000 | 100 |
| Present condensed heterocyclic compound 104 + ethaboxam | 500+5 | 100 |
| Present condensed heterocyclic compound 104 + tolclofos-methyl | 200+2000 | 100 |
| Present condensed heterocyclic compound 104 + tolclofos-methyl | 500+5 | 100 |
| Non-treatment section | - | 0 |

Table 69

| Compounds provided for test | Concentration (ppm) | Mortality rate |
|---|---|---|
| Present condensed heterocyclic compound 104 + captan | 200+2000 | 100 |
| Present condensed heterocyclic compound 104 + captan | 500+5 | 100 |
| Present condensed heterocyclic compound 118 + fludioxonil | 200+2000 | 100 |
| Present condensed heterocyclic compound 118 + fludioxonil | 500+50 | 100 |
| Present condensed heterocyclic compound 118 + fludioxonil | 500+5 | 100 |
| Present condensed heterocyclic compound 118 + ethaboxam | 200+2000 | 100 |
| Present condensed heterocyclic compound 118 + ethaboxam | 500+5 | 100 |
| Present condensed heterocyclic compound 118 + tolclofos-methyl | 200+2000 | 100 |

(continued)

| Compounds provided for test | Concentration (ppm) | Mortality rate |
|---|---|---|
| Present condensed heterocyclic compound 118 + tolclofos-methyl | 500+5 | 100 |
| Present condensed heterocyclic compound 118 + captan | 200+2000 | 100 |
| Present condensed heterocyclic compound 118 + captan | 500+5 | 100 |
| Present condensed heterocyclic compound 119 + fludioxonil | 200+2000 | 100 |
| Present condensed heterocyclic compound 119 + fludioxonil | 500+50 | 100 |
| Present condensed heterocyclic compound 119 + fludioxonil | 500+5 | 100 |
| Present condensed heterocyclic compound 119 + ethaboxam | 200+2000 | 100 |
| Present condensed heterocyclic compound 119 + ethaboxam | 500+5 | 100 |
| Present condensed heterocyclic compound 119 + tolclofos-methyl | 200+2000 | 100 |
| Present condensed heterocyclic compound 119 + tolclofos-methyl | 500+5 | 100 |
| Present condensed heterocyclic compound 119 + captan | 200+2000 | 100 |
| Present condensed heterocyclic compound 119 + captan | 500+5 | 100 |
| Present condensed heterocyclic compound 130 + fludioxonil | 200+2000 | 100 |
| Present condensed heterocyclic compound 130 + fludioxonil | 500+50 | 100 |
| Present condensed heterocyclic compound 130 + fludioxonil | 500+5 | 100 |
| Present condensed heterocyclic compound 130 + ethaboxam | 200+2000 | 100 |
| Present condensed heterocyclic compound 130 + ethaboxam | 500+5 | 100 |
| Present condensed heterocyclic compound 130 + tolclofos-methyl | 200+2000 | 100 |
| Present condensed heterocyclic compound 130 + tolclofos-methyl | 500+5 | 100 |
| Present condensed heterocyclic compound 130 + captan | 200+2000 | 100 |
| Present condensed heterocyclic compound 130 + captan | 500+5 | 100 |
| Present condensed heterocyclic compound 131 + fludioxonil | 200+2000 | 100 |
| Present condensed heterocyclic compound 131 + fludioxonil | 500+50 | 100 |
| Present condensed heterocyclic compound 131 + fludioxonil | 500+5 | 100 |
| Present condensed heterocyclic compound 131 + ethaboxam | 200+2000 | 100 |
| Present condensed heterocyclic compound 131 + ethaboxam | 500+5 | 100 |
| Present condensed heterocyclic compound 131 + tolclofos-methyl | 200+2000 | 100 |
| Present condensed heterocyclic compound 131 + tolclofos-methyl | 500+5 | 100 |
| Present condensed heterocyclic compound 131 + captan | 200+2000 | 100 |
| Present condensed heterocyclic compound 131 + captan | 500+5 | 100 |
| Non-treatment section | - | 0 |

Industrial Applicability

According to the harmful arthropod control composition of the present invention, it is possible to control the harmful arthropod.

## Claims

**1.** A harmful arthropod control composition comprising:

a compound represented by Formula (1)

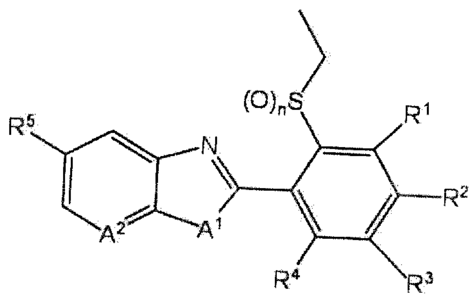

(1)

wherein

A1 represents -NR6-, an oxygen atom, or a sulfur atom,
A2 represents a nitrogen atom or -CH-,
R1, R2, R3, and R4 are the same or different and each represent a C1-C3 chain hydrocarbon group optionally having one or more halogen atoms, a phenyl group optionally having one or more atoms or groups selected from Group Z, a 6-membered heterocyclic group optionally having one or more atoms or groups selected from Group Z, -OR7, -S(O)mR7, a halogen atom, or a hydrogen atom, provided that at least two of R1, R2, R3 and R4 represent hydrogen atoms,
R5 represents a C1-C3 chain hydrocarbon group optionally having one or more atoms or groups selected from Group X, -OR7, -S(O)mR7, or a halogen atom,
R6 represents a C1-C3 chain hydrocarbon group optionally having one or more atoms or groups selected from Group W, a C3-C6 alicyclic hydrocarbon group optionally having one or more atoms or groups selected from Group W, or a hydrogen atom,
R7 represents a C1-C3 chain hydrocarbon group optionally having one or more halogen atoms, or a hydrogen atom,
m represents 0, 1, or 2 and n represents 0, 1, or 2,
provided that, in -S(O)mR7, R7 is not a hydrogen atom when m is 1 or 2,
Group X is a group consisting of a C1-C3 alkoxy group optionally having one or more halogen atoms, a C2-C3 alkenyloxy group optionally having one or more halogen atoms, a C2-C3 alkynyloxy group optionally having one or more halogen atoms, a C1-C3 alkyl sulfanyl group optionally having one or more halogen atoms, a C1-C3 alkyl sulfinyl group optionally having one or more halogen atoms, a C1-C3 alkyl sulfonyl group optionally having one or more halogen atoms, a cyano group, a hydroxy group, and a halogen atom,
Group Z is a group consisting of a C1-C3 chain hydrocarbon group optionally having one or more halogen atoms, a C1-C3 alkoxy group optionally having one or more halogen atoms, a C1-C3 alkyl sulfanyl group optionally having one or more halogen atoms, a C1-C3 alkyl sulfinyl group optionally having one or more halogen atoms, a C1-C3 alkyl sulfonyl group optionally having one or more halogen atoms, a cyano group, a nitro group, and a halogen atom,
Group W is a group consisting of a C1-C3 alkoxy group optionally having one or more halogen atoms, a C2-C3 alkenyloxy group optionally having one or more halogen atoms, C2-C3 alkynyloxy group optionally having one or more halogen atoms, a halogen atom, and hydroxy group, and

one or more fungicidal compounds selected from the following Group (A),

Group (A)
A-1: azoles
tebuconazole, metconazole, difenoconazole, triticonazole, imazalil, triadimenol, fluquinconazole, prochloraz, prothioconazole, diniconazole, diniconazole M, cyproconazole, tetraconazole, ipconazole, triforine, pyrifenox, fenarimol, nuarimol, oxpoconazole fumarate, pefurazoate, triflumizole, azaconazole, bitertanol, bromuconazole, epoxiconazole, fenbuconazole, flusilazole, flutriafol, hexaconazole, imibenconazole, myclobutanil, penconazole, propiconazole, simeconazole, and triadimefon A-2: strobilurins
kresoxim-methyl, azoxystrobin, pyraclostrobin, picoxystrobin, enestrobin, trifloxystrobin, dimoxystrobin, fluoxastrobin, orysastrobin, famoxadone, fenamidone, and
metominostrobin, and a compound represented by the following Formula (2)

Formula (2)

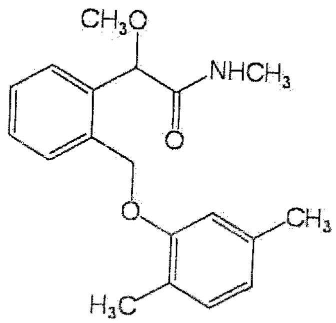

(2)

A-3: phenylamide
metalaxyl, metalaxyl-M, furalaxyl-M, benalaxyl, benalaxyl-M, ofurace, and oxadixyl
A-4: rice blast controlling compounds
probenazole, tiadinil, tricyclazole, pyroquilon, kasugamycin hydrochloride, ferimzone, isotianil, fthalide, and tebufloquin
A-5: rice sheath blight disease controlling compounds Pencycuron, furametpyr, and validamycin
A-6: carboxamides
carboxin, flutolanil, penthiopyrad, fluopyram, penflufen, sedaxane, and fluxapyroxad
A-7: others
fludioxonil, ethaboxam, tolclofos-methyl, and captan.

**2.** The harmful arthropod control composition according to claim 1, wherein the weight ratio of the content of the compound represented by Formula (1) to the content of the one or more fungicidal compounds selected from Group (A) is 10000:1 to 1:100.

**3.** The harmful arthropod control composition according to claim 1, wherein the weight ratio of the content of the compound represented by Formula (1) to the content of the one or more fungicidal compounds selected from Group (A) is 1000:1 to 1:10.

**4.** A method for controlling harmful arthropods, comprising the step of applying an effective amount of the harmful arthropod control composition according to any one of claims 1 to 3 to a plant or soil for cultivating a plant.

**INTERNATIONAL SEARCH REPORT**

International application No. PCT/JP2013/066128

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A01N43/52, A01N37/18, A01N37/24, A01N37/46, A01N37/50, A01N43/12, A01N43/16, A01N43/32, A01N43/36, A01N43/40, A01N43/42, A01N43/54, A01N43/56, A01N43/653, A01N43/76, A01N43/78, A01N43/80, A01N43/88, A01N43/90, A01N47/02, A01N47/04,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2012/086848 A1 (SUMITOMO CHEMICAL CO., LTD.), 28 June 2012 (28.06.2012), claims; paragraph [0307] & TW 201234965 A | 1-4 |
| A | JP 2008-308448 A (Sankyo Agro Co., Ltd.), 25 December 2008 (25.12.2008), claims (Family: none) | 1-4 |
| A | JP 2005-505524 A (Syngenta Ltd.), 24 February 2005 (24.02.2005), claims; paragraph [0036] & US 2004/0209776 A1 & EP 1414818 A1 & WO 2003/011861 A1 | 1-4 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
01 July, 2013 (01.07.13)

Date of mailing of the international search report
09 July, 2013 (09.07.13)

Name and mailing address of the ISA/
Japanese Patent Office

Facsimile No.

Authorized officer

Telephone No.

Form PCT/ISA/210 (second sheet) (July 2009)

INTERNATIONAL SEARCH REPORT

International application No.
PCT/JP2013/066128

Continuation of A. CLASSIFICATION OF SUBJECT MATTER
(International Patent Classification (IPC))

*A01N43/52*(2006.01)i, *A01N37/18*(2006.01)i, *A01N37/24*(2006.01)i, *A01N37/46*(2006.01)i, *A01N37/50*(2006.01)i, *A01N43/12*(2006.01)i, *A01N43/16*(2006.01)i, *A01N43/32*(2006.01)i, *A01N43/36*(2006.01)i, *A01N43/40*(2006.01)i, *A01N43/42*(2006.01)i, *A01N43/54*(2006.01)i, *A01N43/56*(2006.01)i, *A01N43/653*(2006.01)i, *A01N43/76*(2006.01)i, *A01N43/78*(2006.01)i, *A01N43/80*(2006.01)i, *A01N43/88*(2006.01)i, *A01N43/90*(2006.01)i, *A01N47/02*(2006.01)i, *A01N47/04*(2006.01)i, *A01N47/24*(2006.01)i, *A01N57/14*(2006.01)i, *A01P7/04*(2006.01)i

(According to International Patent Classification (IPC) or to both national classification and IPC)

Continuation of B. FIELDS SEARCHED
Minimum documentation searched (International Patent Classification (IPC))

A01N47/24, A01N57/14, A01P7/04

Minimum documentation searched (classification system followed by classification symbols)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9527693 A
- WO 95024413 A
- WO 2001092231 A
- WO 0374491 A
- WO 0310149 A
- WO 06087343 A


### Non-patent literature cited in the description


- The Pesticide Manual. BCPC
- The Pesticide Manual. BCPC, 1072, , 749, , 354, , 1182, , 629, , 1147, , 543, , 928, , 965, , 384
- The Pesticide Manual. BCPC, 688, , 62, , 971, , 910, , 1068, , 1167, , 383, , 538, , 840, , 458
- The Pesticide Manual. BCPC, 737, , 739, , 579, , 74, , 76, , 834, , 847
- The Pesticide Manual. BCPC, 927, , 1134, , 1163, , 999, , 685, , 497
- SHIBUYA INDEX. SHIBUYA INDEX RESEARCH GROUP, 2008, 147
- The Pesticide Manual. BCPC, 871, , 580, , 1187
- The Pesticide Manual. BCPC, 164, , 559, , 877, , 835
- The Pesticide Manual. BCPC, 520, , 435, , 1135, , 154